# EUROPÄISCHE PATENTSCHRIFT

(11) **EP 3 203 841 B1**
(45) Veröffentlichungstag und Bekanntmachung des Hinweises auf die Patenterteilung: **13.06.2018**
(21) Anmeldenummer: 15774616.5
(22) Anmeldetag: 05.10.2015
(51) Int. Cl.: A01N 43/10, A01N 43/40, A01N 43/60, A01N 43/78, A01P 13/00, A01P 21/00, C07D 333/00, C07D 241/00, C07D 277/00, C07D 213/00, A01N 33/02, A01N 43/00, C07D 333/28, C07D 241/16, C07D 277/32, C07D 211/80, A01N 43/76, C07D 213/61, A01N 25/14

(54) **SUBSTITUIERTE 5-HYDROXY-2-PHENYL-3-HETEROARYLPENTANONITRILDERIVATE, VERFAHREN ZU DEREN HERSTELLUNG UND DEREN VERWENDUNG ALS HERBIZIDE UND/ODER PFLANZENWACHSTUMSREGULATOREN**
SUBSTITUTED 5-HYDROXY-2-PHENYL-3-HETEROARYLPENTANONITRILE DERIVATIVES, METHOD FOR THEIR PREPARATION AND THEIR USE AS HERBICIDES AND/OR PLANT GROWTH REGULATORS
DÉRIVÉS DE 5-HYDROXY-2-PHÉNYL-3-HÉTÉROARYLPENTANONITRILE SUBSTITUÉS, LEUR PROCÉDÉ DE FABRICATION ET D'UTILISATION EN TANT QU'HERBICIDES ET/OU RÉGULATEURS DE CROISSANCE DE PLANTES

(30) Priorität: 08.10.2014 EP 14188056
(43) Veröffentlichungstag der Anmeldung: 16.08.2017
(73) Patentinhaber: Bayer CropScience Aktiengesellschaft, 40789 Monheim am Rhein (DE)
(72) Erfinder: MOSRIN, Marc, 50935 Köln (DE); JAKOBI, Harald, 60598 Frankfurt (DE); DIETRICH, Hansjörg, 65835 Liederbach (DE); GATZWEILER, Elmar, 61231 Bad Nauheim (DE); ROSINGER, Christopher Hugh, 65719 Hofheim (DE); SCHMUTZLER, Dirk, 65795 Hattersheim (DE)
(74) Vertreter: BIP Patents
(86) Internationale Anmeldenummer: PCT/EP2015/072886
(87) Internationale Veröffentlichungsnummer: WO 2016/055393

(56) Entgegenhaltungen:
- EP-A1- 0 270 830
- WO-A1-2014/095879
- WO-A1-2014/195253

## Beschreibung

Primär betrifft die vorliegende Erfindung Verbindungen der nachfolgend definierten Formel (I) und deren Verwendung als Herbizide, inbesondere zur Bekämpfung von Unkräutern und/oder Ungräsern in Nutzpflanzenkulturen und/oder als Pflanzenwachstumsregulatoren zur Beeinflussung des Wachstums von Nutzpflanzenkulturen. Die vorliegende Erfindung betrifft ferner herbizide oder pflanzenwachstumsregulierende Mittel umfassend eine oder mehrere Verbindungen der Formel (I). Zudem betrifft die vorliegende Erfindung Verfahren zur Herstellung der Verbindungen der Formel (I).

Bisher bekannte Pflanzenschutzmittel zur selektiven Bekämpfung von Schadpflanzen in Nutzpflanzenkulturen oder Wirkstoffe zur Bekämpfung von unerwünschtem Pflanzenwuchs weisen bei ihrer Anwendung teilweise Nachteile auf, sei es, dass sie (a) keine oder aber eine unzureichende herbizide Wirkung gegen bestimmte Schadpflanzen, (b) ein zu geringes Spektrum der Schadpflanzen, das mit einem Wirkstoff bekämpft werden kann, (c) zu geringe Selektivität in Nutzpflanzenkulturen und/oder (d) ein toxikologisch ungünstiges Profil besitzen. Weiterhin führen manche Wirkstoffe, die als Pflanzenwachstumsregulatoren bei einigen Nutzpflanzen eingesetzt werden können, bei anderen Nutzpflanzen zu unerwünscht verminderten Ernteerträgen oder sind mit der Kulturpflanze nicht oder nur in einem engen Aufwandmengenbereich verträglich. Einige der bekannten Wirkstoffe lassen sich wegen schwer zugänglicher Vorprodukte und Reagenzien im industriellen Maßstab nicht wirtschaftlich herstellen oder besitzen nur unzureichende chemische Stabilitäten. Bei anderen Wirkstoffen hängt die Wirkung zu stark von Umweltbedingungen, wie Wetter- und Bodenverhältnissen ab.

Unter anderem aus den Dokumenten EP-A-0 005 341 (entsprechend US 4,224,052), EP-A-0 266 725, EP-A-0 270 830, WO 2011/098417 A1, WO 2011/003775 A2, WO 2011/003776 A2, WO 2011/073143 A1, WO 2011/042378 A1, WO 2012/126764 A1, WO 2012/126765 A1, WO 2013/010882 A2, WO 2013/092500 A1 und WO 2014/095879 A1 sind herbizide Cyanobutansäurederivate bekannt.

EP-A-0 005 341 beschreibt herbizide Ester und Amide von 4-Cyano-3,4-diphenyl-butansäuren, die an den Phenylresten gegebenfalls substitutiert sind. Gemäß EP-A-0 005 341 eignen sich die threo-Isomere allgemein zur nicht-selektiven Bekämpfung von Schadpflanzen, während die erythro-threo-Isomerengemische für die selektive Bekämpfung von Schadpflanzen in einigen Nutzpflanzenkulturen in Betracht kommen. In EP-A-0 005 341 wird außerdem erwähnt, dass die 2 Enantiomere, die zur threo-Form gehören, unterschiedlich wirksam sind, was am Beispiel der unterschiedlichen Wirkungen der Enantiomeren des Enantiomerenpaares der in den Phenylresten unsubstituierten 4-Cyano-3,4-diphenyl-butansäure untersucht worden ist.

Aus EP-A-0 266 725 sind einige erythro-threo-Isomerengemische bekannt, die selektiv zur Unkrautkontrolle in Reiskulturen eingesetzt werden können.

EP-A-0 270 830 beschreibt, dass threo-Isomere und erythro-threo-Isomerengemische als Pflanzenregulatoren verwendet werden und diese die Ausbildung eines Fruchtstands bei verschiedenen Schadgräsern verhindern können.

Aus WO 2011/003775 A2 sind spezielle Ester von 4-Cyano-3,4-diphenyl-butansäuren bekannt, die als wirksame Herbizide, vorzugsweise in Nutzpflanzenkulturen, eingesetzt werden können.

Aus WO 2011/003776 A2, WO 2011/042378 A1, WO 2011/073143 A1, WO 2012/126764 A1 und WO 2012/126765 A1 sind 4-Cyano-3,4-diphenyl-butansäuren und -säureester bekannt, die an den Phenylresten spezifisch substituiert sind und als wirksame Herbizide, vorzugsweise auch in Nutzpflanzenkulturen eingesetzt werden können.

WO 2013/010882 A2 beschreibt 2,3-Diphenyl-Valeronitrilderivate und deren Verwendung als Herbizide und Pflanzenwachstumsregulatoren.

WO 2013/092500 A1 beschreibt substituierte 4-Cyan-3-phenyl-4-(pyridin-3-yl)butanoate, Verfahren zu deren Herstellung sowie deren Verwendung als Herbizide und Pflanzenwachstumsregulatoren.

WO 2014/095879 A1 beschreibt substituierte 4-Cyan-3-(pyridyl)-4-phenylbutanoate und deren Verwendung als Herbizide und Pflanzenwachstumsregulatoren.

Die herbizide Wirkung dieser bekannten Verbindungen, insbesondere bei niedrigen Aufwandmengen, bzw. deren Verträglichkeit gegenüber Kulturpflanzen bleiben verbesserungswürdig.

Aus den genannten Gründen besteht weiterhin ein Bedarf nach wirkungsstarken Herbiziden und/oder Pflanzenwachstumsregulatoren für die selektive Anwendung in Pflanzenkulturen oder die Anwendung auf Nichtkulturland, wobei diese Wirkstoffe vorzugsweise weitere vorteilhafte Eigenschaften in der Anwendung haben sollten, wie zum Beispiel hinsichtlich ihrer Verträglichkeit gegenüber Kulturpflanzen.

Primäre Aufgabe der vorliegenden Erfindung war die Bereitstellung von Verbindungen mit herbizider Wirkung (Herbizide), die bereits bei relativ niedrigen Aufwandmengen gegen wirtschaftlich wichtige Schadpflanzen hochwirksam sind und vorzugsweise bei guter Wirksamkeit gegen Schadpflanzen selektiv in Kulturpflanzen eingesetzt werden können, und dabei vorzugsweise eine gute Verträglichkeit gegenüber Kulturpflanzen zeigen. Bevorzugt sollten diese herbiziden Verbindungen insbesondere effektiv und effizient gegen ein breites Spektrum an Ungräsern sein, und vorzugsweise zusätzlich eine gute Wirksamkeit gegen viele Unkräuter aufweisen.

Überraschenderweise wurde nun gefunden, dass die nachfolgend definierten Verbindungen der Formel (I) und deren Salze diese Aufgabe erfüllen.

Die vorliegende Erfindung betrifft daher die Verbindungen der Formel (I) und/oder deren Salze, 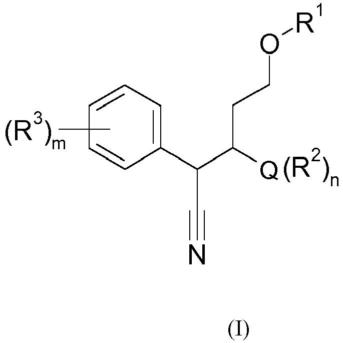 worin
- Q: einen heteroaromatischen Rest bedeutet, ausgewählt aus der Gruppe bestehend aus Pyridinyl, Pyrazinyl, Thienyl und Thiazolyl,
- R¹: Wasserstoff oder eine Gruppe bestehend aus:
Acetyl, Propanoyl, Butanoyl, Pentanoyl, Hexanoyl, 2-Methylpropanoyl (= Isopropanoyl), 2,2-Difluoracetyl, 2,2,2-Trifluoracetyl, C(=O)OMe, Cyclopropancarbonyl, 1-Methylcyclopropancarbonyl, Acryl, Prop-2-inoyl, But-2-inoyl, 2-Methylacryl, Benzoyl, 4-Chlorbenzoyl, 3-Chlorbenzoyl, 2-Chlorbenzoyl, 4-Fluorbenzoyl, 3-Fluorbenzoyl, 2-Fluorbenzoyl, 2,2-Dimethylpropanoyl (= Pivaloyl), 3,3-Dimethylbutanoyl, 2-Nitrobenzoyl, 2-Fluoracetyl, 2-Chloracetyl, 2-Bromacetyl, 2,2-Dichloracetyl, 2-Methoxyacetyl, 2,6-Difluorbenzoyl, C(O)C(=O)OMe, und C(O)CH₂C(O)OMe bedeutet,
- (R²)ₙ: n Substituenten R² bedeuten, wobei R² (wenn n = 1) bzw. jeder der Substituenten R² (wenn n größer als 1 ist) unabhängig voneinander Halogen, Cyano, Nitro, (C₁-C₈)Alkyl, (C₁-C₈)Alkoxy, (C₁-C₈)Alkylthio, (C₁-C₈)Alkylsulfinyl, (C₁-C₈)Alkylsulfonyl, (C₁-C₆)Haloalkyl, (C₂-C₆)Haloalkenyl, (C₁-C₆)Haloalkoxy, (C₁-C₆)Haloalkylthio, (C₁-C₆)Haloalkylsulfinyl, (C₁-C₆)Haloalkylsulfonyl, (C₂-C₆)Alkenyl, (C₂-C₆)Alkinyl, Tri-[(C₁-C₄)alkyl]-silyl oder Tri-[(C₁-C₄)alkyl]-Silyl-(C₁-C₄)alkyl bedeutet,
oder jeweils zwei am heteroaromatischen Rest Q unmittelbar benachbarte R² gemeinsam eine Gruppe der Formel -Z¹-A*¹-Z^{Z}- bedeuten, in welcher
A* für eine Alkylengruppe mit 1 bis 4 C-Atomen steht, die gegebenenfalls durch einen oder mehrere Reste ausgewählt aus der Gruppe bestehend aus Halogen, (C₁-C₄)Alkyl, (C₁-C₄)Haloalkyl, (C₁-C₄)Alkoxy und (C₁-C₄)Haloalkoxy substituiert ist,
Z¹ für eine direkte Bindung, O oder S steht und
Z² für eine direkte Bindung, O oder S steht,
wobei die Gruppe -Z¹-A*-Z²- zusammen mit den an diese Gruppe gebundenen Atomen des heteroaromatischen Restes Q einen 5 oder 6 Ring (d.h. einen Ring mit 5 oder 6 Ringatomen) bilden, und
- (R³)ₘ: m Substituenten R³ bedeutet,
wobei R³ (wenn m = 1) bzw. jeder der Substituenten R³ (wenn m größer als 1) unabhängig voneinander Halogen, Cyano, Nitro, (C₁-C₈)Alkyl, (C₁-C₈)Alkoxy, (C₁-C₈)Alkylthio, (C₁-C₈)Alkylsulfinyl, (C₁-C₈)Alkylsulfonyl, (C₁-C₆)Haloalkyl, (C₂-C₆)Haloalkenyl, (C₁-C₆)Haloalkoxy, (C₁-C₆)Haloalkylthio, (C₁-C₆)Haloalkylsulfinyl, (C₁-C₆)Haloalkylsulfonyl, (C₂-C₆)Alkenyl, (C₂-C₆)Alkinyl, Tri-[(C₁-C₄)alkyl]-silyl, Tri-[(C₁-C₄)alkyl]-silyl-(C₁-C₄)alkyl oder -NR*R** bedeutet, wobei R* und R** unabhängig voneinander und unabhängig von gegebenenfalls vorhandenen weiteren Resten -NR*R** jeweils ausgewählt sind aus der Gruppe bestehend aus H, (C₁-C₈)Alkyl, (C₂-C₈)Alkenyl, (C₂-C₈)Alkinyl, (C₁-C₄)Alkoxy-(C₁-C₄)alkyl, (C₁-C₆)Alkanoyl, [(C₁-C₄)Haloalkyl]-carbonyl, [(C₁-C₄)Alkoxy]-carbonyl, [(C₁-C₄)Haloalkoxy]-carbonyl, (C₃-C₆)Cycloalkyl, (C₃-C₆)Cycloalkyl-(C₁-C₄)alkyl, (C₃-C₆)Cycloalkenyl, (C₃-C₆)Cycloalkenyl-(C₁-C₄)alkyl, Phenyl und Phenyl-(C₁-C₄)alkyl, wobei jeder der genannten Reste (C₃-C₆)Cycloalkyl, (C₃-C₆)Cycloalkyl-(C₁-C₄)alkyl, (C₃-C₆)Cycloalkenyl, (C₃-C₆)Cycloalkenyl-(C₁-C₄)alkyl, Phenyl und Phenyl-(C₁-C₄)alkyl im Cyclus gegebenenfalls durch einen oder mehrere gleiche oder verschiedene Reste R^{bb} substituiert ist, wobei
- R^{bb}: jeweils Halogen, (C₁-C₄)Alkyl, (C₁-C₄)Haloalkyl, (C₁-C₄)Alkoxy oder (C₁-C₄)Haloalkoxy bedeutet, und im Falle von (C₃-C₆)Cycloalkyl, (C₃-C₆)Cycloalkyl-(C₁-C₄)alkyl, (C₃-C₆)Cycloalkenyl, (C₃-C₆)Cycloalkenyl-(C₁-C₄)alkyl R^{bb} zusätzlich Oxo bedeuten kann, oder
NR*R** einen 3- bis 8-gliedrigen Heterocyclus bedeutet, welcher gegebenenfalls zusätzlich zu diesem N-Atom ein oder zwei weitere Heteroringatome ausgewählt aus der Gruppe bestehend aus N, O und S umfasst, und welcher unsubstituiert oder durch einen oder mehrere Reste ausgewählt aus der Gruppe bestehend aus (C₁-C₄)Alkyl, (C₁-C₄)Haloalkyl und Oxo substituiert ist,
oder wobei jeweils zwei am Ring unmittelbar benachbarte Gruppen R³ gemeinsam eine Gruppe der Formel -Z³-A**-Z⁴- bedeuten, in welcher
A** für eine Alkylengruppe mit 1 bis 4 C-Atomen steht, die gegebenenfalls durch einen oder mehrere Reste ausgewählt aus der Gruppe bestehend aus Halogen, (Ci-C₄)Alkyl, (C₁-C₄)Haloalkyl, (C₁-C₄)Alkoxy und (C₁-C₄)Haloalkoxy substituiert ist,
Z³ für eine direkte Bindung, O oder S steht und
Z⁴ für eine direkte Bindung, O oder S steht,
wobei die Gruppe -Z³-A**-Z⁴- zusammen mit den an diese Gruppe gebundenen C-Atomen des Phenylrings einen 5 oder 6 Ring bilden,
- n: 0, oder eine ganze Zahl im Bereich von 1 bis 5, vorzugsweise 0, 1, 2 oder 3 bedeutet, und
- m: 0, 1, 2, 3, 4 oder 5, vorzugsweise 0, 1, 2, oder 3 bedeutet.

Die erfindungsgemäßen Verbindungen der Formel (I) und/oder deren Salze weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die erfindungsgemäßen Verbindungen gut erfaßt.

Die erfindungsgemäßen Verbindungen der Formel (I) und/oder deren Salze weisen beispielsweise im Vergleich zu den in WO 2012/126765 A1 beschriebenen Verbindungen generell eine stärkere Wirkung bei gleicher Dosierung auf, z.B. gegen Ungräser. Ferner weisen die erfindungsgemäßen Verbindungen ein breiteres Wirkspektrum gegen Unräuter auf, d.h. dass mit den erfindungsgemäßen Verbindungen und/oder deren Salzen eine größere Anzahl verschiedener Unkräuter wirkungsvoll bekämpft werden kann.

Sehr wirksam zeigten sich die erfindungsgemäßen Verbindungen der Formel (I) und/oder deren Salze in der Bekämpfung von Schadpflanzen wie Alopecurus myosuroides, Avena fatua, Cyperus esculentus, Echinochloa crus-galli, Lolium multiflorum, Setaria viridis, Abutilon theophrasti, Amaranthus retroflexus, Polygonum convolvulus (= Fallopia convolvulus), Stellaria media, Viola tricolor, und Veronica persica, wobei die bevorzugten bzw. besonders bevorzugten erfindungsgemäßen Verbindungen gegen in den biologischen Tests eine 80%ige bis 100%ige herbizide Wirkung gegen eine, mehrere oder sämtliche der genannten Schadpflanzen zeigten, und dabei gleichzeitig akzeptabler und zumeist sehr geringer Schädigung der Nutzpflanze.

Die erfindungsgemäßen Verbindungen der Formel (I) und/oder deren Salze zeigen zudem eine besonders gute Wirkung im Vorauflaufverfahren, insbesondere gegen Ungräser. Die Wirkung der erfindungsgemäßen Verbindungen im Vorauflauf ist generell besser als die Wirkung im Vorauflauf der in WO 2012/126765 A1 beschriebenen Verbindungen.

Ferner wurde gefunden, dass die erfindungsgemäßen Verbindungen der Formel (I) und/oder deren Salze diese bessere Vorauflaufwirkung besonders selektiv in bestimmten Kulturen sind, insbesondere in Raps, Soja, Baumwolle und Getreide (dabei insbesondere Mais, Gerste, Weizen, Roggen, Hafer, Triticale, Hirsen, Reis).

In der Regel besitzen die erfindungsgemäßen Verbindungen der Formel (I) ein wesentlich breiteres Wirkspektrum.

In der Formel (I) bedeutet die Formel "(R²)ₙ" n Reste R², die als Substituenten am heteroaromatischen Rest Q gebunden sind, wobei die Reste R² im Falle n größer 1 gleich oder verschieden sein können und die jeweils näher genannte Bedeutung haben. Im Fall n = 0 ist der heteroaromatische Rest Q nicht durch Substituenten R² substituiert.

In der Formel (I) bedeutet die Formel "(R³)ₘ" m Reste R³, die als Substituenten am betreffenden Phenylring gebunden sind, wobei die Reste R³ im Falle n größer 1 gleich oder verschieden sein können und die jeweils näher genannte Bedeutung haben. Im Fall n = 0 ist der betreffende Phenylring nicht durch Substituenten R³ substituiert, d.h. alle Ring-C-Atome des Phenylrings in den Positionen 2 bis 6 sind mit einem Wasserstoffatom verbunden.

Die Verbindungen der Formel (I) können im Falle R¹ = H oder im Falle geeigneter saurer Substituenten durch Umsetzung mit Basen Salze bilden, wobei der saure Wasserstoff durch ein für die Landwirtschaft geeignetes Kation ersetzt wird.

Die Verbindungen der Formel (I) können durch Anlagerung einer geeigneten anorganischen oder organischen Säure, an eine basische Gruppe, wie z.B. Amino oder Alkylamino, Salze bilden. Geeignete vorhandene saure Gruppen, wie z.B. Carbonsäuregruppen, können innere Salze mit ihrerseits protonierbaren Gruppen, wie Aminogruppen bilden.

Die Verbindungen der Formel (I) können vorzugsweise in Form landwirtschaftlich einsetzbarer Salze vorliegen, wobei es ansonsten auf die Art des Salzes in der Regel nicht ankommt. Im Allgemeinen kommen dabei die Salze derjenigen Kationen oder die Säureadditionssalze derjenigen Säuren in Betracht, deren Kationen bzw. Anionen die herbizide Wirkung der Verbindungen der Formel (I) nicht negativ beeinflussen.

Es kommen als Kationen insbesondere Ionen der Alkalimetalle, dabei vorzugsweise Lithium, Natrium oder Kalium, der Erdalkalimetalle, dabei vorzugsweise Calcium oder Magnesium, und der Übergangsmetalle, dabei vorzugsweise Mangan, Kupfer, Zink oder Eisen, in Betracht. Ebenso kann als Kation Ammonium (NH₄⁺) oder substituiertes Ammonium verwendet werden, wobei hier ein bis vier Wasserstoffatome durch (C₁-C₄)Alkyl, Hydroxy-(C₁-C₄)alkyl, (C₁-C₄)Alkoxy-(C₁-C₄)alkyl, Hydroxy-(C₁-C₄)alkoxy-(C₁-C₄)alkyl, Phenyl oder Benzyl ersetzt sein können, bevorzugte Ammoniumionen sind Ammonium, Dimethylammonium, Diisopropylammonium, Tetramethylammonium, Tetrabutylammonium, 2-(2-Hydroxyeth-1-oxy)eth-1-ylammonium, Di(2-hydroxyeth-1-yl)ammonium, Trimethylbenzylammonium. Des Weiteren kommen Phosphoniumionen, Sulfoniumionen, vorzugsweise Tri(C₁-C₄)alkyl-sulfonium, insbesondere Trimethylsulfonium, oder Sulfoxoniumionen, vorzugsweise Tri(C₁-C₄)alkyl-sulfoxonium, insbesondere Trimethylsulfoxonium, in Betracht.

Anionen von geeigneten Säureadditionsalzen sind vorzugsweise Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von (C₁-C₄)Alkansäuren, dabei wiederum vorzugsweise Formiat, Acetat, Propionat, Butyrat oder Trifluoracetat.

In der Formel (I) und allen nachfolgenden Formeln werden Bezeichnungen für chemische Reste verwendet, die Sammelbegriffe für die Aufzählung individueller Gruppenmitglieder darstellen oder individuelle chemische Reste spezifisch bezeichnen. Es werden dabei Bezeichnungen verwendet, die dem Fachmann geläufig sind oder die nachstehend definierten Bedeutungen haben.

Ein hydrolysierbarer Rest (siehe Definition von R¹) bedeutet einen unter den Anwendungsbedingungen hydrolysierbaren Rest, beispielsweise einen schon in der Spritzbrühe oder insbesondere unter den physiologischen Bedingungen in Pflanzen hydrolysierbaren Rest, wobei eine Verbindung der Formel (I) mit R¹ ungleich Wasserstoff zur entsprechenden Verbindung der Formel (I), in der R¹ = H (Wasserstoff) hydrolysiert wird. In der Definition der hydrolysierbaren Reste sind auch ausdrücklich die Reste mit R¹ = Kohlenwasserstoffrest oder Heterocyclylrest, wobei die beiden letztgenannten Reste unsubstituiert oder substituiert sind, umfasst, auch wenn sie teilweise vergleichsweise langsam hydrolysierbar sind.

Ein Kohlenwasserstoffrest ist ein aliphatischer, cycloaliphatischer oder aromatischer monocyclischer oder, im Falle eines gegebenenfalls substituierten Kohlenwasserstoffrestes, auch ein bicyclischer oder polycyclischer organischer Rest auf Basis der Elemente Kohlenstoff und Wasserstoff, beispielsweise umfassend die Reste Alkyl, Alkenyl, Alkinyl, Cycloalkyl, Cycloalkenyl, Aryl, Phenyl, Naphthyl, Indanyl, Indenyl, etc. Entsprechendes gilt für Kohlenwasserstoffreste in zusammengesetzte Bedeutungen wie Kohlenwasserstoffoxyresten oder anderen über Heteroatomgruppen gebundene Kohlenwasserstoffresten.

Wenn nicht näher definiert, weisen die Kohlenwasserstoffreste vorzugsweise 1 bis 20 C-Atome, weiter bevorzugt 1 bis 16 C-Atome, insbesondere 1 bis 12 C-Atome auf.

Die Kohlenwasserstoffreste, auch in den speziellen Resten Alkyl, Alkoxy, Haloalkyl, Haloalkoxy, Alkylamino und Alkylthio, sowie die entsprechenden ungesättigten und/oder substituierten Reste können im Kohlenstoffgerüst jeweils geradkettig oder verzweigtkettig (verzweigt) sein.

Der Ausdruck "(C₁-C₄)Alkyl" bedeutet eine Kurzschreibweise für Alkyl mit 1 bis 4 Kohlenstoffatomen, d.h. umfasst die Reste Methyl, Ethyl, 1-Propyl, 2-Propyl, 1-Butyl, 2-Butyl, 2-Methylpropyl oder tert-Butyl. Allgemeine Alkylreste mit einem größeren angegebenen Bereich von C-Atomen, z.B. "(C₁-C₆)Alkyl" umfassen entsprechend auch gradkettige oder verzweigte Alkylreste mit einer größeren Zahl von C-Atomen, d.h. gemäß Beispiel auch die Alkylreste mit 5 und 6 C-Atomen.

Wenn nicht speziell bzw. abweichend angegeben, sind bei den Kohlenwasserstoffresten wie Alkyl-, Alkenyl- und Alkinylresten, auch in zusammengesetzten Resten, die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Vinyl, Allyl, 1-Methyl-2-propenyl, 2-Methyl-2-propenyl, 2-Butenyl, Pentenyl, 2-Methylpentenyl oder Hexenyl group, vorzugsweise Allyl, 1-Methylprop-2-en-1-yl, 2-Methyl-prop-2-en-1-yl, But-2-en-1-yl, But-3-en-1-yl, 1-Methyl-but-3-en-1-yl oder 1-Methyl-but-2-en-1-yl.

Alkenyl schließt insbesondere auch geradkettige oder verweigte Kohlenwasserstoffreste mit mehr als einer Doppelbindung ein, wie 1,3-Butadienyl und 1,4-Pentadienyl, aber auch Allenyl- oder Kumulenylreste mit einer bzw. mehreren kumulierten Doppelbindungen, wie beispielsweise Allenyl (1,2-Propadienyl), 1,2-Butadienyl und 1,2,3-Pentatrienyl;

Alkinyl bedeutet z.B. Propargyl, But-2-in-1-yl, But-3-in-1-yl, 1-Methyl-but-3-in-1-yl. Alkinyl schließt insbesondere auch geradkettige oder verweigte Kohlenwasserstoffreste mit mehr als einer Dreifachbindung oder auch mit einer oder mehreren Dreifachbindungen und einer oder mehreren Doppelbindungen ein, wie beispielsweise 1,3-Butatrienyl bzw. 3-Penten-1-in-1-yl.

Ein 3- bis 9-gliedriger carbocyclischer Ring bedeutet (C₃-C₉)Cycloalkyl oder (C₅-C₉)Cycloalkenyl.

(C₃-C₉)Cycloalkyl bedeutet ein carbocyclisches, gesättigtes Ringsystem mit vorzugsweise 3-9 C-Atomen, z.B. Cyclopropyl, Cyclobutyl, Cyclopentyl, Cyclohexyl, Cycloheptyl, Cyclooctyl oder Cyclononyl. Im Falle von substituiertem Cycloalkyl werden cyclische Systeme mit Substituenten umfasst, wobei die Substituenten auch mit einer Doppelbindung am Cycloalkylrest, z.B. eine Alkylidengruppe wie Methyliden, gebunden sein können.

(C₅-C₉)Cycloalkenyl bedeutet ein carbocyclisches, nicht aromatisches, partiell ungesättigtes Ringsystem mit 5-9 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl. Im Falle von substituiertem Cycloalkenyl gelten die Erläuterungen für substituiertes Cycloalkyl entsprechend

Alkyliden, z.B. auch in der Form (C₁-C₁₀)Alkyliden, bedeutet den Rest eines geradkettigen oder verzweigten Alkans, der über eine Zweifachbindung gebunden ist, wobei die Position der Bindungsstelle noch nicht festgelegt ist. Im Falle eines verzweigten Alkans kommen naturgemäß nur Positionen in Frage, an denen zwei H-Atome durch die Doppelbindung ersetzt werden können; Reste sind z.B. =CH₂, =CH-CH₃, =C(CH₃)-CH₃, =C(CH₃)-C₂H₅ oder =C(C₂H₅)-C₂H₅.

"Halogen" bezieht vorzugsweise auf die Gruppe bestehend aus Fluor, Chlor, Brom und Iod. Haloalkyl, -alkenyl und -alkinyl bedeuten durch gleiche oder verschiedene Halogenatome, vorzugsweise aus der Gruppe Fluor, Chlor und Brom, insbesondere aus der Gruppe Fluor und Chlor, teilweise oder vollständig substituiertes Alkyl, Alkenyl bzw. Alkinyl, z.B. Monohaloalkyl (= Monohalogenalkyl), Perhaloalkyl, CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; Entsprechendes gilt für Haloalkenyl und andere durch Halogen substituierte Reste.

Aryl bedeutet ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl.

Im Falle gegebenenfalls substituiertes Aryl sind auch mehrcyclische Systeme, wie Tetrahydronaphthyl, Indenyl, Indanyl, Fluorenyl, Biphenylyl, umfasst, wobei die Bindungsstelle am aromatischen System ist.

Ein heterocyclischer Rest (Heterocyclyl) enthält mindestens einen heterocyclischen Ring (=carbocyclischer Ring, in dem mindestens ein C-Atom durch ein Heteroatom ersetzt ist, vorzugsweise durch ein Heteroatom aus der Gruppe N, O, S, P, B, Si, Se), der gesättigt, ungesättigt oder heteroaromatisch ist und dabei unsubstituiert oder substituiert sein kann, wobei die Bindungsstelle an einem Ringatom lokalisiert ist. Wenn nicht anders definiert, enthält er vorzugsweise ein oder mehrere, insbesondere 1, 2 oder 3 Heteroatome im heterocyclischen Ring, vorzugsweise aus der Gruppe N, O, und S; vorzugsweise ist er ein aliphatischer Heterocyclylrest mit 3 bis 7 Ringatomen oder ein heteroaromatischer Rest mit 5 oder 6 Ringatomen. Der heterocyclische Rest kann z.B. ein heteroaromatischer Rest oder Ring (Heteroaryl) sein, wie z.B. ein mono-, bi- oder polycyclisches aromatisches System, in dem mindestens 1 Ring ein oder mehrere Heteroatome enthält.

Ist der Heterocyclylrest oder der heterocyclische Ring gegebenenfalls substituiert, kann er mit anderen carbocyclischen oder heterocyclischen Ringen annelliert sein. Bevorzugt sind benzokondensierte (benzoannellierte) heterocyclische bzw. heteroaromatische Ringe.

Im Falle von gegebenenfalls substituiertem Heterocyclyl werden auch mehrcyclische Systeme umfasst, wie beispielsweise 8-Aza-bicyclo[3.2.1]octanyl oder 1-Aza-bicyclo[2.2.1]heptyl. Im Falle von gegebenenfalls substituiertem Heterocyclyl werden auch spirocyclische Systeme umfasst, wie beispielsweise 1-Oxa-5-aza-spiro[2.3]hexyl.

Vorzugsweise ist er ein Rest eines heteroaromatischen Rings mit einem Heteroatom aus der Gruppe N, O und S, beispielsweise der Rest eines Fünf- oder Sechsrings, wie Pyridyl, Pyrrolyl, Thienyl oder Furyl; weiterhin bevorzugt ist er ein Rest eines entsprechenden heteroaromatischen Rings mit 2, 3 oder 4 Heteroatomen, z.B. Pyrimidinyl, Pyridazinyl, Pyrazinyl, Triazinyl, Tetrazinyl, Thiazolyl, Thiadiazolyl, Oxazolyl, Isoxazolyl, Pyrazolyl, Imidazolyl oder Triazolyl oder Tetrazolyl.

Bevorzugt ist er dabei ein Rest eines heteroaromatischen Fünf- oder Sechsrings mit 1 bis 4 Heteroatomen, wie z.B. 1,2,3-Triazolyl, 1,2,4-Triazolyl, Tetrazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Tetrazolyl, 1,2,3-Triazinyl, 1,2,4-Triazinyl, 1,3,5-Triazinyl, 1,2,3,4-Tetrazinyl, 1,2,3,5-Tetrazinyl, 1,2,4,5-Tetrazinyl, Thiazolyl, Isothiazolyl, Oxazolyl, Isoxazolyl, Pyrazolyl, Imidazolyl.

Weiter bevorzugt sind dabei heteroaromatische Reste von Fünfring-Heterocyclen mit 3 N-Atomen, wie 1,2,3-Triazol-1-yl, 1,2,3-Triazol-4-yl, 1,2,3-Triazol-5-yl, 1,2,5-Triazol-1-yl, 1,2,5-Triazol-3-yl, 1,3,4-Triazol-1-yl, 1,3,4-Triazol-2-yl, 1,2,4-Triazol-3-yl, 1,2,4-Triazol-5-yl; weiter bevorzugt sind auch heteroaromatische Reste von Sechsring-Heterocyclen mit 3 N-Atomen, wie 1,3,5-Triazin-2-yl, 1,2,4-Triazin-3-yl, 1,2,4-Triazin-5-yl, 1,2,4-Triazin-6-yl, 1,2,3-Triazin-4-yl, 1,2,3-Triazin-5-yl; weiter bevorzugt sind auch heteroaromatische Reste von Fünfring-Heterocyclen mit zwei N-Atomen und einem O-Atom, wie 1,2,4-Oxadiazol-3-yl; 1,2,4-Oxadiazol-5-yl, 1,3,4-Oxadiazol-2-yl, 1,2,3-Oxadiazol-4-yl, 1,2,3-Oxadiazol-5-yl, 1,2,5-Oxadiazol-3-yl; weiter bevorzugt sind auch heteroaromatische Reste von Fünfring-Heterocyclen mit zwei N-Atomen und einem S-Atom, wie 1,2,4-Thiadiazol-3-yl, 1,2,4-Thiadiazol-5-yl, 1,3,4-Thiadiazol-2-yl, 1,2,3-Thiadiazol-4-yl, 1,2,3-Thiadiazol-5-yl, 1,2,5-Thiadiazol-3-yl; weiter bevorzugt sind auch heteroaromatische Reste von Fünfring-Heterocyclen mit vier N-Atomen, wie 1,2,3,4-Tetrazol-1-yl, 1,2,3,4-Tetrazol-5-yl, 1,2,3,5-Tetrazol-1-yl, 1,2,3,5-Tetrazol-4-yl, 2*H*-1,2,3,4-Tetrazol-5-yl, 1*H*-1,2,3,4-Tetrazol-5-yl, weiter bevorzugt sind auch heteroaromatische Reste von Sechsring-Heterocyclen, wie 1,2,4,5-Tetrazin-3-yl; weiter bevorzugt sind auch heteroaromatische Reste von Fünfring-Heterocyclen mit drei N-Atomen und einem O- oder S-Atom, wie 1,2,3,4-Oxatriazol-5-yl; 1,2,3,5-Oxatriazol-4-yl; 1,2,3,4-Thiatriazol-5-yl;1,2,3,5-Thiatriazol-4-yl; weiter bevorzugt sind auch heteroaromatische Reste von Sechsring-Heterocyclen, wie beispielsweise 1,2,4,6-Thiatriazin-1-yl; 1,2,4,6-Thiatriazin-3-yl; 1,2,4,6-Thiatriazin-5-yl.

Ferner bevorzugt ist der heterocyclische Rest oder Ring ein partiell oder vollständig hydrierter heterocyclischer Rest mit einem Heteroatom aus der Gruppe N, O und S, beispielsweise Oxiranyl, Oxetanyl, Oxolanyl (= Tetrahydrofuryl), Oxanyl, Pyrrolinyl, Pyrrolidyl oder Piperidyl.

Weiterhin bevorzugt ist er auch ein partiell oder vollständig hydrierter heterocyclischer Rest mit 2 Heteroatomen aus der Gruppe N, O und S, beispielsweise Piperazinyl, Dioxolanyl, Oxazolinyl, Isoxazolinyl, Oxazolidinyl, Isoxazolidinyl und Morpholinyl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Bevorzugte Beispiele für Heterocyclyl sind ein heterocyclischer Rest mit 3 bis 6 Ringatomen aus der Gruppe Pyridyl, Thienyl, Furyl, Pyrrolyl, Oxiranyl, 2-Oxetanyl, 3-Oxetanyl, Oxolanyl (= Tetrahydrofuryl), Pyrrolidyl, Piperidyl, insbesondere Oxiranyl, 2-Oxetanyl, 3-Oxetanyl oder Oxolanyl, oder ist ein heterocyclischer Rest mit zwei oder drei Heteroatomen, beispielsweise Pyrimidinyl, Pyridazinyl, Pyrazinyl, Triazinyl, Thiazolyl, Thiadiazolyl, Oxazolyl, Isoxazolyl, Pyrazolyl, Triazolyl, Piperazinyl, Dioxolanyl, Oxazolinyl, Isoxazolinyl, Oxazolidinyl, Isoxazolidinyl oder Morpholinyl.

Bevorzugte heterocyclische Reste sind auch benzokondensierte oder benzoannellierte heteroaromatische Ringe, beispielsweise Benzofuryl, Benzisofuryl, Benzothiophenyl, Benzisothiophenyl, Isobenzothiophenyl, Indolyl, Isoindolyl, Indazolyl, Benzimidazolyl, Benztriazolyl, Benzoxazolyl, 1,2-Benzisoxazolyl, 2,1-Benzisoxazolyl, Benzothiazolyl, 1,2-Benzisothiazolyl, 2,1-Benzoisothiazolyl, 1,2,3-Benzoxadiazolyl, 2,1,3-Benzoxadiazolyl, 1,2,3-Benzothiadiazolyl, 2,1,3-Benzothiadiazolyl, Chinolyl (Chinolinyl), Isochinolyl (Isochinolinyl), Chinnolinyl, Phtalazinyl, Chinazolinyl, Chinoxalinyl, Naphthyridinyl, Benzotriazinyl, Purinyl, Pteridinyl, Indolizinyl, Benzo-1,3-dioxylyl, 4H-Benzo-1,3-dioxinyl, und 4H-Benzo-1,4-dioxinyl, und wo es möglich ist, N-Oxide und Salze davon.

Wenn ein Grundkörper "durch einen oder mehrere Reste" aus einer Aufzählung von Resten (= Gruppe) oder einer generisch definierten Gruppe von Resten substituiert ist, so schließt dies jeweils die gleichzeitige Substitution durch mehrere gleiche und/oder strukturell unterschiedliche Reste ein.

Substituierte Reste, wie ein substituierter Alkyl-, Alkenyl-, Alkinyl-, Cycloalkyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Alkylthio, Hydroxy, Amino, Nitro, Carboxy, Cyano, Azido, Alkoxycarbonyl, Alkylcarbonyl, Formyl, Carbamoyl, Mono-und Dialkylaminocarbonyl, substituiertes Amino, wie Acylamino, Mono- und Dialkylamino, und Alkylsulfinyl, Alkylsulfonyl und, im Falle cyclischer Reste, auch Alkyl, Haloalkyl, Alkylthio-alkyl, Alkoxy-alkyl, gegebenfalls substituiertes Mono- und Dialkyl-aminoalkyl und Hydroxy-alkyl bedeuten; im Begriff "substituierte Reste" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Phenyl, Phenoxy etc. eingeschlossen. Im Falle von substituierten cyclischen Resten mit aliphatischen Anteilen im Ring werden auch cyclische Systeme mit solchen Substituenten umfasst, die mit einer Doppelbindung am Ring gebunden sind, z.B. mit einer Alkylidengruppe wie Methyliden oder Ethyliden substituiert sind.

Der Ausdruck "Reste aus der Gruppe" gefolgt von einer Gruppe (Liste der Substituenten), wo immer verwendet, ist gleichbedeutend mit "Reste ausgewählt aus der Gruppe bestehend aus" (Liste der Substituenten).

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.

Bevorzugte Substituenten für die Substituentenebenen sind dabei Amino, Hydroxy, Halogen, Nitro, Cyano, Mercapto, Carboxy, Carbonamid, SF₅, Aminosulfonyl, Alkyl, Cycloalkyl, Alkenyl, Cycloalkenyl, Alkinyl, Monoalkyl-amino, Dialkyl-amino, N-Alkanoyl-amino, Alkoxy, Alkenyloxy, Alkinyloxy, Cycloalkoxy, Cycloalkenyloxy, Alkoxy-carbonyl, Alkenyloxy-carbonyl, Alkinyloxycarbonyl, Aryloxycarbonyl, Alkanoyl, Alkenyl-carbonyl, Alkinyl-carbonyl, Aryl-carbonyl, Alkylthio, Cycloalkylthio, Alkenylthio, Cycloalkenylthio, Alkinylthio, Alkylsulfinyl, Alkylsulfonyl, Monoalkylaminosulfonyl, Dialkyl-aminosulfonyl, N-Alkyl-aminocarbonyl, N,N-Dialkyl-aminocarbonyl, N-Alkanoyl-amino-carbonyl, N-Alkanoyl-N-alkyl-aminocarbonyl, Aryl, Aryloxy, Benzyl, Benzyloxy, Benzylthio, Arylthio, Arylamino und Benzylamino.

Zwei Substituenten können auch gemeinsam eine gesättigte oder ungesättigte KohlenwasserstoffBrücke, oder eine entsprechende Brücke, in denen C-Atome, CH-Gruppen oder CH₂-Gruppen durch Heteroatome ersetzt sind, bilden und damit einen ankondensierten oder annellierten Cyclus bilden. Bevorzugt werden dabei benzokondensierte Systeme auf Basis der Grundkörper gebildet.

Gegebenenfalls substituiertes Phenyl bedeutet vorzugsweise unsubstituiertes Phenyl, oder Phenyl, das durch einen oder mehrere Reste aus der Gruppe Halogen, Cyano, (Ci-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkoxy-(C₁-C₄)alkoxy, (C₁-C₄)Alkylthio und Nitro substituiert ist, insbesondere Phenyl, das gegebenenfalls durch einen oder mehrere Reste aus der Gruppe Halogen, (Ci-C₄)Alkyl, (C₁-C₄)Haloalkyl und (C₁-C₄)Alkoxy substituiert ist.

Bei Substituenten mit C-Atomen sind solche mit 1 bis 6 C-Atomen bevorzugt, vorzugsweise solche mit 1 bis 4 C-Atomen, insbesondere solche mit 1 oder 2 C-Atomen. Bevorzugt sind in der Regel Substituenten aus der Gruppe Halogen, z.B. Fluor und Chlor, (Ci-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy, Fluor und Chlor.

Substituiertes Amino wie mono- oder disubstituiertes Amino bedeutet einen Rest aus der Gruppe der substituierten Aminoreste, welche beispielsweise durch einen bzw. zwei gleiche oder verschiedene Reste aus der Gruppe Alkyl, Alkoxy, Acyl und Aryl N-substituiert sind; vorzugsweise Mono- und Dialkyl-amino, Mono- und Diarylamino, Acylamino, N-Alkyl-N-arylamino, N-Alkyl-N-acylamino sowie N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Acyl bedeutet einen Rest einer organischen Säure, der formal durch Abtrennen einer Hydroxygruppe an der Säurefunktion entsteht, wobei der organische Rest in der Säure auch über ein Heteroatom mit der Säurefunktion verbunden sein kann. Beispiele für Acyl sind die Reste einer Carbonsäure und Reste davon abgeleiteter Säuren wie der Thiocarbonsäure, gegebenenfalls N-substituierten Iminocarbonsäuren oder der Rest von Kohlensäuremonoestern, N-substituierter Carbaminsäure, Sulfonsäuren, Sulfinsäuren, N-substituierter Sulfonamidsäuren, Phosphonsäuren oder Phosphinsäuren.

Acyl bedeutet beispielsweise Formyl, Alkylcarbonyl, dabei vorzugsweise [(C₁-C₄)Alkyl]-carbonyl, Phenylcarbonyl, Alkyloxycarbonyl, Phenyloxycarbonyl, Benzyloxycarbonyl, Alkylsulfonyl, Alkylsulfinyl, N-Alkyl-1-iminoalkyl und andere Reste von organischen Säuren. Dabei können die Reste jeweils im Alkyl- oder Phenylteil wiederum substituiert sein, beispielsweise im Alkylteil durch einen oder mehrere Reste aus der Gruppe Halogen, Alkoxy, Phenyl und Phenoxy; Beispiele für Substituenten im Phenylteil sind die bereits weiter oben allgemein für substituiertes Phenyl erwähnten Substituenten.

Acyl bedeutet vorzugsweise einen Acylrest im engeren Sinne, d.h. einen Rest einer organischen Säure, bei der die Säuregruppe direkt mit dem C-Atom eines organischen Restes verbunden ist, beispielsweise Formyl, Alkylcarbonyl wie Acetyl oder [(C₁-C₄)Alkyl]-carbonyl, Phenylcarbonyl, Alkylsulfonyl, Alkylsulfinyl und andere Reste von organischen Säuren.

In einer bevorzugten Ausgestaltung bedeutet Acyl einen Alkanoylrest mit 1 bis 8 C-Atomen, bevorzugt mit 1 bis 6 C-Atomen, insbesondere bevorzugt mit 1 bis 4 C-Atomen. (C₁-C₄)Alkanoyl bedeutet dabei den Rest einer Alkansäure mit 1 bis 6 C-Atomen nach Abtrennen der OH-Gruppe der Säuregruppe, d.h. Formyl, Acetyl, n-Propionyl, i-Propionyl, n-, i-, sec.- oder tert.-Butanoyl, n-, i-, sec.- oder tert.-Pentanoyl, n-, i-, oder sec.-Hexanoyl.

Die "yl-Position" eines Restes bezeichnet das C-Atom mit der freien Bindung.

Erfindungsgemäße Verbindungen der Formel (I) und/oder deren Salze werden im Rahmen des vorliegenden Textes auch abgekürzt als "Verbindungen (I)" bezeichnet.

Gegenstand der Erfindung sind auch alle Stereoisomeren, die von Formel (I) umfasst sind, und deren Gemische. Verbindungen der Formel (I) enthalten zwei oder mehr asymmetrische C-Atome, und können auch Doppelbindungen enthalten, deren Stereochemie in der allgemeinen Formel (I) nicht gesondert angegeben ist. Die durch ihre spezifische Raumform definierten möglichen Stereoisomeren, wie Enantiomere, Diastereomere, Z- und E-Isomere sind alle von der Formel (I) umfasst und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Die erfindungsgemäßen Verbindungen der Formel (I) umfassen alle Stereoisomeren, welche aufgrund der Asymmetriezentren und/oder Doppelbindungen im Molekül, deren Konfiguration in den jeweiligen Strukturformeln nicht speziell bezeichnet oder nicht speziell angegeben sind, auftreten können, und deren Mischung, inklusive der racemischen Gemische und der teilweise mit bestimmten Stereoisomeren angereicherten Mischungen.

Gegenstand der Erfindung sind auch alle Tautomere der Verbindungen der Formel (I), die durch Verschiebung eines Wasserstoffatoms entstehen können (z.B. Keto-Enol-Tautomere). Die Tautomere sind ebenfalls von der Verbindung der Formel (I) umfasst, auch wenn die Formel (I) nur eines der jeweiligen im Gleichgewicht stehenden bzw. ineinander umwandelbaren Tautomere formal richtig beschreibt.

Die Verbindungen der Formel (I) umfassen auch alle physikalischen Formen, in denen diese in Reinsubstanz oder gegebenenfalls in Mischung mit anderen Stoffen auftreten können, insbesondere auch polymorphe Kristallformen der Verbindungen der Formel (I) oder deren Salze oder Lösungsmitteladditionsverbindungen (z.B. Hydrate).

Vor allem aus den Gründen der höheren herbiziden Wirkung, besseren Selektivität und/oder besseren Herstellbarkeit sind erfindungsgemäße Verbindungen der genannten Formel (I) oder deren Salze bzw. deren erfindungsgemäße Verwendung von besonderem Interesse, worin einzelne Reste eine der bereits genannten oder im folgenden genannten bevorzugten Bedeutungen haben, oder insbesondere solche, worin eine oder mehrere der bereits genannten oder im Folgenden genannten bevorzugten Bedeutungen kombiniert auftreten.

Unabhängig von den jeweils anderen Resten aus der Gruppe R¹, (R²)ₙ und (R³)ₘ und der den allgemeinen Resten entsprechenden Unterbedeutungen, vorzugsweise in Kombination mit bevorzugten Bedeutungen von einem oder mehreren dieser Reste, sind erfindungsgemäße Verbindungen mit nachfolgend aufgeführten bevorzugten Bedeutungen der betreffenden Reste von besonderem Interesse.

Erfindungsgemäß besonders bevorzugt sind Verbindungen der Formel (I) und/oder deren Salze, in denen Q Pyridyl bedeutet, entsprechend den nachfolgend definierten Formeln (I-1), (I-2) bzw. (I-3): 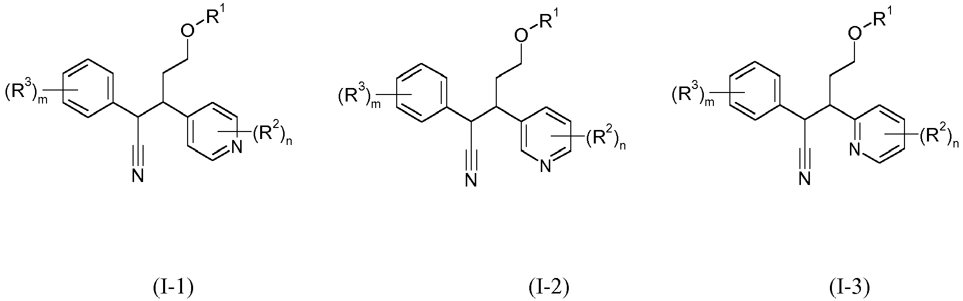 wobei
R¹, R², R³, m und n jeweils die vorstehend oder nachfolgend definierte Bedeutung haben.

Insbesondere bevorzugte erfindungsgemäße Verbindungen der Formel (I) entsprechen der oben definierten Formel (I-2), d.h. eine erfindungsgemäß besonders bevorzugte Bedeutung von Q ist 3-Pyridyl.

Bevorzugte erfindungsgemäße Verbindungen sind Verbindungen der Formel (I), in denen R¹ die in der nachfolgenden Tabelle U1 angegebene Bedeutung hat (siehe Beispielteil).

In Formel (I) bedeutet insbesondere bevorzugt R¹ Wasserstoff oder eine Gruppe ausgewählt aus der Gruppe bestehend aus:
Acetyl, Propanoyl, Butanoyl, Pentanoyl, Hexanoyl, 2-Methylpropanoyl (= Isopropanoyl), 2,2-Difluoracetyl, 2,2,2-Trifluoracetyl, C(=O)OMe, Cyclopropancarbonyl, 1 -Methylcyclopropancarbonyl, Acryl, Prop-2-inoyl, But-2-inoyl, 2-Methylacryl, Benzoyl, 4-Chlorbenzoyl, 3-Chlorbenzoyl, 2-Chlorbenzoyl, 4-Fluorbenzoyl, 3-Fluorbenzoyl, 2-Fluorbenzoyl, 2,2-Dimethylpropanoyl (= Pivaloyl), 3,3-Dimethylbutanoyl, und 2-Nitrobenzoyl.

Weiter bevorzugt sind erfindungsgemäße Verbindungen der Formel (I) oder deren Salze, in denen
- (R²)ₙ: n Substituenten R² bedeutet,
wobei R² (wenn n = 1) oder jeder derSubstituenten R² (wenn n größer als 1) unabhängig voneinander Halogen, Cyano, Nitro, (C₁-C₆)Alkyl, (C₁-C₆)Alkoxy, (C₁-C₆)Alkylthio, (C₁-C₆)Alkylsulfinyl, (C₁-C₆)Alkylsulfonyl, (C₁-C₆)Haloalkyl, (C₂-C₆)Haloalkenyl, (C₁-C₆)Haloalkoxy, (C₁-C₆)Haloalkylthio, (C₁-C₆)Haloalkylsulfinyl, (Ci-C₆)Haloalkylsulfonyl, (C₂-C₆)Alkenyl, (C₂-C₆)Alkinyl, Tri-[(C₁-C₄)alkyl]-silyl oder Tri-[(C₁-C₄)alkyl]-silyl-(C₁-C₄)alkyl bedeutet,
und/oder
- (R³)ₘ: m Substituenten R³ bedeutet,
wobei R³ (wenn m = 1) oder jeder der Substituenten R³ (wenn m größer als 1) unabhängig voneinander Halogen, Cyano, Nitro, (C₁-C₆)Alkyl, (C₁-C₆)Alkoxy, (C₁-C₆)Alkylthio, (C₁-C₆)Alkylsulfinyl, (C₁-C₆)Alkylsulfonyl, (C₁-C₆)Haloalkyl, (C₂-C₆)Haloalkenyl, (C₁-C₆)Haloalkoxy, (C₁-C₆)Haloalkylthio, (C₁-C₆)Haloalkylsulfinyl, (C₁-C₆)Haloalkylsulfonyl, (C₂-C₆)Alkenyl, (C₂-C₆)Alkinyl, NR*R**, Tri-[(C₁-C₄)alkyl]-silyl oder Tri-[(C₁-C₄)alkyl]-silyl-(C₁-C₄)alkyl bedeutet,
oder wobei jeweils zwei am Ring unmittelbar benachbarte Gruppen R³ gemeinsam eine Gruppe der Formel -Z³-A**-Z⁴ bedeuten, in welcher
A** für eine Alkylengruppe mit 1 bis 4 C-Atomen steht, die gegebenenfalls durch einen oder mehrere Reste aus der Gruppe Halogen, (Ci-C₄)Alkyl, (C₁-C₄)Haloalkyl, (C₁-C₄)Alkoxy und (C₁-C₄)Haloalkoxy substituiert ist,
Z³ für eine direkte Bindung, O oder S steht und
Z⁴ für eine direkte Bindung, O oder S steht,
wobei die Gruppe -Z³-A**-Z⁴ zusammen mit den an die Gruppe gebundenen C-Atomen des Phenylrings einen ankondensierten 5 oder 6 Ring bilden,
- R*, R**: jeweils unabhängig voneinander oder gemeinsam mit dem N-Atom die oben angegebene Bedeutung haben,
- n, m: jeweils unabhängig voneinander 0, 1, 2, 3, 4 oder 5 bedeuten, vorzugsweise 0, 1, 2 oder 3, insbesondere 0, 1 oder 2 bedeuten,
wobei ferner vorzugsweise
R¹ Wasserstoff bedeutet oder eine Gruppe ausgewählt aus der Gruppe bestehend aus:
Acetyl, Propanoyl, Butanoyl, Pentanoyl, Hexanoyl, 2-Methylpropanoyl (= Isopropanoyl), 2,2-Difluoracetyl, 2,2,2-Trifluoracetyl, C(=O)OMe, Cyclopropancarbonyl, 1 -Methylcyclopropancarbonyl, Acryl, Prop-2-inoyl, But-2-inoyl, 2-Methylacryl, Benzoyl, 4-Chlorbenzoyl, 3-Chlorbenzoyl, 2-Chlorbenzoyl, 4-Fluorbenzoyl, 3-Fluorbenzoyl, 2-Fluorbenzoyl, 2,2-Dimethylpropanoyl (= Pivaloyl), 3,3-Dimethylbutanoyl, 2-Nitrobenzoyl, 2-Fluoracetyl, 2-Chloracetyl, 2-Bromacetyl, 2,2-Dichloracetyl, 2-Methoxyacetyl, 2,6-Difluorbenzoyl, C(O)C(=O)OMe, und C(O)CH₂C(O)OMe.

Weiter bevorzugt sind erfindungsgemäße Verbindungen der Formel (I) oder deren Salze bzw. der unten definierten Formel (Ia) oder deren Salze, in denen
- (R²)ₙ: n Substituenten R² bedeutet,
wobei, im Falle dass n = 1 ist, der Substituent R² oder, im Falle dass n größer als 1 ist, jeder der Substituenten R² unabhängig voneinander
Halogen, Cyano, Nitro, (C₁-C₄)Alkyl, (C₁-C₄)Alkoxy, (C₁-C₄)Alkylthio, (C₁-C₄)Alkylsulfinyl, (C₁-C₄)Alkylsulfonyl, (C₁-C₄)Haloalkyl, (C₂-C₄)Haloalkenyl, (C₁-C₄)Haloalkoxy, (C₁-C₄)Haloalkylthio, (C₂-C₄)Haloalkylsulfinyl, (C₁-C₄)Haloalkylsulfonyl, (C₂-C₄)Alkenyl, (C₂-C₄)Alkinyl, Tri-[(C₁-C₄)Alkyl]-silyl oder Tri-[(C₁-C₄)Alkyl]-silyl -(C₁-C₄)Alkyl bedeutet und
- n: 0, 1, 2, 3 oder 4, vorzugsweise 0, 1, 2 oder 3, insbesondere 0, 1 oder 2 bedeutet,
und/oder
- (R³)ₘ: m Substituenten R³ bedeutet,
wobei, im Falle dass m = 1 ist, der Substituent R³ oder, im Falle dass m größer als 1 ist, jeder der Substituenten R³ unabhängig voneinander Halogen, Cyano, Nitro, (C₁-C₄)Alkyl, (C₁-C₄)Alkoxy, (C₁-C₄)Alkylthio, (C₁-C₄)Alkylsulfinyl, (C₁-C₄)Alkylsulfonyl, (C₁-C₄)Haloalkyl, (C₂-C₄)Haloalkenyl, (C₁-C₄)Haloalkoxy, (C₁-C₄)Haloalkylthio, (C₁-C₄)Haloalkylsulfinyl, (C₁-C₄)Haloalkylsulfonyl, (C₂-C₆)Alkenyl, (C₂-C₆)Alkinyl oder Tri-[(C₁-C₄)Alkyl]silyl-Z^{b}-, wobei Z^{b} = für eine kovalente Bindung oder (C₁-C₄)Alkylen steht, bedeutet, oder
jeweils zwei am Ring unmittelbar benachbarte Gruppen R³ gemeinsam eine Gruppe der Formel -Z³-A**-Z⁴ bedeuten,
wobei
A** für eine Alkylengruppe steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z³ für O oder S steht, und
Z⁴ für O oder S steht, wobei die Gruppe -Z³-A**-Z⁴ zusammen mit den an die Gruppe gebundenen C-Atomen des Phenylrings einen ankondensierten 5 oder 6 Ring bilden,
- m: 0, 1, 2, 3, 4 oder 5, vorzugsweise 0, 1, 2, 3 oder 4, insbesondere 0, 1, 2 oder 3 bedeutet.

Weiter bevorzugt sind erfindungsgemäße Verbindungen der Formel (I) oder deren Salze bzw. der unten definierten Formel (Ia) oder deren Salze, in denen
- (R²)ₙ: n Substituenten R² bedeutet,
wobei, im Falle dass n = 1 ist, der Substituent R² oder, im Falle dass n größer als 1 ist, jeder der Substituenten R² unabhängig voneinander Fluor, Chlor, Brom, Iod, Cyano, Nitro, Methyl, Ethyl, Methoxy, Methylthio, Methylsulfinyl, Methylsulfonyl, Trifluormethyl, Trifluormethoxy, Trifluoralkylthio, Trifluormethylsulfinyl oder Trifluormethylsulfonyl bedeutet, und
- (R³)ₘ: m Substituenten R³ bedeutet,
wobei, im Falle dass m = 1 ist, der Substituent Rest R³ oder, im Falle dass m größer als 1 ist, jeder der Substituenten R³ unabhängig voneinander Fluor, Chlor, Brom, Iod, Cyano, Nitro, Methyl, Methoxy (dabei vorzugsweise 2-Methoxy, 3-Methoxy), Methylthio, Methylsulfinyl, Methylsulfonyl, Trifluormethyl, Trifluormethoxy, Trifluoralkylthio, Trifluormethylsulfinyl oder Trifluormethylsulfonyl bedeutet, und
- m: 0, 1, 2, 3 oder 4, bevorzugt 0, 1, 2 oder 3 bedeutet, und
- n: 0, 1, 2 oder 3, bevorzugt 0, 1 oder 2 bedeutet,
und ferner vorzugsweise
- R¹: Wasserstoff bedeutet oder eine Gruppe ausgewählt aus der Gruppe bestehend aus: Acetyl, Propanoyl, Butanoyl, Pentanoyl, Hexanoyl, 2-Methylpropanoyl (= Isopropanoyl), 2,2-Difluoracetyl, 2,2,2-Trifluoracetyl, C(=O)OMe, Cyclopropancarbonyl, 1-Methylcyclopropancarbonyl, Acryl, Prop-2-inoyl, But-2-inoyl, 2-Methylacryl, Benzoyl, 4-Chlorbenzoyl, 3-Chlorbenzoyl, 2-Chlorbenzoyl, 4-Fluorbenzoyl, 3-Fluorbenzoyl, 2-Fluorbenzoyl, 2,2-Dimethylpropanoyl (= Pivaloyl), 3,3-Dimethylbutanoyl, und 2-Nitrobenzoyl.

Insbesondere bevorzugt sind erfindungsgemäße Verbindungen der Formel (I) oder deren Salze bzw. der unten definierten Formel (Ia) oder deren Salze, in denen
- (R²)ₙ: n Substituenten R² bedeutet, wobei, im Falle dass n = 1 ist, der Substituent R² oder, im Falle dass n größer als 1 ist, jeder der Substituenten R² unabhängig voneinander Fluor, Chlor, Brom, Iod, Cyano, Nitro, Methyl, Ethyl, oder Methoxy bedeutet.

Insbesondere bevorzugt sind erfindungsgemäße Verbindungen der Formel (I) oder deren Salze bzw. der unten definierten Formel (Ia) oder deren Salze, in denen
- (R³)ₘ: m Substituenten R³ bedeutet, 2-Brom, 3-Brom, 4-Brom, 2-Chlor, 3-Chlor, 4-Chlor, 2-Fluor, 3-Fluor, 4-Fluor, 2-Cyano, 3-Cyano, 4-Cyano, 2-Methyl, 3-Methyl, 4-Methyl, 2-Ethyl, 3-Ethyl, 4-Ethyl, 2-CF₃, 3-CF₃, 4-CF₃, 2-Methoxy, 3-Methoxy, 4-Methoxy, 2-Ethoxy, 3-Ethoxy, 4-Ethoxy, 2-Methylthio, 3-Methylthio, 4-Methylthio, 2-Methylsulfinyl, 3-Methylsulfinyl, 4-Methylsulfinyl, 2-Methylsulfonyl, 3-Methylsulfonyl, 4-Methylsulfonyl, 2-Nitro, 3-Nitro, 4-Nitro, 2,3-Dimethyl, 2,4-Dimethyl, 2,5-Dimethyl, 2,6-Dimethyl, 3,4-Dimethyl, 3,5-Dimethyl, 2,3-Difluor, 2,4-Difluor, 2,5-Difluor, 2,6-Difluor, 3,4-Difluor, 3,5-Difluor, 2,3-Dichlor, 2,4-Dichlor, 2,5-Dichlor, 2,6-Dichlor, 3,4-Dichlor, 3,5-Dichlor, 2,5-Dicyano, 2,6-Dicyano, (2-Cl-3-F), (2-Cl-4-F), (2-Cl-5-F), (2-Cl-6-F), (3-Cl-2-F), (3-Cl-4-F), (3-Cl-5-F), (3-Cl-6-F), (4-Cl-2-F), (4-Cl-3-F), (4-Br-2-F), (4-Br-3-F), (4-CN-3-F), (4-NO₂-3-F), (4-OMe-3-F), (3-Br-4-F), (3-CN-4-F), (3-NO₂-4-F), (3-CN-4-Cl), (3-NO₂-4-Cl), (5-CN-2-F), 2,3,4-Trifluor, 2,3,5-Trifluor, 2,3,6-Trifluor, 2,4,6-Trifluor, 3,4,5-Trifluor, 2,3,4-Trichlor, 2,3,5-Trichlor, 2,3,6-Trichlor, 2,4,6-Trichlor, 3,4,5-Trichlor oder (2,6-F₂-4-Cl) bedeutet.

Bevorzugte erfindungsgemäße Verbindungen sind Verbindungen der nachfolgend dargestellten Formel (Ia), (entsprechend Verbindungen der Formel (I), in denen der Rest R¹ = Wasserstoff bedeutet), deren Salze und deren Ester 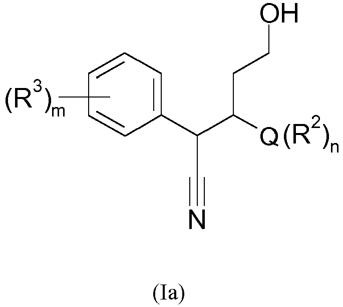 worin Q, R², R³, m und n jeweils die vorstehend bzw. nachstehend angebenen Bedeutungen haben, vorzugsweise die als bevorzugt angegebenen Bedeutungen, bevorzugt die als bevorzugt angegebenen Bedeutungen, besonders bevorzugt die als bevorzugt angegebenen Bedeutungen, und insbesondere (bevorzugt) die als insbesondere (bevorzugt) angegebenen Bedeutungen.

In einer bevorzugten Ausgestaltung der vorliegenden Erfindung ist m + n > 0, d.h. dass vorzugsweise mindestens einer der Reste R² und R³ nicht Wasserstoff bedeutet.

Erfindungsgemäß bevorzugt sind Verbindungen der Formel (I) bzw. (Ia), oder deren Salze, worin n > 0 ist.

Erfindungsgemäß ebenfalls bevorzugt sind Verbindungen der Formel (I) bzw. (Ia), oder deren Salze, worin m > 0 ist.

In einer bevorzugten Ausgestaltung der vorliegenden Erfindung ist m + n > 1, d.h. dass vorzugsweise mindestens zwei Reste R², mindestens zwei Reste R³ oder mindestens ein Rest R² und mindestens ein Rest R³ jeweils nicht Wasserstoff bedeuten.

In bevorzugten erfindungsgemäßen Verbindungen der Formel (I) bzw. (Ia), oder deren Salzen, beträgt die Summe aus m + n = 2, 3, 4, oder 5.

In besonders bevorzugten erfindungsgemäßen Verbindungen der Formel (I) bzw. (Ia) ist n größer oder gleich 1 und ein oder mehrere Reste R² bedeuten Halogen, vorzugsweise Halogen ausgewählt aus der Gruppe bestehend aus F, Cl und Br.

In besonders bevorzugten erfindungsgemäßen Verbindungen der Formel (I) bzw. (Ia) ist m größer oder gleich 1 und ein oder mehrere Reste R³ bedeuten Halogen, vorzugsweise Halogen aus der Gruppe bestehend aus F, Cl und Br.

Insbesondere bevorzugte erfindungsgemäße Verbindungen der Formel (Ia) entsprechen der oben definierten Formel (I-2), in der R¹ = Wasserstoff ist.

Ebenfalls insbesondere bevorzugt sind erfindungsgemäße Verbindungen der Formel (I) oder deren Salze, in denen
- (R²)ₙ: n Substituenten R² bedeutet,
wobei, im Falle dass n = 1 ist, der Substituent R² oder, im Falle dass n größer als 1 ist, jeder der Substituenten R² unabhängig voneinander Fluor, Chlor, Brom, Iod, Cyano, Nitro, Methyl, Ethyl, oder Methoxy bedeutet,
- (R³)ₘ: m Substituenten R³ bedeutet, wobei, im Falle dass m = 1 ist, der Substituent Rest R³ oder, im Falle dass m größer als 1 ist, jeder der Substituenten R³ unabhängig voneinander 2-Brom, 3-Brom, 4-Brom, 2-Chlor, 3-Chlor, 4-Chlor, 2-Fluor, 3-Fluor, 4-Fluor, 2-Cyano, 3-Cyano, 4-Cyano, 2-Methyl, 3-Methyl, 4-Methyl, 2-Ethyl, 3-Ethyl, 4-Ethyl, 2-CF₃, 3-CF₃, 4-CF₃, 2-Methoxy, 3-Methoxy, 4-Methoxy, 2-Ethoxy, 3-Ethoxy, 4-Ethoxy, 2-Methylthio, 3-Methylthio, 4-Methylthio, 2-Methylsulfinyl, 3-Methylsulflnyl, 4-Methylsulfinyl, 2-Methylsulfonyl, 3-Methylsulfonyl, 4-Methylsulfonyl, 2-Nitro, 3-Nitro, 4-Nitro, 2,3-Dimethyl, 2,4-Dimethyl, 2,5-Dimethyl, 2,6-Dimethyl, 3,4-Dimethyl, 3,5-Dimethyl, 2,3-Difluor, 2,4-Difluor, 2,5-Difluor, 2,6-Difluor, 3,4-Difluor, 3,5-Difluor, 2,3-Dichlor, 2,4-Dichlor, 2,5-Dichlor, 2,6-Dichlor, 3,4-Dichlor, 3,5-Dichlor, 2,5-Dicyano, 2,6-Dicyano, (2-Cl-3-F), (2-Cl-4-F), (2-Cl-5-F), (2-Cl-6-F), (3-Cl-2-F), (3-Cl-4-F), (3-Cl-5-F), (3-Cl-6-F), (4-Cl-2-F), (4-Cl-3-F), (4-Br-2-F), (4-Br-3-F), (4-CN-3-F), (4-NO₂-3-F), (4-OMe-3-F), (3-Br-4-F), (3-CN-4-F), (3-NO₂-4-F), (3-CN-4-Cl), (3-NO₂-4-Cl), (5-CN-2-F), 2,3,4-Trifluor, 2,3,5-Trifluor, 2,3,6-Trifluor, 2,4,6-Trifluor, 3,4,5-Trifluor, 2,3,4-Trichlor, 2,3,5-Trichlor, 2,3,6-Trichlor, 2,4,6-Trichlor, 3,4,5-Trichlor oder (2,6-F₂-4-Cl) bedeutet,
- R¹: Wasserstoff bedeutet oder eine Gruppe ausgewählt aus der Gruppe bestehend aus: Acetyl, Propanoyl, Butanoyl, Pentanoyl, Hexanoyl, 2-Methylpropanoyl (= Isopropanoyl), 2,2-Difluoracetyl, 2,2,2-Trifluoracetyl, C(=O)OMe, Cyclopropancarbonyl, 1-Methylcyclopropancarbonyl, Acryl, Prop-2-inoyl, But-2-inoyl, 2-Methylacryl, Benzoyl, 4-Chlorbenzoyl, 3-Chlorbenzoyl, 2-Chlorbenzoyl, 4-Fluorbenzoyl, 3-Fluorbenzoyl, 2-Fluorbenzoyl, 2,2-Dimethylpropanoyl (= Pivaloyl), 3,3-Dimethylbutanoyl, und 2-Nitrobenzoyl,
- m: 0, 1, 2 oder 3 bedeutet, und
- n: 0, 1 oder 2 bedeutet,
wobei vorzugsweise die Summe aus m + n = 2, 3, 4, oder 5 beträgt.

Bevorzugte erfindungsgemäße Verbindungen sind Verbindungen der Formel (I) bzw. (Ia), in denen Q(R²)ₙ die in der nachfolgenden Tabelle 1 angegebene Bedeutung hat.

Bevorzugte erfindungsgemäße Verbindungen sind Verbindungen der Formel (I) bzw. (Ia), in denen (R³)ₘ die in der nachfolgenden Tabelle 1 angegebene Bedeutung hat.

Bevorzugte erfindungsgemäße Verbindungen sind Verbindungen der Formel (I) bzw. der Formel (Ia) wie in der nachfolgenden Tabelle 1 definiert.

In der nachfolgenden Tabelle 1 sind bevorzugte Bedeutungen von Q(R²)ₙ und (R³)ₘ angegeben. Die dabei verwendeten Abkürzungen und Schreibweisen sind im Beispielteil erläutert.

**Tabelle 1: Bevorzugte Bedeutungen von Q(R²)ₙ und (R³)ₘ in erfindungsgemäßen Verbindungen der Formel (I) bzw. der Formel (Ia):**

| Nr. | Q(R²)ₙ | (R³)ₘ |
|---|---|---|
| 1 | 6-Fluorpyridin-2-yl | H |
| 2 | 6-Fluorpyridin-2-yl | 2-F |
| 3 | 6-Fluorpyridin-2-yl | 3-F |
| 4 | 6-Fluorpyridin-2-yl | 4-F |
| 5 | 6-Fluorpyridin-2-yl | 3-Cl |
| 6 | 6-Fluorpyridin-2-yl | 4-Cl |
| 7 | 6-Fluorpyridin-2-yl | 3-Br |
| 8 | 6-Fluorpyridin-2-yl | 4-Br |
| 9 | 6-Fluorpyridin-2-yl | 3-I |
| 10 | 6-Fluorpyridin-2-yl | 3-CN |
| 11 | 6-Fluorpyridin-2-yl | 4-CN |
| 12 | 6-Fluorpyridin-2-yl | 3-NO₂ |
| 13 | 6-Fluorpyridin-2-yl | 3-Me |
| 14 | 6-Fluorpyridin-2-yl | 4-Me |
| 15 | 6-Fluorpyridin-2-yl | 2,3-F₂ |
| 16 | 6-Fluorpyridin-2-yl | 2,4-F₂ |
| 17 | 6-Fluorpyridin-2-yl | 2,5-F₂ |
| 18 | 6-Fluorpyridin-2-yl | 2,6-F₂ |
| 19 | 6-Fluorpyridin-2-yl | 3,4-F₂ |
| 20 | 6-Fluorpyridin-2-yl | 3,5-F₂ |
| 21 | 6-Fluorpyridin-2-yl | 3,4,5-F₃ |
| 22 | 6-Fluorpyridin-2-yl | 3-F, 4-Cl |
| 23 | 6-Fluorpyridin-2-yl | 3-F, 4-Br |
| 24 | 6-Fluorpyridin-2-yl | 3-CN, 4-F |
| 25 | 6-Fluorpyridin-2-yl | 3-Br, 4-F |
| 26 | 6-Fluorpyridin-2-yl | 3-Cl, 4-F |
| 27 | 6-Fluorpyridin-2-yl | 3,4-Cl₂ |
| 28 | 5-Fluorpyridin-2-yl | H |
| 29 | 5-Fluorpyridin-2-yl | 2-F |
| 30 | 5-Fluorpyridin-2-yl | 3-F |
| 31 | 5-Fluorpyridin-2-yl | 4-F |
| 32 | 5-Fluorpyridin-2-yl | 3-Cl |
| 33 | 5-Fluorpyridin-2-yl | 4-Cl |
| 34 | 5-Fluorpyridin-2-yl | 3-Br |
| 35 | 5-Fluorpyridin-2-yl | 4-Br |
| 36 | 5-Fluorpyridin-2-yl | 3-I |
| 37 | 5-Fluorpyridin-2-yl | 3-CN |
| 38 | 5-Fluorpyridin-2-yl | 4-CN |
| 39 | 5-Fluorpyridin-2-yl | 3-NO₂ |
| 40 | 5-Fluorpyridin-2-yl | 3-Me |
| 41 | 5-Fluorpyridin-2-yl | 4-Me |
| 42 | 5-Fluorpyridin-2-yl | 2,3-F₂ |
| 43 | 5-Fluorpyridin-2-yl | 2,4-F₂ |
| 44 | 5-Fluorpyridin-2-yl | 2,5-F₂ |
| 45 | 5-Fluorpyridin-2-yl | 2,6-F₂ |
| 46 | 5-Fluorpyridin-2-yl | 3,4-F₂ |
| 47 | 5-Fluorpyridin-2-yl | 3,5-F₂ |
| 48 | 5-Fluorpyridin-2-yl | 3,4,5-F₃ |
| 49 | 5-Fluorpyridin-2-yl | 3-F, 4-Cl |
| 50 | 5-Fluorpyridin-2-yl | 3-F, 4-Br |
| 51 | 5-Fluorpyridin-2-yl | 3-CN, 4-F |
| 52 | 5-Fluorpyridin-2-yl | 3-Br, 4-F |
| 53 | 5-Fluorpyridin-2-yl | 3-Cl, 4-F |
| 54 | 5-Fluorpyridin-2-yl | 3,4-Cb |
| 55 | 4-Fluorpyridin-2-yl | H |
| 56 | 4-Fluorpyridin-2-yl | 2-F |
| 57 | 4-Fluorpyridin-2-yl | 3-F |
| 58 | 4-Fluorpyridin-2-yl | 4-F |
| 59 | 4-Fluorpyridin-2-yl | 3-Cl |
| 60 | 4-Fluorpyridin-2-yl | 4-Cl |
| 61 | 4-Fluorpyridin-2-yl | 3-Br |
| 62 | 4-Fluorpyridin-2-yl | 4-Br |
| 63 | 4-Fluorpyridin-2-yl | 3-I |
| 64 | 4-Fluorpyridin-2-yl | 3-CN |
| 65 | 4-Fluorpyridin-2-yl | 4-CN |
| 66 | 4-Fluorpyridin-2-yl | 3-NO₂ |
| 67 | 4-Fluorpyridin-2-yl | 3-Me |
| 68 | 4-Fluorpyridin-2-yl | 4-Me |
| 69 | 4-Fluorpyridin-2-yl | 2,3-F₂ |
| 70 | 4-Fluorpyridin-2-yl | 2,4-F₂ |
| 71 | 4-Fluorpyridin-2-yl | 2,5-F₂ |
| 72 | 4-Fluorpyridin-2-yl | 2,6-F₂ |
| 73 | 4-Fluorpyridin-2-yl | 3,4-F₂ |
| 74 | 4-Fluorpyridin-2-yl | 3,5-F₂ |
| 75 | 4-Fluorpyridin-2-yl | 3,4,5-F₃ |
| 76 | 4-Fluorpyridin-2-yl | 3-F, 4-Cl |
| 77 | 4-Fluorpyridin-2-yl | 3-F, 4-Br |
| 78 | 4-Fluorpyridin-2-yl | 3-CN, 4-F |
| 79 | 4-Fluorpyridin-2-yl | 3-Br, 4-F |
| 80 | 4-Fluorpyridin-2-yl | 3-Cl, 4-F |
| 81 | 4-Fluorpyridin-2-yl | 3,4-Cb |
| 82 | 3-Fluorpyridin-2-yl | H |
| 83 | 3-Fluorpyridin-2-yl | 2-F |
| 84 | 3-Fluorpyridin-2-yl | 3-F |
| 85 | 3-Fluorpyridin-2-yl | 4-F |
| 86 | 3-Fluorpyridin-2-yl | 3-Cl |
| 87 | 3-Fluorpyridin-2-yl | 4-Cl |
| 88 | 3-Fluorpyridin-2-yl | 3-Br |
| 89 | 3-Fluorpyridin-2-yl | 4-Br |
| 90 | 3-Fluorpyridin-2-yl | 3-I |
| 91 | 3-Fluorpyridin-2-yl | 3-CN |
| 92 | 3-Fluorpyridin-2-yl | 4-CN |
| 93 | 3-Fluorpyridin-2-yl | 3-NO₂ |
| 94 | 3-Fluorpyridin-2-yl | 3-Me |
| 95 | 3-Fluorpyridin-2-yl | 4-Me |
| 96 | 3-Fluorpyridin-2-yl | 2,3-F₂ |
| 97 | 3-Fluorpyridin-2-yl | 2,4-F₂ |
| 98 | 3-Fluorpyridin-2-yl | 2,5-F₂ |
| 99 | 3-Fluorpyridin-2-yl | 2,6-F₂ |
| 100 | 3-Fluorpyridin-2-yl | 3,4-F₂ |
| 101 | 3-Fluorpyridin-2-yl | 3,5-F₂ |
| 102 | 3-Fluorpyridin-2-yl | 3,4,5-F₃ |
| 103 | 3-Fluorpyridin-2-yl | 3-F, 4-Cl |
| 104 | 3-Fluorpyridin-2-yl | 3-F, 4-Br |
| 105 | 3-Fluorpyridin-2-yl | 3-CN, 4-F |
| 106 | 3-Fluorpyridin-2-yl | 3-Br, 4-F |
| 107 | 3-Fluorpyridin-2-yl | 3-Cl, 4-F |
| 108 | 3-Fluorpyridin-2-yl | 3,4-Cl₂ |
| 109 | 6-Chlorpyridin-2-yl | H |
| 110 | 6-Chlorpyridin-2-yl | 2-F |
| 111 | 6-Chlorpyridin-2-yl | 3-F |
| 112 | 6-Chlorpyridin-2-yl | 4-F |
| 113 | 6-Chlorpyridin-2-yl | 3-Cl |
| 114 | 6-Chlorpyridin-2-yl | 4-Cl |
| 115 | 6-Chlorpyridin-2-yl | 3-Br |
| 116 | 6-Chlorpyridin-2-yl | 4-Br |
| 117 | 6-Chlorpyridin-2-yl | 3-I |
| 118 | 6-Chlorpyridin-2-yl | 3-CN |
| 119 | 6-Chlorpyridin-2-yl | 4-CN |
| 120 | 6-Chlorpyridin-2-yl | 3-NO₂ |
| 121 | 6-Chlorpyridin-2-yl | 3-Me |
| 122 | 6-Chlorpyridin-2-yl | 4-Me |
| 123 | 6-Chlorpyridin-2-yl | 2,3-F₂ |
| 124 | 6-Chlorpyridin-2-yl | 2,4-F₂ |
| 125 | 6-Chlorpyridin-2-yl | 2,5-F₂ |
| 126 | 6-Chlorpyridin-2-yl | 2,6-F₂ |
| 127 | 6-Chlorpyridin-2-yl | 3,4-F₂ |
| 128 | 6-Chlorpyridin-2-yl | 3,5-F₂ |
| 129 | 6-Chlorpyridin-2-yl | 3,4,5-F₃ |
| 130 | 6-Chlorpyridin-2-yl | 3-F, 4-Cl |
| 131 | 6-Chlorpyridin-2-yl | 3-F, 4-Br |
| 132 | 6-Chlorpyridin-2-yl | 3-CN, 4-F |
| 133 | 6-Chlorpyridin-2-yl | 3-Br, 4-F |
| 134 | 6-Chlorpyridin-2-yl | 3-Cl, 4-F |
| 135 | 6-Chlorpyridin-2-yl | 3,4-Cl₂ |
| 136 | 5-Chlorpyridin-2-yl | H |
| 137 | 5-Chlorpyridin-2-yl | 2-F |
| 138 | 5-Chlorpyridin-2-yl | 3-F |
| 139 | 5-Chlorpyridin-2-yl | 4-F |
| 140 | 5-Chlorpyridin-2-yl | 3-Cl |
| 141 | 5-Chlorpyridin-2-yl | 4-Cl |
| 142 | 5-Chlorpyridin-2-yl | 3-Br |
| 143 | 5-Chlorpyridin-2-yl | 4-Br |
| 144 | 5-Chlorpyridin-2-yl | 3-I |
| 145 | 5-Chlorpyridin-2-yl | 3-CN |
| 146 | 5-Chlorpyridin-2-yl | 4-CN |
| 147 | 5-Chlorpyridin-2-yl | 3-NO₂ |
| 148 | 5-Chlorpyridin-2-yl | 3-Me |
| 149 | 5-Chlorpyridin-2-yl | 4-Me |
| 150 | 5-Chlorpyridin-2-yl | 2,3-F₂ |
| 151 | 5-Chlorpyridin-2-yl | 2,4-F₂ |
| 152 | 5-Chlorpyridin-2-yl | 2,5-F₂ |
| 153 | 5-Chlorpyridin-2-yl | 2,6-F₂ |
| 154 | 5-Chlorpyridin-2-yl | 3,4-F₂ |
| 155 | 5-Chlorpyridin-2-yl | 3,5-F₂ |
| 156 | 5-Chlorpyridin-2-yl | 3,4,5-F₃ |
| 157 | 5-Chlorpyridin-2-yl | 3-F, 4-Cl |
| 158 | 5-Chlorpyridin-2-yl | 3-F, 4-Br |
| 159 | 5-Chlorpyridin-2-yl | 3-CN, 4-F |
| 160 | 5-Chlorpyridin-2-yl | 3-Br, 4-F |
| 161 | 5-Chlorpyridin-2-yl | 3-Cl, 4-F |
| 162 | 5-Chlorpyridin-2-yl | 3,4-Cl₂ |
| 163 | 4-Chlorpyridin-2-yl | H |
| 164 | 4-Chlorpyridin-2-yl | 2-F |
| 165 | 4-Chlorpyridin-2-yl | 3-F |
| 166 | 4-Chlorpyridin-2-yl | 4-F |
| 167 | 4-Chlorpyridin-2-yl | 3-Cl |
| 168 | 4-Chlorpyridin-2-yl | 4-Cl |
| 169 | 4-Chlorpyridin-2-yl | 3-Br |
| 170 | 4-Chlorpyridin-2-yl | 4-Br |
| 171 | 4-Chlorpyridin-2-yl | 3-I |
| 172 | 4-Chlorpyridin-2-yl | 3-CN |
| 173 | 4-Chlorpyridin-2-yl | 4-CN |
| 174 | 4-Chlorpyridin-2-yl | 3-NO₂ |
| 175 | 4-Chlorpyridin-2-yl | 3-Me |
| 176 | 4-Chlorpyridin-2-yl | 4-Me |
| 177 | 4-Chlorpyridin-2-yl | 2,3-F₂ |
| 178 | 4-Chlorpyridin-2-yl | 2,4-F₂ |
| 179 | 4-Chlorpyridin-2-yl | 2,5-F₂ |
| 180 | 4-Chlorpyridin-2-yl | 2,6-F₂ |
| 181 | 4-Chlorpyridin-2-yl | 3,4-F₂ |
| 182 | 4-Chlorpyridin-2-yl | 3,5-F₂ |
| 183 | 4-Chlorpyridin-2-yl | 3,4,5-F₃ |
| 184 | 4-Chlorpyridin-2-yl | 3-F,4-Cl |
| 185 | 4-Chlorpyridin-2-yl | 3-F, 4-Br |
| 186 | 4-Chlorpyridin-2-yl | 3-CN, 4-F |
| 187 | 4-Chlorpyridin-2-yl | 3-Br, 4-F |
| 188 | 4-Chlorpyridin-2-yl | 3-Cl, 4-F |
| 189 | 4-Chlorpyridin-2-yl | 3,4-Cb |
| 190 | 3-Chlorpyridin-2-yl | H |
| 191 | 3-Chlorpyridin-2-yl | 2-F |
| 192 | 3-Chlorpyridin-2-yl | 3-F |
| 193 | 3-Chlorpyridin-2-yl | 4-F |
| 194 | 3-Chlorpyridin-2-yl | 3-Cl |
| 195 | 3-Chlorpyridin-2-yl | 4-Cl |
| 196 | 3-Chlorpyridin-2-yl | 3-Br |
| 197 | 3-Chlorpyridin-2-yl | 4-Br |
| 198 | 3-Chlorpyridin-2-yl | 3-I |
| 199 | 3-Chlorpyridin-2-yl | 3-CN |
| 200 | 3-Chlorpyridin-2-yl | 4-CN |
| 201 | 3-Chlorpyridin-2-yl | 3-NO₂ |
| 202 | 3-Chlorpyridin-2-yl | 3-Me |
| 203 | 3-Chlorpyridin-2-yl | 4-Me |
| 204 | 3-Chlorpyridin-2-yl | 2,3-F₂ |
| 205 | 3-Chlorpyridin-2-yl | 2,4-F₂ |
| 206 | 3-Chlorpyridin-2-yl | 2,5-F₂ |
| 207 | 3-Chlorpyridin-2-yl | 2,6-F₂ |
| 208 | 3 -Chlorpyridin-2-yl | 3,4-F₂ |
| 209 | 3-Chlorpyridin-2-yl | 3,5-F₂ |
| 210 | 3-Chlorpyridin-2-yl | 3,4,5-F₃ |
| 211 | 3-Chlorpyridin-2-yl | 3-F, 4-Cl |
| 212 | 3-Chlorpyridin-2-yl | 3-F, 4-Br |
| 213 | 3-Chlorpyridin-2-yl | 3-CN, 4-F |
| 214 | 3-Chlorpyridin-2-yl | 3-Br, 4-F |
| 215 | 3-Chlorpyridin-2-yl | 3-Cl, 4-F |
| 216 | 3-Chlorpyridin-2-yl | 3,4-Cb |
| 217 | 2-Fluorpyridin-3-yl | H |
| 218 | 2-Fluorpyridin-3-yl | 2-F |
| 219 | 2-Fluorpyridin-3-yl | 3-F |
| 220 | 2-Fluorpyridin-3-yl | 4-F |
| 221 | 2-Fluorpyridin-3-yl | 3-Cl |
| 222 | 2-Fluorpyridin-3-yl | 4-Cl |
| 223 | 2-Fluorpyridin-3-yl | 3-Br |
| 224 | 2-Fluorpyridin-3-yl | 4-Br |
| 225 | 2-Fluorpyridin-3-yl | 3-I |
| 226 | 2-Fluorpyridin-3-yl | 3-CN |
| 227 | 2-Fluorpyridin-3-yl | 4-CN |
| 228 | 2-Fluorpyridin-3-yl | 3-NO₂ |
| 229 | 2-Fluorpyridin-3-yl | 3-Me |
| 230 | 2-Fluorpyridin-3-yl | 4-Me |
| 231 | 2-Fluorpyridin-3-yl | 2,3-F₂ |
| 232 | 2-Fluorpyridin-3-yl | 2,4-F₂ |
| 233 | 2-Fluorpyridin-3-yl | 2,5-F₂ |
| 234 | 2-Fluorpyridin-3-yl | 2,6-F₂ |
| 235 | 2-Fluorpyridin-3-yl | 3,4-F₂ |
| 236 | 2-Fluorpyridin-3-yl | 3,5-F₂ |
| 237 | 2-Fluorpyridin-3-yl | 3,4,5-F₃ |
| 238 | 2-Fluorpyridin-3-yl | 3-F, 4-Cl |
| 239 | 2-Fluorpyridin-3-yl | 3-F, 4-Br |
| 240 | 2-Fluorpyridin-3-yl | 3-CN, 4-F |
| 241 | 2-Fluorpyridin-3-yl | 3-Br, 4-F |
| 242 | 2-Fluorpyridin-3-yl | 3-Cl, 4-F |
| 243 | 2-Fluorpyridin-3-yl | 3,4-Cb |
| 244 | 6-Fluorpyridin-3-yl | H |
| 245 | 6-Fluorpyridin-3-yl | 2-F |
| 246 | 6-Fluorpyridin-3-yl | 3-F |
| 247 | 6-Fluorpyridin-3-yl | 4-F |
| 248 | 6-Fluorpyridin-3-yl | 3-Cl |
| 249 | 6-Fluorpyridin-3-yl | 4-Cl |
| 250 | 6-Fluorpyridin-3-yl | 3-Br |
| 251 | 6-Fluorpyridin-3-yl | 4-Br |
| 252 | 6-Fluorpyridin-3-yl | 3-I |
| 253 | 6-Fluorpyridin-3-yl | 3-CN |
| 254 | 6-Fluorpyridin-3-yl | 4-CN |
| 255 | 6-Fluorpyridin-3-yl | 3-NO₂ |
| 256 | 6-Fluorpyridin-3-yl | 3-Me |
| 257 | 6-Fluorpyridin-3-yl | 4-Me |
| 258 | 6-Fluorpyridin-3-yl | 2,3-F₂ |
| 259 | 6-Fluorpyridin-3-yl | 2,4-F₂ |
| 260 | 6-Fluorpyridin-3-yl | 2,5-F₂ |
| 261 | 6-Fluorpyridin-3-yl | 2,6-F₂ |
| 262 | 6-Fluorpyridin-3-yl | 3,4-F₂ |
| 263 | 6-Fluorpyridin-3-yl | 3,5-F₂ |
| 264 | 6-Fluorpyridin-3-yl | 3,4,5-F₃ |
| 265 | 6-Fluorpyridin-3-yl | 3-F, 4-Cl |
| 266 | 6-Fluorpyridin-3-yl | 3-F, 4-Br |
| 267 | 6-Fluorpyridin-3-yl | 3-CN, 4-F |
| 268 | 6-Fluorpyridin-3-yl | 3-Br, 4-F |
| 269 | 6-Fluorpyridin-3-yl | 3-Cl, 4-F |
| 270 | 6-Fluorpyridin-3-yl | 3,4-Cb |
| 271 | 5-Fluorpyridin-3-yl | H |
| 272 | 5-Fluorpyridin-3-yl | 2-F |
| 273 | 5-Fluorpyridin-3-yl | 3-F |
| 274 | 5-Fluorpyridin-3-yl | 4-F |
| 275 | 5-Fluorpyridin-3-yl | 3-Cl |
| 276 | 5-Fluorpyridin-3-yl | 4-Cl |
| 277 | 5-Fluorpyridin-3-yl | 3-Br |
| 278 | 5-Fluorpyridin-3-yl | 4-Br |
| 279 | 5-Fluorpyridin-3-yl | 3-I |
| 280 | 5-Fluorpyridin-3-yl | 3-CN |
| 281 | 5-Fluorpyridin-3-yl | 4-CN |
| 282 | 5-Fluorpyridin-3-yl | 3-NO₂ |
| 283 | 5-Fluorpyridin-3-yl | 3-Me |
| 284 | 5-Fluorpyridin-3-yl | 4-Me |
| 285 | 5-Fluorpyridin-3-yl | 2,3-F₂ |
| 286 | 5-Fluorpyridin-3-yl | 2,4-F₂ |
| 287 | 5-Fluorpyridin-3-yl | 2,5-F₂ |
| 288 | 5-Fluorpyridin-3-yl | 2,6-F₂ |
| 289 | 5-Fluorpyridin-3-yl | 3,4-F₂ |
| 290 | 5-Fluorpyridin-3-yl | 3,5-F₂ |
| 291 | 5-Fluorpyridin-3-yl | 3,4,5-F₃ |
| 292 | 5-Fluorpyridin-3-yl | 3-F, 4-Cl |
| 293 | 5-Fluorpyridin-3-yl | 3-F, 4-Br |
| 294 | 5-Fluorpyridin-3-yl | 3-CN, 4-F |
| 295 | 5-Fluorpyridin-3-yl | 3-Br, 4-F |
| 296 | 5-Fluorpyridin-3-yl | 3-Cl, 4-F |
| 297 | 5-Fluorpyridin-3-yl | 3,4-Cb |
| 298 | 4-Fluorpyridin-3-yl | H |
| 299 | 4-Fluorpyridin-3-yl | 2-F |
| 300 | 4-Fluorpyridin-3-yl | 3-F |
| 301 | 4-Fluorpyridin-3-yl | 4-F |
| 302 | 4-Fluorpyridin-3-yl | 3-Cl |
| 303 | 4-Fluorpyridin-3-yl | 4-Cl |
| 304 | 4-Fluorpyridin-3-yl | 3-Br |
| 305 | 4-Fluorpyridin-3-yl | 4-Br |
| 306 | 4-Fluorpyridin-3-yl | 3-I |
| 307 | 4-Fluorpyridin-3-yl | 3-CN |
| 308 | 4-Fluorpyridin-3-yl | 4-CN |
| 309 | 4-Fluorpyridin-3-yl | 3-NO₂ |
| 310 | 4-Fluorpyridin-3-yl | 3-Me |
| 311 | 4-Fluorpyridin-3-yl | 4-Me |
| 312 | 4-Fluorpyridin-3-yl | 2,3-F₂ |
| 313 | 4-Fluorpyridin-3-yl | 2,4-F₂ |
| 314 | 4-Fluorpyridin-3-yl | 2,5-F₂ |
| 315 | 4-Fluorpyridin-3-yl | 2,6-F₂ |
| 316 | 4-Fluorpyridin-3-yl | 3,4-F₂ |
| 317 | 4-Fluorpyridin-3-yl | 3,5-F₂ |
| 318 | 4-Fluorpyridin-3-yl | 3,4,5-F₃ |
| 319 | 4-Fluorpyridin-3-yl | 3-F, 4-Cl |
| 320 | 4-Fluorpyridin-3-yl | 3-F, 4-Br |
| 321 | 4-Fluorpyridin-3-yl | 3-CN, 4-F |
| 322 | 4-Fluorpyridin-3-yl | 3-Br, 4-F |
| 323 | 4-Fluorpyridin-3-yl | 3-Cl, 4-F |
| 324 | 4-Fluorpyridin-3-yl | 3,4-Cb |
| 325 | 2-Chlorpyridin-3-yl | H |
| 326 | 2-Chlorpyridin-3-yl | 2-F |
| 327 | 2-Chlorpyridin-3-yl | 3-F |
| 328 | 2-Chlorpyridin-3-yl | 4-F |
| 329 | 2-Chlorpyridin-3-yl | 3-Cl |
| 330 | 2-Chlorpyridin-3-yl | 4-Cl |
| 331 | 2-Chlorpyridin-3-yl | 3-Br |
| 332 | 2-Chlorpyridin-3-yl | 4-Br |
| 333 | 2-Chlorpyridin-3-yl | 3-I |
| 334 | 2-Chlorpyridin-3-yl | 3-CN |
| 335 | 2-Chlorpyridin-3-yl | 4-CN |
| 336 | 2-Chlorpyridin-3-yl | 3-NO₂ |
| 337 | 2-Chlorpyridin-3-yl | 3-Me |
| 338 | 2-Chlorpyridin-3-yl | 4-Me |
| 339 | 2-Chlorpyridin-3-yl | 2,3-F₂ |
| 340 | 2-Chlorpyridin-3-yl | 2,4-F₂ |
| 341 | 2-Chlorpyridin-3-yl | 2,5-F₂ |
| 342 | 2-Chlorpyridin-3-yl | 2,6-F₂ |
| 343 | 2-Chlorpyridin-3-yl | 3,4-F₂ |
| 344 | 2-Chlorpyridin-3 -yl | 3,5-F₂ |
| 345 | 2-Chlorpyridin-3-yl | 3,4,5-F₃ |
| 346 | 2-Chlorpyridin-3-yl | 3-F, 4-Cl |
| 347 | 2-Chlorpyridin-3-yl | 3-F, 4-Br |
| 348 | 2-Chlorpyridin-3-yl | 3-CN, 4-F |
| 349 | 2-Chlorpyridin-3-yl | 3-Br, 4-F |
| 350 | 2-Chlorpyridin-3-yl | 3-Cl, 4-F |
| 351 | 2-Chlorpyridin-3-yl | 3,4-Cl₂ |
| 352 | 6-Chlorpyridin-3-yl | H |
| 353 | 6-Chlorpyridin-3-yl | 2-F |
| 354 | 6-Chlorpyridin-3-yl | 3-F |
| 355 | 6-Chlorpyridin-3-yl | 4-F |
| 356 | 6-Chlorpyridin-3-yl | 3-Cl |
| 357 | 6-Chlorpyridin-3-yl | 4-Cl |
| 358 | 6-Chlorpyridin-3-yl | 3-Br |
| 359 | 6-Chlorpyridin-3-yl | 4-Br |
| 360 | 6-Chlorpyridin-3-yl | 3-I |
| 361 | 6-Chlorpyridin-3-yl | 3-CN |
| 362 | 6-Chlorpyridin-3-yl | 4-CN |
| 363 | 6-Chlorpyridin-3-yl | 3-NO₂ |
| 364 | 6-Chlorpyridin-3-yl | 3-Me |
| 365 | 6-Chlorpyridin-3-yl | 4-Me |
| 366 | 6-Chlorpyridin-3-yl | 2,3-F₂ |
| 367 | 6-Chlorpyridin-3 -yl | 2,4-F₂ |
| 368 | 6-Chlorpyridin-3-yl | 2,5-F₂ |
| 369 | 6-Chlorpyridin-3-yl | 2,6-F₂ |
| 370 | 6-Chlorpyridin-3-yl | 3,4-F₂ |
| 371 | 6-Chlorpyridin-3-yl | 3,5-F₂ |
| 372 | 6-Chlorpyridin-3-yl | 3,4,5-F₃ |
| 373 | 6-Chlorpyridin-3-yl | 3-F, 4-Cl |
| 374 | 6-Chlorpyridin-3-yl | 3-F, 4-Br |
| 375 | 6-Chlorpyridin-3-yl | 3-CN, 4-F |
| 376 | 6-Chlorpyridin-3-yl | 3-Br, 4-F |
| 377 | 6-Chlorpyridin-3-yl | 3-Cl, 4-F |
| 378 | 6-Chlorpyridin-3 -yl | 3,4-Cl₂ |
| 379 | 5-Chlorpyridin-3-yl | H |
| 380 | 5-Chlorpyridin-3-yl | 2-F |
| 381 | 5-Chlorpyridin-3-yl | 3-F |
| 382 | 5-Chlorpyridin-3-yl | 4-F |
| 383 | 5-Chlorpyridin-3-yl | 3-Cl |
| 384 | 5-Chlorpyridin-3-yl | 4-Cl |
| 385 | 5-Chlorpyridin-3-yl | 3-Br |
| 386 | 5-Chlorpyridin-3-yl | 4-Br |
| 387 | 5-Chlorpyridin-3-yl | 3-I |
| 388 | 5-Chlorpyridin-3-yl | 3-CN |
| 389 | 5-Chlorpyridin-3-yl | 4-CN |
| 390 | 5-Chlorpyridin-3-yl | 3-NO₂ |
| 391 | 5-Chlorpyridin-3-yl | 3-Me |
| 392 | 5-Chlorpyridin-3-yl | 4-Me |
| 393 | 5-Chlorpyridin-3-yl | 2,3-F₂ |
| 394 | 5-Chlorpyridin-3-yl | 2,4-F₂ |
| 395 | 5-Chlorpyridin-3-yl | 2,5-F₂ |
| 396 | 5-Chlorpyridin-3-yl | 2,6-F₂ |
| 397 | 5-Chlorpyridin-3-yl | 3,4-F₂ |
| 398 | 5-Chlorpyridin-3-yl | 3,5-F₂ |
| 399 | 5-Chlorpyridin-3-yl | 3,4,5-F₃ |
| 400 | 5-Chlorpyridin-3-yl | 3-F, 4-Cl |
| 401 | 5-Chlorpyridin-3-yl | 3-F, 4-Br |
| 402 | 5-Chlorpyridin-3-yl | 3-CN, 4-F |
| 403 | 5-Chlorpyridin-3-yl | 3-Br, 4-F |
| 404 | 5-Chlorpyridin-3-yl | 3-Cl, 4-F |
| 405 | 5-Chlorpyridin-3-yl | 3,4-Cl₂ |
| 406 | 4-Chlorpyridin-3-yl | H |
| 407 | 4-Chlorpyridin-3-yl | 2-F |
| 408 | 4-Chlorpyridin-3-yl | 3-F |
| 409 | 4-Chlorpyridin-3-yl | 4-F |
| 410 | 4-Chlorpyridin-3-yl | 3-Cl |
| 411 | 4-Chlorpyridin-3-yl | 4-Cl |
| 412 | 4-Chlorpyridin-3-yl | 3-Br |
| 413 | 4-Chlorpyridin-3-yl | 4-Br |
| 414 | 4-Chlorpyridin-3-yl | 3-I |
| 415 | 4-Chlorpyridin-3-yl | 3-CN |
| 416 | 4-Chlorpyridin-3-yl | 4-CN |
| 417 | 4-Chlorpyridin-3-yl | 3-NO₂ |
| 418 | 4-Chlorpyridin-3-yl | 3-Me |
| 419 | 4-Chlorpyridin-3-yl | 4-Me |
| 420 | 4-Chlorpyridin-3-yl | 2,3-F₂ |
| 421 | 4-Chlorpyridin-3-yl | 2,4-F₂ |
| 422 | 4-Chlorpyridin-3-yl | 2,5-F₂ |
| 423 | 4-Chlorpyridin-3-yl | 2,6-F₂ |
| 424 | 4-Chlorpyridin-3-yl | 3,4-F₂ |
| 425 | 4-Chlorpyridin-3-yl | 3,5-F₂ |
| 426 | 4-Chlorpyridin-3-yl | 3,4,5-F₃ |
| 427 | 4-Chlorpyridin-3-yl | 3-F, 4-Cl |
| 428 | 4-Chlorpyridin-3-yl | 3-F, 4-Br |
| 429 | 4-Chlorpyridin-3-yl | 3-CN, 4-F |
| 430 | 4-Chlorpyridin-3-yl | 3-Br, 4-F |
| 431 | 4-Chlorpyridin-3-yl | 3-Cl, 4-F |
| 432 | 4-Chlorpyridin-3-yl | 3,4-Cl₂ |
| 433 | 3-Fluorpyridin-4-yl | H |
| 434 | 3-Fluorpyridin-4-yl | 2-F |
| 435 | 3-Fluorpyridin-4-yl | 3-F |
| 436 | 3-Fluorpyridin-4-yl | 4-F |
| 437 | 3-Fluorpyridin-4-yl | 3-Cl |
| 438 | 3-Fluorpyridin-4-yl | 4-Cl |
| 439 | 3-Fluorpyridin-4-yl | 3-Br |
| 440 | 3-Fluorpyridin-4-yl | 4-Br |
| 441 | 3-Fluorpyridin-4-yl | 3-I |
| 442 | 3-Fluorpyridin-4-yl | 3-CN |
| 443 | 3-Fluorpyridin-4-yl | 4-CN |
| 444 | 3-Fluorpyridin-4-yl | 3-NO₂ |
| 445 | 3-Fluorpyridin-4-yl | 3-Me |
| 446 | 3-Fluorpyridin-4-yl | 4-Me |
| 447 | 3-Fluorpyridin-4-yl | 2,3-F₂ |
| 448 | 3-Fluorpyridin-4-yl | 2,4-F₂ |
| 449 | 3-Fluorpyridin-4-yl | 2,5-F₂ |
| 450 | 3-Fluorpyridin-4-yl | 2,6-F₂ |
| 451 | 3-Fluorpyridin-4-yl | 3,4-F₂ |
| 452 | 3-Fluorpyridin-4-yl | 3,5-F₂ |
| 453 | 3-Fluorpyridin-4-yl | 3,4,5-F₃ |
| 454 | 3-Fluorpyridin-4-yl | 3-F, 4-Cl |
| 455 | 3-Fluorpyridin-4-yl | 3-F, 4-Br |
| 456 | 3-Fluorpyridin-4-yl | 3-CN, 4-F |
| 457 | 3-Fluorpyridin-4-yl | 3-Br, 4-F |
| 458 | 3-Fluorpyridin-4-yl | 3-Cl, 4-F |
| 459 | 3-Fluorpyridin-4-yl | 3,4-Cl₂ |
| 460 | 2-Fluorpyridin-4-yl | H |
| 461 | 2-Fluorpyridin-4-yl | 2-F |
| 462 | 2-Fluorpyridin-4-yl | 3-F |
| 463 | 2-Fluorpyridin-4-yl | 4-F |
| 464 | 2-Fluorpyridin-4-yl | 3-Cl |
| 465 | 2-Fluorpyridin-4-yl | 4-Cl |
| 466 | 2-Fluorpyridin-4-yl | 3-Br |
| 467 | 2-Fluorpyridin-4-yl | 4-Br |
| 468 | 2-Fluorpyridin-4-yl | 3-I |
| 469 | 2-Fluorpyridin-4-yl | 3-CN |
| 470 | 2-Fluorpyridin-4-yl | 4-CN |
| 471 | 2-Fluorpyridin-4-yl | 3-NO₂ |
| 472 | 2-Fluorpyridin-4-yl | 3-Me |
| 473 | 2-Fluorpyridin-4-yl | 4-Me |
| 474 | 2-Fluorpyridin-4-yl | 2,3-F₂ |
| 475 | 2-Fluorpyridin-4-yl | 2,4-F₂ |
| 476 | 2-Fluorpyridin-4-yl | 2,5-F₂ |
| 477 | 2-Fluorpyridin-4-yl | 2,6-F₂ |
| 478 | 2-Fluorpyridin-4-yl | 3,4-F₂ |
| 479 | 2-Fluorpyridin-4-yl | 3,5-F₂ |
| 480 | 2-Fluorpyridin-4-yl | 3,4,5-F₃ |
| 481 | 2-Fluorpyridin-4-yl | 3-F, 4-Cl |
| 482 | 2-Fluorpyridin-4-yl | 3-F, 4-Br |
| 483 | 2-Fluorpyridin-4-yl | 3-CN, 4-F |
| 484 | 2-Fluorpyridin-4-yl | 3-Br, 4-F |
| 485 | 2-Fluorpyridin-4-yl | 3-Cl, 4-F |
| 486 | 2-Fluorpyridin-4-yl | 3,4-Cl₂ |
| 487 | 3-Chlorpyridin-4-yl | H |
| 488 | 3-Chlorpyridin-4-yl | 2-F |
| 489 | 3-Chlorpyridin-4-yl | 3-F |
| 490 | 3-Chlorpyridin-4-yl | 4-F |
| 491 | 3-Chlorpyridin-4-yl | 3-Cl |
| 492 | 3-Chlorpyridin-4-yl | 4-Cl |
| 493 | 3-Chlorpyridin-4-yl | 3-Br |
| 494 | 3-Chlorpyridin-4-yl | 4-Br |
| 495 | 3-Chlorpyridin-4-yl | 3-I |
| 496 | 3-Chlorpyridin-4-yl | 3-CN |
| 497 | 3-Chlorpyridin-4-yl | 4-CN |
| 498 | 3-Chlorpyridin-4-yl | 3-NO₂ |
| 499 | 3-Chlorpyridin-4-yl | 3-Me |
| 500 | 3-Chlorpyridin-4-yl | 4-Me |
| 501 | 3-Chlorpyridin-4-yl | 2,3-F₂ |
| 502 | 3-Chlorpyridin-4-yl | 2,4-F₂ |
| 503 | 3-Chlorpyridin-4-yl | 2,5-F₂ |
| 504 | 3-Chlorpyridin-4-yl | 2,6-F₂ |
| 505 | 3-Chlorpyridin-4-yl | 3,4-F₂ |
| 506 | 3-Chlorpyridin-4-yl | 3,5-F₂ |
| 507 | 3-Chlorpyridin-4-yl | 3,4,5-F₃ |
| 508 | 3-Chlorpyridin-4-yl | 3-F, 4-Cl |
| 509 | 3-Chlorpyridin-4-yl | 3-F, 4-Br |
| 510 | 3-Chlorpyridin-4-yl | 3-CN, 4-F |
| 511 | 3-Chlorpyridin-4-yl | 3-Br, 4-F |
| 512 | 3-Chlorpyridin-4-yl | 3-Cl, 4-F |
| 513 | 3-Chlorpyridin-4-yl | 3,4-Cl₂ |
| 514 | 2-Chlorpyridin-4-yl | H |
| 515 | 2-Chlorpyridin-4-yl | 2-F |
| 516 | 2-Chlorpyridin-4-yl | 3-F |
| 517 | 2-Chlorpyridin-4-yl | 4-F |
| 518 | 2-Chlorpyridin-4-yl | 3-Cl |
| 519 | 2-Chlorpyridin-4-yl | 4-Cl |
| 520 | 2-Chlorpyridin-4-yl | 3-Br |
| 521 | 2-Chlorpyridin-4-yl | 4-Br |
| 522 | 2-Chlorpyridin-4-yl | 3-I |
| 523 | 2-Chlorpyridin-4-yl | 3-CN |
| 524 | 2-Chlorpyridin-4-yl | 4-CN |
| 525 | 2-Chlorpyridin-4-yl | 3-NO₂ |
| 526 | 2-Chlorpyridin-4-yl | 3-Me |
| 527 | 2-Chlorpyridin-4-yl | 4-Me |
| 528 | 2-Chlorpyridin-4-yl | 2,3-F₂ |
| 529 | 2-Chlorpyridin-4-yl | 2,4-F₂ |
| 530 | 2-Chlorpyridin-4-yl | 2,5-F₂ |
| 531 | 2-Chlorpyridin-4-yl | 2,6-F₂ |
| 532 | 2-Chlorpyridin-4-yl | 3,4-F₂ |
| 533 | 2-Chlorpyridin-4-yl | 3,5-F₂ |
| 534 | 2-Chlorpyridin-4-yl | 3,4,5-F₃ |
| 535 | 2-Chlorpyridin-4-yl | 3-F, 4-Cl |
| 536 | 2-Chlorpyridin-4-yl | 3-F, 4-Br |
| 537 | 2-Chlorpyridin-4-yl | 3-CN, 4-F |
| 538 | 2-Chlorpyridin-4-yl | 3-Br, 4-F |
| 539 | 2-Chlorpyridin-4-yl | 3-Cl, 4-F |
| 540 | 2-Chlorpyridin-4-yl | 3,4-Cl₂ |
| 541 | 2,3-Difluorpyridin-4-yl | H |
| 542 | 2,3-Difluorpyridin-4-yl | 2-F |
| 543 | 2,3-Difluorpyridin-4-yl | 3-F |
| 544 | 2,3-Difluorpyridin-4-yl | 4-F |
| 545 | 2,3-Difluorpyridin-4-yl | 3-Cl |
| 546 | 2,3-Difluorpyridin-4-yl | 4-Cl |
| 547 | 2,3-Difluorpyridin-4-yl | 3-Br |
| 548 | 2,3-Difluorpyridin-4-yl | 4-Br |
| 549 | 2,3-Difluorpyridin-4-yl | 3-I |
| 550 | 2,3-Difluorpyridin-4-yl | 3-CN |
| 551 | 2,3-Difluorpyridin-4-yl | 4-CN |
| 552 | 2,3-Difluorpyridin-4-yl | 3-NO₂ |
| 553 | 2,3-Difluorpyridin-4-yl | 3-Me |
| 554 | 2,3-Difluorpyridin-4-yl | 4-Me |
| 555 | 2,3-Difluorpyridin-4-yl | 2,3-F₂ |
| 556 | 2,3-Difluorpyridin-4-yl | 2,4-F₂ |
| 557 | 2,3-Difluorpyridin-4-yl | 2,5-F₂ |
| 558 | 2,3-Difluorpyridin-4-yl | 2,6-F₂ |
| 559 | 2,3-Difluorpyridin-4-yl | 3,4-F₂ |
| 560 | 2,3-Difluorpyridin-4-yl | 3,5-F₂ |
| 561 | 2,3-Difluorpyridin-4-yl | 3,4,5-F₃ |
| 562 | 2,3-Difluorpyridin-4-yl | 3-F, 4-Cl |
| 563 | 2,3-Difluorpyridin-4-yl | 3-F, 4-Br |
| 564 | 2,3-Difluorpyridin-4-yl | 3-CN, 4-F |
| 565 | 2,3-Difluorpyridin-4-yl | 3-Br, 4-F |
| 566 | 2,3-Difluorpyridin-4-yl | 3-Cl, 4-F |
| 567 | 2,3-Difluorpyridin-4-yl | 3,4-Cl₂ |
| 568 | 2,5-Difluorpyridin-4-yl | H |
| 569 | 2,5-Difluorpyridin-4-yl | 2-F |
| 570 | 2,5-Difluorpyridin-4-yl | 3-F |
| 571 | 2,5-Difluorpyridin-4-yl | 4-F |
| 572 | 2,5-Difluorpyridin-4-yl | 3-Cl |
| 573 | 2,5-Difluorpyridin-4-yl | 4-Cl |
| 574 | 2,5-Difluorpyridin-4-yl | 3-Br |
| 575 | 2,5-Difluorpyridin-4-yl | 4-Br |
| 576 | 2,5-Difluorpyridin-4-yl | 3-I |
| 577 | 2,5-Difluorpyridin-4-yl | 3-CN |
| 578 | 2,5-Difluorpyridin-4-yl | 4-CN |
| 579 | 2,5-Difluorpyridin-4-yl | 3-NO₂ |
| 580 | 2,5-Difluorpyridin-4-yl | 3-Me |
| 581 | 2,5-Difluorpyridin-4-yl | 4-Me |
| 582 | 2,5-Difluorpyridin-4-yl | 2,3-F₂ |
| 583 | 2,5-Difluorpyridin-4-yl | 2,4-F₂ |
| 584 | 2,5-Difluorpyridin-4-yl | 2,5-F₂ |
| 585 | 2,5-Difluorpyridin-4-yl | 2,6-F₂ |
| 586 | 2,5-Difluorpyridin-4-yl | 3,4-F₂ |
| 587 | 2,5-Difluorpyridin-4-yl | 3,5-F₂ |
| 588 | 2,5-Difluorpyridin-4-yl | 3,4,5-F₃ |
| 589 | 2,5-Difluorpyridin-4-yl | 3-F, 4-Cl |
| 590 | 2,5-Difluorpyridin-4-yl | 3-F, 4-Br |
| 591 | 2,5-Difluorpyridin-4-yl | 3-CN, 4-F |
| 592 | 2,5-Difluorpyridin-4-yl | 3-Br, 4-F |
| 593 | 2,5-Difluorpyridin-4-yl | 3-Cl, 4-F |
| 594 | 2,5-Difluorpyridin-4-yl | 3,4-Cl₂ |
| 595 | 3,5-Difluorpyridin-4-yl | H |
| 596 | 3,5-Difluorpyridin-4-yl | 2-F |
| 597 | 3,5-Difluorpyridin-4-yl | 3-F |
| 598 | 3,5-Difluorpyridin-4-yl | 4-F |
| 599 | 3,5-Difluorpyridin-4-yl | 3-Cl |
| 600 | 3,5-Difluorpyridin-4-yl | 4-Cl |
| 601 | 3,5-Difluorpyridin-4-yl | 3-Br |
| 602 | 3,5-Difluorpyridin-4-yl | 4-Br |
| 603 | 3,5-Difluorpyridin-4-yl | 3-I |
| 604 | 3,5-Difluorpyridin-4-yl | 3-CN |
| 605 | 3,5-Difluorpyridin-4-yl | 4-CN |
| 606 | 3,5-Difluorpyridin-4-yl | 3-NO₂ |
| 607 | 3,5-Difluorpyridin-4-yl | 3-Me |
| 608 | 3,5-Difluorpyridin-4-yl | 4-Me |
| 609 | 3,5-Difluorpyridin-4-yl | 2,3-F₂ |
| 610 | 3,5-Difluorpyridin-4-yl | 2,4-F₂ |
| 611 | 3,5-Difluorpyridin-4-yl | 2,5-F₂ |
| 612 | 3,5-Difluorpyridin-4-yl | 2,6-F₂ |
| 613 | 3,5-Difluorpyridin-4-yl | 3,4-F₂ |
| 614 | 3,5-Difluorpyridin-4-yl | 3,5-F₂ |
| 615 | 3,5-Difluorpyridin-4-yl | 3,4,5-F₃ |
| 616 | 3,5-Difluorpyridin-4-yl | 3-F, 4-Cl |
| 617 | 3,5-Difluorpyridin-4-yl | 3-F, 4-Br |
| 618 | 3,5-Difluorpyridin-4-yl | 3-CN, 4-F |
| 619 | 3,5-Difluorpyridin-4-yl | 3-Br, 4-F |
| 620 | 3,5-Difluorpyridin-4-yl | 3-Cl, 4-F |
| 621 | 3,5-Difluorpyridin-4-yl | 3,4-Cl₂ |
| 622 | 2,4-Difluorpyridin-3-yl | H |
| 623 | 2,4-Difluorpyridin-3-yl | 2-F |
| 624 | 2,4-Difluorpyridin-3-yl | 3-F |
| 625 | 2,4-Difluorpyridin-3-yl | 4-F |
| 626 | 2,4-Difluorpyridin-3-yl | 3-Cl |
| 627 | 2,4-Difluorpyridin-3-yl | 4-Cl |
| 628 | 2,4-Difluorpyridin-3-yl | 3-Br |
| 629 | 2,4-Difluorpyridin-3-yl | 4-Br |
| 630 | 2,4-Difluorpyridin-3-yl | 3-I |
| 631 | 2,4-Difluorpyridin-3-yl | 3-CN |
| 632 | 2,4-Difluorpyridin-3-yl | 4-CN |
| 633 | 2,4-Difluorpyridin-3-yl | 3-NO₂ |
| 634 | 2,4-Difluorpyridin-3-yl | 3-Me |
| 635 | 2,4-Difluorpyridin-3-yl | 4-Me |
| 636 | 2,4-Difluorpyridin-3-yl | 2,3-F₂ |
| 637 | 2,4-Difluorpyridin-3-yl | 2,4-F₂ |
| 638 | 2,4-Difluorpyridin-3-yl | 2,5-F₂ |
| 639 | 2,4-Difluorpyridin-3-yl | 2,6-F₂ |
| 640 | 2,4-Difluorpyridin-3-yl | 3,4-F₂ |
| 641 | 2,4-Difluorpyridin-3-yl | 3,5-F₂ |
| 642 | 2,4-Difluorpyridin-3-yl | 3,4,5-F₃ |
| 643 | 2,4-Difluorpyridin-3-yl | 3-F, 4-Cl |
| 644 | 2,4-Difluorpyridin-3-yl | 3-F, 4-Br |
| 645 | 2,4-Difluorpyridin-3-yl | 3-CN, 4-F |
| 646 | 2,4-Difluorpyridin-3-yl | 3-Br, 4-F |
| 647 | 2,4-Difluorpyridin-3-yl | 3-Cl, 4-F |
| 648 | 2,4-Difluorpyridin-3-yl | 3,4-Cl₂ |
| 649 | 2,5-Difluorpyridin-3-yl | H |
| 650 | 2,5-Difluorpyridin-3-yl | 2-F |
| 651 | 2,5-Difluorpyridin-3-yl | 3-F |
| 652 | 2,5-Difluorpyridin-3-yl | 4-F |
| 653 | 2,5-Difluorpyridin-3-yl | 3-Cl |
| 654 | 2,5-Difluorpyridin-3-yl | 4-Cl |
| 655 | 2,5-Difluorpyridin-3-yl | 3-Br |
| 656 | 2,5-Difluorpyridin-3-yl | 4-Br |
| 657 | 2,5-Difluorpyridin-3-yl | 3-I |
| 658 | 2,5-Difluorpyridin-3-yl | 3-CN |
| 659 | 2,5-Difluorpyridin-3-yl | 4-CN |
| 660 | 2,5-Difluorpyridin-3-yl | 3-NO₂ |
| 661 | 2,5-Difluorpyridin-3-yl | 3-Me |
| 662 | 2,5-Difluorpyridin-3-yl | 4-Me |
| 663 | 2,5-Difluorpyridin-3-yl | 2,3-F₂ |
| 664 | 2,5-Difluorpyridin-3-yl | 2,4-F₂ |
| 665 | 2,5-Difluorpyridin-3-yl | 2,5-F₂ |
| 666 | 2,5-Difluorpyridin-3-yl | 2,6-F₂ |
| 667 | 2,5-Difluorpyridin-3-yl | 3,4-F₂ |
| 668 | 2,5-Difluorpyridin-3-yl | 3,5-F₂ |
| 669 | 2,5-Difluorpyridin-3-yl | 3,4,5-F₃ |
| 670 | 2,5-Difluorpyridin-3-yl | 3-F, 4-Cl |
| 671 | 2,5-Difluorpyridin-3-yl | 3-F, 4-Br |
| 672 | 2,5-Difluorpyridin-3-yl | 3-CN, 4-F |
| 673 | 2,5-Difluorpyridin-3-yl | 3-Br, 4-F |
| 674 | 2,5-Difluorpyridin-3-yl | 3-Cl, 4-F |
| 675 | 2,5-Difluorpyridin-3-yl | 3,4-Cl₂ |
| 676 | 2,6-Difluorpyridin-3-yl | H |
| 677 | 2,6-Difluorpyridin-3-yl | 2-F |
| 678 | 2,6-Difluorpyridin-3-yl | 3-F |
| 679 | 2,6-Difluorpyridin-3-yl | 4-F |
| 680 | 2,6-Difluorpyridin-3-yl | 3-Cl |
| 681 | 2,6-Difluorpyridin-3-yl | 4-Cl |
| 682 | 2,6-Difluorpyridin-3-yl | 3-Br |
| 683 | 2,6-Difluorpyridin-3-yl | 4-Br |
| 684 | 2,6-Difluorpyridin-3-yl | 3-I |
| 685 | 2,6-Difluorpyridin-3-yl | 3-CN |
| 686 | 2,6-Difluorpyridin-3-yl | 4-CN |
| 687 | 2,6-Difluorpyridin-3-yl | 3-NO₂ |
| 688 | 2,6-Difluorpyridin-3-yl | 3-Me |
| 689 | 2,6-Difluorpyridin-3-yl | 4-Me |
| 690 | 2,6-Difluorpyridin-3-yl | 2,3-F₂ |
| 691 | 2,6-Difluorpyridin-3-yl | 2,4-F₂ |
| 692 | 2,6-Difluorpyridin-3-yl | 2,5-F₂ |
| 693 | 2,6-Difluorpyridin-3-yl | 2,6-F₂ |
| 694 | 2,6-Difluorpyridin-3-yl | 3,4-F₂ |
| 695 | 2,6-Difluorpyridin-3-yl | 3,5-F₂ |
| 696 | 2,6-Difluorpyridin-3-yl | 3,4,5-F₃ |
| 697 | 2,6-Difluorpyridin-3-yl | 3-F, 4-Cl |
| 698 | 2,6-Difluorpyridin-3-yl | 3-F, 4-Br |
| 699 | 2,6-Difluorpyridin-3-yl | 3-CN, 4-F |
| 700 | 2,6-Difluorpyridin-3-yl | 3-Br, 4-F |
| 701 | 2,6-Difluorpyridin-3-yl | 3-Cl, 4-F |
| 702 | 2,6-Difluorpyridin-3-yl | 3,4-Cl₂ |
| 703 | 5,6-Difluorpyridin-3-yl | H |
| 704 | 5,6-Difluorpyridin-3-yl | 2-F |
| 705 | 5,6-Difluorpyridin-3-yl | 3-F |
| 706 | 5,6-Difluorpyridin-3-yl | 4-F |
| 707 | 5,6-Difluorpyridin-3-yl | 3-Cl |
| 708 | 5,6-Difluorpyridin-3-yl | 4-Cl |
| 709 | 5,6-Difluorpyridin-3-yl | 3-Br |
| 710 | 5,6-Difluorpyridin-3-yl | 4-Br |
| 711 | 5,6-Difluorpyridin-3-yl | 3-I |
| 712 | 5,6-Difluorpyridin-3-yl | 3-CN |
| 713 | 5,6-Difluorpyridin-3-yl | 4-CN |
| 714 | 5,6-Difluorpyridin-3-yl | 3-NO₂ |
| 715 | 5,6-Difluorpyridin-3-yl | 3-Me |
| 716 | 5,6-Difluorpyridin-3-yl | 4-Me |
| 717 | 5,6-Difluorpyridin-3-yl | 2,3-F₂ |
| 718 | 5,6-Difluorpyridin-3-yl | 2,4-F₂ |
| 719 | 5,6-Difluorpyridin-3-yl | 2,5-F₂ |
| 720 | 5,6-Difluorpyridin-3-yl | 2,6-F₂ |
| 721 | 5,6-Difluorpyridin-3-yl | 3,4-F₂ |
| 722 | 5,6-Difluorpyridin-3-yl | 3,5-F₂ |
| 723 | 5,6-Difluorpyridin-3-yl | 3,4,5-F₃ |
| 724 | 5,6-Difluorpyridin-3-yl | 3-F, 4-Cl |
| 725 | 5,6-Difluorpyridin-3-yl | 3-F, 4-Br |
| 726 | 5,6-Difluorpyridin-3-yl | 3-CN, 4-F |
| 727 | 5,6-Difluorpyridin-3-yl | 3-Br, 4-F |
| 728 | 5,6-Difluorpyridin-3-yl | 3-Cl, 4-F |
| 729 | 5,6-Difluorpyridin-3-yl | 3,4-Cl₂ |
| 730 | 4,5-Difluorpyridin-3-yl | H |
| 731 | 4,5-Difluorpyridin-3-yl | 2-F |
| 732 | 4,5-Difluorpyridin-3-yl | 3-F |
| 733 | 4,5-Difluorpyridin-3-yl | 4-F |
| 734 | 4,5-Difluorpyridin-3-yl | 3-Cl |
| 735 | 4,5-Difluorpyridin-3-yl | 4-Cl |
| 736 | 4,5-Difluorpyridin-3-yl | 3-Br |
| 737 | 4,5-Difluorpyridin-3-yl | 4-Br |
| 738 | 4,5-Difluorpyridin-3-yl | 3-I |
| 739 | 4,5-Difluorpyridin-3-yl | 3-CN |
| 740 | 4,5-Difluorpyridin-3-yl | 4-CN |
| 741 | 4,5-Difluorpyridin-3-yl | 3-NO₂ |
| 742 | 4,5-Difluorpyridin-3-yl | 3-Me |
| 743 | 4,5-Difluorpyridin-3-yl | 4-Me |
| 744 | 4,5-Difluorpyridin-3-yl | 2,3-F₂ |
| 745 | 4,5-Difluorpyridin-3-yl | 2,4-F₂ |
| 746 | 4,5-Difluorpyridin-3-yl | 2,5-F₂ |
| 747 | 4,5-Difluorpyridin-3-yl | 2,6-F₂ |
| 748 | 4,5-Difluorpyridin-3-yl | 3,4-F₂ |
| 749 | 4,5-Difluorpyridin-3-yl | 3,5-F₂ |
| 750 | 4,5-Difluorpyridin-3-yl | 3,4,5-F₃ |
| 751 | 4,5-Difluorpyridin-3-yl | 3-F, 4-Cl |
| 752 | 4,5-Difluorpyridin-3-yl | 3-F, 4-Br |
| 753 | 4,5-Difluorpyridin-3-yl | 3-CN, 4-F |
| 754 | 4,5-Difluorpyridin-3-yl | 3-Br, 4-F |
| 755 | 4,5-Difluorpyridin-3-yl | 3-Cl, 4-F |
| 756 | 4,5-Difluorpyridin-3-yl | 3,4-Cl₂ |
| 757 | 4,6-Difluorpyridin-3-yl | H |
| 758 | 4,6-Difluorpyridin-3-yl | 2-F |
| 759 | 4,6-Difluorpyridin-3-yl | 3-F |
| 760 | 4,6-Difluorpyridin-3-yl | 4-F |
| 761 | 4,6-Difluorpyridin-3-yl | 3-Cl |
| 762 | 4,6-Difluorpyridin-3-yl | 4-Cl |
| 763 | 4,6-Difluorpyridin-3-yl | 3-Br |
| 764 | 4,6-Difluorpyridin-3-yl | 4-Br |
| 765 | 4,6-Difluorpyridin-3-yl | 3-I |
| 766 | 4,6-Difluorpyridin-3-yl | 3-CN |
| 767 | 4,6-Difluorpyridin-3-yl | 4-CN |
| 768 | 4,6-Difluorpyridin-3-yl | 3-NO₂ |
| 769 | 4,6-Difluorpyridin-3-yl | 3-Me |
| 770 | 4,6-Difluorpyridin-3-yl | 4-Me |
| 771 | 4,6-Difluorpyridin-3-yl | 2,3-F₂ |
| 772 | 4,6-Difluorpyridin-3-yl | 2,4-F₂ |
| 773 | 4,6-Difluorpyridin-3-yl | 2,5-F₂ |
| 774 | 4,6-Difluorpyridin-3-yl | 2,6-F₂ |
| 775 | 4,6-Difluorpyridin-3-yl | 3,4-F₂ |
| 776 | 4,6-Difluorpyridin-3-yl | 3,5-F₂ |
| 777 | 4,6-Difluorpyridin-3-yl | 3,4,5-F₃ |
| 778 | 4,6-Difluorpyridin-3-yl | 3-F, 4-Cl |
| 779 | 4,6-Difluorpyridin-3-yl | 3-F, 4-Br |
| 780 | 4,6-Difluorpyridin-3-yl | 3-CN, 4-F |
| 781 | 4,6-Difluorpyridin-3-yl | 3-Br, 4-F |
| 782 | 4,6-Difluorpyridin-3-yl | 3-Cl, 4-F |
| 783 | 4,6-Difluorpyridin-3-yl | 3,4-Cl₂ |
| 784 | 3,5-Difluorpyridin-2-yl | H |
| 785 | 3,5-Difluorpyridin-2-yl | 2-F |
| 786 | 3,5-Difluorpyridin-2-yl | 3-F |
| 787 | 3,5-Difluorpyridin-2-yl | 4-F |
| 788 | 3,5-Difluorpyridin-2-yl | 3-Cl |
| 789 | 3,5-Difluorpyridin-2-yl | 4-Cl |
| 790 | 3,5-Difluorpyridin-2-yl | 3-Br |
| 791 | 3,5-Difluorpyridin-2-yl | 4-Br |
| 792 | 3,5-Difluorpyridin-2-yl | 3-I |
| 793 | 3,5-Difluorpyridin-2-yl | 3-CN |
| 794 | 3,5-Difluorpyridin-2-yl | 4-CN |
| 795 | 3,5-Difluorpyridin-2-yl | 3-NO₂ |
| 796 | 3,5-Difluorpyridin-2-yl | 3-Me |
| 797 | 3,5-Difluorpyridin-2-yl | 4-Me |
| 798 | 3,5-Difluorpyridin-2-yl | 2,3-F₂ |
| 799 | 3,5-Difluorpyridin-2-yl | 2,4-F₂ |
| 800 | 3,5-Difluorpyridin-2-yl | 2,5-F₂ |
| 801 | 3,5-Difluorpyridin-2-yl | 2,6-F₂ |
| 802 | 3,5-Difluorpyridin-2-yl | 3,4-F₂ |
| 803 | 3,5-Difluorpyridin-2-yl | 3,5-F₂ |
| 804 | 3,5-Difluorpyridin-2-yl | 3,4,5-F₃ |
| 805 | 3,5-Difluorpyridin-2-yl | 3-F, 4-Cl |
| 806 | 3,5-Difluorpyridin-2-yl | 3-F, 4-Br |
| 807 | 3,5-Difluorpyridin-2-yl | 3-CN, 4-F |
| 808 | 3,5-Difluorpyridin-2-yl | 3-Br, 4-F |
| 809 | 3,5-Difluorpyridin-2-yl | 3-Cl, 4-F |
| 810 | 3,5-Difluorpyridin-2-yl | 3,4-Cl₂ |
| 811 | 3,6-Difluorpyridin-2-yl | H |
| 812 | 3,6-Difluorpyridin-2-yl | 2-F |
| 813 | 3,6-Difluorpyridin-2-yl | 3-F |
| 814 | 3,6-Difluorpyridin-2-yl | 4-F |
| 815 | 3,6-Difluorpyridin-2-yl | 3-Cl |
| 816 | 3,6-Difluorpyridin-2-yl | 4-Cl |
| 817 | 3,6-Difluorpyridin-2-yl | 3-Br |
| 818 | 3,6-Difluorpyridin-2-yl | 4-Br |
| 819 | 3,6-Difluorpyridin-2-yl | 3-I |
| 820 | 3,6-Difluorpyridin-2-yl | 3-CN |
| 821 | 3,6-Difluorpyridin-2-yl | 4-CN |
| 822 | 3,6-Difluorpyridin-2-yl | 3-NO₂ |
| 823 | 3,6-Difluorpyridin-2-yl | 3-Me |
| 824 | 3,6-Difluorpyridin-2-yl | 4-Me |
| 825 | 3,6-Difluorpyridin-2-yl | 2,3-F₂ |
| 826 | 3,6-Difluorpyridin-2-yl | 2,4-F₂ |
| 827 | 3,6-Difluorpyridin-2-yl | 2,5-F₂ |
| 828 | 3,6-Difluorpyridin-2-yl | 2,6-F₂ |
| 829 | 3,6-Difluorpyridin-2-yl | 3,4-F₂ |
| 830 | 3,6-Difluorpyridin-2-yl | 3,5-F₂ |
| 831 | 3,6-Difluorpyridin-2-yl | 3,4,5-F₃ |
| 832 | 3,6-Difluorpyridin-2-yl | 3-F, 4-Cl |
| 833 | 3,6-Difluorpyridin-2-yl | 3-F, 4-Br |
| 834 | 3,6-Difluorpyridin-2-yl | 3-CN, 4-F |
| 835 | 3,6-Difluorpyridin-2-yl | 3-Br, 4-F |
| 836 | 3,6-Difluorpyridin-2-yl | 3-Cl, 4-F |
| 837 | 3,6-Difluorpyridin-2-yl | 3,4-Cl₂ |
| 838 | 4,6-Difluorpyridin-2-yl | H |
| 839 | 4,6-Difluorpyridin-2-yl | 2-F |
| 840 | 4,6-Difluorpyridin-2-yl | 3-F |
| 841 | 4,6-Difluorpyridin-2-yl | 4-F |
| 842 | 4,6-Difluorpyridin-2-yl | 3-Cl |
| 843 | 4,6-Difluorpyridin-2-yl | 4-Cl |
| 844 | 4,6-Difluorpyridin-2-yl | 3-Br |
| 845 | 4,6-Difluorpyridin-2-yl | 4-Br |
| 846 | 4,6-Difluorpyridin-2-yl | 3-I |
| 847 | 4,6-Difluorpyridin-2-yl | 3-CN |
| 848 | 4,6-Difluorpyridin-2-yl | 4-CN |
| 849 | 4,6-Difluorpyridin-2-yl | 3-NO₂ |
| 850 | 4,6-Difluorpyridin-2-yl | 3-Me |
| 851 | 4,6-Difluorpyridin-2-yl | 4-Me |
| 852 | 4,6-Difluorpyridin-2-yl | 2,3-F₂ |
| 853 | 4,6-Difluorpyridin-2-yl | 2,4-F₂ |
| 854 | 4,6-Difluorpyridin-2-yl | 2,5-F₂ |
| 855 | 4,6-Difluorpyridin-2-yl | 2,6-F₂ |
| 856 | 4,6-Difluorpyridin-2-yl | 3,4-F₂ |
| 857 | 4,6-Difluorpyridin-2-yl | 3,5-F₂ |
| 858 | 4,6-Difluorpyridin-2-yl | 3,4,5-F₃ |
| 859 | 4,6-Difluorpyridin-2-yl | 3-F, 4-Cl |
| 860 | 4,6-Difluorpyridin-2-yl | 3-F, 4-Br |
| 861 | 4,6-Difluorpyridin-2-yl | 3-CN, 4-F |
| 862 | 4,6-Difluorpyridin-2-yl | 3-Br, 4-F |
| 863 | 4,6-Difluorpyridin-2-yl | 3-Cl, 4-F |
| 864 | 4,6-Difluorpyridin-2-yl | 3,4-Cl₂ |
| 865 | 5,6-Difluorpyridin-2-yl | H |
| 866 | 5,6-Difluorpyridin-2-yl | 2-F |
| 867 | 5,6-Difluorpyridin-2-yl | 3-F |
| 868 | 5,6-Difluorpyridin-2-yl | 4-F |
| 869 | 5,6-Difluorpyridin-2-yl | 3-Cl |
| 870 | 5,6-Difluorpyridin-2-yl | 4-Cl |
| 871 | 5,6-Difluorpyridin-2-yl | 3-Br |
| 872 | 5,6-Difluorpyridin-2-yl | 4-Br |
| 873 | 5,6-Difluorpyridin-2-yl | 3-I |
| 874 | 5,6-Difluorpyridin-2-yl | 3-CN |
| 875 | 5,6-Difluorpyridin-2-yl | 4-CN |
| 876 | 5,6-Difluorpyridin-2-yl | 3-NO₂ |
| 877 | 5,6-Difluorpyridin-2-yl | 3-Me |
| 878 | 5,6-Difluorpyridin-2-yl | 4-Me |
| 879 | 5,6-Difluorpyridin-2-yl | 2,3-F₂ |
| 880 | 5,6-Difluorpyridin-2-yl | 2,4-F₂ |
| 881 | 5,6-Difluorpyridin-2-yl | 2,5-F₂ |
| 882 | 5,6-Difluorpyridin-2-yl | 2,6-F₂ |
| 883 | 5,6-Difluorpyridin-2-yl | 3,4-F₂ |
| 884 | 5,6-Difluorpyridin-2-yl | 3,5-F₂ |
| 885 | 5,6-Difluorpyridin-2-yl | 3,4,5-F₃ |
| 886 | 5,6-Difluorpyridin-2-yl | 3-F, 4-Cl |
| 887 | 5,6-Difluorpyridin-2-yl | 3-F, 4-Br |
| 888 | 5,6-Difluorpyridin-2-yl | 3-CN, 4-F |
| 889 | 5,6-Difluorpyridin-2-yl | 3-Br, 4-F |
| 890 | 5,6-Difluorpyridin-2-yl | 3-Cl, 4-F |
| 891 | 5,6-Difluorpyridin-2-yl | 3,4-Cl₂ |
| 892 | 4,5-Difluorpyridin-2-yl | H |
| 893 | 4,5-Difluorpyridin-2-yl | 2-F |
| 894 | 4,5-Difluorpyridin-2-yl | 3-F |
| 895 | 4,5-Difluorpyridin-2-yl | 4-F |
| 896 | 4,5-Difluorpyridin-2-yl | 3-Cl |
| 897 | 4,5-Difluorpyridin-2-yl | 4-Cl |
| 898 | 4,5-Difluorpyridin-2-yl | 3-Br |
| 899 | 4,5-Difluorpyridin-2-yl | 4-Br |
| 900 | 4,5-Difluorpyridin-2-yl | 3-I |
| 901 | 4,5-Difluorpyridin-2-yl | 3-CN |
| 902 | 4,5-Difluorpyridin-2-yl | 4-CN |
| 903 | 4,5-Difluorpyridin-2-yl | 3-NO₂ |
| 904 | 4,5-Difluorpyridin-2-yl | 3-Me |
| 905 | 4,5-Difluorpyridin-2-yl | 4-Me |
| 906 | 4,5-Difluorpyridin-2-yl | 2,3-F₂ |
| 907 | 4,5-Difluorpyridin-2-yl | 2,4-F₂ |
| 908 | 4,5-Difluorpyridin-2-yl | 2,5-F₂ |
| 909 | 4,5-Difluorpyridin-2-yl | 2,6-F₂ |
| 910 | 4,5-Difluorpyridin-2-yl | 3,4-F₂ |
| 911 | 4,5-Difluorpyridin-2-yl | 3,5-F₂ |
| 912 | 4,5-Difluorpyridin-2-yl | 3,4,5-F₃ |
| 913 | 4,5-Difluorpyridin-2-yl | 3-F, 4-Cl |
| 914 | 4,5-Difluorpyridin-2-yl | 3-F, 4-Br |
| 915 | 4,5-Difluorpyridin-2-yl | 3-CN, 4-F |
| 916 | 4,5-Difluorpyridin-2-yl | 3-Br, 4-F |
| 917 | 4,5-Difluorpyridin-2-yl | 3-Cl, 4-F |
| 918 | 4,5-Difluorpyridin-2-yl | 3,4-Cl₂ |
| 919 | 6-Methoxypyridin-2-yl | H |
| 920 | 6-Methoxypyridin-2-yl | 2-F |
| 921 | 6-Methoxypyridin-2-yl | 3-F |
| 922 | 6-Methoxypyridin-2-yl | 4-F |
| 923 | 6-Methoxypyridin-2-yl | 3-Cl |
| 924 | 6-Methoxypyridin-2-yl | 4-Cl |
| 925 | 6-Methoxypyridin-2-yl | 3-Br |
| 926 | 6-Methoxypyridin-2-yl | 4-Br |
| 927 | 6-Methoxypyridin-2-yl | 3-I |
| 928 | 6-Methoxypyridin-2-yl | 3-CN |
| 929 | 6-Methoxypyridin-2-yl | 4-CN |
| 930 | 6-Methoxypyridin-2-yl | 3-NO₂ |
| 931 | 6-Methoxypyridin-2-yl | 3-Me |
| 932 | 6-Methoxypyridin-2-yl | 4-Me |
| 933 | 6-Methoxypyridin-2-yl | 2,3-F₂ |
| 934 | 6-Methoxypyridin-2-yl | 2,4-F₂ |
| 935 | 6-Methoxypyridin-2-yl | 2,5-F₂ |
| 936 | 6-Methoxypyridin-2-yl | 2,6-F₂ |
| 937 | 6-Methoxypyridin-2-yl | 3,4-F₂ |
| 938 | 6-Methoxypyridin-2-yl | 3,5-F₂ |
| 939 | 6-Methoxypyridin-2-yl | 3,4,5-F₃ |
| 940 | 6-Methoxypyridin-2-yl | 3-F, 4-Cl |
| 941 | 6-Methoxypyridin-2-yl | 3-F, 4-Br |
| 942 | 6-Methoxypyridin-2-yl | 3-CN, 4-F |
| 943 | 6-Methoxypyridin-2-yl | 3-Br, 4-F |
| 944 | 6-Methoxypyridin-2-yl | 3-Cl, 4-F |
| 945 | 6-Methoxypyridin-2-yl | 3,4-Cl₂ |
| 946 | 5-Methoxypyridin-2-yl | H |
| 947 | 5-Methoxypyridin-2-yl | 2-F |
| 948 | 5-Methoxypyridin-2-yl | 3-F |
| 949 | 5-Methoxypyridin-2-yl | 4-F |
| 950 | 5-Methoxypyridin-2-yl | 3-Cl |
| 951 | 5-Methoxypyridin-2-yl | 4-Cl |
| 952 | 5-Methoxypyridin-2-yl | 3-Br |
| 953 | 5-Methoxypyridin-2-yl | 4-Br |
| 954 | 5-Methoxypyridin-2-yl | 3-I |
| 955 | 5-Methoxypyridin-2-yl | 3-CN |
| 956 | 5-Methoxypyridin-2-yl | 4-CN |
| 957 | 5-Methoxypyridin-2-yl | 3-NO₂ |
| 958 | 5-Methoxypyridin-2-yl | 3-Me |
| 959 | 5-Methoxypyridin-2-yl | 4-Me |
| 960 | 5-Methoxypyridin-2-yl | 2,3-F₂ |
| 961 | 5-Methoxypyridin-2-yl | 2,4-F₂ |
| 962 | 5-Methoxypyridin-2-yl | 2,5-F₂ |
| 963 | 5-Methoxypyridin-2-yl | 2,6-F₂ |
| 964 | 5-Methoxypyridin-2-yl | 3,4-F₂ |
| 965 | 5-Methoxypyridin-2-yl | 3,5-F₂ |
| 966 | 5-Methoxypyridin-2-yl | 3,4,5-F₃ |
| 967 | 5-Methoxypyridin-2-yl | 3-F, 4-Cl |
| 968 | 5-Methoxypyridin-2-yl | 3-F, 4-Br |
| 969 | 5-Methoxypyridin-2-yl | 3-CN, 4-F |
| 970 | 5-Methoxypyridin-2-yl | 3-Br, 4-F |
| 971 | 5-Methoxypyridin-2-yl | 3-Cl, 4-F |
| 972 | 5-Methoxypyridin-2-yl | 3,4-Cl₂ |
| 973 | 4-Methoxypyridin-2-yl | H |
| 974 | 4-Methoxypyridin-2-yl | 2-F |
| 975 | 4-Methoxypyridin-2-yl | 3-F |
| 976 | 4-Methoxypyridin-2-yl | 4-F |
| 977 | 4-Methoxypyridin-2-yl | 3-Cl |
| 978 | 4-Methoxypyridin-2-yl | 4-Cl |
| 979 | 4-Methoxypyridin-2-yl | 3-Br |
| 980 | 4-Methoxypyridin-2-yl | 4-Br |
| 981 | 4-Methoxypyridin-2-yl | 3-I |
| 982 | 4-Methoxypyridin-2-yl | 3-CN |
| 983 | 4-Methoxypyridin-2-yl | 4-CN |
| 984 | 4-Methoxypyridin-2-yl | 3-NO₂ |
| 985 | 4-Methoxypyridin-2-yl | 3-Me |
| 986 | 4-Methoxypyridin-2-yl | 4-Me |
| 987 | 4-Methoxypyridin-2-yl | 2,3-F₂ |
| 988 | 4-Methoxypyridin-2-yl | 2,4-F₂ |
| 989 | 4-Methoxypyridin-2-yl | 2,5-F₂ |
| 990 | 4-Methoxypyridin-2-yl | 2,6-F₂ |
| 991 | 4-Methoxypyridin-2-yl | 3,4-F₂ |
| 992 | 4-Methoxypyridin-2-yl | 3,5-F₂ |
| 993 | 4-Methoxypyridin-2-yl | 3,4,5-F₃ |
| 994 | 4-Methoxypyridin-2-yl | 3-F, 4-Cl |
| 995 | 4-Methoxypyridin-2-yl | 3-F, 4-Br |
| 996 | 4-Methoxypyridin-2-yl | 3-CN, 4-F |
| 997 | 4-Methoxypyridin-2-yl | 3-Br, 4-F |
| 998 | 4-Methoxypyridin-2-yl | 3-Cl, 4-F |
| 999 | 4-Methoxypyridin-2-yl | 3,4-Cl₂ |
| 1000 | 3-Methoxypyridin-2-yl | H |
| 1001 | 3-Methoxypyridin-2-yl | 2-F |
| 1002 | 3-Methoxypyridin-2-yl | 3-F |
| 1003 | 3-Methoxypyridin-2-yl | 4-F |
| 1004 | 3-Methoxypyridin-2-yl | 3-Cl |
| 1005 | 3-Methoxypyridin-2-yl | 4-Cl |
| 1006 | 3-Methoxypyridin-2-yl | 3-Br |
| 1007 | 3-Methoxypyridin-2-yl | 4-Br |
| 1008 | 3-Methoxypyridin-2-yl | 3-I |
| 1009 | 3-Methoxypyridin-2-yl | 3-CN |
| 1010 | 3-Methoxypyridin-2-yl | 4-CN |
| 1011 | 3-Methoxypyridin-2-yl | 3-NO₂ |
| 1012 | 3-Methoxypyridin-2-yl | 3-Me |
| 1013 | 3-Methoxypyridin-2-yl | 4-Me |
| 1014 | 3-Methoxypyridin-2-yl | 2,3-F₂ |
| 1015 | 3-Methoxypyridin-2-yl | 2,4-F₂ |
| 1016 | 3-Methoxypyridin-2-yl | 2,5-F₂ |
| 1017 | 3-Methoxypyridin-2-yl | 2,6-F₂ |
| 1018 | 3-Methoxypyridin-2-yl | 3,4-F₂ |
| 1019 | 3-Methoxypyridin-2-yl | 3,5-F₂ |
| 1020 | 3-Methoxypyridin-2-yl | 3,4,5-F₃ |
| 1021 | 3-Methoxypyridin-2-yl | 3-F, 4-Cl |
| 1022 | 3-Methoxypyridin-2-yl | 3-F, 4-Br |
| 1023 | 3-Methoxypyridin-2-yl | 3-CN, 4-F |
| 1024 | 3-Methoxypyridin-2-yl | 3-Br, 4-F |
| 1025 | 3-Methoxypyridin-2-yl | 3-Cl, 4-F |
| 1026 | 3-Methoxypyridin-2-yl | 3,4-Cl₂ |
| 1027 | 4-Methoxypyridin-3-yl | H |
| 1028 | 4-Methoxypyridin-3-yl | 2-F |
| 1029 | 4-Methoxypyridin-3-yl | 3-F |
| 1030 | 4-Methoxypyridin-3-yl | 4-F |
| 1031 | 4-Methoxypyridin-3-yl | 3-Cl |
| 1032 | 4-Methoxypyridin-3-yl | 4-Cl |
| 1033 | 4-Methoxypyridin-3-yl | 3-Br |
| 1034 | 4-Methoxypyridin-3-yl | 4-Br |
| 1035 | 4-Methoxypyridin-3-yl | 3-I |
| 1036 | 4-Methoxypyridin-3-yl | 3-CN |
| 1037 | 4-Methoxypyridin-3-yl | 4-CN |
| 1038 | 4-Methoxypyridin-3-yl | 3-NO₂ |
| 1039 | 4-Methoxypyridin-3-yl | 3-Me |
| 1040 | 4-Methoxypyridin-3-yl | 4-Me |
| 1041 | 4-Methoxypyridin-3-yl | 2,3-F₂ |
| 1042 | 4-Methoxypyridin-3-yl | 2,4-F₂ |
| 1043 | 4-Methoxypyridin-3-yl | 2,5-F₂ |
| 1044 | 4-Methoxypyridin-3-yl | 2,6-F₂ |
| 1045 | 4-Methoxypyridin-3-yl | 3,4-F₂ |
| 1046 | 4-Methoxypyridin-3-yl | 3,5-F₂ |
| 1047 | 4-Methoxypyridin-3-yl | 3,4,5-F₃ |
| 1048 | 4-Methoxypyridin-3-yl | 3-F, 4-Cl |
| 1049 | 4-Methoxypyridin-3-yl | 3-F, 4-Br |
| 1050 | 4-Methoxypyridin-3-yl | 3-CN, 4-F |
| 1051 | 4-Methoxypyridin-3-yl | 3-Br, 4-F |
| 1052 | 4-Methoxypyridin-3-yl | 3-Cl, 4-F |
| 1053 | 4-Methoxypyridin-3-yl | 3,4-Cl₂ |
| 1054 | 5-Methoxypyridin-3-yl | H |
| 1055 | 5-Methoxypyridin-3-yl | 2-F |
| 1056 | 5-Methoxypyridin-3-yl | 3-F |
| 1057 | 5-Methoxypyridin-3-yl | 4-F |
| 1058 | 5-Methoxypyridin-3-yl | 3-Cl |
| 1059 | 5-Methoxypyridin-3-yl | 4-Cl |
| 1060 | 5-Methoxypyridin-3-yl | 3-Br |
| 1061 | 5-Methoxypyridin-3-yl | 4-Br |
| 1062 | 5-Methoxypyridin-3-yl | 3-I |
| 1063 | 5-Methoxypyridin-3-yl | 3-CN |
| 1064 | 5-Methoxypyridin-3-yl | 4-CN |
| 1065 | 5-Methoxypyridin-3-yl | 3-NO₂ |
| 1066 | 5-Methoxypyridin-3-yl | 3-Me |
| 1067 | 5-Methoxypyridin-3-yl | 4-Me |
| 1068 | 5-Methoxypyridin-3-yl | 2,3-F₂ |
| 1069 | 5-Methoxypyridin-3-yl | 2,4-F₂ |
| 1070 | 5-Methoxypyridin-3-yl | 2,5-F₂ |
| 1071 | 5-Methoxypyridin-3-yl | 2,6-F₂ |
| 1072 | 5-Methoxypyridin-3-yl | 3,4-F₂ |
| 1073 | 5-Methoxypyridin-3-yl | 3,5-F₂ |
| 1074 | 5-Methoxypyridin-3-yl | 3,4,5-F₃ |
| 1075 | 5-Methoxypyridin-3-yl | 3-F, 4-Cl |
| 1076 | 5-Methoxypyridin-3-yl | 3-F, 4-Br |
| 1077 | 5-Methoxypyridin-3-yl | 3-CN, 4-F |
| 1078 | 5-Methoxypyridin-3-yl | 3-Br, 4-F |
| 1079 | 5-Methoxypyridin-3-yl | 3-Cl, 4-F |
| 1080 | 5-Methoxypyridin-3-yl | 3,4-Cl₂ |
| 1081 | 6-Methoxypyridin-3-yl | H |
| 1082 | 6-Methoxypyridin-3-yl | 2-F |
| 1083 | 6-Methoxypyridin-3-yl | 3-F |
| 1084 | 6-Methoxypyridin-3-yl | 4-F |
| 1085 | 6-Methoxypyridin-3-yl | 3-Cl |
| 1086 | 6-Methoxypyridin-3-yl | 4-Cl |
| 1087 | 6-Methoxypyridin-3-yl | 3-Br |
| 1088 | 6-Methoxypyridin-3-yl | 4-Br |
| 1089 | 6-Methoxypyridin-3-yl | 3-I |
| 1090 | 6-Methoxypyridin-3-yl | 3-CN |
| 1091 | 6-Methoxypyridin-3-yl | 4-CN |
| 1092 | 6-Methoxypyridin-3-yl | 3-NO₂ |
| 1093 | 6-Methoxypyridin-3-yl | 3-Me |
| 1094 | 6-Methoxypyridin-3-yl | 4-Me |
| 1095 | 6-Methoxypyridin-3-yl | 2,3-F₂ |
| 1096 | 6-Methoxypyridin-3-yl | 2,4-F₂ |
| 1097 | 6-Methoxypyridin-3-yl | 2,5-F₂ |
| 1098 | 6-Methoxypyridin-3-yl | 2,6-F₂ |
| 1099 | 6-Methoxypyridin-3-yl | 3,4-F₂ |
| 1100 | 6-Methoxypyridin-3-yl | 3,5-F₂ |
| 1101 | 6-Methoxypyridin-3-yl | 3,4,5-F₃ |
| 1102 | 6-Methoxypyridin-3-yl | 3-F, 4-Cl |
| 1103 | 6-Methoxypyridin-3-yl | 3-F, 4-Br |
| 1104 | 6-Methoxypyridin-3-yl | 3-CN, 4-F |
| 1105 | 6-Methoxypyridin-3-yl | 3-Br, 4-F |
| 1106 | 6-Methoxypyridin-3-yl | 3-Cl, 4-F |
| 1107 | 6-Methoxypyridin-3-yl | 3,4-Cl₂ |
| 1108 | 2-Methoxypyridin-3-yl | H |
| 1109 | 2-Methoxypyridin-3-yl | 2-F |
| 1110 | 2-Methoxypyridin-3-yl | 3-F |
| 1111 | 2-Methoxypyridin-3-yl | 4-F |
| 1112 | 2-Methoxypyridin-3-yl | 3-Cl |
| 1113 | 2-Methoxypyridin-3-yl | 4-Cl |
| 1114 | 2-Methoxypyridin-3-yl | 3-Br |
| 1115 | 2-Methoxypyridin-3-yl | 4-Br |
| 1116 | 2-Methoxypyridin-3-yl | 3-I |
| 1117 | 2-Methoxypyridin-3-yl | 3-CN |
| 1118 | 2-Methoxypyridin-3-yl | 4-CN |
| 1119 | 2-Methoxypyridin-3-yl | 3-NO₂ |
| 1120 | 2-Methoxypyridin-3-yl | 3-Me |
| 1121 | 2-Methoxypyridin-3-yl | 4-Me |
| 1122 | 2-Methoxypyridin-3-yl | 2,3-F₂ |
| 1123 | 2-Methoxypyridin-3-yl | 2,4-F₂ |
| 1124 | 2-Methoxypyridin-3-yl | 2,5-F₂ |
| 1125 | 2-Methoxypyridin-3-yl | 2,6-F₂ |
| 1126 | 2-Methoxypyridin-3-yl | 3,4-F₂ |
| 1127 | 2-Methoxypyridin-3-yl | 3,5-F₂ |
| 1128 | 2-Methoxypyridin-3-yl | 3,4,5-F₃ |
| 1129 | 2-Methoxypyridin-3-yl | 3-F, 4-Cl |
| 1130 | 2-Methoxypyridin-3-yl | 3-F, 4-Br |
| 1131 | 2-Methoxypyridin-3-yl | 3-CN, 4-F |
| 1132 | 2-Methoxypyridin-3-yl | 3-Br, 4-F |
| 1133 | 2-Methoxypyridin-3-yl | 3-Cl, 4-F |
| 1134 | 2-Methoxypyridin-3-yl | 3,4-Cl₂ |
| 1135 | 2-Methoxypyridin-4-yl | H |
| 1136 | 2-Methoxypyridin-4-yl | 2-F |
| 1137 | 2-Methoxypyridin-4-yl | 3-F |
| 1138 | 2-Methoxypyridin-4-yl | 4-F |
| 1139 | 2-Methoxypyridin-4-yl | 3-Cl |
| 1140 | 2-Methoxypyridin-4-yl | 4-Cl |
| 1141 | 2-Methoxypyridin-4-yl | 3-Br |
| 1142 | 2-Methoxypyridin-4-yl | 4-Br |
| 1143 | 2-Methoxypyridin-4-yl | 3-I |
| 1144 | 2-Methoxypyridin-4-yl | 3-CN |
| 1145 | 2-Methoxypyridin-4-yl | 4-CN |
| 1146 | 2-Methoxypyridin-4-yl | 3-NO₂ |
| 1147 | 2-Methoxypyridin-4-yl | 3-Me |
| 1148 | 2-Methoxypyridin-4-yl | 4-Me |
| 1149 | 2-Methoxypyridin-4-yl | 2,3-F₂ |
| 1150 | 2-Methoxypyridin-4-yl | 2,4-F₂ |
| 1151 | 2-Methoxypyridin-4-yl | 2,5-F₂ |
| 1152 | 2-Methoxypyridin-4-yl | 2,6-F₂ |
| 1153 | 2-Methoxypyridin-4-yl | 3,4-F₂ |
| 1154 | 2-Methoxypyridin-4-yl | 3,5-F₂ |
| 1155 | 2-Methoxypyridin-4-yl | 3,4,5-F₃ |
| 1156 | 2-Methoxypyridin-4-yl | 3-F, 4-Cl |
| 1157 | 2-Methoxypyridin-4-yl | 3-F, 4-Br |
| 1158 | 2-Methoxypyridin-4-yl | 3-CN, 4-F |
| 1159 | 2-Methoxypyridin-4-yl | 3-Br, 4-F |
| 1160 | 2-Methoxypyridin-4-yl | 3-Cl, 4-F |
| 1161 | 2-Methoxypyridin-4-yl | 3,4-Cl₂ |
| 1162 | 3-Methoxypyridin-4-yl | H |
| 1163 | 3-Methoxypyridin-4-yl | 2-F |
| 1164 | 3-Methoxypyridin-4-yl | 3-F |
| 1165 | 3-Methoxypyridin-4-yl | 4-F |
| 1166 | 3-Methoxypyridin-4-yl | 3-Cl |
| 1167 | 3-Methoxypyridin-4-yl | 4-Cl |
| 1168 | 3-Methoxypyridin-4-yl | 3-Br |
| 1169 | 3-Methoxypyridin-4-yl | 4-Br |
| 1170 | 3-Methoxypyridin-4-yl | 3-I |
| 1171 | 3-Methoxypyridin-4-yl | 3-CN |
| 1172 | 3-Methoxypyridin-4-yl | 4-CN |
| 1173 | 3-Methoxypyridin-4-yl | 3-NO₂ |
| 1174 | 3-Methoxypyridin-4-yl | 3-Me |
| 1175 | 3-Methoxypyridin-4-yl | 4-Me |
| 1176 | 3-Methoxypyridin-4-yl | 2,3-F₂ |
| 1177 | 3-Methoxypyridin-4-yl | 2,4-F₂ |
| 1178 | 3-Methoxypyridin-4-yl | 2,5-F₂ |
| 1179 | 3-Methoxypyridin-4-yl | 2,6-F₂ |
| 1180 | 3-Methoxypyridin-4-yl | 3,4-F₂ |
| 1181 | 3-Methoxypyridin-4-yl | 3,5-F₂ |
| 1182 | 3-Methoxypyridin-4-yl | 3,4,5-F₃ |
| 1183 | 3-Methoxypyridin-4-yl | 3-F, 4-Cl |
| 1184 | 3-Methoxypyridin-4-yl | 3-F, 4-Br |
| 1185 | 3-Methoxypyridin-4-yl | 3-CN, 4-F |
| 1186 | 3-Methoxypyridin-4-yl | 3-Br, 4-F |
| 1187 | 3-Methoxypyridin-4-yl | 3-Cl, 4-F |
| 1188 | 3-Methoxypyridin-4-yl | 3,4-Cl₂ |
| 1189 | 3,5-Dichlorpyrazin-2-yl | H |
| 1190 | 3,5-Dichlorpyrazin-2-yl | 2-F |
| 1191 | 3,5-Dichlorpyrazin-2-yl | 3-F |
| 1192 | 3,5-Dichlorpyrazin-2-yl | 4-F |
| 1193 | 3,5-Dichlorpyrazin-2-yl | 3-Cl |
| 1194 | 3,5-Dichlorpyrazin-2-yl | 4-Cl |
| 1195 | 3,5-Dichlorpyrazin-2-yl | 3-Br |
| 1196 | 3,5-Dichlorpyrazin-2-yl | 4-Br |
| 1197 | 3,5-Dichlorpyrazin-2-yl | 3-I |
| 1198 | 3,5-Dichlorpyrazin-2-yl | 3-CN |
| 1199 | 3,5-Dichlorpyrazin-2-yl | 4-CN |
| 1200 | 3,5-Dichlorpyrazin-2-yl | 3-NO₂ |
| 1201 | 3,5-Dichlorpyrazin-2-yl | 3-Me |
| 1202 | 3,5-Dichlorpyrazin-2-yl | 4-Me |
| 1203 | 3,5-Dichlorpyrazin-2-yl | 2,3-F₂ |
| 1204 | 3,5-Dichlorpyrazin-2-yl | 2,4-F2 |
| 1205 | 3,5-Dichlorpyrazin-2-yl | 2,5-F₂ |
| 1206 | 3,5-Dichlorpyrazin-2-yl | 2,6-F₂ |
| 1207 | 3,5-Dichlorpyrazin-2-yl | 3,4-F₂ |
| 1208 | 3,5-Dichlorpyrazin-2-yl | 3,5-F₂ |
| 1209 | 3,5-Dichlorpyrazin-2-yl | 3,4,5-F₃ |
| 1210 | 3,5-Dichlorpyrazin-2-yl | 3-F, 4-Cl |
| 1211 | 3,5-Dichlorpyrazin-2-yl | 3-F, 4-Br |
| 1212 | 3,5-Dichlorpyrazin-2-yl | 3-CN, 4-F |
| 1213 | 3,5-Dichlorpyrazin-2-yl | 3-Br, 4-F |
| 1214 | 3,5-Dichlorpyrazin-2-yl | 3-Cl, 4-F |
| 1215 | 3,5-Dichlorpyrazin-2-yl | 3,4-Cl₂ |
| 1216 | 3,6-Dichlorpyrazin-2-yl | H |
| 1217 | 3,6-Dichlorpyrazin-2-yl | 2-F |
| 1218 | 3,6-Dichlorpyrazin-2-yl | 3-F |
| 1219 | 3,6-Dichlorpyrazin-2-yl | 4-F |
| 1220 | 3,6-Dichlorpyrazin-2-yl | 3-Cl |
| 1221 | 3,6-Dichlorpyrazin-2-yl | 4-Cl |
| 1222 | 3,6-Dichlorpyrazin-2-yl | 3-Br |
| 1223 | 3,6-Dichlorpyrazin-2-yl | 4-Br |
| 1224 | 3,6-Dichlorpyrazin-2-yl | 3-I |
| 1225 | 3,6-Dichlorpyrazin-2-yl | 3-CN |
| 1226 | 3,6-Dichlorpyrazin-2-yl | 4-CN |
| 1227 | 3,6-Dichlorpyrazin-2-yl | 3-NO₂ |
| 1228 | 3,6-Dichlorpyrazin-2-yl | 3-Me |
| 1229 | 3,6-Dichlorpyrazin-2-yl | 4-Me |
| 1230 | 3,6-Dichlorpyrazin-2-yl | 2,3-F₂ |
| 1231 | 3,6-Dichlorpyrazin-2-yl | 2,4-F2 |
| 1232 | 3,6-Dichlorpyrazin-2-yl | 2,5-F₂ |
| 1233 | 3,6-Dichlorpyrazin-2-yl | 2,6-F₂ |
| 1234 | 3,6-Dichlorpyrazin-2-yl | 3,4-F₂ |
| 1235 | 3,6-Dichlorpyrazin-2-yl | 3,5-F₂ |
| 1236 | 3,6-Dichlorpyrazin-2-yl | 3,4,5-F₃ |
| 1237 | 3,6-Dichlorpyrazin-2-yl | 3-F, 4-Cl |
| 1238 | 3,6-Dichlorpyrazin-2-yl | 3-F, 4-Br |
| 1239 | 3,6-Dichlorpyrazin-2-yl | 3-CN, 4-F |
| 1240 | 3,6-Dichlorpyrazin-2-yl | 3-Br, 4-F |
| 1241 | 3,6-Dichlorpyrazin-2-yl | 3-Cl, 4-F |
| 1242 | 3,6-Dichlorpyrazin-2-yl | 3,4-Cl₂ |
| 1243 | 6-Chlorpyrazin-2-yl | H |
| 1244 | 6-Chlorpyrazin-2-yl | 2-F |
| 1245 | 6-Chlorpyrazin-2-yl | 3-F |
| 1246 | 6-Chlorpyrazin-2-yl | 4-F |
| 1247 | 6-Chlorpyrazin-2-yl | 3-Cl |
| 1248 | 6-Chlorpyrazin-2-yl | 4-Cl |
| 1249 | 6-Chlorpyrazin-2-yl | 3-Br |
| 1250 | 6-Chlorpyrazin-2-yl | 4-Br |
| 1251 | 6-Chlorpyrazin-2-yl | 3-I |
| 1252 | 6-Chlorpyrazin-2-yl | 3-CN |
| 1253 | 6-Chlorpyrazin-2-yl | 4-CN |
| 1254 | 6-Chlorpyrazin-2-yl | 3-NO₂ |
| 1255 | 6-Chlorpyrazin-2-yl | 3-Me |
| 1256 | 6-Chlorpyrazin-2-yl | 4-Me |
| 1257 | 6-Chlorpyrazin-2-yl | 2,3-F₂ |
| 1258 | 6-Chlorpyrazin-2-yl | 2,4-F₂ |
| 1259 | 6-Chlorpyrazin-2-yl | 2,5-F₂ |
| 1260 | 6-Chlorpyrazin-2-yl | 2,6-F₂ |
| 1261 | 6-Chlorpyrazin-2-yl | 3,4-F₂ |
| 1262 | 6-Chlorpyrazin-2-yl | 3,5-F₂ |
| 1263 | 6-Chlorpyrazin-2-yl | 3,4,5-F₃ |
| 1264 | 6-Chlorpyrazin-2-yl | 3-F, 4-Cl |
| 1265 | 6-Chlorpyrazin-2-yl | 3-F, 4-Br |
| 1266 | 6-Chlorpyrazin-2-yl | 3-CN, 4-F |
| 1267 | 6-Chlorpyrazin-2-yl | 3-Br, 4-F |
| 1268 | 6-Chlorpyrazin-2-yl | 3-Cl, 4-F |
| 1269 | 6-Chlorpyrazin-2-yl | 3,4-Cl₂ |
| 1270 | 5-Chlorpyrazin-2-yl | H |
| 1271 | 5-Chlorpyrazin-2-yl | 2-F |
| 1272 | 5-Chlorpyrazin-2-yl | 3-F |
| 1273 | 5-Chlorpyrazin-2-yl | 4-F |
| 1274 | 5-Chlorpyrazin-2-yl | 3-Cl |
| 1275 | 5-Chlorpyrazin-2-yl | 4-Cl |
| 1276 | 5-Chlorpyrazin-2-yl | 3-Br |
| 1277 | 5-Chlorpyrazin-2-yl | 4-Br |
| 1278 | 5-Chlorpyrazin-2-yl | 3-I |
| 1279 | 5-Chlorpyrazin-2-yl | 3-CN |
| 1280 | 5-Chlorpyrazin-2-yl | 4-CN |
| 1281 | 5-Chlorpyrazin-2-yl | 3-NO₂ |
| 1282 | 5-Chlorpyrazin-2-yl | 3-Me |
| 1283 | 5-Chlorpyrazin-2-yl | 4-Me |
| 1284 | 5-Chlorpyrazin-2-yl | 2,3-F₂ |
| 1285 | 5-Chlorpyrazin-2-yl | 2,4-F₂ |
| 1286 | 5-Chlorpyrazin-2-yl | 2,5-F₂ |
| 1287 | 5-Chlorpyrazin-2-yl | 2,6-F₂ |
| 1288 | 5-Chlorpyrazin-2-yl | 3,4-F₂ |
| 1289 | 5-Chlorpyrazin-2-yl | 3,5-F₂ |
| 1290 | 5-Chlorpyrazin-2-yl | 3,4,5-F₃ |
| 1291 | 5-Chlorpyrazin-2-yl | 3-F, 4-Cl |
| 1292 | 5-Chlorpyrazin-2-yl | 3-F, 4-Br |
| 1293 | 5-Chlorpyrazin-2-yl | 3-CN, 4-F |
| 1294 | 5-Chlorpyrazin-2-yl | 3-Br, 4-F |
| 1295 | 5-Chlorpyrazin-2-yl | 3-Cl, 4-F |
| 1296 | 5-Chlorpyrazin-2-yl | 3,4-Cl₂ |
| 1297 | 3-Chlorpyrazin-2-yl | H |
| 1298 | 3-Chlorpyrazin-2-yl | 2-F |
| 1299 | 3-Chlorpyrazin-2-yl | 3-F |
| 1300 | 3-Chlorpyrazin-2-yl | 4-F |
| 1301 | 3-Chlorpyrazin-2-yl | 3-Cl |
| 1302 | 3-Chlorpyrazin-2-yl | 4-Cl |
| 1303 | 3-Chlorpyrazin-2-yl | 3-Br |
| 1304 | 3-Chlorpyrazin-2-yl | 4-Br |
| 1305 | 3-Chlorpyrazin-2-yl | 3-I |
| 1306 | 3-Chlorpyrazin-2-yl | 3-CN |
| 1307 | 3-Chlorpyrazin-2-yl | 4-CN |
| 1308 | 3-Chlorpyrazin-2-yl | 3-NO₂ |
| 1309 | 3-Chlorpyrazin-2-yl | 3-Me |
| 1310 | 3-Chlorpyrazin-2-yl | 4-Me |
| 1311 | 3-Chlorpyrazin-2-yl | 2,3-F₂ |
| 1312 | 3-Chlorpyrazin-2-yl | 2,4-F₂ |
| 1313 | 3-Chlorpyrazin-2-yl | 2,5-F₂ |
| 1314 | 3-Chlorpyrazin-2-yl | 2,6-F₂ |
| 1315 | 3-Chlorpyrazin-2-yl | 3,4-F₂ |
| 1316 | 3-Chlorpyrazin-2-yl | 3,5-F₂ |
| 1317 | 3-Chlorpyrazin-2-yl | 3,4,5-F₃ |
| 1318 | 3-Chlorpyrazin-2-yl | 3-F, 4-Cl |
| 1319 | 3-Chlorpyrazin-2-yl | 3-F, 4-Br |
| 1320 | 3-Chlorpyrazin-2-yl | 3-CN, 4-F |
| 1321 | 3-Chlorpyrazin-2-yl | 3-Br, 4-F |
| 1322 | 3-Chlorpyrazin-2-yl | 3-Cl, 4-F |
| 1323 | 3-Chlorpyrazin-2-yl | 3,4-Cl₂ |
| 1324 | 5-Chlor-3-thienyl | H |
| 1325 | 5-Chlor-3-thienyl | 2-F |
| 1326 | 5-Chlor-3-thienyl | 3-F |
| 1327 | 5-Chlor-3-thienyl | 4-F |
| 1328 | 5-Chlor-3-thienyl | 3-Cl |
| 1329 | 5-Chlor-3-thienyl | 4-Cl |
| 1330 | 5-Chlor-3-thienyl | 3-Br |
| 1331 | 5-Chlor-3-thienyl | 4-Br |
| 1332 | 5-Chlor-3-thienyl | 3-I |
| 1333 | 5-Chlor-3-thienyl | 3-CN |
| 1334 | 5-Chlor-3-thienyl | 4-CN |
| 1335 | 5-Chlor-3-thienyl | 3-NO₂ |
| 1336 | 5-Chlor-3-thienyl | 3-Me |
| 1337 | 5-Chlor-3-thienyl | 4-Me |
| 1338 | 5-Chlor-3-thienyl | 2,3-F₂ |
| 1339 | 5-Chlor-3-thienyl | 2,4-F₂ |
| 1340 | 5-Chlor-3-thienyl | 2,5-F₂ |
| 1341 | 5-Chlor-3-thienyl | 2,6-F₂ |
| 1342 | 5-Chlor-3-thienyl | 3,4-F₂ |
| 1343 | 5-Chlor-3-thienyl | 3,5-F₂ |
| 1344 | 5-Chlor-3-thienyl | 3,4,5-F₃ |
| 1345 | 5-Chlor-3-thienyl | 3-F, 4-Cl |
| 1346 | 5-Chlor-3-thienyl | 3-F, 4-Br |
| 1347 | 5-Chlor-3-thienyl | 3-CN, 4-F |
| 1348 | 5-Chlor-3-thienyl | 3-Br, 4-F |
| 1349 | 5-Chlor-3-thienyl | 3-Cl, 4-F |
| 1350 | 5-Chlor-3-thienyl | 3,4-Cl₂ |
| 1351 | 4-Chlor-3-thienyl | H |
| 1352 | 4-Chlor-3-thienyl | 2-F |
| 1353 | 4-Chlor-3-thienyl | 3-F |
| 1354 | 4-Chlor-3-thienyl | 4-F |
| 1355 | 4-Chlor-3-thienyl | 3-Cl |
| 1356 | 4-Chlor-3-thienyl | 4-Cl |
| 1357 | 4-Chlor-3-thienyl | 3-Br |
| 1358 | 4-Chlor-3-thienyl | 4-Br |
| 1359 | 4-Chlor-3-thienyl | 3-I |
| 1360 | 4-Chlor-3-thienyl | 3-CN |
| 1361 | 4-Chlor-3-thienyl | 4-CN |
| 1362 | 4-Chlor-3-thienyl | 3-NO₂ |
| 1363 | 4-Chlor-3-thienyl | 3-Me |
| 1364 | 4-Chlor-3-thienyl | 4-Me |
| 1365 | 4-Chlor-3-thienyl | 2,3-F₂ |
| 1366 | 4-Chlor-3-thienyl | 2,4-F₂ |
| 1367 | 4-Chlor-3-thienyl | 2,5-F₂ |
| 1368 | 4-Chlor-3-thienyl | 2,6-F₂ |
| 1369 | 4-Chlor-3-thienyl | 3,4-F₂ |
| 1370 | 4-Chlor-3-thienyl | 3,5-F₂ |
| 1371 | 4-Chlor-3-thienyl | 3,4,5-F₃ |
| 1372 | 4-Chlor-3-thienyl | 3-F, 4-Cl |
| 1373 | 4-Chlor-3-thienyl | 3-F, 4-Br |
| 1374 | 4-Chlor-3-thienyl | 3-CN, 4-F |
| 1375 | 4-Chlor-3-thienyl | 3-Br, 4-F |
| 1376 | 4-Chlor-3-thienyl | 3-Cl, 4-F |
| 1377 | 4-Chlor-3-thienyl | 3,4-Cl₂ |
| 1378 | 2-Chlor-3-thienyl | H |
| 1379 | 2-Chlor-3-thienyl | 2-F |
| 1380 | 2-Chlor-3-thienyl | 3-F |
| 1381 | 2-Chlor-3-thienyl | 4-F |
| 1382 | 2-Chlor-3-thienyl | 3-Cl |
| 1383 | 2-Chlor-3-thienyl | 4-Cl |
| 1384 | 2-Chlor-3-thienyl | 3-Br |
| 1385 | 2-Chlor-3-thienyl | 4-Br |
| 1386 | 2-Chlor-3-thienyl | 3-I |
| 1387 | 2-Chlor-3-thienyl | 3-CN |
| 1388 | 2-Chlor-3-thienyl | 4-CN |
| 1389 | 2-Chlor-3-thienyl | 3-NO₂ |
| 1390 | 2-Chlor-3-thienyl | 3-Me |
| 1391 | 2-Chlor-3-thienyl | 4-Me |
| 1392 | 2-Chlor-3-thienyl | 2,3-F₂ |
| 1393 | 2-Chlor-3-thienyl | 2,4-F₂ |
| 1394 | 2-Chlor-3-thienyl | 2,5-F₂ |
| 1395 | 2-Chlor-3-thienyl | 2,6-F₂ |
| 1396 | 2-Chlor-3-thienyl | 3,4-F₂ |
| 1397 | 2-Chlor-3-thienyl | 3,5-F₂ |
| 1398 | 2-Chlor-3-thienyl | 3,4,5-F₃ |
| 1399 | 2-Chlor-3-thienyl | 3-F, 4-Cl |
| 1400 | 2-Chlor-3-thienyl | 3-F, 4-Br |
| 1401 | 2-Chlor-3-thienyl | 3-CN, 4-F |
| 1402 | 2-Chlor-3-thienyl | 3-Br, 4-F |
| 1403 | 2-Chlor-3-thienyl | 3-Cl, 4-F |
| 1404 | 2-Chlor-3-thienyl | 3,4-Cl₂ |
| 1405 | 2,5-Dichlor-3-thienyl | H |
| 1406 | 2,5-Dichlor-3-thienyl | 2-F |
| 1407 | 2,5-Dichlor-3-thienyl | 3-F |
| 1408 | 2,5-Dichlor-3-thienyl | 4-F |
| 1409 | 2,5-Dichlor-3-thienyl | 3-Cl |
| 1410 | 2,5-Dichlor-3-thienyl | 4-Cl |
| 1411 | 2,5-Dichlor-3-thienyl | 3-Br |
| 1412 | 2,5-Dichlor-3-thienyl | 4-Br |
| 1413 | 2,5-Dichlor-3-thienyl | 3-I |
| 1414 | 2,5-Dichlor-3-thienyl | 3-CN |
| 1415 | 2,5-Dichlor-3-thienyl | 4-CN |
| 1416 | 2,5-Dichlor-3-thienyl | 3-NO₂ |
| 1417 | 2,5-Dichlor-3-thienyl | 3-Me |
| 1418 | 2,5-Dichlor-3-thienyl | 4-Me |
| 1419 | 2,5-Dichlor-3-thienyl | 2,3-F₂ |
| 1420 | 2,5-Dichlor-3-thienyl | 2,4-F₂ |
| 1421 | 2,5-Dichlor-3-thienyl | 2,5-F₂ |
| 1422 | 2,5-Dichlor-3-thienyl | 2,6-F₂ |
| 1423 | 2,5-Dichlor-3-thienyl | 3,4-F₂ |
| 1424 | 2,5-Dichlor-3-thienyl | 3,5-F₂ |
| 1425 | 2,5-Dichlor-3-thienyl | 3,4,5-F₃ |
| 1426 | 2,5-Dichlor-3-thienyl | 3-F, 4-Cl |
| 1427 | 2,5-Dichlor-3-thienyl | 3-F, 4-Br |
| 1428 | 2,5-Dichlor-3-thienyl | 3-CN, 4-F |
| 1429 | 2,5-Dichlor-3-thienyl | 3-Br, 4-F |
| 1430 | 2,5-Dichlor-3-thienyl | 3-Cl, 4-F |
| 1431 | 2,5-Dichlor-3-thienyl | 3,4-Cl₂ |
| 1432 | 5-Chlor-2-thienyl | H |
| 1433 | 5-Chlor-2-thienyl | 2-F |
| 1434 | 5-Chlor-2-thienyl | 3-F |
| 1435 | 5-Chlor-2-thienyl | 4-F |
| 1436 | 5-Chlor-2-thienyl | 3-Cl |
| 1437 | 5-Chlor-2-thienyl | 4-Cl |
| 1438 | 5-Chlor-2-thienyl | 3-Br |
| 1439 | 5-Chlor-2-thienyl | 4-Br |
| 1440 | 5-Chlor-2-thienyl | 3-I |
| 1441 | 5-Chlor-2-thienyl | 3-CN |
| 1442 | 5-Chlor-2-thienyl | 4-CN |
| 1443 | 5-Chlor-2-thienyl | 3-NO₂ |
| 1444 | 5-Chlor-2-thienyl | 3-Me |
| 1445 | 5-Chlor-2-thienyl | 4-Me |
| 1446 | 5-Chlor-2-thienyl | 2,3-F₂ |
| 1447 | 5-Chlor-2-thienyl | 2,4-F₂ |
| 1448 | 5-Chlor-2-thienyl | 2,5-F₂ |
| 1449 | 5-Chlor-2-thienyl | 2,6-F₂ |
| 1450 | 5-Chlor-2-thienyl | 3,4-F₂ |
| 1451 | 5-Chlor-2-thienyl | 3,5-F₂ |
| 1452 | 5-Chlor-2-thienyl | 3,4,5-F₃ |
| 1453 | 5-Chlor-2-thienyl | 3-F, 4-Cl |
| 1454 | 5-Chlor-2-thienyl | 3-F, 4-Br |
| 1455 | 5-Chlor-2-thienyl | 3-CN, 4-F |
| 1456 | 5-Chlor-2-thienyl | 3-Br, 4-F |
| 1457 | 5-Chlor-2-thienyl | 3-Cl, 4-F |
| 1458 | 5-Chlor-2-thienyl | 3,4-Cl₂ |
| 1459 | 4-Chlor-2-thienyl | H |
| 1460 | 4-Chlor-2-thienyl | 2-F |
| 1461 | 4-Chlor-2-thienyl | 3-F |
| 1462 | 4-Chlor-2-thienyl | 4-F |
| 1463 | 4-Chlor-2-thienyl | 3-Cl |
| 1464 | 4-Chlor-2-thienyl | 4-Cl |
| 1465 | 4-Chlor-2-thienyl | 3-Br |
| 1466 | 4-Chlor-2-thienyl | 4-Br |
| 1467 | 4-Chlor-2-thienyl | 3-I |
| 1468 | 4-Chlor-2-thienyl | 3-CN |
| 1469 | 4-Chlor-2-thienyl | 4-CN |
| 1470 | 4-Chlor-2-thienyl | 3-NO₂ |
| 1471 | 4-Chlor-2-thienyl | 3-Me |
| 1472 | 4-Chlor-2-thienyl | 4-Me |
| 1473 | 4-Chlor-2-thienyl | 2,3-F₂ |
| 1474 | 4-Chlor-2-thienyl | 2,4-F₂ |
| 1475 | 4-Chlor-2-thienyl | 2,5-F₂ |
| 1476 | 4-Chlor-2-thienyl | 2,6-F₂ |
| 1477 | 4-Chlor-2-thienyl | 3,4-F₂ |
| 1478 | 4-Chlor-2-thienyl | 3,5-F₂ |
| 1479 | 4-Chlor-2-thienyl | 3,4,5-F₃ |
| 1480 | 4-Chlor-2-thienyl | 3-F, 4-Cl |
| 1481 | 4-Chlor-2-thienyl | 3-F, 4-Br |
| 1482 | 4-Chlor-2-thienyl | 3-CN, 4-F |
| 1483 | 4-Chlor-2-thienyl | 3-Br, 4-F |
| 1484 | 4-Chlor-2-thienyl | 3-Cl, 4-F |
| 1485 | 4-Chlor-2-thienyl | 3,4-C1₂ |
| 1486 | 3-Chlor-2-thienyl | H |
| 1487 | 3-Chlor-2-thienyl | 2-F |
| 1488 | 3-Chlor-2-thienyl | 3-F |
| 1489 | 3-Chlor-2-thienyl | 4-F |
| 1490 | 3-Chlor-2-thienyl | 3-Cl |
| 1491 | 3-Chlor-2-thienyl | 4-Cl |
| 1492 | 3-Chlor-2-thienyl | 3-Br |
| 1493 | 3-Chlor-2-thienyl | 4-Br |
| 1494 | 3-Chlor-2-thienyl | 3-I |
| 1495 | 3-Chlor-2-thienyl | 3-CN |
| 1496 | 3-Chlor-2-thienyl | 4-CN |
| 1497 | 3-Chlor-2-thienyl | 3-NO₂ |
| 1498 | 3-Chlor-2-thienyl | 3-Me |
| 1499 | 3-Chlor-2-thienyl | 4-Me |
| 1500 | 3-Chlor-2-thienyl | 2,3-F₂ |
| 1501 | 3-Chlor-2-thienyl | 2,4-F₂ |
| 1502 | 3-Chlor-2-thienyl | 2,5-F₂ |
| 1503 | 3-Chlor-2-thienyl | 2,6-F₂ |
| 1504 | 3-Chlor-2-thienyl | 3,4-F₂ |
| 1505 | 3-Chlor-2-thienyl | 3,5-F₂ |
| 1506 | 3-Chlor-2-thienyl | 3,4,5-F₃ |
| 1507 | 3-Chlor-2-thienyl | 3-F, 4-Cl |
| 1508 | 3-Chlor-2-thienyl | 3-F, 4-Br |
| 1509 | 3-Chlor-2-thienyl | 3-CN, 4-F |
| 1510 | 3-Chlor-2-thienyl | 3-Br, 4-F |
| 1511 | 3-Chlor-2-thienyl | 3-Cl, 4-F |
| 1512 | 3-Chlor-2-thienyl | 3,4-Cl₂ |
| 1513 | 2-Brom-1,3-thiazol-5-yl | H |
| 1514 | 2-Brom-1,3-thiazol-5-yl | 2-F |
| 1515 | 2-Brom-1,3-thiazol-5-yl | 3-F |
| 1516 | 2-Brom-1,3-thiazol-5-yl | 4-F |
| 1517 | 2-Brom-1,3-thiazol-5-yl | 3-Cl |
| 1518 | 2-Brom-1,3-thiazol-5-yl | 4-Cl |
| 1519 | 2-Brom-1,3-thiazol-5-yl | 3-Br |
| 1520 | 2-Brom-1,3-thiazol-5-yl | 4-Br |
| 1521 | 2-Brom-1,3-thiazol-5-yl | 3-I |
| 1522 | 2-Brom-1,3-thiazol-5-yl | 3-CN |
| 1523 | 2-Brom-1,3-thiazol-5-yl | 4-CN |
| 1524 | 2-Brom-1,3-thiazol-5-yl | 3-NO₂ |
| 1525 | 2-Brom-1,3-thiazol-5-yl | 3-Me |
| 1526 | 2-Brom-1,3-thiazol-5-yl | 4-Me |
| 1527 | 2-Brom-1,3-thiazol-5-yl | 2,3-F₂ |
| 1528 | 2-Brom-1,3-thiazol-5-yl | 2,4-F₂ |
| 1529 | 2-Brom-1,3-thiazol-5-yl | 2,5-F₂ |
| 1530 | 2-Brom-1,3-thiazol-5-yl | 2,6-F₂ |
| 1531 | 2-Brom-1,3-thiazol-5-yl | 3,4-F₂ |
| 1532 | 2-Brom-1,3-thiazol-5-yl | 3,5-F₂ |
| 1533 | 2-Brom-1,3-thiazol-5-yl | 3,4,5-F₃ |
| 1534 | 2-Brom-1,3-thiazol-5-yl | 3-F, 4-Cl |
| 1535 | 2-Brom-1,3-thiazol-5-yl | 3-F, 4-Br |
| 1536 | 2-Brom-1,3-thiazol-5-yl | 3-CN, 4-F |
| 1537 | 2-Brom-1,3-thiazol-5-yl | 3-Br, 4-F |
| 1538 | 2-Brom-1,3-thiazol-5-yl | 3-Cl, 4-F |
| 1539 | 2-Brom-1,3-thiazol-5-yl | 3,4-Cl₂ |
| 1540 | 2-Chlor-1,3-thiazol-5-yl | H |
| 1541 | 2-Chlor-1,3-thiazol-5-yl | 2-F |
| 1542 | 2-Chlor-1,3-thiazol-5-yl | 3-F |
| 1543 | 2-Chlor-1,3-thiazol-5-yl | 4-F |
| 1544 | 2-Chlor-1,3-thiazol-5-yl | 3-Cl |
| 1545 | 2-Chlor-1,3-thiazol-5-yl | 4-Cl |
| 1546 | 2-Chlor-1,3-thiazol-5-yl | 3-Br |
| 1547 | 2-Chlor-1,3-thiazol-5-yl | 4-Br |
| 1548 | 2-Chlor-1,3-thiazol-5-yl | 3-I |
| 1549 | 2-Chlor-1,3-thiazol-5-yl | 3-CN |
| 1550 | 2-Chlor-1,3-thiazol-5-yl | 4-CN |
| 1551 | 2-Chlor-1,3-thiazol-5-yl | 3-NO₂ |
| 1552 | 2-Chlor-1,3-thiazol-5-yl | 3-Me |
| 1553 | 2-Chlor-1,3-thiazol-5-yl | 4-Me |
| 1554 | 2-Chlor-1,3-thiazol-5-yl | 2,3-F₂ |
| 1555 | 2-Chlor-1,3-thiazol-5-yl | 2,4-F2 |
| 1556 | 2-Chlor-1,3-thiazol-5-yl | 2,5-F₂ |
| 1557 | 2-Chlor-1,3-thiazol-5-yl | 2,6-F₂ |
| 1558 | 2-Chlor-1,3-thiazol-5-yl | 3,4-F₂ |
| 1559 | 2-Chlor-1,3-thiazol-5-yl | 3,5-F₂ |
| 1560 | 2-Chlor-1,3-thiazol-5-yl | 3,4,5-F₃ |
| 1561 | 2-Chlor-1,3-thiazol-5-yl | 3-F, 4-Cl |
| 1562 | 2-Chlor-1,3-thiazol-5-yl | 3-F, 4-Br |
| 1563 | 2-Chlor-1,3-thiazol-5-yl | 3-CN, 4-F |
| 1564 | 2-Chlor-1,3-thiazol-5-yl | 3-Br, 4-F |
| 1565 | 2-Chlor-1,3-thiazol-5-yl | 3-Cl, 4-F |
| 1566 | 2-Chlor-1,3-thiazol-5-yl | 3,4-Cl₂ |
| 1567 | 5-Fluor-3-thienyl | H |
| 1568 | 5-Fluor-3-thienyl | 2-F |
| 1569 | 5-Fluor-3-thienyl | 3-F |
| 1570 | 5-Fluor-3-thienyl | 4-F |
| 1571 | 5-Fluor-3-thienyl | 3-Cl |
| 1572 | 5-Fluor-3-thienyl | 4-Cl |
| 1573 | 5-Fluor-3-thienyl | 3-Br |
| 1574 | 5-Fluor-3-thienyl | 4-Br |
| 1575 | 5-Fluor-3-thienyl | 3-I |
| 1576 | 5-Fluor-3-thienyl | 3-CN |
| 1577 | 5-Fluor-3-thienyl | 4-CN |
| 1578 | 5-Fluor-3-thienyl | 3-NO₂ |
| 1579 | 5-Fluor-3-thienyl | 3-Me |
| 1580 | 5-Fluor-3-thienyl | 4-Me |
| 1581 | 5-Fluor-3-thienyl | 2,3-F₂ |
| 1582 | 5-Fluor-3-thienyl | 2,4-F2 |
| 1583 | 5-Fluor-3-thienyl | 2,5-F₂ |
| 1584 | 5-Fluor-3-thienyl | 2,6-F₂ |
| 1585 | 5-Fluor-3-thienyl | 3,4-F₂ |
| 1586 | 5-Fluor-3-thienyl | 3,5-F₂ |
| 1587 | 5-Fluor-3-thienyl | 3,4,5-F₃ |
| 1588 | 5-Fluor-3-thienyl | 3-F, 4-Cl |
| 1589 | 5-Fluor-3-thienyl | 3-F, 4-Br |
| 1590 | 5-Fluor-3-thienyl | 3-CN, 4-F |
| 1591 | 5-Fluor-3-thienyl | 3-Br, 4-F |
| 1592 | 5-Fluor-3-thienyl | 3-Cl, 4-F |
| 1593 | 5-Fluor-3-thienyl | 3,4-Cl₂ |
| 1594 | 4-Fluor-3-thienyl | H |
| 1595 | 4-Fluor-3-thienyl | 2-F |
| 1596 | 4-Fluor-3-thienyl | 3-F |
| 1597 | 4-Fluor-3-thienyl | 4-F |
| 1598 | 4-Fluor-3-thienyl | 3-Cl |
| 1599 | 4-Fluor-3-thienyl | 4-Cl |
| 1600 | 4-Fluor-3-thienyl | 3-Br |
| 1601 | 4-Fluor-3-thienyl | 4-Br |
| 1602 | 4-Fluor-3-thienyl | 3-I |
| 1603 | 4-Fluor-3-thienyl | 3-CN |
| 1604 | 4-Fluor-3-thienyl | 4-CN |
| 1605 | 4-Fluor-3-thienyl | 3-NO₂ |
| 1606 | 4-Fluor-3-thienyl | 3-Me |
| 1607 | 4-Fluor-3-thienyl | 4-Me |
| 1608 | 4-Fluor-3-thienyl | 2,3-F₂ |
| 1609 | 4-Fluor-3-thienyl | 2,4-F2 |
| 1610 | 4-Fluor-3-thienyl | 2,5-F₂ |
| 1611 | 4-Fluor-3-thienyl | 2,6-F₂ |
| 1612 | 4-Fluor-3-thienyl | 3,4-F₂ |
| 1613 | 4-Fluor-3-thienyl | 3,5-F₂ |
| 1614 | 4-Fluor-3-thienyl | 3,4,5-F₃ |
| 1615 | 4-Fluor-3-thienyl | 3-F, 4-Cl |
| 1616 | 4-Fluor-3-thienyl | 3-F, 4-Br |
| 1617 | 4-Fluor-3-thienyl | 3-CN, 4-F |
| 1618 | 4-Fluor-3-thienyl | 3-Br, 4-F |
| 1619 | 4-Fluor-3-thienyl | 3-Cl, 4-F |
| 1620 | 4-Fluor-3-thienyl | 3,4-Cl₂ |
| 1621 | 2-Fluor-3-thienyl | H |
| 1622 | 2-Fluor-3-thienyl | 2-F |
| 1623 | 2-Fluor-3-thienyl | 3-F |
| 1624 | 2-Fluor-3-thienyl | 4-F |
| 1625 | 2-Fluor-3-thienyl | 3-Cl |
| 1626 | 2-Fluor-3-thienyl | 4-Cl |
| 1627 | 2-Fluor-3-thienyl | 3-Br |
| 1628 | 2-Fluor-3-thienyl | 4-Br |
| 1629 | 2-Fluor-3-thienyl | 3-I |
| 1630 | 2-Fluor-3-thienyl | 3-CN |
| 1631 | 2-Fluor-3-thienyl | 4-CN |
| 1632 | 2-Fluor-3-thienyl | 3-NO₂ |
| 1633 | 2-Fluor-3-thienyl | 3-Me |
| 1634 | 2-Fluor-3-thienyl | 4-Me |
| 1635 | 2-Fluor-3-thienyl | 2,3-F₂ |
| 1636 | 2-Fluor-3-thienyl | 2,4-F2 |
| 1637 | 2-Fluor-3-thienyl | 2,5-F₂ |
| 1638 | 2-Fluor-3-thienyl | 2,6-F₂ |
| 1639 | 2-Fluor-3-thienyl | 3,4-F₂ |
| 1640 | 2-Fluor-3-thienyl | 3,5-F₂ |
| 1641 | 2-Fluor-3-thienyl | 3,4,5-F₃ |
| 1642 | 2-Fluor-3-thienyl | 3-F, 4-Cl |
| 1643 | 2-Fluor-3-thienyl | 3-F, 4-Br |
| 1644 | 2-Fluor-3-thienyl | 3-CN, 4-F |
| 1645 | 2-Fluor-3-thienyl | 3-Br, 4-F |
| 1646 | 2-Fluor-3-thienyl | 3-Cl, 4-F |
| 1647 | 2-Fluor-3-thienyl | 3,4-Cl₂ |
| 1648 | 2,5-Difluor-3-thienyl | H |
| 1649 | 2,5-Difluor-3-thienyl | 2-F |
| 1650 | 2,5-Difluor-3-thienyl | 3-F |
| 1651 | 2,5-Difluor-3-thienyl | 4-F |
| 1652 | 2,5-Difluor-3-thienyl | 3-Cl |
| 1653 | 2,5-Difluor-3-thienyl | 4-Cl |
| 1654 | 2,5-Difluor-3-thienyl | 3-Br |
| 1655 | 2,5-Difluor-3-thienyl | 4-Br |
| 1656 | 2,5-Difluor-3-thienyl | 3-I |
| 1657 | 2,5-Difluor-3-thienyl | 3-CN |
| 1658 | 2,5-Difluor-3-thienyl | 4-CN |
| 1659 | 2,5-Difluor-3-thienyl | 3-NO₂ |
| 1660 | 2,5-Difluor-3-thienyl | 3-Me |
| 1661 | 2,5-Difluor-3-thienyl | 4-Me |
| 1662 | 2,5-Difluor-3-thienyl | 2,3-F₂ |
| 1663 | 2,5-Difluor-3-thienyl | 2,4-F₂ |
| 1664 | 2,5-Difluor-3-thienyl | 2,5-F₂ |
| 1665 | 2,5-Difluor-3-thienyl | 2,6-F₂ |
| 1666 | 2,5-Difluor-3-thienyl | 3,4-F₂ |
| 1667 | 2,5-Difluor-3-thienyl | 3,5-F₂ |
| 1668 | 2,5-Difluor-3-thienyl | 3,4,5-F₃ |
| 1669 | 2,5-Difluor-3-thienyl | 3-F, 4-Cl |
| 1670 | 2,5-Difluor-3-thienyl | 3-F, 4-Br |
| 1671 | 2,5-Difluor-3-thienyl | 3-CN, 4-F |
| 1672 | 2,5-Difluor-3-thienyl | 3-Br, 4-F |
| 1673 | 2,5-Difluor-3-thienyl | 3-Cl, 4-F |
| 1674 | 2,5-Difluor-3-thienyl | 3,4-Cl₂ |
| 1675 | 5-Fluor-2-thienyl | H |
| 1676 | 5-Fluor-2-thienyl | 2-F |
| 1677 | 5-Fluor-2-thienyl | 3-F |
| 1678 | 5-Fluor-2-thienyl | 4-F |
| 1679 | 5-Fluor-2-thienyl | 3-Cl |
| 1680 | 5-Fluor-2-thienyl | 4-Cl |
| 1681 | 5-Fluor-2-thienyl | 3-Br |
| 1682 | 5-Fluor-2-thienyl | 4-Br |
| 1683 | 5-Fluor-2-thienyl | 3-I |
| 1684 | 5-Fluor-2-thienyl | 3-CN |
| 1685 | 5-Fluor-2-thienyl | 4-CN |
| 1686 | 5-Fluor-2-thienyl | 3-NO₂ |
| 1687 | 5-Fluor-2-thienyl | 3-Me |
| 1688 | 5-Fluor-2-thienyl | 4-Me |
| 1689 | 5-Fluor-2-thienyl | 2,3-F₂ |
| 1690 | 5-Fluor-2-thienyl | 2,4-F2 |
| 1691 | 5-Fluor-2-thienyl | 2,5-F₂ |
| 1692 | 5-Fluor-2-thienyl | 2,6-F₂ |
| 1693 | 5-Fluor-2-thienyl | 3,4-F₂ |
| 1694 | 5-Fluor-2-thienyl | 3,5-F₂ |
| 1695 | 5-Fluor-2-thienyl | 3,4,5-F₃ |
| 1696 | 5-Fluor-2-thienyl | 3-F, 4-Cl |
| 1697 | 5-Fluor-2-thienyl | 3-F, 4-Br |
| 1698 | 5-Fluor-2-thienyl | 3-CN, 4-F |
| 1699 | 5-Fluor-2-thienyl | 3-Br, 4-F |
| 1700 | 5-Fluor-2-thienyl | 3-Cl, 4-F |
| 1701 | 5-Fluor-2-thienyl | 3,4-Cl₂ |
| 1702 | 4-Fluor-2-thienyl | H |
| 1703 | 4-Fluor-2-thienyl | 2-F |
| 1704 | 4-Fluor-2-thienyl | 3-F |
| 1705 | 4-Fluor-2-thienyl | 4-F |
| 1706 | 4-Fluor-2-thienyl | 3-Cl |
| 1707 | 4-Fluor-2-thienyl | 4-Cl |
| 1708 | 4-Fluor-2-thienyl | 3-Br |
| 1709 | 4-Fluor-2-thienyl | 4-Br |
| 1710 | 4-Fluor-2-thienyl | 3-I |
| 1711 | 4-Fluor-2-thienyl | 3-CN |
| 1712 | 4-Fluor-2-thienyl | 4-CN |
| 1713 | 4-Fluor-2-thienyl | 3-NO₂ |
| 1714 | 4-Fluor-2-thienyl | 3-Me |
| 1715 | 4-Fluor-2-thienyl | 4-Me |
| 1716 | 4-Fluor-2-thienyl | 2,3-F₂ |
| 1717 | 4-Fluor-2-thienyl | 2,4-F2 |
| 1718 | 4-Fluor-2-thienyl | 2,5-F₂ |
| 1719 | 4-Fluor-2-thienyl | 2,6-F₂ |
| 1720 | 4-Fluor-2-thienyl | 3,4-F₂ |
| 1721 | 4-Fluor-2-thienyl | 3,5-F₂ |
| 1722 | 4-Fluor-2-thienyl | 3,4,5-F₃ |
| 1723 | 4-Fluor-2-thienyl | 3-F, 4-Cl |
| 1724 | 4-Fluor-2-thienyl | 3-F, 4-Br |
| 1725 | 4-Fluor-2-thienyl | 3-CN, 4-F |
| 1726 | 4-Fluor-2-thienyl | 3-Br, 4-F |
| 1727 | 4-Fluor-2-thienyl | 3-Cl, 4-F |
| 1728 | 4-Fluor-2-thienyl | 3,4-Cl₂ |
| 1729 | 3-Fluor-2-thienyl | H |
| 1730 | 3-Fluor-2-thienyl | 2-F |
| 1731 | 3-Fluor-2-thienyl | 3-F |
| 1732 | 3-Fluor-2-thienyl | 4-F |
| 1733 | 3-Fluor-2-thienyl | 3-Cl |
| 1734 | 3-Fluor-2-thienyl | 4-Cl |
| 1735 | 3-Fluor-2-thienyl | 3-Br |
| 1736 | 3-Fluor-2-thienyl | 4-Br |
| 1737 | 3-Fluor-2-thienyl | 3-I |
| 1738 | 3-Fluor-2-thienyl | 3-CN |
| 1739 | 3-Fluor-2-thienyl | 4-CN |
| 1740 | 3-Fluor-2-thienyl | 3-NO₂ |
| 1741 | 3-Fluor-2-thienyl | 3-Me |
| 1742 | 3-Fluor-2-thienyl | 4-Me |
| 1743 | 3-Fluor-2-thienyl | 2,3-F₂ |
| 1744 | 3-Fluor-2-thienyl | 2,4-F2 |
| 1745 | 3-Fluor-2-thienyl | 2,5-F₂ |
| 1746 | 3-Fluor-2-thienyl | 2,6-F₂ |
| 1747 | 3-Fluor-2-thienyl | 3,4-F₂ |
| 1748 | 3-Fluor-2-thienyl | 3,5-F₂ |
| 1749 | 3-Fluor-2-thienyl | 3,4,5-F₃ |
| 1750 | 3-Fluor-2-thienyl | 3-F, 4-Cl |
| 1751 | 3-Fluor-2-thienyl | 3-F, 4-Br |
| 1752 | 3-Fluor-2-thienyl | 3-CN, 4-F |
| 1753 | 3-Fluor-2-thienyl | 3-Br, 4-F |
| 1754 | 3-Fluor-2-thienyl | 3-Cl, 4-F |
| 1755 | 3-Fluor-2-thienyl | 3,4-Cl₂ |
| 1756 | 1,3-Thiazol-5-yl | H |
| 1757 | 1,3-Thiazol-5-yl | 2-F |
| 1758 | 1,3-Thiazol-5-yl | 3-F |
| 1759 | 1,3-Thiazol-5-yl | 4-F |
| 1760 | 1,3-Thiazol-5-yl | 3-Cl |
| 1761 | 1,3-Thiazol-5-yl | 4-Cl |
| 1762 | 1,3-Thiazol-5-yl | 3-Br |
| 1763 | 1,3-Thiazol-5-yl | 4-Br |
| 1764 | 1,3-Thiazol-5-yl | 3-I |
| 1765 | 1,3-Thiazol-5-yl | 3-CN |
| 1766 | 1,3-Thiazol-5-yl | 4-CN |
| 1767 | 1,3-Thiazol-5-yl | 3-NO₂ |
| 1768 | 1,3-Thiazol-5-yl | 3-Me |
| 1769 | 1,3-Thiazol-5-yl | 4-Me |
| 1770 | 1,3-Thiazol-5-yl | 2,3-F₂ |
| 1771 | 1,3-Thiazol-5-yl | 2,4-F2 |
| 1772 | 1,3-Thiazol-5-yl | 2,5-F₂ |
| 1773 | 1,3-Thiazol-5-yl | 2,6-F₂ |
| 1774 | 1,3-Thiazol-5-yl | 3,4-F₂ |
| 1775 | 1,3-Thiazol-5-yl | 3,5-F₂ |
| 1776 | 1,3-Thiazol-5-yl | 3,4,5-F₃ |
| 1777 | 1,3-Thiazol-5-yl | 3-F, 4-Cl |
| 1778 | 1,3-Thiazol-5-yl | 3-F, 4-Br |
| 1779 | 1,3-Thiazol-5-yl | 3-CN, 4-F |
| 1780 | 1,3-Thiazol-5-yl | 3-Br, 4-F |
| 1781 | 1,3-Thiazol-5-yl | 3-Cl, 4-F |
| 1782 | 1,3-Thiazol-5-yl | 3,4-Cl₂ |
| 1783 * | 1,3-Oxazol-5-yl | H |
| 1784 * | 1,3-Oxazol-5-yl | 2-F |
| 1785 * | 1,3-Oxazol-5-yl | 3-F |
| 1786 * | 1,3-Oxazol-5-yl | 4-F |
| 1787 * | 1,3-Oxazol-5-yl | 3-Cl |
| 1788 * | 1,3-Oxazol-5-yl | 4-Cl |
| 1789 * | 1,3-Oxazol-5-yl | 3-Br |
| 1790 * | 1,3-Oxazol-5-yl | 4-Br |
| 1791 * | 1,3-Oxazol-5-yl | 3-I |
| 1792 * | 1,3-Oxazol-5-yl | 3-CN |
| 1793 * | 1,3-Oxazol-5-yl | 4-CN |
| 1794 * | 1,3-Oxazol-5-yl | 3-NO₂ |
| 1795 * | 1,3-Oxazol-5-yl | 3-Me |
| 1796 * | 1,3-Oxazol-5-yl | 4-Me |
| 1797 * | 1,3-Oxazol-5-yl | 2,3-F₂ |
| 1798 * | 1,3-Oxazol-5-yl | 2,4-F2 |
| 1799 * | 1,3-Oxazol-5-yl | 2,5-F₂ |
| 1800 * | 1,3-Oxazol-5-yl | 2,6-F₂ |
| 1801 * | 1,3-Oxazol-5-yl | 3,4-F₂ |
| 1802 * | 1,3-Oxazol-5-yl | 3,5-F₂ |
| 1803 * | 1,3-Oxazol-5-yl | 3,4,5-F₃ |
| 1804 * | 1,3-Oxazol-5-yl | 3-F, 4-Cl |
| 1805 * | 1,3-Oxazol-5-yl | 3-F, 4-Br |
| 1806 * | 1,3-Oxazol-5-yl | 3-CN, 4-F |
| 1807 * | 1,3-Oxazol-5-yl | 3-Br, 4-F |
| 1808 * | 1,3-Oxazol-5-yl | 3-Cl, 4-F |
| 1809 * | 1,3-Oxazol-5-yl | 3,4-Cl₂ |

| | | |
|---|---|---|
| *nicht Gegenstand der Erfindung | | |

Ebenfalls bevorzugte erfindungsgemäße Verbindungen der Formel (I) bzw. (Ia) sind im untenstehenden Beispielteil beschrieben.

Für bevorzugte erfindungsgemäße Verbindungen der Formel (I) bzw. (Ia) ist m größer oder gleich 1, bevorzugt größer oder gleich 2, und es gilt:
ein, mehrere oder sämtliche Reste R² sind ausgewählt aus der Gruppe bestehend aus F, Cl, Br, I, Methyl, und CN,
   und
ein, mehrere oder sämtliche Reste R³ sind ausgewählt aus der Gruppe bestehend aus F, Cl, Br, Methyl, Methoxy, Nitro und CN.

Für bevorzugte erfindungsgemäße Verbindungen der Formel (I) bzw. (Ia) gilt:
n ist größer oder gleich 1, bevorzugt größer oder gleich 2,
m ist größer oder gleich 1, bevorzugt größer oder gleich 2,
ein, mehrere oder sämtliche Reste R² sind ausgewählt aus der Gruppe bestehend aus F, Cl, Br, I, Methyl, und CN,
   und
ein, mehrere oder sämtliche Reste R³ sind ausgewählt aus der Gruppe bestehend aus F, Cl, Br, Methyl, Methoxy, und CN.

Besonders bevorzugt sind erfindungsgemäße Verbindungen der Formel (I) bzw. (Ia), oder deren Salze, in denen gilt:
n = 1, 2 oder 3, und mindestens ein R² ist ausgewählt, vorzugsweise sind sämtliche R² ausgewählt, aus der Gruppe bestehend aus Fluor, Chlor und Brom,
   und/oder
m = 1, 2 oder 3, und mindestens ein R³ ist ausgewählt, vorzugsweise sind sämtliche R³ ausgewählt, aus der Gruppe bestehend aus Fluor und Chlor.

Erfindungsgemäß ebenfalls bevorzugt sind Verbindungen der Formel (I) und/oder deren Salze, worin (R³)ₘ 3-Chlor, 4-Chlor, 2,3-Dichlor, 2,4-Dichlor, 2,5-Dichlor, 2,6-Dichlor, 3,4-Dichlor, 3,5-Dichlor, 2-Fluor, 3-Fluor, 4-Fluor, 2,3-Difluor, 2,4-Difluor, 2,5-Difluor, 2,6-Difluor, 3,4-Difluor, 3,5-Difluor, 2,3,6-Trifluor, 2,4,6-Trifluor, (3-Cl-2-F), (3-Cl-4-F), (3-Cl-5-F), (3-Cl-6-F), (4-Cl-2-F), (4-Cl-3-F) oder (2,6-F₂-4-Cl) bedeutet.

Erfindungsgemäß ebenfalls bevorzugt sind Verbindungen der Formel (Ia) und/oder deren Salze, worin (R³)ₘ 3-Chlor, 4-Chlor, 2,3-Dichlor, 2,4-Dichlor, 2,5-Dichlor, 2,6-Dichlor, 3,4-Dichlor, 3,5-Dichlor, 2-Fluor, 3-Fluor, 4-Fluor, 2,3-Difluor, 2,4-Difluor, 2,5-Difluor, 2,6-Difluor, 3,4-Difluor, 3,5-Difluor, 2,3,6-Trifluor, 2,4,6-Trifluor, (3-Cl-2-F), (3-Cl-4-F), (3-Cl-5-F), (3-Cl-6-F), (4-Cl-2-F), (4-Cl-3-F) oder (2,6-F₂-4-Cl) bedeutet.

Unter den erfindungsgemäßen Verbindungen mit den vorstehend genannten Bedeutungen sind solche bevorzugt, in denen die jeweils als bevorzugt, jeweils als besonders bevorzugt bzw. jeweils als insbesondere bevorzugt definierten Strukturelemente Q, R¹, (R²)ₙ und (R³)ₘ kombiniert miteinander verwirklicht sind.

Die erfindungsgemäßen Verbindungen der Formel (I) können (2,3)-erythro-Konfiguration aufweisen, d.h. den Verbindungen der nachfolgenden Formeln (I eryrthro-1) und (I eryrthro-2) entsprechen:

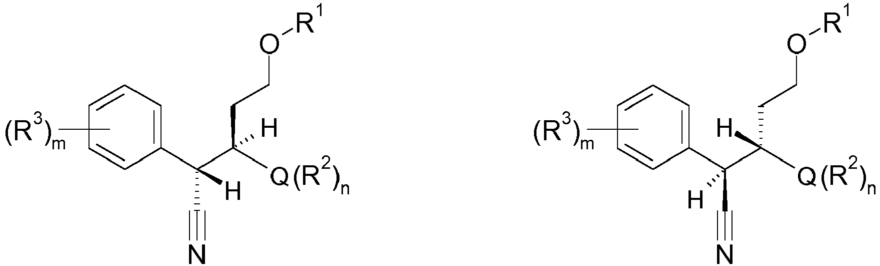

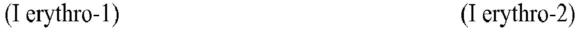

Ferner wurde gefunden, dass die erfindungsgemäßen Verbindungen der Formel (I) und/oder deren Salze bzw. (Ia) und/oder deren Salze, die (2,3)-threo-konfiguriert sind, eine bessere herbizide Eigenschaften aufweisen als die entsprechenden (2,3)-erythro-konfigurierten Verbindungen. Daher sind (2,3)-threo-konfigurierte Verbindungen der Formel (I) bzw. (Ia) sowie deren Salze bevorzugt. Gleiches gilt entsprechend für erfindungsgemäße Mittel.

Erfindungsgemäß entsprechend bevorzugt sind Mischungen und Mittel, in denen das molare Verhältnis einer (2,3)-threo-konfigurierten Verbindung der Formel (I) zu der entsprechenden (2,3)-erythro-konfigurierten Verbindung der Formel (I) größer als 1 ist, weiter bevorzugt größer als 2, besonders bevorzugt größer als 3, insbesondere bevorzugt größer als 4.

Erfindungsgemäß ebenfalls bevorzugt sind Mischungen und Mittel, in denen das Gewichtsverhältnis der Gesamtmenge an (2,3)-threo-konfigurierten Verbindungen der Formeln (I) bzw. (Ia) zu der Gesamtmenge an (2,3)-erythro-konfigurierten Verbindungen der Formeln (I) bzw. (Ia) größer als 1 ist, weiter bevorzugt größer als 2, besonders bevorzugt größer als 3, insbesondere bevorzugt größer als 4.

Überraschenderweise wurde ferner gefunden, dass optisch aktive threo-konfigurierte Verbindungen der Formel (I) und deren Salze besonders gute herbizide Wirkungen bei zugleich vorteilhaften Selektivitäten gegenüber einigen Nutzpflanzen aufweisen.

Dementsprechend sind im Rahmen der vorliegenden Erfindung Verbindungen der Formel (I) und deren Salze bevorzugt, welche (2,3)-threo-Konfiguration aufweisen, d.h. Verbindungen der nachfolgenden Formeln (I threo-1) und (I threo-2) 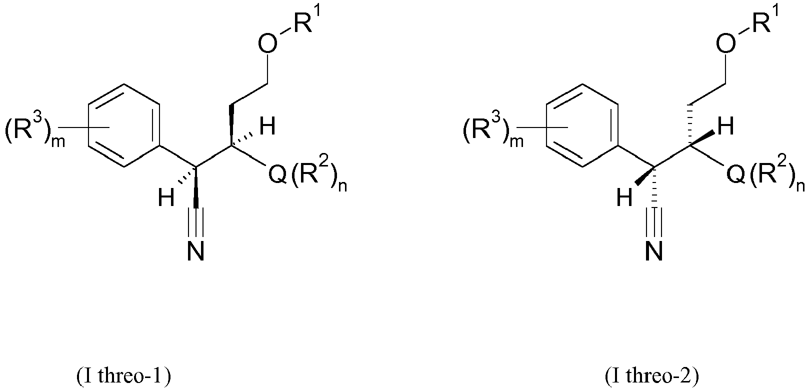 worin
R¹, R², R³, n und m die jeweils vorstehend genannte Bedeutung haben, vorzugsweise eine der jeweils als bevorzugt bzw. besonders bevorzugt angegebenen Bedeutungen.

Im Rahmen der vorliegenden Erfindung Verbindungen der Formel (Ia) und deren Salze bevorzugt, welche (2,3)-threo-Konfiguration aufweisen, d.h. Verbindungen der nachfolgenden Formeln (Ia threo-1) und/oder (Ia threo-2) 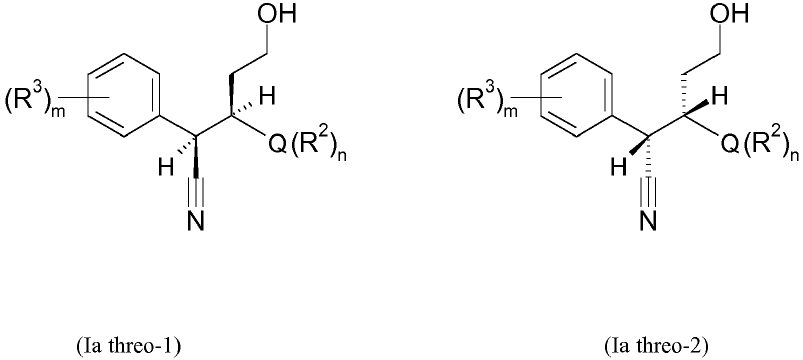 worin
R², R³, n und m die jeweils vorstehend genannte Bedeutung haben, vorzugsweise eine der jeweils als bevorzugt bzw. besonders bevorzugt bzw. insbesondere bevorzugt angegebenen Bedeutungen.

Die stereochemische Konfiguration am C-Atom in Position 2 des Pentanonitrilderivats der Formeln (I) bzw. (Ia) weist vorzugsweise eine stereochemische Reinheit von 60 bis 100 % (*S*) auf, bevorzugt von 70 bis 100 % (*S*), weiter bevorzugt von 80 bis 100 % (*S*), insbesondere bevorzugt von 90 bis 100 % (*S*), und die stereochemische Konfiguration am C-Atom in Position 3 des Pentanonitrilderivats weist vorzugsweise eine stereochemische Reinheit von 60 bis 100 % (*S*) auf, bevorzugt von 70 bis 100 % (*S*), weiter bevorzugt von 80 bis 100 % (*S*), insbesondere bevorzugt von 90 bis 100 % (*S*), jeweils bezogen auf die Gesamtmenge der betreffenden threo-Enantiomeren.

Die stereochemische Konfiguration am C-Atom in Position 2 des Pentanonitrilderivats der Formeln (I) bzw. (Ia) weist vorzugsweise eine stereochemische Reinheit von 60 bis 100 % (*R*) auf, bevorzugt von 70 bis 100 % (*R*), weiter bevorzugt von 80 bis 100 % (*R*), insbesondere bevorzugt von 90 bis 100 % (*R*), und die stereochemische Konfiguration am C-Atom in Position 3 des Pentanonitrilderivats weist vorzugsweise eine stereochemische Reinheit von 60 bis 100 % (R) auf, bevorzugt von 70 bis 100 % (*R*), weiter bevorzugt von 80 bis 100 % (*R*), insbesondere bevorzugt von 90 bis 100 % (*R*), jeweils bezogen auf die Gesamtmenge der betreffenden threo-Enantiomeren.

Aufgrund ihres noch besseren Eigenschaftsprofils, insbesondere ihrer noch höheren herbiziden Wirkung, sind insbesondere die (2*R*,3*R*)-konfigurierten Pentanonitrilderivate der Formeln (I) bzw. (Ia) bevorzugt, vorzugsweise in den vorstehend angegebenen stereochemischen Reinheiten. Folglich sind Verbindungen der Formeln (I threo-1) bzw. (Ia threo-1) im Rahmen der vorliegenden Erfindung beonders bevorzugt. Die absolute Konfiguration dieser (2*R*,3*R*)-konfigurierten Pentanonitrilderivate wurde mittels Röntgenstrukturanalyse zugeordnet.

Das vorstehend Gesagte gilt entsprechend für erfindungsgemäße Mischungen und erfindungsgemäße Mittel.

Die erfindungsgemäßen Verbindungen der Formel (I) können durch verschiedene Verfahren hergestellt werden.

In den nachfolgend beschriebenen Verfahren werden zum Teil Lösemittel (gleichbedeutend mit "Lösungsmittel") verwendet. In diesem Zusammenhang bezeichnen "inerte Lösemittel" jeweils Lösemittel, die unter den jeweiligen Reaktionsbedingungen inert sind, jedoch nicht unter beliebigen Reaktionsbedingungen inert sein müssen. In den nachfolgenden Verfahren können die beschriebenen Reaktionen alternativ auch in einem Mikrowellenreaktor durchgeführt werden.

Die erfindungsgemäßen Verbindungen können nach an sich bekannten Methoden hergestellt werden. Dabei kann beispielsweise von Verbindungen der Formel (E) ausgegangen werden. Verbindungen der Formel (E) sind aus dem Stand der Technik bekannt, z.B. aus WO 2014/095879 A1. Das nachfolgende Schema zeigt exemplarisch, wie die erfindungsgemäßen Verbindungen hergestellt werden können.

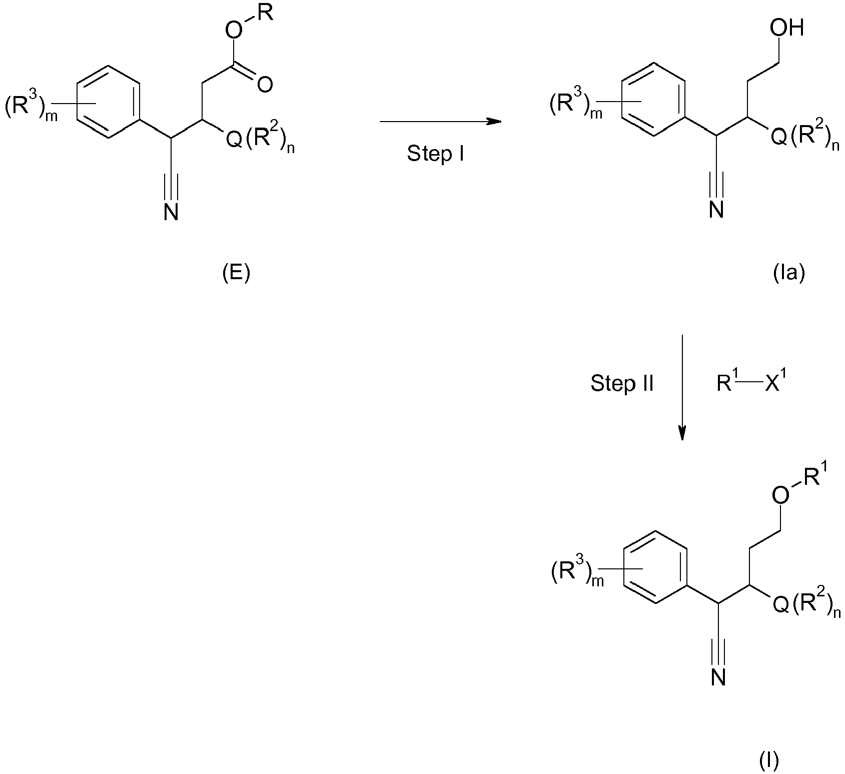

Q, R¹, R², R³, n und m haben dabei jeweils die oben genannte (bevorzugte bzw. besonders bevorzugte) Bedeutung, und R bedeutet Wasserstoff oder einen organischen Rest, vorzugsweise einen Rest ausgewählt aus der Gruppe der vorstehend für R¹ definierten Reste.

Zu Schritt "Step 1" des vorstehenden Schemas:
Methoden zur selektiven Reduktion von Verbindungen der Formel (E) zu entsprechenden Verbindungen der Formel (Ia) sind dem Fachmann aus dem Stand der Technik bekannt und beispielsweise in European Journal of Organic Chemistry 2009, (11), 1806-1811 oder in WO 2012/076513 A1 beschrieben. Vorzugsweise wird die selektive Reduktion vorliegend unter Verwendung von Borhydriden durchgeführt, bevorzugt unter Verwendung von Borhydriden von Alkalimetallborhydriden, insbesondere von Lithium-, Natrium- oder Kaliumborhydrid, dabei wiederum bevorzugt in Gegenwart eines Lithiumhalogenids, bevorzugt in Gegenwart von Lithiumchlorid. Diese Reduktion kann dabei in einem protischen organischen Lösungsmittel, wie beispielsweise Ethanol (siehe beispielsweise WO 2011/014383), oder einem aprotischen organischen Lösungsmittel, wie beispielsweise Tetrahydrofuran (THF), durchgeführt werden, dabei vorzugsweise durch Erhitzen unter Rückfluss.

Zu Schritt "Step 2" des vorstehenden Schemas:
Die vorliegend optionale, weitere Umsetzung (nukleophile Substitution) des in Schritt "Step 1" erhaltenen primären Alkohols (Ia) mit R¹-X¹ (wobei X¹ eine Abgangsgruppe bedeutet) zu Verbindungen der Formel (I) ist dem Fachmann grundsätzlich bekannt und beispielsweise in Journal of Organic Chemistry 2011, 76(3), 811-819 beschrieben. Die nukleophile Substitution wird dabei vorzugsweise in Gegenwart von 4-(Dimethylamino)pyridin durchgeführt. Die Reaktion kann beispielweise auch in einem aprotischen organischen Lösungsmittel wie Tetrahydrofuran (THF) oder Dichlormethan durchgeführt werden, gegebenenfalls bei erhöhter Temperatur, z.B. durch Erhitzen unter Rückfluss.

Die vorliegende Erfindung betrifft entsprechend auch ein Verfahren zur Herstellung einer erfindungsgemäßen Verbindung der Formel (I) und/oder deren Salz, dadurch gekennzeichnet, dass eine Verbindung der Formel (E) 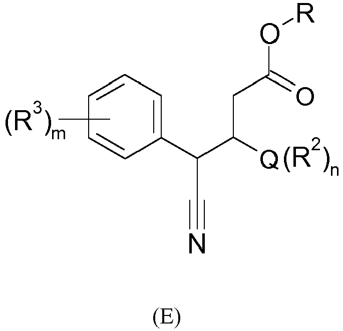 mittels Reduktion zu einer Verbindung der Formel (Ia) 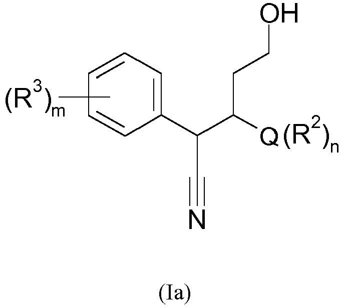 umgesetzt wird, und Verbindung (Ia) gegebenenfalls weiter zu einer Verbindung der Formel (I) umgesetzt wird, sofern R¹ darin nicht Wasserstoff ist,
wobei
R Wasserstoff oder ein organischer Rest ist, vorzugsweise ein Rest ausgewählt aus der Gruppe der vorstehend für R¹ definierten Reste, vorzugsweise ausgewählt aus einer der als bevorzugt gekennzeichneten Reste,
   und
R², R³, m und n jeweils vorstehend definierte Bedeutung haben, und vorzugsweise jeweils ausgewählt sind aus einer der als bevorzugt gekennzeichneten Gruppen an Resten.

Die für die Herstellung der Verbindungen der Formel (Ia) bzw. (I) eingesetzten Ausgangsstoffe (E) sind gemäß der zitierten Literatur bekannt oder können analog der zitierten Literatur hergestellt werden.

Zur Herstellung der erfindungsgemäßen Verbindungen (Ia) bzw. (I) können auch die entsprechenden Diastereomerengemische in Form ihrer racemischen Gemische verwendet werden.

Sofern stereochemisch angereicherte Verbindungen der oben genannten Formel (E) in das erfindungsgemäße Verfahren eingesetzt werden, können die entsprechenden stereochemisch angereicherten Verbindungen der Formel (Ia) bzw. (I) erhalten werden.

Geeignete Lösungsmittel hierfür sind beispielsweise organische Lösungsmittel wie:
- aliphatische Kohlenwasserstoffe wie Pentan, Hexan, Cyclohexan oder Petrolether;
- aromatische Kohlenwasserstoffe wie Toluol, o-, m- oder p-Xylol,
- halogenierte Kohlenwasserstoffe wie Methylenchlorid, Chloroform oder Chlorbenzol,
- Ether wie Diethylether, Diisopropylether, tert.-Butylmethylether, Dioxan, Anisol und Tetrahydrofuran (THF),
- Nitrile wie Acetonitril oder Propionitril,
- Ketone wie Aceton, Methylethylketon, Diethylketon oder tert.-Butylmethylketon,
- Dimethylsulfoxid, Dimethylformamid, Dimethylacetamid, Sulfolan, sowie
- Gemische aus den genannten organischen Lösungsmitteln.

Geeignet sind in Einzelfällen auch anorganische Lösungsmittel wie Wasser oder Gemische organischer Lösungsmittel mit Wasser.

Geeignete Bedingungen und Katalysatoren für die Herstellung von Verbindungen der Formel (I), in den R¹ nicht H ist, aus Verbindungen der Formel (Ia), beispielsweise duch Veresterung, sind dem Fachmann bekannt.

Die Umsetzung von Verbindungen der Formel (Ia) zu Verbindungen der Formel (I), in den R¹ nicht H ist, kann in Gegenwart einer Base erfolgen, beispielsweise einer Base aus der Gruppe der anorganischer Basen wie der Alkalimetall- und Erdalkalimetallhydroxide, zum Beispiel (z.B.) Lithiumhydroxid, Natriumhydroxid, Kaliumhydroxid oder Calciumhydroxid, der Alkalimetall- und Erdalkalimetalloxide, zum Beispiel Lithiumoxid, Natriumoxid, Calziumoxid oder Magnesiumoxid, der Alkalimetall- und Erdalkalimetallhydride, z.B. Lithiumhydrid, Natriumhydrid, Kaliumhydrid oder Calziumhydrid, der Alkalimetallamide, zum Beispiel Lithiumamid, Natriumamid oder Kaliumamid, der Alkalimetall- und Erdalkalimetallcarbonate, z.B. Lithiumcarbonat, Kaliumcarbonat oder Calciumcarbonat, der Alkalimetallhydrogencarbonate, z.B. Natriumhydrogencarbonat, oder der metallorganischen Verbindungen wie vorzugsweise der Alkalimetallalkyle, z.B. Methyllithium, Butyllithium oder Phenyllithium, der Alkylmagnesiumhalogenide, z.B. Methylmagnesiumchlorid, oder der Alkalimetall- und Erdalkalimetallalkoholate, z.B. Natriummethanolat, Natriumethanolat, Kaliumethanolat, Kalium-tert-butanolat oder Dimethoxymagnesium.

Auch können als Basen organische Basen eingesetzt werden, beispielsweise aus der Gruppe der tertiären aliphatischen Amine, z.B. Trimethylamin, Triethylamin, Tributylamin, Diisopropylethylamin oder N-Methylpiperidin, oder der aromatischen tertiären Amine, z.B. Pyridin oder substituierte Pyridine wie Collidin, Lutidin oder 4-Dimethylaminopyridin, oder der bicyclische Amine wie 7-Methyl-1,5,7-triazabicyclo[4.4.0]- dec-5-en oder 1,8-Diazabicyclo[5.4.0]undec-7en (DBU).

Bevorzugte Basen sind beispielsweise Lithiumhydroxid, Kaliumcarbonat, Kalium-tert-butanolat, Lithium-bis(trimethylsilyl)amid, Pyridine, subsitutierte Pyridine, 7-Methyl-1,5,7-triazabicyclo[4.4.0]-dec-5-en oder DBU.

Die Menge an Base kann im Allgemeinen breit variiert weren. Beispielsweise kann es sinnvoll sein, die Base in katalytischen Mengen, im Unterschuss, äquimolar oder im Überschuß einzusetzen. Eine vorzugsweise flüssige organische Base kann gegebenenfalls auch als Lösungsmittel verwendet werden.

Geeignete Katalysatoren für die Umsetzung von Verbindungen der Formel (Ia) zu Verbindungen der Formel (I), in den R¹ nicht H ist, können auch saure Katalysatoren sein, bespielsweise aus der Gruppe der anorganischen Säuren, z.B. Broensted-Säuren, wie Fluorwasserstoffsäure, Salzsäure, Bromwasserstoffsäure, Schwefelsäure oder Perchlorsäure, oder Lewis-Säuren wie Bortrifluorid, Aluminiumtrichlorid, Eisen-III-chlorid, Zinn-IV-chlorid, Titan-IV-chlorid, Scandium-III-triflat oder Zink-II-chlorid, sowie der organischen Säuren, z.B. Ameisensäure, Essigsäure, Propionsäure, Oxalsäure, Toluolsulfonsäure, Benzolsulfonsäure, Camphersulfonsäure, Citronensäure oder Trifluoressigsäure.

Die erhaltenen Reaktionsgemische werden in üblicher Weise aufgearbeitet, z.B. durch Mischen mit Wasser, Trennung der Phasen und gegebenenfalls chromatographische Reinigung der Rohprodukte. Die Verbindungen fallen z.T. in Form farbloser oder schwach bräunlicher, zäher Öle an, die unter vermindertem Druck und bei mäßig erhöhter Temperatur von flüchtigen Anteilen befreit oder gereinigt werden.

Sofern die Verbindungen als Feststoffe erhalten werden, kann die Reinigung auch durch Umkristallisieren oder Digerieren erfolgen. Sofern einzelne Verbindungen (I) nicht auf den voranstehend beschriebenen Wegen zufriedenstellend zugänglich sind, können sie durch Derivatisierung anderer Verbindungen (I) bzw. (Ia) hergestellt werden.

Als Isolierungs-, Reinigungs- und Stereoisomerenauftrennungsverfahren von Verbindungen der Formel (I) bzw. (Ia) kommen Methoden in Frage, die dem Fachmann aus analogen Fällen allgemein bekannt sind, z.B. durch physikalische Verfahren wie Kristallisation, Chromatographieverfahren, vor allem Säulenchromatographie und HPLC (Hochdruckflüssigchromatographie), Destillation, gegebenenfalls unter reduziertem Druck, Extraktion und andere Verfahren, können gegebenfalls verbleibende Gemische in der Regel durch chromatographische Trennung, z.B. an chiralen Festphasen, getrennt werden. Für präparative Mengen oder im industriellen Maßstab kommen Verfahren in Frage wie Kristallisation, z.B. diastereomerer Salze, die aus den Diastereomerengemischen mit optisch aktiven Säuren und gegebenenfalls bei vorhandenen sauren Gruppen mit optisch aktiven Basen erhalten werden können.

Zur Racemattrennung durch Kristallisation diastereomerer Salze kommen als optisch aktive Säure z.B. Camphersulfonsäure, Camphersäure, Bromcamphersulfonsäure, Chinasäure, Weinsäure, Dibenzoylweinsäure und andere analoge Säure in Betracht; als optisch aktive Basen kommen z.B. Chinin, Cinchonin, Chinidin, Brucin, 1-(S)- oder 1-(R)-Phenylethylamin und andere analoge Basen in Frage.

Die Kristallisationen werden dann meist in wässrigen, alkoholischen oder wässrig-organischen Lösungsmittel durchgeführt, wobei das Diastereomer mit der geringeren Löslichkeit gegebenenfalls nach Animpfen zunächst ausfällt. Das eine Enantiomer der Verbindung der Formel (I) wird danach aus dem ausgefällten Salz oder das andere aus dem Kristallisat durch Ansäuern bzw. mit Base freigesetzt.

Alternativ zu den genannten Racemattrennungsverfahren eignen sich zur Herstellung der (threo-)Enantiomeren prinzipiell auch enantioselektive Verfahren ausgehend von stereochemisch reinen Ausgangsstoffen.

Zur Herstellung der Säureadditionssalze der Verbindungen der Formel (I) kommen allgemein folgende Säuren in Frage: Halogenwasserstoffsäuren wie Chlorwasserstoffsäure oder Bromwasserstoffsäure, weiterhin Phosphorsäure, Salpetersäure, Schwefelsäure, mono- oder bifunktionelle Carbonsäuren und Hydroxycarbonsäuren wie Essigsäure, Maleinsäure, Bernsteinsäure, Fumarsäure, Weinsäure, Citronensäure, Salicylsäure, Sorbinsäure oder Milchsäure, sowie Sulfonsäuren wie p-Toluolsulfonsäure oder 1,5-Naphtalindisulfonsäure. Die Säureadditionsverbindungen der Formel (I) können in an sich bekannter Weise nach den üblichen Salzbildungsmethoden erhalten werden, z.B. durch Lösen einer Verbindung der Formel (I) in einem geeigneten organischen Lösungsmittel wie z.B. Methanol, Aceton, Methylenchlorid oder Benzol und Hinzufügen der Säure bei Temperaturen von 0 bis 100 °C und in bekannter Weise, z.B. durch Abfiltrieren, isoliert und gegebenfalls durch Waschen mit einem inerten organischen Lösemittel gereinigt werden.

Basenadditionssalze der Verbindungen der Formel (I) können beispielsweise in polaren Lösungsmitteln wie z.B. Wasser, Methanol oder Aceton bei Temperaturen von 0 bis 100 °C hergestellt werden. Geeignete Basen zur Herstellung der solcher Salze sind beispielsweise Alkalicarbonate, wie Kaliumcarbonat, Alkali- und Erdalkalihydroxide, z.B. NaOH oder KOH, Alkali- und Erdalkalihydride, z.B. NaH, Alkali- und Erdalalkoholate, z.B. Natriummethanolat oder Kalium-tert.-butylat, oder Ammoniak, Ethanolamin oder quartäres Ammoniumhydroxid.

Eine Kollektion aus Verbindungen der Formel (I) können zusätzlich in parallelisierter oder kombinatorischer Weise hergestellt werden, wobei dies in manueller, teilweise automatisierter oder vollständig automatisierter Weise geschehen kann. Dabei ist es möglich, sowohl die Reaktionsdurchführung, die Aufarbeitung oder die Reinigung der Produkte bzw. Zwischenstufen zu automatisieren.

Zur parallelisierten Reaktionsdurchführung und Aufarbeitung können eine Reihe von im Handel erhältlichen Geräten verwendet werden wie sie beispielsweise von den Firmen Stem Corporation, Woodrolfe Road, Tollesbury, Essex, CM9 8SE, England oder H + P Labortechnik GmbH, Bruckmannring 28, 85764 Oberschleißheim, Deutschland angeboten werden. Für die parallelisierte Aufreinigung von Verbindungen (I) oder von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA. Die aufgeführten Apparaturen ermöglichen eine modulare Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise von Roboter bedient werden. Derartige Automationssysteme können zum Beispiel von der Firma Zymark Corporation, Zymark Center, Hopkinton, MA 01748, USA bezogen werden.

Neben den beschriebenen Methoden kann die Herstellung von Verbindungen der Formel (I) vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepaßten Synthese an ein Syntheseharz gebunden. Festphasen unterstützte Synthesemethoden sind in der Fachliteratur hinreichend beschrieben, z.B.: Barry A. Bunin in "The Combinatorial Index", Verlag Academic Press, 1998.

Die Verwendung von Festphasen unterstützten Synthesemethoden erlaubt eine Reihe von literaturbekannten Protokollen, die wiederum manuell oder automatisiert ausgeführt werden können. Zum Beispiel kann die sogenannte "Teebeutelmethode" (US 4,631,211) mit Produkten der Firma IRORI, 11149 North Torrey Pines Road, La Jolla, CA 92037, USA teilweise automatisiert werden. Die Automatisierung von Festphasen unterstützter Parallelsynthese gelingt beispielsweise durch Apparaturen der Firmen Argonaut Technologies, Inc., 887 Industrial Road, San Carlos, CA 94070, USA oder MultiSynTech GmbH, Wullener Feld 4, 58454 Witten, Deutschland.

Die Herstellung gemäß den hier beschriebenen Verfahren liefert Verbindungen der Formel (I) in Form von Substanzkollektionen oder -bibliotheken. Gegenstand der vorliegenden Erfindung sind daher auch Bibliotheken der Verbindungen der Formel (I), die mindestens zwei Verbindungen der Formel (I) enthalten, und deren Vorprodukte.

Gegenstand der vorliegenden Erfindung ist ferner ein Verfahren zur Bekämpfung von Schadpflanzen und/oder zur Wachstumsregulierung von Pflanzen, dadurch gekennzeichnet, dass eine wirksame Menge
- einer oder mehrerer Verbindungen der Formel (I) und/oder deren Salzen, wie oben definiert,
- einer oder mehrerer Verbindungen der Formel (Ia) und/oder deren Salzen, wie oben definiert, oder
- eines Mittels, wie oben definiert,
auf die (Schad)Pflanzen, (Schad)Pflanzensamen, den Boden, in dem oder auf dem die (Schad)Pflanzen wachsen, oder die Anbaufläche appliziert wird.

Gegenstand der vorliegenden Erfindung ist auch ein Verfahren zur Bekämpfung von unerwünschten Pflanzen, vorzugsweise in Nutzpflanzenkulturen, dadurch gekennzeichnet, dass eine wirksame Menge
- einer oder mehrerer Verbindungen der Formel (I) und/oder deren Salzen, wie oben definiert,
- einer oder mehrerer Verbindungen der Formel (Ia) und/oder deren Salzen, wie oben definiert, oder
- eines erfindungsgemäßen Mittels, wie oben definiert,

auf unerwünschte Pflanzen (z.B. Schadpflanzen wie mono- oder dikotyle Unkräuter oder unerwünschte Kulturpflanzen), das Saatgut der unerwünschten Pflanzen (d.h. Pflanzensamen, z.B. Körner, Samen oder vegetative Vermehrungsorgane wie Knollen oder Sprossteile mit Knospen), den Boden, in dem oder auf dem die unerwünschte Pflanzen wachsen, (z.B. den Boden von Kulturland oder Nicht-Kulturland) oder die Anbaufläche (d.h. Fläche, auf der die unerwünschte Pflanzen wachsen werden) appliziert wird.

Gegenstand der vorliegenden Erfindung ist ferner auch Verfahren zur Bekämpfung zur Wachstumsregulierung von Pflanzen, vorzugsweise von Nutzpflanzen, dadurch gekennzeichnet, dass eine wirksame Menge
- einer oder mehrerer Verbindungen der Formel (I) und/oder deren Salzen, wie oben definiert,
- einer oder mehrerer Verbindungen der Formel (Ia) und/oder deren Salzen, wie oben definiert, oder
- eines erfindungsgemäßen Mittels, wie oben definiert,
die Pflanze, das Saatgut der Pflanze (d.h. Pflanzensamen, z.B. Körner, Samen oder vegetative Vermehrungsorgane wie Knollen oder Sprossteile mit Knospen), den Boden, in dem oder auf dem die Pflanzen wachsen, (z.B. den Boden von Kulturland oder Nicht-Kulturland) oder die Anbaufläche (d.h. Fläche, auf der die Pflanzen wachsen werden) appliziert wird.

Dabei können die erfindungsgemäßen Verbindungen bzw. die erfindungsgemäßen Mittel z.B. im Vorsaat- (ggf. auch durch Einarbeitung in den Boden), Vorauflauf- und/oder Nachauflaufverfahren ausgebracht werden. Im einzelnen seien beispielhaft einige Vertreter der mono- und dikotylen Unkrautflora genannt, die durch die die erfindungsgemäßen Verbindungen kontrolliert werden können, ohne dass durch die Nennung eine Beschränkung auf bestimmte Arten erfolgen soll.

Vorzugsweise werden in einem erfindungsgemäßen Verfahren zur Bekämpfung von Schadpflanzen oder zur Wachstumsregulierung von Pflanzen eine oder mehrere Verbindungen der Formel (I), (Ia) und/oder deren Salze zur Bekämpfung von Schadpflanzen oder zur Wachstumsregulierung in Kulturen von Nutzpflanzen oder Zierpflanzen eingesetzt, wobei die Nutzpflanzen oder Zierpflanzen in einer bevorzugten Ausgestaltung transgene Pflanzen sind.

Die erfindungsgemäßen Verbindungen Formel (I) bzw. (Ia) und/oder deren Salze eignen sich zur Bekämpfung der folgenden Gattungen von monokotylen und dikotylen Schadpflanzen:
Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Schadpflanzen der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen der Schadpflanzen (Ungräser und/oder Unkräuter) auf die Erdoberfläche appliziert (Vorauflaufverfahren), so wird entweder das Auflaufen der Ungras- bzw. Unkrautkeimlinge vollständig verhindert oder diese wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativem Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen und/oder deren Salze in wirtschaftlich bedeutenden transgenen Kulturen von Nutz und Zierpflanzen, z.B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.

Vorzugsweise können die erfindungsgemäßen Verbindungen auch als Herbizide in Nutzpflanzenkulturen eingesetzt werden, welche gegenüber den phytotoxischen Wirkungen der Herbizide resistent sind bzw. gentechnisch resistent gemacht worden sind.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z.B. Hitze, Kälte, Trockenheit, Salz und ultraviolette Strahlung liegen.

Bevorzugt ist die Anwendung der erfindungsgemäßen Verbindungen der Formel (I) oder deren Salze in wirtschaftlich bedeutenden transgenen Kulturen von Nutz-und Zierpflanzen, z.B. von Getreide wie Weizen, Gerste, Roggen, Hafer, Triticale, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden.

Zahlreiche molekularbiologische Techniken, mit denen neue transgene Pflanzen mit veränderten Eigenschaften hergestellt werden können, sind dem Fachmann bekannt. Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z.B. Dicamba oder gegen Herbizide, die essentielle Pflanzenenzyme, z.B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) und/oder deren Salze als Herbizide zur Bekämpfung von Schadpflanzen in Kulturen von Nutz- oder Zierpflanzen, gegebenenfalls in transgenen Kulturpflanzen.

Bevorzugt ist die Verwendung in Getreide, dabei vorzugsweise Mais, Weizen, Gerste, Roggen, Hafer, Hirse, oder Reis, im Vor- oder Nachauflauf.

Bevorzugt ist auch die Verwendung in Soja im Vor- oder Nachauflauf.

Die erfindungsgemäße Verwendung zur Bekämpfung von Schadpflanzen oder zur Wachstumsregulierung von Pflanzen schließt auch den Fall ein, bei dem der Wirkstoff der Formel (I) oder dessen Salz erst nach der Ausbringung auf der Pflanze, in der Pflanze oder im Boden aus einer Vorläufersubstanz ("Prodrug") gebildet wird.

Gegenstand der Erfindung ist auch die Verwendung einer oder mehrerer Verbindungen der Formel (I) oder deren Salzen bzw. eines erfindungsgemäßen Mittels (wie nachstehend definiert) (in einem Verfahren) zur Bekämpfung von Schadpflanzen oder zur Wachstumsregulierung von Pflanzen, dadurch gekennzeichnet, dass man eine wirksame Menge einer oder mehreren Verbindungen der Formel (I) oder deren Salzen auf die Pflanzen (Schadpflanzen, ggf. zusammen mit den Nutzpflanzen) Pflanzensamen, den Boden, in dem oder auf dem die Pflanzen wachsen, oder die Anbaufläche appliziert.

Gegenstand der Erfindung ist auch ein herbizides oder pflanzenwachstumsregulierendes Mittel, dadurch gekennzeichnet, dass das Mittel
(a) eine oder mehrere Verbindungen der Formel (I) und/oder deren Salze enthält wie oben definiert, vorzugsweise eine oder mehrere Verbindungen der Formel (Ia) und/oder deren Salze, jeweils wie oben definiert,
   und
(b) ein oder mehrere weitere Stoffe ausgewählt aus den Gruppen (i) und/oder (ii):
   (i) ein oder mehrere weitere agrochemisch wirksame Stoffe, vorzugsweise ausgewählt aus der Gruppe bestehend aus Insektiziden, Akariziden, Nematiziden, weiteren Herbiziden (d.h. solche, die nicht der oben definierten Formel (I) entsprechen), Fungiziden, Safenern, Düngemitteln und/oder weiteren Wachstumsregulatoren,
   (ii) ein oder mehrere im Pflanzenschutz übliche Formulierungshilfsmittel.

Ein erfindungsgemäßes herbizides oder pflanzenwachstumsregulierendes Mittel, umfasst vorzugsweise ein, zwei, drei oder mehr im Pflanzenschutz übliche Formulierungshilfsmittel (ii) ausgewählt aus der Gruppe bestehend aus Tensiden, Emulgatoren, Dispergiermitteln, Filmbildnern, Verdickungsmitteln, anorganischen Salzen, Stäubemitteln, bei 25 °C und 1013 mbar festen Trägerstoffen, vorzugsweise adsorptionsfähigen, granulierten Inertmaterialien, Netzmitteln, Antioxidationsmitteln, Stabilisatoren, Puffersubstanzen, Antischaummitteln, Wasser, organischen Lösungsmitteln, vorzugsweise bei 25 °C und 1013 mbar mit Wasser in jedem beliebigen Verhältnis mischbare organische Lösungsmittel.

Die erfindungsgemäßen Verbindungen (I) können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) und/oder deren Salze enthalten.

Die Verbindungen der Formel (I) und/oder deren Salze können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen und die Formulierungshilfsmittel wie Inertmaterialien, Tenside, Lösungsmittel und weitere Zusatzstoffe sind dem Fachmann bekannt, und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesellschaft, Stuttgart 1976; Winnacker-Küchler, "Chemische Technologie", Band 7, C. Hanser Verlag München, 4. Aufl. 1986.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalinsulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calcium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulaten siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen, vorzugsweise herbizide oder pflanzenwachstumsregulierende Mittel der vorliegenden Erfindung enthalten vorzugsweise eine Gesamtmenge von 0,1 bis 99 Gew.-%, bevorzugt 0,5 bis 95 Gew.-%, weiter bevorzugt 1 bis 90 Gew.-%, insbesondere bevorzugt 2 bis 80 Gew.-%, an Wirkstoffen der Formel (I) und deren Salzen.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel. Beispiele für Formulierungshilfsmittel sind unter anderem in "Chemistry and Technology of Agrochemical Formulations", ed. D. A. Knowles, Kluwer Academic Publishers (1998) beschrieben.

Die Verbindungen der Formel (I) oder deren Salze können als solche oder in Form ihrer Zubereitungen (Formulierungen) mit anderen pestizid wirksamen Stoffen, wie z.B. Insektiziden, Akariziden, Nematiziden, Herbiziden, Fungiziden, Safenern, Düngemitteln und/oder Wachstumsregulatoren kombiniert eingesetzt werden, z.B. als Fertigformulierung oder als Tankmischungen. Die Kombinationsformulierungen können dabei auf Basis der obengenannten Formulierungen hergestellt werden, wobei die physikalischen Eigenschaften und Stabilitäten der zu kombinierenden Wirkstoffe zu berücksichtigen sind.

Als Kombinationspartner für die erfindungsgemäßen Verbindungen der Formel (I) in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. in Weed Research 26 (1986) 441-445 oder "The Pesticide Manual", 16th edition, The British Crop Protection Council and the Royal Soc. of Chemistry, 2012 und der dort zitierten Literatur beschrieben sind.

Von besonderem Interesse ist die selektive Bekämpfung von Schadpflanzen in Kulturen von Nutz-und Zierpflanzen. Obgleich die erfindungsgemäßen Verbindungen (I) bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen erfindungsgemäßer Verbindungen (I) von besonderem Interesse, welche die Verbindungen (I) bzw. deren Kombinationen mit anderen Herbiziden oder Pestiziden und Safenern enthalten. Die Safener, welche in einem antidotisch wirksamen Gehalt eingesetzt werden, reduzieren die phytotoxischen Nebenwirkungen der eingesetzten Herbizide/Pestizide, z.B. in wirtschaftlich bedeutenden Kulturen wie Getreide (Weizen, Gerste, Roggen, Mais, Reis, Hirse), Zuckerrübe, Zuckerrohr, Raps, Baumwolle und Soja, vorzugsweise Getreide.

Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200:1 bis 1:200, vorzugsweise 100:1 bis 1:100, insbesondere 20:1 bis 1:20. Die Safener können analog den Verbindungen (I) oder deren Mischungen mit weiteren Herbiziden/Pestiziden formuliert werden und als Fertigformulierung oder Tankmischung mit den Herbiziden bereitgestellt und angewendet werden.

Zur Anwendung werden die in handelsüblicher Form vorliegenden Herbizid- oder Herbizid-Safener-Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Äußere Bedingungen wie Temperatur, Feuchtigkeit etc. beeinflussen zu einem gewissen Teil die Aufwandmenge der Verbindungen der Formel (I) und/oder deren Salze. Die Aufwandmenge kann dabei innerhalb weiter Grenzen variieren. Für die Anwendung als Herbizid zur Bekämpfung von Schadpflanzen liegt die Gesamtmenge an Verbindungen der Formel (I) und deren Salze vorzugsweise im Bereich von 0,001 bis 10,0 kg/ha, bevorzugt im Bereich von 0,005 bis 5 kg/ha, weiter bevorzugt im Bereich von 0,01 bis 1,5 kg/ha, insbesondere bevorzugt im Bereich von 0,05 bis 1 kg/ha. Dies gilt sowohl für die Anwendung im Vorauflauf oder im Nachauflauf, wobei die Anwendung im Vorauflauf wegen der signifikant höheren Wirksamkeit bevorzugt ist.

Bei der Anwendung von Verbindungen der Formel (I) und/oder deren Salzen als Pflanzenwachstumsregulator, beispielsweise als Halmverkürzer bei Kulturpflanzen, wie sie oben genannt worden sind, vorzugsweise bei Getreidepflanzen wie Weizen, Gerste, Roggen, Triticale, Hirse, Reis oder Mais, liegt die Gesamt-Aufwandmenge vorzugsweise im Bereich von 0,001 bis 2 kg/ha, vorzugsweise im Bereich von 0,005 bis 1 kg/ha, insbesondere im Bereich von 10 bis 500 g/ha, ganz besonders bevorzugt im Bereich von 20 bis 250 g/ha. Dies gilt sowohl für die Anwendung im Vorauflauf oder im Nachauflauf.

Die Applikation als Halmverkürzer kann in verschiedenen Stadien des Wachstums der Pflanzen erfolgen. Bevorzugt ist beispielsweise die Anwendung nach der Bestockung am Beginn des Längenwachstums.

Alternativ kommt bei der Anwendung als Pflanzenwachstumsregulator auch die Behandlung des Saatguts in Frage, welche die unterschiedlichen Saatgutbeiz- und Beschichtungstechniken einschließt. Die Aufwandmenge hängt dabei von den einzelnen Techniken ab und kann in Vorversuchen ermittelt werden.

### Beispielteil

### Abkürzungen und Schreibweisen

Die Symbole ">" und "<" bedeuten "größer als" beziehungsweise "kleiner als". Das Symbol "≥" bedeutet "größer als oder gleich", das Symbol "≤" bedeutet "kleiner als oder gleich".

Wenn im Rahmen der Beschreibung und der Beispiele Bezeichnungen "*R*" und "*S*" für die absolute Konfiguration an einem Chiralitätszentrum der Stereoisomeren der Formeln (I) bzw. (Ia) angegeben ist, folgt diese der RS-Nomenklatur der Cahn-Ingold-Prelog-Regeln.

Verwendete Abkürzungen und Schreibweisen:
- Bsp.: = Beispiel Nummer
- H: = Wasserstoff(-atom)
- Me: = Methyl
- Et: = Ethyl
- n-Pr: = n-Propyl
- i-Pr: = Isopropyl
- n-Bu: = n-Butyl
- i-Bu: = Isobutyl
- F, Cl, Br, I: = Fluor, Chlor, Brom bzw. Iod entsprechend den üblichen chemischen Atomsymbolen
- MeO oder OMe: = Methoxy
- CN: = Cyano
- NO₂: = Nitro

Die Position eines Substituenten an einem (Phenyl)Ring, beispielsweise in Position 2 oder Position 3, ist dem Symbol oder der Abkürzung des Restes vorangestellt, z.B.
- 2-Cl: = 2-Chlor
- 2-Me: = 2-Methyl
- 3-NO₂: = 3-Nitro

Nummerierungen der Substituentenpositionen bei di- oder trisubstituiertem Substitutionsmuster sind analog vorangestellt, z.B.
- 2,3,6-F₃ oder 2,3,6-F3: = 2,3,6-Trifluor (z.B. als Substitution am Phenylring)
- 2,3-F₂ oder 2,3-F2: = 2,3-Difluor (z.B. als Substitution am Phenylring)

Andere Abkürzungen sind analog der oben angegebenen Beispiele zu verstehen.
- "(R²)ₙ = "H": = unsubstituierter heteroaromatischer Rest Q (n = 0)
- "(R³)ₘ = "H": = unsubstituierter Phenylring (m = 0)

Im Übrigen gelten die üblichen chemischen Symbole und Formeln, wie z.B. CH₂ für Methylen oder CF₃ für Trifluormethyl oder OH für Hydroxyl. Zusammengesetzte Bedeutungen sind entsprechend aus den genannten Abkürzungen zusammengesetzt definiert.

Nachfolgend sind beispielhaft einige Synthesebeispiele von Verbindungen der Formel (I) beschrieben. In den Beispielen beziehen sich Mengenangaben (auch die Prozentangaben) auf das Gewicht, sofern nichts anderes angegeben ist.

### Synthesebeispiele

### 1a) Herstellung von Methyl-4-(3-brom-4-fluorphenyl)-4-cyan-3-(3,5-difluorpyridin-4-yl)butanoat:

Zu 0,413 g (2,07 mmol) Methyl-(2E)-3-(3,5-difluorpyridin-4-yl)acrylat und 0,443 g (2,07 mmol) (3-Brom-4-fluorphenyl)acetonitril in 21,0 ml Toluol und 1 ml DMF wurden unter Schutzgas (Argon) 46 mg (0,41 mmol) Kalium-tert-butanolat gegeben und 5 h bei 60°C gerührt. Nach Entfernen des Lösemittels unter reduziertem Druck wurde der Rückstand in Ethylacetat aufgenommen und zweimal mit je 50 ml Wasser gewaschen. Die vereinigten organischen Phasen wurden über Natriumsulfat getrocknet und das Lösemittel unter reduziertem Druck entfernt. Nach Chromatographie des Rückstands an Kieselgel (Ethylacetat / Heptan = 15:85) wurden 0,501 g (58% d. Th.) der diastereomeren Methyl-4-(3-brom-4-fluorphenyl)-4-cyan-3-(3,5-difluorpyridin-4-yl)butanoate erhalten.

### 1b) Herstellung von 2-(3-Brom-4-fluorphenyl)-3-(3,5-difluorpyridin-4-yl)-5-hydroxypentannitril:

Zu 0,125 g (2,32 mmol) Kaliumborhydrid und 0,049 g (1,16 mmol) Lithiumchlorid in 2 ml THF wurden unter Schutzgas (Argon) 0,480 g (1,16 mmol) der gemäß Schritt 1a) erhaltenen Methyl-4-(3-brom-4-fluorphenyl)-4-cyan-3-(3,5-difluorpyridin-4-yl)butanoate in 3 ml THF zugegeben und 5 h bei 70°C gerührt. Nach Abkühlung auf 0 °C wurde Wasser zugegeben. Der Rückstand wurde anschließend in Ethylacetat aufgenommen und zweimal mit je 50 ml Wasser gewaschen. Die vereinigten organischen Phasen wurden über Natriumsulfat getrocknet und das Lösemittel unter reduziertem Druck entfernt. Nach Chromatographie des Rückstands an Kieselgel (Ethylacetat / Heptan = 15:85) wurden 73 mg (16% d. Th.) der diastereomeren 2-(3-Brom-4-fluorphenyl)-3-(3,5-difluorpyridin-4-yl)-5-hydroxypentannitrile erhalten (erythro:threo = 49:51, Vergleich der Dubletts im ¹H-NMR in CDCl₃ bei 4,24 und 4,16 ppm).

### 1c) Herstellung von Methyl-4-cyan-4-(3,4-difluorphenyl)-3-(5-fluorpyridin-3-yl)butanoat:

Zu 1,006 g (5,55 mmol) Methyl-(2E)-3-(5-fluorpyridin-3-yl)acrylat und 0,850 g (5,55 mmol) (3,4-Difluorphenyl)acetonitril in 45,0 ml Toluol und 2 ml DMF wurden unter Schutzgas (Argon) 125 mg (1,11 mmol) Kalium-tert-butanolat gegeben und 5 h bei 60°C gerührt. Nach Entfernen des Lösemittels unter reduziertem Druck wurde der Rückstand in Ethylacetat aufgenommen und zweimal mit je 50 ml Wasser gewaschen. Die vereinigten organischen Phasen wurden über Natriumsulfat getrocknet und das Lösemittel unter reduziertem Druck entfernt. Nach Chromatographie des Rückstands an Kieselgel (Ethylacetat / Heptan = 15:85) wurden 1,050 g (57% d. Th.) der diastereomeren Methyl-4-cyan-4-(3,4-difluorphenyl)-3-(5-fluorpyridin-3-yl)butanoate erhalten.

### 1d) Herstellung von 2-(3,4-Difluorphenyl)-3-(5-fluorpyridin-3-yl)-5-hydroxypentannitril:

Zu 0,339 g (6,28 mmol) Kaliumborhydrid und 0,133 g (3,14 mmol) Lithiumchlorid in 10 ml THF wurden unter Schutzgas (Argon) 1,050 g (3,14 mmol) der gemäß Schritt 1c) erhaltenen Methyl-4-cyan-4-(3,4-difluorphenyl)-3-(5-fluorpyridin-3-yl)butanoate in 5 ml THF zugegeben und 5 h bei 70°C gerührt. Nach Abkühlung auf 0 °C wurde Wasser zugegeben. Der Rückstand wurde anschließend in Ethylacetat aufgenommen und zweimal mit je 50 ml Wasser gewaschen. Die vereinigten organischen Phasen wurden über Natriumsulfat getrocknet und das Lösemittel unter reduziertem Druck entfernt. Nach Chromatographie des Rückstands an Kieselgel (Ethylacetat / Heptan = 15:85) wurden 330 mg (35% d. Th.) der diastereomeren 2-(3,4-Difluorphenyl)-3-(5-fluorpyridin-3-yl)-5-hydroxypentannitrile erhalten (erythro:threo = 59:41, Vergleich der Dubletts im ¹H-NMR in CDCl₃ bei 4,29 und 4,04 ppm).

### 1e) Herstellung von (2R,3R)-2-(3,4-Difluorphenyl)-3-(5-fluorpyridin-3-yl)-5-hydroxypentannitril:

Durch präparative Chromatographie [(80 ml/min n-Heptan/2-Propanol (80:20)] von 100 mg des wie oben gemäß Schritt 1d) erhaltenen Diastereomerengemischs (gelöst in 4,0 ml Methanol) an einer chiralen Festphase [Chiralpak IC, 20 µm, (250 x 50)-mm Säule] wurden 19 mg (2R,3R)-2-(3,4-Difluorphenyl)-3-(5-fluorpyridin-3-yl)-5-hydroxypentannitril erhalten, welches als vorletztes der vier Stereoisomere (Retentionszeit: 11,9 min) eluiert wurde. Die absolute Konfiguration dieser verbindung wurde anschließend mittels Röntgenstrukturanalyse zugeordnet.

### 1f) Herstellung von 4-(3-Brom-4-fluorphenyl)-4-cyan-3-(3,5-difluorpyridin-4-yl)butylchloracetat:

Zu 35 mg (0,09 mmol) 2-(3-Brom-4-fluorphenyl)-3-(3,5-difluorpyridin-4-yl)-5-hydroxypentannitril (erhalten gemäß aus obigem Schritt 1b)), 4-(Dimethylamino)pyridin 7 mg (0,05 mmol) und 18 mg (0,18 mmol) Triethylamin in 3 ml Dichlormethan wurden unter Schutzgas (Argon) 21 mg (0,18 mmol) Chloracetylchlorid gegeben und 3 h bei 25°C gerührt. Der Rückstand wurde in Ethylacetat aufgenommen und zweimal mit je 50 ml Wasser gewaschen. Die vereinigten organischen Phasen wurden danach über Natriumsulfat getrocknet und das Lösemittel unter reduziertem Druck entfernt. Nach Chromatographie des Rückstands an Kieselgel (Ethylacetat / Heptan = 15:85) wurden 17 mg (41% d. Th.) der diastereomeren 4-(3-Brom-4-fluorphenyl)-4-cyan-3-(3,5-difluorpyridin-4-yl)butyl-chloracetate erhalten (erythro:threo = 48:52, Vergleich der Singuletts im ¹H-NMR in CDCl₃ bei 8,42 und 8,25 ppm).

### Definition und Beschreibung bevorzugter erfindungsgemäßer Verbindungen - Analytische und physikalische Daten

Nachfolgendend sind erfindungsgemäße Verbindungen der Formel (I) mit entsprechenden einzelnen Nummern (= Beispielnummern) angegeben, wobei die jeweilige Verbindungsbezeichnung sich zusammensetzt aus der Verbindungsnummer der chemischen Formel (I), die sich auf dieselbe Nummer bezieht wie in der jeweiligen Zeile der Tabelle angegeben, d.h. dass die Strukturkombination der Gruppen Q(R²)ₙ und (R³)ₘ gemäß der entsprechenden fortlaufenden Eintragsnummer (nachfolgend "Zeilennummer", wobei die Kopfzeile der Tabelle 1 nicht mitgezählt wird) aus Tabelle 1 definiert ist.

Auf diese Weise wird die jeweilige chemische Struktur einer erfindungsgemäßen Verbindung der Formel (I) eindeutig definiert, was an folgenden Beispielen verdeutlicht werden soll:
Die Verbindungsbezeichnung "215a" identifiziert die Verbindung der Formel (Ia) [entsprechend Formel (I), worin R¹ = H (= Wasserstoff) bedeutet], in der Q(R²)ₙ = 3-Chlorpyridin-2-yl und (R³)ₘ = 3-Cl, 4-F gemäß Zeilennnummer 215 aus Tabelle 1 ist.

Die Verbindungsbezeichnung "296a" identifiziert die Verbindung der Formel (Ia) [entsprechend Formel (I), worin R¹ = H (= Wasserstoff) bedeutet], in der Q(R²)ₙ = 5-Fluorpyridin-3-yl und (R³)ₘ = 3-Cl, 4-F gemäß Zeilennnummer 296 aus Tabelle 1 ist.

Die Verbindungsbezeichnung "599a" identifiziert die Verbindung der Formel (Ia) [entsprechend Formel (I), worin R¹ = H (= Wasserstoff) bedeutet], in der Q(R²)ₙ = 3,5-Difluorpyridin-4-yl und (R³)ₘ = 3-Cl gemäß Zeilennummer 599 aus Tabelle 1 ist.

Die Verbindungsbezeichnung "1295a" identifiziert die Verbindung der Formel (Ia) [entsprechend Formel (I), worin R¹ = H (= Wasserstoff) bedeutet], in der Q(R²)ₙ = 5-Chlorpyrazin-2-yl und (R³)ₘ = 3-Cl, 4-F gemäß Zeilennnummer 1295 aus Tabelle 1 ist.

Die Verbindungsbezeichnung "1477a" identifiziert die Verbindung der Formel (Ia) [entsprechend Formel (I), worin R¹ = H (= Wasserstoff) bedeutet], in der Q(R²)ₙ = 4-Chlor-2-thienyl und (R³)ₘ = 3,4-F₂ gemäß Zeilennnummer 1477 aus Tabelle 1 ist.

Definitionen von R¹ in bevorzugten erfindungsgemäßen Verbindungen der Formel (I) sind in der nachfolgenden Tabelle U1 dargestellt.

**Tabelle U1:**

| Formel | R¹ in Formel (I) |
|---|---|
| (Ia) | H (Wasserstoff) |
| (Ibb) | Acetyl |
| (Ibc) | Propanoyl |
| (Ibd) | Butanoyl |
| (Ibe) | 2-Methylpropanoyl (= Isopropanoyl) |
| (Ibf) | 2,2-Difluoracetyl |
| (Ibg) | 2,2,2-Trifluoracetyl |
| (Ibh) | C(O)OMe |
| (Ibi) | Cyclopropancarbonyl |
| (Ibj) | 1-Methylcyclopropancarbonyl |
| (Ibk) | Acryl |
| (Ibl) | Prop-2-inoyl |
| (Ibm) | But-2-inoyl |
| (Ibn) | 2-Methylacryl |
| (Ibo) | Benzoyl |
| (Ibp) | 4-Chlorbenzoyl |
| (Ibq) | 3-Chlorbenzoyl |
| (Ibr) | 2-Chlorbenzoyl |
| (Ibs) | 4-Fluorbenzoyl |
| (Ibt) | 3-Fluorbenzoyl |
| (Ibu) | 2-Fluorbenzoyl |
| (Ibv) | 2,2-Dimethylpropanoyl (= Pivaloyl) |
| (Ibw) | 3,3-Dimethylbutanoyl |
| (Ibx) | Pentanoyl |
| (Iby) | Hexanoyl |
| (Ibz) | 2-Nitrobenzoyl |
| (Ibaa) | 2-Fluoracetyl |
| (Ibab) | 2-Chloracetyl |
| (Ibac) | 2-Bromacetyl |
| (Ibad) | 2,2-Dichloracetyl |
| (Ibae) | 2-Methoxyacetyl |
| (Ibaf) | 2,6-Difluorbenzoyl |
| (Ibag) | C(O)C(O)OMe |
| (Ibah) | C(O)CH₂C(O)OMe |

Beispiele für erfindungsgemäße Verbindungen der Formeln (Ia) und (Ibb) bis (Ibah) sind die Verbindungen der betreffenden Formeln (Ia) und (Ibb) bis (Ibah) gemäß vorstehender Tabelle U1, worin die Strukturkombination der Gruppen Q(R²)ₙ und (R³)ₘ gemäß einer Zeilennummer aus Tabelle 1 definiert ist.

So identifiziert beispielsweise die Verbindungsbezeichnung "619bab" die Verbindung der Formel (I), worin R¹ = 2-Chloracetyl bedeutet, und Q(R²)ₙ = 3,5-Difluorpyridin-4-yl und (R³)ₘ = 3-Br, 4-F gemäß Zeilennummer 619 aus Tabelle 1 ist.

Die Verbindungsbezeichnung "599bae" identifiziert die Verbindung der Formel (I), worin R¹ = 2-Methoxyacetyl bedeutet, und Q(R²)ₙ = 3,5-Difluorpyridin-4-yl und (R³)ₘ = 3-Cl gemäß Zeilennummer 599 aus Tabelle 1 ist.

Diese Verbindungen können beispielsweise in Form eines erythro-threo-Gemischs im Mengenverhältnis 70:30 bis 30:70, zum Beispiel in Form eines racemischen erythro-threo-Gemischs, vorliegen.

Erläuterung zur Angabe der Beispiele in den nachfolgenden Tabellen 2a bis 2d:
In den nachfolgenden Tabellen 2a bis 2d sind die erfindungsgemäßen Verbindungen entsprechenden einzelnen Nummern (= Beispielnummern) zugeordnet, wobei die jeweilige Beispielnummer sich zusammensetzt aus der Verbindungsnummer der chemischen Formel (I), die sich auf dieselbe Nummer bezieht wie in der jeweiligen Zeile der Tabelle angegeben, gefolgt von der jeweiligen stereoisomeren Form. Die jeweilige stereoisomere Form entspricht den oben definierten Formeln (I threo-1), (I threo-2), (I erythro-1) bzw. (I erythro-2), oder Mischungen dieser Stereoisomere.

Entsprechendes gilt für die Zuordnung von racemischen oder optisch aktiven threo-Stereoisomeren oder erythro-Stereoisomeren der erfindungsgemäßen Verbindungen.

Auf diese Weise werden die Stereoisomeren der jeweiligen eindeutig definierten chemischen Struktur einer erfindungsgemäßen Verbindung der Formel (I) angegeben, was an folgenden Beispielen verdeutlicht werden soll:
Die Verbindungsbezeichnung "532a erythro" identifiziert ein Gemisch der "erythro" Isomere der Verbindung der Formel (I), worin R¹ = H (= Wasserstoff) bedeutet, und Q(R²)ₙ = 2-Chlorpyridin-4-yl und (R³)ₘ = 3,4-F₂ gemäß Zeilennnummer 532 aus Tabelle 1 ist.

Die Verbindungsbezeichnung "208a threo" identifiziert ein Gemisch der "threo" Isomere der Verbindung der Formel (I), worin R¹ = H (= Wasserstoff) bedeutet, und Q(R²)ₙ = 3-Chlorpyridin-2-yl und (R³)ₘ = 3,4-F₂ gemäß Zeilennnummer 208 aus Tabelle 1 ist.

Die Verbindungsbezeichnung "289a threo-2" identifiziert das "threo-2" Isomere der Verbindung der Formel (I) [entsprechend Formel (I threo-2)], worin R¹ = H (= Wasserstoff) bedeutet, und Q(R²)ₙ = 5-Fluorpyridin-3-yl und (R³)ₘ = 3,4-F₂ gemäß Zeilennnummer 289 aus Tabelle 1 ist.

Die Verbindungsbezeichnung "532a erythro-1" identifiziert das "erythro-1" Isomere der Verbindung der Formel (I) [entsprechend Formel (I erythro-1)], worin R¹ = H (= Wasserstoff) bedeutet, und Q(R²)ₙ = 2-Chlorpyridin-4-yl und (R³)ₘ = 3,4-F₂ gemäß Zeilennnummer 532 aus Tabelle 1 ist.

**Tabellen 2a bis 2d:** Bevorzugte erfindungsgemäße Verbindungen der Formeln (I threo-1), (I threo-2), (I erythro-1) bzw. (I erythro-2)

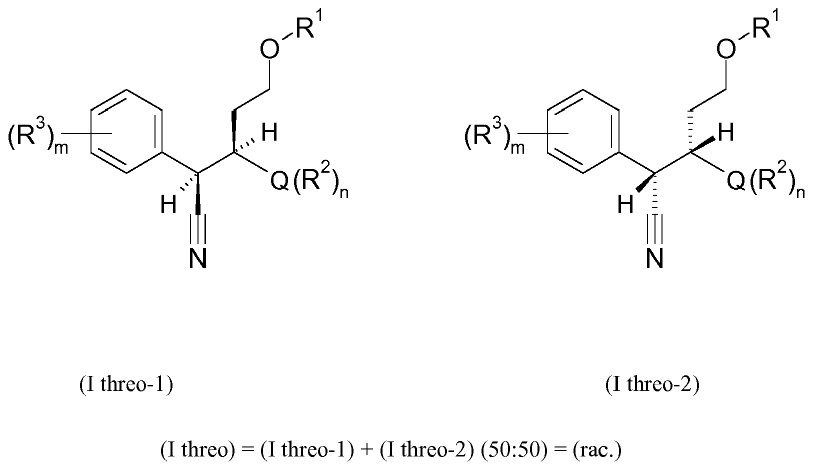

### Tabelle 2a: Bevorzugte (I threo-1) - Enantiomere

Tabelle 2a beschreibt die Verbindungen der Formeln (1 threo-1) bis (1809 threo-1), worin die Strukturkombination der Gruppen Q(R²)ₙ und (R³)ₘ gemäß einer Zeilennummer aus der Tabelle 1 definiert ist, wie vorstehend ausführlich erläutert, wobei R¹ vorzugsweise ausgewählt ist aus Tabelle U1, und bevorzugt R¹ = H (Wasserstoff) bedeutet.

### Tabelle 2b: Bevorzugte (I threo-2) - Enantiomere

Tabelle 2b beschreibt die Verbindungen der Formeln (1 threo-2) bis (1809 threo-2), worin die Strukturkombination der Gruppen Q(R²)ₙ und (R³)ₘ gemäß einer Zeilennummer aus der Tabelle 1 definiert ist, wie vorstehend ausführlich erläutert, wobei R¹ vorzugsweise ausgewählt ist aus Tabelle U1, und bevorzugt R¹ = H (Wasserstoff) bedeutet.

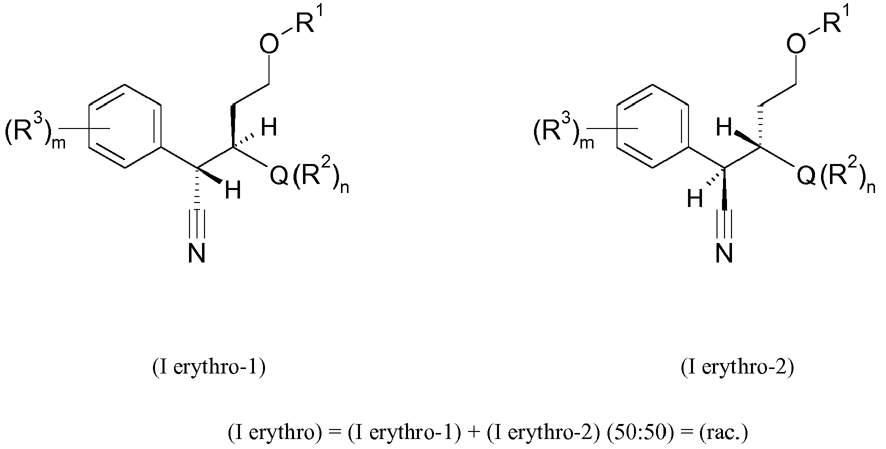

### Tabelle 2c: Bevorzugte (I erythro-1) - Enantiomere

Tabelle 2c beschreibt die Verbindungen der Formeln (1 erythro-1) bis (1809 erythro-1), worin die Strukturkombination der Gruppen Q(R²)ₙ und (R³)ₘ gemäß einer Zeilennummer aus der Tabelle 1 definiert ist, wie vorstehend ausführlich erläutert, wobei R¹ vorzugsweise ausgewählt ist aus Tabelle U1, und bevorzugt R¹ = H (Wasserstoff) bedeutet.

### Tabelle 2d: Bevorzugte (I erythro-2) - Enantiomere

Tabelle 2d beschreibt die Verbindungen der Formeln (1 erythro-2) bis (1809 erythro-2), worin die Strukturkombination der Gruppen Q(R²)ₙ und (R³)ₘ gemäß einer Zeilennummer aus der Tabelle 1 definiert ist, wie vorstehend ausführlich erläutert, wobei R¹ vorzugsweise ausgewählt ist aus Tabelle U1, und bevorzugt R¹ = H (Wasserstoff) bedeutet.

### Physikalische Daten einiger erfindungsgemäßer Verbindungen:

### Testmethoden:

1) NMR = ¹H-NMR-Daten (400 MHz, CDCl₃); charakteristische chemische Verschiebungen [in ppm] sind beim jeweiligen Beispiel angegeben.
2) HPLC = Hochdruckflüssigchromatographie (High Performance Liquid Chromatography), Säule: Zorbax Eclipse, 50x3,0, C18 1,8 µm, Laufmittel: Wasser + 0,06% Ameisensäure / Acrylnitril + 0,06% Ameisensäure, Gradient: 90:10, nach 2min 5:95; Detektor: DAD (210 - 400 nm); Retentionszeit (Rtz.) beim jeweiligen Beispiel angegeben.
3) HPLC-Chiral = HPLC an chiraler Säule, Säule: Chiralpak IC, 250 x 4,6mm, 5µm DAIC 83325, Detektorwellenlänge: 210 nm; Säulentemperatur 25°C
   Eluent a: (n-Heptan : 2-Propanol), (60 : 40), Chromasolv, Fluss: 1,0 mL/min
   Eluent b: (n-Heptan : 2-Propanol), (70 : 30), Chromasolv, Fluss: 1,0 mL/min
   Eluent c: (n-Heptan : 2-Propanol), (80 : 20), Chromasolv, Fluss: 1,0 mL/min
   Eluent d: (n-Heptan : 2-Propanol), (90 : 10), Chromasolv, Fluss: 0,6 mL/min

### NMR-Daten ausgewählter erfindungsgemäßer Verbindungen

¹H-NMR-Daten (CDCl₃) - Chemische Verschiebung ausgewählter charakteristischer Signale in ppm.

### NMR - Peak- Listenverfahren

Die ¹H-NMR-Daten (nachfolgend auch 1H-NMR genannt) ausgewählter Beispiele werden in Form von 1H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispiels hat daher die Form:
δ₁ (Intensität₁); δ₂ (Intensität₂);........; δᵢ (Intensitätᵢ);......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von 1H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu 1H-NMR-Peaklisten können der Research Disclosure Database Number 564025 entnommen werden.

| |
|---|
| Verbindung 215a: 1H-NMR (400,0 MHz, CDCl3): δ = 8,577(10,7);8,573(11,0);8,565(11,1);8,562(10,9);8,524(6,5);8,520(6,7);8,512(6,8);8,509(6,5);7,729(11,1);7,725(1 1,2);7,708(12,4);7,705(11,8);7,539(8,0);7,535(7,9);7,518(15,9);7,515(8,7);7,414(8,9);7,409(9,8);7,398(9,1);7,392 (9,5);7,359(1,4);7,292(2,8);7,260(1373,1);7,251(10,7);7,245(7,0);7,239(5,8);7,235(7,8);7,227(15,1);7,224(8,3);7, 216(14,9);7,210(5,7);7,207(12,2);7,196(13,8);7,192(6,9);7,181(6,4);7,175(6,8);7,166(13,1);7,158(1,8);7,145(21,2 );7,124(8,8);7,115(9,4);7,103(9,0);7,094(8,5);7,083(8,6);7,046(2,6);7,040(2,5);7,035(2,9);7,029(2,6);7,025(4,6);7, 019(4,5);7,013(4,6);7,008(4,4);6,996(8,2);6,984(8,8);6,963(12,5);6,942(4,6);6,914(1,0);4,362(13,3);4,357(9,1);4, 338(16,0);4,332(10,9);4,163(2,9);4,144(7,3);4,133(5,4);4,128(3,7);4,120(10,4);4,109(8,1);4,103(2,9);4,096(3,7);4 ,085(3,6);3,637(2,4);3,623(4,6);3,609(5,6);3,596(6,3);3,581(3,2);3,524(3,3);3,510(6,7);3,496(7,6);3,492(4,3);3,48 2(10,3);3,476(5,7);3,468(5,7);3,458(3,7);3,448(3,5);3,430(2,6);3,415(5,5);3,401(6,5);3,395(6,4);3,387(3,9);3,382( |
| 6,1);3,374(3,9);3,367(4,2);3,354(3,6);2,413(4,9);2,398(7,4);2,382(6,8);2,379(8,4);2,365(4,9);2,110(1,9);2,097(3,6 );2,087(2,2);2,073(4,2);2,062(5,0);2,052(2,8);2,038(4,6);2,025(2,2);2,005(2,4);1,882(2,8);1,870(3,1);1,866(3,3);1, 862(3,3);1,855(3,1);1,851(3,6);1,847(4,9);1,836(4,7);1,832(2,8);1,827(2,8);1,821(2,6);1,816(2,5);1,801(2,0);1,58 7(6,3);0,157(2,0);0,146(1,6);0,008(16,7);0,000(516,3);-0,009(15,4);-0,050(1,6);-0,149(1,6) |
| Verbindung 1484a: 1H-NMR (400,0 MHz, CDCl3): δ= 8,003(1,8);7,301(2,2);7,295(2,4);7,284(2,4);7,279(2,3);7,263(50,3);7,220(4,5);7,214(4,8);7,203(4,6);7,197(4,6);7, 131(1,3);7,110(6,2);7,089(14,8);7,083(2,2);7,077(2,4);7,068(7,4);7,062(0,8);7,056(0,6);7,050(0,6);7,019(3,1);7,0 14(3,1);7,009(3,5);7,000(6,4);6,997(6,9);6,992(2,2);6,987(2,1);6,981(2,0);6,973(11,3);6,969(11,7);6,718(14,0);6, 717(14,2);6,715(14,3);4,190(6,7);4,176(7,0);4,010(3,4);3,993(3,7);3,813(1,8);3,801(4,0);3,787(3,7);3,774(5,2);3, 761(2,5);3,750(0,9);3,741(1,1);3,736(1,1);3,724(1,4);3,714(1,3);3,709(1,3);3,699(1,1);3,623(2,4);3,612(2,7);3,60 2(2,8);3,596(2,2);3,590(2,8);3,585(2,2);3,575(2,6);3,564(2,9);3,556(1,0);3,546(3,2);3,532(3,6);3,522(2,5);3,517(3 ,5);3,508(3,8);3,493(3,1);3,484(15,8);3,479(1,4);3,466(1,1);3,454(1,0);2,958(16,0);2,880(13,6);2,879(13,4);2,279 (0,5);2,270(0,6);2,264(0,6);2,255(1,1);2,244(0,9);2,240(0,6);2,235(0,8);2,230(0,9);2,220(1,3);2,211(0,7);2,205(0, 6);2,196(0,6);2,189(0,7);2,176(1,2);2,168(0,7);2,163(0,9);2,154(2,6);2,141(3,5);2,133(2,0);2,127(1,8);2,119(2,9); 2,114(1,8);2,106(1,9);2,102(3,3);2,090(3,1);2,078(3,4);2,066(2,6);2,054(1,2);2,042(1,2);2,030(0,6);2,018(0,6);2,0 07(1,0);1,996(0,7);1,989(0,6);1,983(0,7);1,979(1,1);1,972(0,9);1,968(0,7);1,954(0,5);1,944(0,8);1,496(6,6);0,008( 0,6);0,000(19,0) |
| Verbindung 181a: 1H-NMR (400,0MHz,CDCl3): δ = 8,523(3,7);8,520(1,5);8,517(1,6);8,513(3,7);8,481(5,3);8,472(5,3);7,263(50,0);7,259(12,4);7,200(1,1);7,186(2,3); 7,180(1,4);7,176(1,6);7,170(2,8);7,166(4,8);7,162(5,3);7,157(5,1);7,154(4,0);7,147(1,2);7,086(0,3);7,063(1,6);7,0 61(1,3);7,057(1,2);7,046(2,2);7,032(2,7);7,016(2,6);7,002(1,5);6,990(1,4);6,987(1,5);6,978(1,5);6,974(2,0);6,969( 1,5);6,960(1,4);6,957(1,4);6,930(6,1);6,927(5,8);6,866(1,9);6,863(1,6);6,859(1,5);6,852(1,6);4,381(4,8);4,364(5,0 );4,317(3,8);4,301(3,9);3,684(1,3);3,677(1,8);3,675(1,8);3,667(2,2);3,658(2,1);3,657(2,0);3,649(1,5);3,563(1,0);3, 554(1,7);3,545(2,1);3,536(2,0);3,527(1,2);3,488(4,1);3,458(1,0);3,451(1,1);3,442(2,0);3,435(2,4);3,426(1,3);3,42 0(2,9);3,415(2,5);3,404(1,4);3,398(1,3);3,377(1,5);3,370(1,7);3,361(2,0);3,359(1,8);3,354(2,0);3,352(1,8);3,343(1 ,5);3,336(1,4);3,330(1,2);3,322(1,3);3,315(1,4);3,312(1,3);3,307(1,4);3,304(1,3);3,297(1,2);3,289(1,0);2,403(0,6); 2,397(0,7);2,393(0,7);2,387(1,2);2,380(1,3);2,371(1,3);2,364(1,6);2,358(1,0);2,354(0,9);2,349(0,8);2,261(0,9);2,2 54(1,7);2,246(1,0);2,243(1,0);2,236(1,9);2,230(1,6);2,223(0,7);2,220(0,7);2,213(1,2);2,205(0,6);2,001(0,7);1,994( 1,2);1,985(1,2);1,977(1,7);1,970(2,0);1,962(1,7);1,953(2,1);1,945(1,7);1,915(2,2);1,833(0,9);1,825(1,1);1,817(1,2 );1,810(1,4);1,801(1,4);1,794(0,9);1,787(0,8);1,778(0,6);0,005(0,6);0,000(12,8);-0,006(0,5) |
| Verbindung 1515a: 1H-NMR (400,0 MHz, CDCl3): δ = 7,361(0,5);7,351(0,7);7,348(1,0);7,338(2,5);7,328(2,1);7,324(3,6);7,315(3,2);7,311(2,1);7,301(1,9);7,291(4,1);7,2 62(50,0);7,114(12,4);7,092(0,4);7,085(0,3);7,072(0,5);7,068(0,6);7,058(1,0);7,052(1,8);7,047(1,8);7,036(3,3);7,0 33(4,1);7,019(2,6);7,010(0,8);7,007(1,0);6,995(0,8);6,992(1,0);6,969(3,8);6,956(3,5);6,930(2,2);6,927(3,0);6,914( 2,2);6,911(2,9);4,280(5,6);4,271(5,7);4,061(1,8);4,050(1,9);3,851(1,5);3,843(3,0);3,834(2,9);3,825(3,5);3,816(1,7 );3,761(0,6);3,755(1,0);3,749(1,1);3,739(2,5);3,730(3,8);3,723(2,5);3,714(2,9);3,705(1,4);3,640(1,7);3,633(1,9);3, 625(2,1);3,622(1,9);3,618(2,1);3,615(1,8);3,607(1,6);3,600(1,5);3,495(0,6);3,490(2,8);3,478(0,9);3,471(0,9);3,46 0(0,5);3,453(0,5);2,259(0,5);2,252(0,6);2,243(0,6);2,236(0,6);2,230(1,0);2,221(1,4);2,215(0,9);2,212(0,9);2,207(2 ,1);2,198(2,3);2,192(1,3);2,189(1,2);2,183(1,7);2,174(0,9);2,110(1,0);2,103(1,7);2,095(1,9);2,087(2,3);2,079(2,1); 2,071(1,3);2,064(1,1);2,055(0,6);1,939(0,5);1,932(0,3);1,928(0,3);1,921(0,6);1,916(0,6);1,897(0,5);1,556(7,6);0,0 00(14,4);-0,006(0,5) |
| Verbindung 1461a: 1H-NMR (400,0 MHz, CDCl3): δ = 8,004(1,0);7,343(1,1);7,328(1,4);7,324(3,8);7,309(4,0);7,304(5,0);7,289(4,9);7,284(3,1);7,269(3,2);7,261(50,1);7, 054(0,9);7,052(1,1);7,047(1,1);7,045(1,5);7,043(1,9);7,041(2,0);7,036(2,1);7,034(2,6);7,031(2,5);7,027(3,6);7,02 4(4,7);7,022(5,5);7,020(4,4);7,016(3,6);7,013(3,8);7,009(2,2);7,004(3,9);7,001(2,8);6,999(2,0);6,995(1,9);6,992(2 ,1);6,988(6,3);6,985(7,2);6,982(2,3);6,976(1,2);6,963(2,1);6,956(15,1);6,952(16,0);6,934(3,7);6,933(3,5);6,904(2, 7);6,899(3,4);6,893(2,1);6,880(2,7);6,875(3,3);6,870(2,1);6,727(5,1);6,726(5,0);6,724(5,1);6,722(4,8);6,708(9,2); 6,707(9,3);6,704(9,4);4,202(6,9);4,187(7,2);4,043(3,7);4,026(4,0);3,800(1,8);3,787(3,8);3,774(3,8);3,761(4,9);3,7 48(2,4);3,733(1,0);3,724(1,2);3,719(1,2);3,707(1,7);3,697(1,5);3,692(1,4);3,682(1,2);3,614(2,4);3,603(2,7);3,593( 3,6);3,587(2,5);3,581(4,9);3,576(3,3);3,566(6,3);3,554(4,4);3,543(3,5);3,528(1,7);3,508(1,3);3,496(1,5);3,484(10, 9);3,472(1,6);3,469(1,4);3,457(1,2);3,445(1,1);2,956(9,3);2,879(7,8);2,878(7,6);2,281(0,6);2,272(0,6);2,266(0,6); 2,258(1,2);2,247(0,9);2,243(0,7);2,238(0,8);2,232(0,9);2,223(1,5);2,214(0,8);2,208(0,7);2,199(0,7);2,187(0,7);2,1 74(1,3);2,166(0,7);2,161(0,9);2,152(2,7);2,139(3,7);2,131(2,0);2,125(1,8);2,118(3,2);2,111(1,8);2,104(1,8);2,099( 3,4);2,087(3,0);2,075(3,4);2,063(2,5);2,052(1,2);2,039(1,8);2,028(1,6);2,017(0,8);2,010(0,7);2,004(0,7);2,000(1,0 |
| );1,993(0,9);1,989(0,8);1,982(0,5);1,976(0,6);1,965(0,7);1,954(0,5);1,449(4,9);0,008(0,6);0,000(19,6);-0,009(0,5) |
| Verbindung 1677a: 1H-NMR (400,0 MHz, CDCl3): δ = 8,001(1,8);7,334(1,9);7,327(3,8);7,319(2,2);7,312(5,7);7,306(6,6);7,300(4,3);7,292(6,9);7,286(5,1);7,279(2,8);7,2 72(5,1);7,262(65,8);7,043(1,6);7,040(4,0);7,037(4,2);7,034(4,5);7,031(3,7);7,019(9,5);7,016(8,0);7,013(7,7);7,01 0(6,5);7,007(2,8);7,006(2,9);7,005(3,1);7,003(3,7);7,001(4,5);6,999(4,0);6,998(4,2);6,995(4,0);6,992(3,7);6,989(3 ,0);6,978(4,9);6,977(5,7);6,974(9,2);6,971(6,4);6,964(2,2);6,958(4,9);6,957(5,1);6,955(6,4);6,954(6,1);6,953(5,9); 6,952(6,1);6,946(3,0);6,940(1,8);6,915(4,5);6,910(5,5);6,905(3,5);6,892(4,5);6,886(5,5);6,881(3,5);6,394(3,5);6,3 93(3,5);6,385(7,2);6,383(5,0);6,380(7,1);6,378(7,3);6,376(5,3);6,375(5,0);6,370(13,2);6,362(7,7);6,360(7,3);6,26 6(12,9);6,261(13,1);6,256(11,0);6,251(12,0);6,250(8,3);6,245(6,7);6,240(5,7);6,236(5,6);4,150(11,5);4,136(12,0); 3,992(6,1);3,974(6,5);3,803(3,0);3,790(6,3);3,776(6,6);3,763(8,5);3,750(5,6);3,741(1,9);3,736(1,9);3,724(2,7);3,7 14(2,4);3,709(2,3);3,699(2,1);3,641(4,2);3,629(4,7);3,620(4,9);3,614(3,6);3,608(4,8);3,602(3,5);3,593(3,6);3,581( 3,3);3,549(2,2);3,537(2,5);3,526(2,6);3,522(2,1);3,514(2,6);3,510(2,2);3,499(2,0);3,487(1,9);3,481(6,3);3,467(1,9 );3,461(2,6);3,452(4,1);3,446(4,0);3,443(3,6);3,437(3,2);3,433(3,2);3,428(3,5);3,422(3,7);3,414(1,8);3,408(1,5);2, 956(16,0);2,878(14,1);2,877(14,0);2,276(0,9);2,267(0,9);2,261(1,0);2,252(1,8);2,243(1,2);2,241(1,4);2,238(1,1);2 ,232(1,3);2,229(1,2);2,226(1,4);2,218(2,2);2,209(1,2);2,203(1,1);2,194(1,0);2,142(1,1);2,129(2,3);2,121(1,2);2,11 6(1,4);2,107(4,5);2,094(6,2);2,086(3,3);2,080(2,9);2,072(5,4);2,064(3,1);2,059(2,9);2,052(5,8);2,040(5,3);2,028(5 ,7);2,016(3,7);2,004(2,2);1,992(2,3);1,980(1,1);1,972(1,1);1,960(1,6);1,950(1,2);1,943(1,1);1,937(1,1);1,932(1,6); 1,925(1,4);1,921(1,2);1,915(0,9);1,908(0,9);1,898(1,2);1,886(0,8);1,498(12,3);0,008(0,7);0,000(25,4);-0,009(0,7) |
| Verbindung 1153a: 1H-NMR (400,0 MHz, CDCl3): δ = 8,095(2,9);8,090(5,8);8,087(3,3);8,082(5,6);7,264(29,0);7,127(1,4);7,119(0,9);7,113(3,1);7,105(1,7);7,0 97(3,0);7,089(1,6);7,083(1,6);7,075(0,7);7,065(0,7);7,061(0,7);7,053(0,8);7,048(1,1);7,043(0,8);7,035(0,7);7,031( 0,7);7,013(1,5);7,010(1,6);7,001(1,7);6,997(2,3);6,992(1,7);6,984(1,5);6,980(1,5);6,914(1,0);6,907(0,8);6,900(0,9 );6,886(2,2);6,879(1,7);6,871(1,9);6,666(2,1);6,664(2,4);6,658(5,6);6,652(4,2);6,650(3,9);6,499(3,5);6,476(7,7);4, 192(5,3);4,181(5,3);4,031(0,4);3,987(2,4);3,974(2,5);3,912(50,0);3,724(1,4);3,715(2,9);3,706(3,0);3,698(3,3);3,6 89(1,6);3,659(0,6);3,652(0,8);3,649(0,8);3,642(1,3);3,634(0,9);3,631(0,9);3,624(0,7);3,494(1,7);3,488(3,2);3,480( 1,9);3,476(1,7);3,471(1,9);3,468(1,7);3,462(1,5);3,454(1,4);3,381(0,7);3,373(0,8);3,365(1,0);3,357(1,0);3,348(0,7 );3,340(0,6);3,278(0,7);3,268(1,6);3,259(3,3);3,253(2,3);3,248(2,2);3,242(2,8);3,232(1,3);2,272(0,3);2,266(0,4);2, 262(0,4);2,256(0,7);2,249(0,7);2,240(0,7);2,233(0,8);2,227(0,5);2,223(0,4);2,217(0,4);2,110(0,6);2,101(1,1);2,09 6(0,8);2,091(1,1);2,087(2,4);2,077(3,6);2,070(3,8);2,063(6,4);2,054(6,4);2,046(6,1);2,038(4,2);2,031(2,1);2,022(2 ,0);2,014(1,1);0,000(8,3) |
| Verbindung 532a threo: 1H-NMR (400,0 MHz, CDCl3): δ = 8,341(15,5);8,339(15,4);8,328(16,0);8,326(15,5);8,308(0,8);7,519(4,8);7,379(0,6);7,310(1,0);7,293(1,7);7,260(90 3,9);7,229(0,7);7,210(1,3);7,163(5,1);7,142(8,1);7,138(6,3);7,121(7,6);7,116(20,9);7,112(19,5);7,102(5,3);7,097( 10,7);7,084(5,1);7,078(5,8);7,070(5,1);7,058(4,8);7,052(4,9);7,032(12,4);7,028(11,3);7,018(11,4);7,015(10,7);6,9 96(5,2);6,975(0,8);6,918(3,2);6,913(4,0);6,909(5,6);6,904(4,3);6,898(4,3);6,892(3,7);6,887(4,7);6,883(3,3);6,877( 2,4);5,299(4,5);4,032(13,8);4,014(14,6);3,709(2,1);3,696(3,1);3,683(4,1);3,672(3,6);3,658(2,6);3,384(4,7);3,374( 5,3);3,366(5,1);3,356(10,5);3,346(7,1);3,338(5,6);3,328(5,2);2,288(2,0);2,278(2,2);2,273(2,4);2,263(4,3);2,252(4, 3);2,242(3,4);2,238(4,3);2,228(5,8);2,218(3,2);2,214(3,0);2,204(2,6);2,170(0,6);2,088(2,8);2,077(5,2);2,067(3,0); 2,061(2,7);2,050(5,2);2,042(4,5);2,031(2,0);2,025(2,2);2,014(3,4);2,004(1,9);1,541(60,1);1,333(3,2);1,284(6,3);1, 256(5,0);0,880(0,8);0,146(1,0);0,008(9,2);0,000(342,5);-0,006(4,9);-0,009(11,0);-0,051(0,6);-0,150(1,1) |
| Verbindung 1288a: 1H-NMR (400,0 MHz, CDCl3): δ = 8,620(14,8);8,617(14,8);8,565(12,7);8,562(12,6);8,189(16,0);8,185(15,7);8,010(0,7);7,963(13,7);7,960( 13,4);7,520(1,1);7,261(212,1);7,215(2,8);7,211(0,6);7,195(5,3);7,191(3,5);7,180(3,4);7,174(7,3);7,170(5,7);7,162 (3,3);7,154(4,1);7,149(5,2);7,135(3,0);7,129(3,1);7,077(2,7);7,056(4,8);7,053(3,7);7,046(3,1);7,041(4,3);7,036(6, 4);7,032(6,8);7,023(4,7);7,017(4,5);7,011(4,5);7,005(2,9);6,997(4,1);6,991(2,8);6,978(2,5);6,973(2,6);6,860(1,8); 6,856(2,2);6,851(3,2);6,846(2,3);6,840(2,2);6,835(2,0);6,829(2,5);6,825(1,8);6,820(1,3);4,292(9,2);4,285(8,4);4,2 70(9,6);4,260(8,7);3,728(2,0);3,717(2,8);3,701(2,9);3,690(3,2);3,676(2,3);3,645(2,3);3,633(4,5);3,619(4,6);3,605( 6,3);3,594(6,0);3,583(3,3);3,577(4,0);3,570(6,1);3,561(3,6);3,552(2,9);3,548(3,1);3,543(2,5);3,536(2,5);3,346(2,7 );3,335(3,0);3,323(5,3);3,319(3,3);3,312(5,8);3,308(3,2);3,297(5,0);3,286(4,9);3,272(2,3);3,261(2,1);2,958(5,6);2, 882(4,8);2,489(1,1);2,480(1,2);2,475(1,2);2,466(1,8);2,454(2,0);2,441(1,8);2,431(2,2);2,422(1,4);2,417(1,3);2,40 8(1,1);2,236(1,3);2,225(2,7);2,215(1,5);2,209(1,4);2,198(2,9);2,191(2,4);2,180(1,1);2,174(1,1);2,163(2,0);2,153(0 ,9);2,063(1,0);2,052(1,9);2,040(1,5);2,028(3,0);2,017(3,8);2,004(2,8);1,992(3,3);1,980(1,6);1,960(1,7);1,948(2,9); 1,935(2,4);1,925(3,6);1,913(3,1);1,902(1,3);1,890(1,5);1,878(0,9);1,485(1,6);0,008(2,6);0,000(79,4);-0,009(2,3) |
| Verbindung 1477a: 1H-NMR (400,0 MHz, CDCl3): δ = 8,003(1,7);7,262(60,8);7,166(1,4);7,152(2,8);7,145(2,5);7,142(1,8);7,131(4,7);7,127(3,4);7,121(2,6);7,111(3,5);7, 106(4,6);7,100(2,0);7,097(1,6);7,091(1,6);7,086(3,2);7,079(1,4);7,073(1,6);7,071(1,5);7,065(1,4);7,052(1,3);7,04 6(1,4);7,016(2,3);7,010(2,5);6,997(3,0);6,994(7,3);6,991(9,2);6,983(2,8);6,971(2,9);6,966(15,2);6,962(14,1);6,95 8(2,2);6,954(1,4);6,947(1,2);6,941(1,0);6,936(1,3);6,931(1,0);6,927(0,7);6,903(1,7);6,899(2,1);6,894(2,9);6,889(2 ,2);6,883(2,2);6,878(1,8);6,873(2,3);6,868(1,7);6,863(1,2);6,722(10,6);6,721(11,5);6,719(11,3);6,718(11,0);6,715 (6,8);6,714(6,4);6,711(6,2);6,710(5,8);4,199(7,1);4,185(7,4);4,010(3,9);3,993(4,2);3,813(2,0);3,800(4,4);3,787(4, 0);3,773(5,7);3,761(2,7);3,749(1,1);3,740(1,3);3,735(1,3);3,723(1,7);3,713(1,6);3,708(1,6);3,698(1,4);3,622(2,7); 3,610(3,0);3,601(3,1);3,595(2,4);3,589(3,1);3,583(2,4);3,574(3,0);3,567(1,4);3,562(2,5);3,559(1,3);3,549(3,4);3,5 36(4,0);3,526(2,6);3,521(3,1);3,515(2,3);3,512(4,1);3,504(1,9);3,498(2,0);3,492(2,0);3,489(1,7);3,485(7,5);3,480( 1,9);3,478(1,6);3,465(1,4);3,454(1,3);2,958(15,6);2,957(16,0);2,880(13,7);2,878(14,0);2,288(0,6);2,279(0,7);2,27 3(0,7);2,264(1,3);2,253(1,1);2,249(0,8);2,244(0,9);2,239(1,1);2,229(1,6);2,220(0,9);2,214(0,8);2,205(0,7);2,192(0 ,7);2,178(1,2);2,171(0,7);2,165(0,9);2,156(2,9);2,143(3,8);2,135(2,3);2,130(2,1);2,121(3,4);2,107(4,4);2,095(3,4); 2,083(3,8);2,071(2,9);2,060(1,3);2,048(1,3);2,036(0,7);2,019(0,8);2,008(1,2);1,998(0,8);1,991(0,8);1,984(0,8);1,9 80(1,3);1,974(1,1);1,970(0,9);1,963(0,6);1,956(0,6);1,946(0,9);1,935(0,6);1,508(3,9);0,008(0,7);0,000(24,0);-0,009(0,7) |
| Verbindung 1542a: 1H-NMR (400,0 MHz, CDCl3): δ = 7,996(2,0);7,368(1,2);7,353(1,7);7,347(4,4);7,332(5,0);7,327(5,9);7,313(5,9);7,307(3,6);7,292(3,2);7,266(32,8);7, 259(11,9);7,087(20,3);7,078(1,6);7,076(1,6);7,072(1,6);7,070(1,7);7,056(4,1);7,050(4,7);7,036(8,0);7,032(6,2);7, 015(6,6);7,010(4,4);6,991(2,4);6,980(6,2);6,960(4,9);6,941(3,4);6,936(4,3);6,931(2,6);6,918(3,3);6,913(4,3);6,90 8(2,6);4,294(8,1);4,280(8,5);4,068(4,7);4,052(5,1);3,853(2,1);3,840(4,3);3,826(4,1);3,813(5,5);3,800(2,7);3,761(1 ,2);3,751(1,5);3,747(1,6);3,734(3,2);3,724(3,1);3,719(2,7);3,709(5,0);3,695(4,8);3,686(3,2);3,681(3,8);3,672(4,3); 3,658(2,2);3,647(2,7);3,636(3,1);3,625(3,1);3,620(2,7);3,614(3,2);3,609(2,6);3,598(2,4);3,587(2,2);3,506(1,5);3,4 95(1,7);3,480(8,0);3,470(2,1);3,454(1,4);3,443(1,3);2,959(16,0);2,877(13,6);2,288(0,7);2,278(0,8);2,273(0,8);2,2 64(1,3);2,253(1,4);2,249(1,0);2,239(2,3);2,228(2,1);2,218(1,8);2,212(1,6);2,203(3,3);2,190(3,2);2,182(2,1);2,176( 1,9);2,168(2,3);2,155(1,5);2,116(1,6);2,104(2,4);2,092(3,0);2,080(3,2);2,069(2,9);2,057(1,7);2,046(1,3);2,033(0,9 );1,949(0,8);1,939(1,5);1,929(0,9);1,922(0,9);1,911(1,7);1,904(1,5);1,894(0,7);1,887(0,7);1,876(1,2);1,866(0,7);1, 729(4,4);0,008(0,5);0,000(12,4) |
| Verbindung 1565a: 1H-NMR (400,0 MHz, CDCl3): δ = 8,005(1,3);7,351(2,1);7,345(2,2);7,334(2,0);7,328(2,0);7,2724(9,5);7,2715(9,3);7,264(57,7);7,258(4,0);7,252(3,7) ;7,163(1,3);7,142(4,7);7,117(17,9);7,109(2,4);7,103(2,1);7,097(5,7);7,088(0,6);7,082(0,5);7,060(2,4);7,054(2,3);7 ,050(2,6);7,044(2,4);7,039(1,5);7,033(1,5);7,028(1,4);7,022(1,3);4,269(5,0);4,255(5,2);4,046(3,2);4,029(3,5);3,86 6(1,4);3,854(2,9);3,840(2,7);3,827(3,7);3,814(1,8);3,783(0,8);3,773(1,0);3,769(1,0);3,759(1,1);3,756(1,2);3,746(1 ,2);3,742(1,2);3,732(1,0);3,713(0,8);3,704(0,9);3,697(0,8);3,686(1,6);3,676(2,2);3,669(1,1);3,661(3,4);3,658(2,8); 3,653(2,1);3,647(3,6);3,638(3,1);3,636(3,0);3,630(2,0);3,625(3,6);3,620(1,9);3,609(1,7);3,598(1,5);3,521(1,1);3,5 10(1,2);3,496(1,5);3,487(16,0);3,469(1,0);3,459(0,9);2,960(11,7);2,881(9,8);2,880(9,9);2,263(0,5);2,259(0,5);2,2 49(0,8);2,238(1,0);2,232(0,9);2,228(0,8);2,224(1,1);2,219(1,0);2,217(1,0);2,213(1,2);2,210(0,9);2,204(1,3);2,196( 2,0);2,190(0,8);2,183(2,0);2,174(1,4);2,169(1,3);2,162(1,3);2,160(1,4);2,147(1,0);2,112(1,1);2,101(1,5);2,100(1,5 );2,089(2,0);2,077(2,0);2,066(1,9);2,054(1,1);2,043(0,8);2,041(0,8);2,030(0,6);1,939(0,5);1,929(1,1);1,918(0,6);1, 911(0,6);1,901(1,2);1,893(1,0);1,866(0,9);1,564(3,9);0,008(0,7);0,000(21,5);-0,009(0,6) |
| Verbindung 1516a: 1H-NMR (400,0 MHz, CDCl3): δ = 7,262(50,0);7,256(5,1);7,222(1,7);7,214(1,9);7,208(2,1);7,200(1,9);7,150(2,6);7,142(3,0);7,136(3,5);7,1 27(3,2);7,100(7,5);7,085(0,5);7,077(0,6);7,071(2,2);7,057(3,9);7,051(3,9);7,047(1,7);7,043(2,4);7,037(5,9);7,022( 2,6);4,259(3,2);4,250(3,3);4,038(2,0);4,027(2,1);3,847(0,9);3,839(1,9);3,830(1,8);3,821(2,2);3,813(1,0);3,787(0,4 );3,758(0,6);3,751(0,7);3,748(0,7);3,741(0,9);3,730(1,3);3,724(1,2);3,719(0,6);3,712(1,0);3,706(0,6);3,700(0,7);3, 694(1,4);3,685(1,7);3,678(1,2);3,669(1,7);3,660(0,9);3,634(1,1);3,627(1,2);3,620(1,3);3,616(1,2);3,612(1,3);3,60 9(1,1);3,602(1,0);3,595(0,9);3,499(0,6);3,492(0,9);3,490(0,8);3,483(1,0);3,476(1,0);3,465(0,6);3,458(0,5);2,285(0 ,3);2,279(0,3);2,276(0,4);2,269(0,6);2,262(0,6);2,253(0,6);2,246(0,7);2,239(0,4);2,236(0,4);2,230(0,4);2,227(0,5); 2,218(0,7);2,212(0,6);2,209(0,6);2,203(1,3);2,195(1,4);2,189(0,8);2,186(0,7);2,180(1,0);2,171(0,6);2,099(0,6);2,0 92(1,0);2,084(1,2);2,076(1,4);2,068(1,3);2,060(0,8);2,052(0,7);2,044(0,4);1,927(0,3);1,920(0,6);1,913(0,4);1,908( 0,4);1,901(0,7);1,896(0,7);1,890(0,3);1,885(0,3);1,878(0,5);1,541(6,6);0,005(0,7);0,000(13,6);-0,006(0,5) |
| Verbindung 208a: 1H-NMR (400,0 MHz, CDCl3): δ = 8,570(11,1);8,567(11,4);8,559(11,4);8,555(11,2);8,516(6,7);8,512(6,9);8,505(6,9);8,501(6,8);7,718(14,0);7,715(1 3,8);7,698(15,5);7,694(14,7);7,535(8,8);7,531(8,7);7,522(1,0);7,515(9,8);7,511(9,4);7,263(118,6);7,219(15,9);7,2 14(5,0);7,207(18,3);7,199(16,3);7,196(6,0);7,190(6,5);7,187(19,0);7,181(8,5);7,180(8,7);7,176(4,9);7,169(4,8);7, 164(5,4);7,160(7,3);7,155(7,5);7,135(6,4);7,110(12,6);7,102(5,9);7,098(13,8);7,089(11,1);7,078(10,7);7,071(1,9); 7,024(2,6);7,003(4,9);6,999(3,7);6,995(3,1);6,989(3,4);6,982(4,3);6,977(6,2);6,970(3,6);6,968(3,3);6,962(3,4);6,9 58(4,1);6,949(2,8);6,944(3,1);6,906(2,2);6,903(2,5);6,901(2,3);6,896(3,5);6,893(2,6);6,891(2,3);6,887(2,3);6,881 1,9);6,875(2,3);6,871(1,7);6,866(1,3);4,363(8,6);4,356(12,9);4,337(11,1);4,333(16,0);4,158(2,7);4,141(8,1);4,131 (5,7);4,123(3,5);4,117(9,3);4,107(8,1);4,098(2,7);4,094(3,9);4,083(3,6);3,625(1,9);3,611(3,9);3,597(4,7);3,583(5, 5);3,569(2,8);3,519(2,8);3,505(5,6);3,491(6,7);3,483(11,2);3,478(9,2);3,464(6,9);3,455(2,4);3,449(3,2);3,437(3,0) ;3,421(2,2);3,408(4,6);3,394(5,3);3,388(5,5);3,380(3,5);3,374(5,2);3,367(3,4);3,360(3,5);3,347(3,1);2,955(4,5);2, 880(3,8);2,878(3,7);2,405(5,2);2,390(7,5);2,388(7,6);2,374(6,6);2,372(7,7);2,356(4,7);2,103(2,0);2,089(4,0);2,07 9(2,2);2,075(2,3);2,068(3,3);2,065(4,4);2,055(5,6);2,052(2,8);2,045(3,0);2,041(2,8);2,031(5,3);2,017(2,5);1,883(2 ,5);1,872(2,8);1,868(3,0);1,863(3,0);1,857(3,0);1,853(3,3);1,848(4,5);1,838(4,5);1,834(2,5);1,829(2,4);1,823(2,2); 1,818(2,3);1,814(2,0);1,803(1,8);1,624(2,7);0,008(1,4);0,000(44,8);-0,009(1,2) |
| Verbindung 208a threo: 1H-NMR (400,0 MHz, CDCl3): δ = 8,574(1,9);8,570(2,0);8,563(2,0);8,559(2,0);8,519(10,6);8,515(11,0);8,508(10,9);8,504(10,9);7,723(2,4);7,720(2,4 );7,703(2,6);7,699(2,5);7,538(13,3);7,534(13,7);7,517(15,0);7,513(14,7);7,261(206,1);7,223(2,7);7,217(0,9);7,211 (3,7);7,202(3,2);7,199(1,0);7,191(3,4);7,182(1,6);7,178(1,0);7,172(0,9);7,167(1,1);7,162(1,4);7,157(1,5);7,138(1, 2);7,112(16,2);7,100(15,5);7,091(14,3);7,080(14,1);7,024(4,2);7,003(7,8);7,000(5,1);6,995(4,9);6,989(5,3);6,983( 6,8);6,978(9,3);6,971(5,7);6,968(5,2);6,962(5,5);6,958(6,5);6,950(4,2);6,944(4,8);6,909(3,6);6,905(3,9);6,903(3,7 );6,899(5,6);6,895(4,2);6,893(3,7);6,889(3,7);6,884(2,9);6,877(3,7);6,874(2,7);6,868(2,1);4,366(12,7);4,360(2,8); 4,341(16,0);4,160(4,3);4,143(7,2);4,134(4,0);4,126(4,6);4,117(5,6);4,109(1,8);4,100(3,6);4,085(0,7);3,631(3,3);3, 617(6,8);3,603(8,4);3,589(9,6);3,575(4,9);3,523(0,6);3,509(1,1);3,495(1,5);3,488(5,1);3,481(2,1);3,472(7,5);3,46 1(3,9);3,454(5,6);3,443(5,1);3,427(3,7);3,412(1,0);3,398(1,1);3,392(1,1);3,384(0,7);3,378(1,1);3,371(0,7);3,364(0 ,7);3,351(0,6);2,409(8,5);2,393(12,7);2,377(12,6);2,361(7,8);2,093(0,7);2,069(0,8);2,058(1,0);2,048(0,5);2,045(0, 5);2,034(0,9);1,873(0,5);1,868(0,6);1,864(0,6);1,858(0,6);1,853(0,6);1,849(0,8);1,838(0,8);1,607(3,5);0,008(2,1); 0,000(77,2);-0,009(2,2) |
| Verbindung 1531a: 1H-NMR (400,0 MHz, CDCl3): δ = 7,288(3,5);7,262(50,0);7,188(0,4);7,174(1,0);7,168(1,6);7,155(3,2);7,139(3,2);7,131(12,3);7,125(1,8);7, 117(0,8);7,112(0,6);7,109(0,5);7,104(0,5);7,100(0,4);7,085(0,3);7,051(1,5);7,047(1,6);7,039(1,7);7,034(2,4);7,02 9(1,6);7,021(1,5);7,017(1,4);6,980(0,6);6,973(0,5);6,966(0,6);6,922(2,2);6,915(1,7);6,907(1,9);4,268(5,1);4,260(5 ,2);4,040(1,4);4,029(1,5);3,857(1,4);3,849(2,9);3,840(2,7);3,831(3,3);3,823(1,6);3,772(0,4);3,765(0,6);3,763(0,5); 3,756(0,6);3,747(0,7);3,745(0,7);3,738(0,7);3,731(0,5);3,724(0,8);3,718(0,5);3,710(1,6);3,701(2,7);3,694(1,9);3,6 92(1,7);3,685(2,7);3,676(1,4);3,642(1,7);3,635(1,9);3,628(2,1);3,624(1,9);3,621(2,1);3,618(1,7);3,610(1,5);3,603( 1,4);3,504(0,5);3,497(0,6);3,490(2,3);3,481(0,8);3,470(0,4);3,463(0,4);2,252(0,4);2,245(0,5);2,236(0,5);2,228(0,5 );2,222(0,9);2,214(1,2);2,208(0,9);2,204(0,9);2,199(1,9);2,190(2,2);2,184(1,3);2,181(1,2);2,175(1,5);2,166(0,9);2, 107(1,0);2,100(1,6);2,092(1,9);2,084(2,0);2,076(1,9);2,068(1,3);2,061(1,0);2,052(0,6);1,925(0,5);1,907(0,6);1,90 1(0,6);1,883(0,4);1,566(5,3);0,000(14,6);-0,006(0,5) |
| Verbindung 1272a: 1H-NMR (400,0 MHz, CDCl3): δ = 8,623(13,3);8,620(13,8);8,565(11,5);8,561(11,5);8,162(16,0);8,159(15,6);7,926(13,9);7,922(13,8);7,519 (6,5);7,383(2,4);7,369(2,7);7,364(5,2);7,349(5,1);7,344(3,4);7,329(3,5);7,310(1,5);7,308(0,7);7,291(1,3);7,286(1, 7);7,277(2,8);7,271(5,2);7,270(5,8);7,269(7,2);7,260(1202,0);7,247(4,0);7,231(2,5);7,226(5,9);7,212(4,5);7,206(3 ,8);7,198(1,2);7,192(2,8);7,169(1,0);7,081(8,0);7,078(6,4);7,075(4,7);7,060(7,4);7,058(7,4);7,054(6,3);7,052(4,4); 7,043(4,2);7,038(5,1);7,033(4,7);7,020(3,9);7,016(4,1);7,010(2,3);6,996(6,6);6,969(2,1);6,967(2,1);6,963(2,2);6,9 60(2,4);6,948(3,4);6,946(3,8);6,942(3,6);6,939(4,2);6,927(1,8);6,925(1,9);6,921(1,9);6,918(2,0);6,888(5,0);6,886( 4,5);6,881(3,7);6,876(4,2);6,870(6,5);6,858(3,5);6,852(3,5);6,847(2,1);4,302(8,8);4,280(14,4);4,254(8,3);3,725(2, 1);3,711(2,6);3,698(3,0);3,687(2,9);3,684(3,0);3,673(2,5);3,639(2,2);3,626(4,1);3,613(6,3);3,599(5,5);3,590(5,8); 3,586(5,1);3,581(5,0);3,566(3,9);3,556(3,7);3,348(2,6);3,337(4,0);3,326(4,5);3,321(2,9);3,314(5,9);3,310(4,4);3,3 03(3,3);3,299(4,3);3,287(4,6);3,276(2,4);2,502(1,0);2,493(1,2);2,488(1,4);2,479(2,1);2,467(1,8);2,453(1,9);2,444( 2,7);2,435(1,6);2,429(1,4);2,421(1,3);2,276(1,5);2,265(3,0);2,254(1,4);2,249(1,5);2,238(3,0);2,230(2,5);2,220(1,2 );2,214(1,2);2,203(2,1);2,192(1,0);2,089(1,0);2,078(2,0);2,065(1,6);2,053(2,7);2,043(4,0);2,030(2,7);2,017(3,6);2, 006(2,1);1,977(1,8);1,964(2,6);1,954(2,2);1,952(2,4);1,942(3,3);1,929(3,0);1,919(1,2);1,908(1,3);1,894(0,9);1,52 9(6,9);1,255(1,0);0,331(1,0);0,238(0,8);0,157(1,8);0,146(1,2);0,008(12,9);0,000(468,1);-0,009(13,6);-0,150(1,4) |
| Verbindung 1700a: 1H-NMR (400,0 MHz, CDCl3): δ = 8,005(1,8);7,291(3,2);7,285(3,4);7,274(3,2);7,269(3,7);7,262(60,2);7,230(6,1);7,224(6,4);7,213(6,3);7,207(6,3);7, 123(1,7);7,116(5,3);7,102(6,4);7,094(14,3);7,086(3,5);7,081(7,6);7,073(11,9);7,069(3,7);7,065(1,2);7,059(1,0);7, 053(0,8);7,044(4,5);7,038(4,3);7,032(4,8);7,027(4,4);7,022(2,8);7,016(2,5);7,011(2,4);7,005(2,2);6,998(0,5);6,37 8(3,1);6,377(3,1);6,373(5,8);6,372(5,8);6,369(6,4);6,364(10,8);6,355(6,5);6,282(10,0);6,278(10,1);6,272(8,2);6,2 68(8,1);6,261(5,1);6,256(5,1);6,251(4,3);6,247(4,2);4,138(9,2);4,124(9,5);3,950(5,1);3,931(5,5);3,819(2,4);3,806( 5,3);3,792(5,1);3,779(7,2);3,770(1,8);3,766(3,6);3,761(1,7);3,756(1,6);3,744(2,1);3,734(1,9);3,729(1,9);3,719(1,7 );3,653(3,3);3,641(3,7);3,632(3,8);3,626(2,9);3,620(3,8);3,614(2,9);3,605(2,8);3,593(2,6);3,562(1,7);3,550(1,9);3, 538(2,1);3,535(1,7);3,526(2,1);3,523(1,8);3,511(1,6);3,499(1,4);3,485(6,8);3,458(0,7);3,450(1,0);3,441(1,0);3,43 3(2,1);3,429(2,2);3,420(3,2);3,413(3,5);3,404(3,2);3,396(3,3);3,390(2,6);3,381(1,3);3,375(1,2);2,958(16,0);2,880( 13,4);2,879(12,9);2,281(0,7);2,272(0,7);2,267(0,8);2,258(1,5);2,247(1,2);2,243(0,9);2,237(1,0);2,232(1,2);2,223( 1,8);2,214(0,9);2,208(0,9);2,199(0,8);2,146(0,8);2,133(1,7);2,125(0,9);2,120(1,1);2,111(3,5);2,098(4,8);2,090(2,8 );2,085(2,4);2,077(4,2);2,071(2,5);2,063(2,5);2,059(4,6);2,047(4,3);2,035(4,6);2,023(3,4);2,012(1,7);2,000(1,7);1, 988(0,8);1,952(0,9);1,941(1,4);1,930(0,9);1,923(0,9);1,917(1,0);1,913(1,5);1,906(1,3);1,902(1,0);1,896(0,8);1,88 8(0,8);1,878(1,1);1,867(0,7);1,489(10,1);0,008(0,7);0,000(22,5);-0,009(0,7) |
| Verbindung 1538a: 1H-NMR (400,0 MHz, CDCl3): δ = 7,345(0,7);7,341(0,7);7,334(0,7);7,330(0,7);7,300(2,5);7,262(50,0);7,253(2,3);7,250(2,1);7,154(0,6);7,1 42(8,3);7,130(2,0);7,126(1,2);7,116(4,3);7,107(0,7);7,102(2,8);7,093(0,3);7,085(0,4);7,045(1,3);7,041(1,4);7,038( 1,5);7,034(1,4);7,032(1,1);7,027(1,0);7,024(1,0);7,020(0,8);4,258(3,3);4,249(3,4);4,039(1,1);4,028(1,1);3,858(0,9 );3,850(1,9);3,841(1,8);3,832(2,2);3,824(1,1);3,773(0,3);3,766(0,4);3,764(0,4);3,755(0,5);3,748(0,4);3,746(0,5);3, 740(0,5);3,735(0,4);3,730(0,3);3,723(0,6);3,716(0,4);3,711(0,4);3,706(1,2);3,697(1,8);3,690(1,2);3,687(1,1);3,68 1(1,8);3,672(0,9);3,644(1,1);3,638(1,2);3,630(1,3);3,627(1,2);3,623(1,3);3,620(1,2);3,612(1,0);3,605(0,9);3,505(0 ,4);3,498(0,4);3,491(2,3);3,481(0,5);3,471(0,3);2,243(0,3);2,236(0,4);2,227(0,4);2,220(0,7);2,211(0,9);2,206(0,6); 2,203(0,7);2,197(1,3);2,188(1,4);2,182(0,8);2,179(0,8);2,173(1,0);2,164(0,6);2,103(0,6);2,095(1,0);2,088(1,2);2,0 80(1,4);2,072(1,3);2,064(0,8);2,056(0,6);2,049(0,4);1,925(0,3);1,906(0,4);1,901(0,4);1,556(3,1);0,005(0,7);0,000( 14,1);-0,006(0,5) |
| Verbindung 532a erythro: 1H-NMR (400,0 MHz, CDCl3): δ = 8,341(1,2);8,321(12,7);8,320(12,5);8,309(12,9);8,307(12,7);7,519(4,3);7,360(0,9);7,309(0,9);7,292(1,8);7,286(1,4 );7,283(1,6);7,271(3,9);7,269(5,3);7,260(812,3);7,210(2,6);7,163(0,5);7,156(4,4);7,135(7,0);7,132(5,1);7,115(6,6) ;7,111(7,8);7,097(1,1);7,090(4,7);7,036(13,8);7,033(14,0);7,032(16,0);7,019(4,5);7,013(4,3);7,001(4,1);6,996(8,9 );6,992(14,5);6,988(11,4);6,979(10,3);6,975(11,6);6,969(4,0);6,866(2,4);6,861(3,4);6,856(4,5);6,852(3,3);6,845(3 ,5);6,840(2,9);6,834(3,8);6,830(2,7);6,825(1,9);5,299(12,0);4,260(11,2);4,244(11,5);4,032(1,0);4,014(1,0);3,783( 2,3);3,770(2,5);3,757(2,8);3,748(2,8);3,735(1,3);3,531(1,2);3,520(2,3);3,509(2,5);3,498(2,9);3,472(1,9);3,461(1,1 );3,351(3,1);3,336(5,6);3,328(3,8);3,320(3,4);3,313(5,7);3,297(2,9);2,194(1,1);2,180(1,7);2,173(1,4);2,167(1,5);2, 159(4,4);2,146(5,1);2,137(3,7);2,135(2,9);2,131(3,1);2,123(3,9);2,115(3,0);2,110(3,0);2,103(4,9);2,092(4,3);2,08 0(5,2);2,068(4,3);2,056(1,7);2,044(3,6);2,032(1,2);1,541(142,4);1,491(0,8);1,440(1,0);1,425(1,0);1,362(5,0);1,33 3(2,7);1,284(3,9);1,256(5,8);0,880(1,2);0,146(1,0);0,032(0,8);0,008(9,6);0,000(325,0);-0,009(9,2);-0,050(1,0);-0,150(0,9) |
| Verbindung 647a: 1H-NMR (600,1 MHz,CDCl3): δ = 8,185(0,3);8,181(0,3);8,041(0,3);8,031(0,4);8,027(0,4);8,018(0,3);7,449(0,3);7,446(0,3);7,438(0,3);7,43 3(0,4);7,261(50,0);7,211(0,4);7,197(0,7);7,058(0,3);7,054(0,3);7,051(0,3);7,047(0,5);7,037(0,4);7,032(0,4);7,025( 0,7);7,011(0,8);6,842(0,4);6,833(0,4);6,828(0,4);6,819(0,3);4,211(0,4);4,194(0,4);4,144(0,7);4,126(0,7);3,758(0,4 );3,749(0,4);3,740(0,4);1,557(3,5);0,000(13,9);-0,006(0,5) |
| Verbindung 1137a: 1H-NMR (600,1MHz,CDCl3): δ = 8,095(3,2);8,086(8,3);8,076(5,9);7,303(1,9);7,290(4,4);7,279(4,1);7,277(4,2);7,263(42,9);7,024(1,7);7,0 21(1,8);7,010(4,0);7,007(3,4);6,996(3,2);6,983(3,0);6,970(2,1);6,952(2,0);6,946(4,5);6,933(4,6);6,900(3,2);6,884( 3,2);6,700(2,4);6,691(2,4);6,671(4,7);6,663(4,6);6,528(4,4);6,487(8,6);4,193(5,8);4,181(5,9);4,027(0,4);4,012(3,0 );4,000(3,0);3,908(50,0);3,708(1,5);3,699(3,0);3,690(3,2);3,681(3,4);3,672(1,7);3,645(0,8);3,638(1,0);3,635(1,0); 3,628(1,6);3,620(1,1);3,617(1,0);3,610(0,9);3,488(3,6);3,485(2,0);3,476(2,0);3,471(2,0);3,467(1,8);3,462(2,1);3,4 58(1,8);3,453(1,6);3,445(1,5);3,378(0,9);3,370(1,0);3,362(1,3);3,354(1,2);3,345(0,9);3,337(0,8);3,298(1,9);3,288( 3,2);3,282(3,3);3,277(2,4);3,272(2,9);3,262(1,9);2,265(0,4);2,259(0,5);2,255(0,5);2,249(0,8);2,242(0,9);2,232(0,9 );2,226(1,0);2,220(0,6);2,216(0,6);2,210(0,5);2,107(0,8);2,097(1,5);2,095(1,4);2,083(2,9);2,074(4,4);2,066(4,7);2, 060(6,6);2,050(6,6);2,043(6,4);2,034(4,8);2,027(2,9);2,019(2,3);2,011(1,4);0,000(11,5) |
| Verbindung 1558a: 1H-NMR (400,0 MHz, CDCl3): δ = 7,997(2,2);7,266(38,5);7,258(12,2);7,199(1,5);7,179(3,9);7,159(6,1);7,136(6,6);7,125(2,3);7,115(4,9);7,105(20,5) ;7,093(1,9);7,067(2,6);7,061(2,8);7,049(2,8);7,042(3,8);7,035(3,0);7,022(2,6);7,017(2,7);6,997(1,3);6,992(1,7);6, 987(2,2);6,983(1,9);6,977(1,8);6,971(1,5);6,966(1,8);6,961(1,5);6,939(2,9);6,934(3,6);6,929(3,0);6,923(2,8);6,91 8(2,6);6,913(3,0);6,908(2,4);4,283(7,9);4,269(8,2);4,050(4,7);4,033(5,2);3,861(2,1);3,849(4,3);3,835(4,2);3,822(5 ,4);3,809(2,7);3,779(1,3);3,769(1,6);3,765(1,7);3,752(2,0);3,742(2,0);3,738(2,0);3,728(1,6);3,716(1,4);3,707(1,6); 3,699(1,4);3,689(2,8);3,680(3,7);3,666(4,8);3,657(3,6);3,652(5,4);3,642(5,9);3,630(5,3);3,625(3,3);3,619(3,5);3,6 14(2,8);3,603(2,4);3,592(2,2);3,517(1,5);3,506(1,8);3,491(2,6);3,483(7,9);3,466(1,5);3,455(1,4);2,961(16,0);2,87 8(13,9);2,280(0,7);2,270(0,8);2,266(0,9);2,256(1,3);2,245(1,6);2,232(2,5);2,220(2,5);2,210(2,1);2,205(1,9);2,196( 3,5);2,183(3,2);2,174(2,2);2,169(2,1);2,160(2,4);2,148(1,5);2,113(1,7);2,101(2,5);2,090(3,1);2,078(3,3);2,067(2,9 );2,055(1,7);2,043(1,4);2,031(0,9);1,937(0,9);1,927(1,6);1,917(1,0);1,910(0,9);1,900(1,9);1,892(1,7);1,882(0,8);1, 875(0,8);1,864(1,4);1,854(0,7);1,706(4,1);0,008(0,7);0,000(14,6);-0,008(0,6) |
| Verbindung 1295a: 1H-NMR (400,0 MHz, CDCl3): δ = 8,623(14,4);8,620(15,2);8,570(12,8);8,566(13,8);8,199(15,6);8,196(16,0);7,971(13,3);7,968(14,3);7,519 (4,0);7,385(7,9);7,367(6,7);7,260(668,8);7,237(14,3);7,231(13,7);7,220(11,5);7,214(10,7);7,171(18,4);7,168(19,9 );7,157(11,6);7,153(17,4);7,038(5,1);7,017(11,7);6,996(12,9);6,981(5,7);6,976(6,1);6,970(6,5);6,965(6,1);4,281(1 5,1);4,260(12,8);4,256(11,9);3,733(2,4);3,721(4,4);3,706(4,1);3,693(4,5);3,681(3,3);3,645(3,0);3,632(5,5);3,619( 5,8);3,605(8,3);3,593(7,4);3,579(7,2);3,569(8,6);3,556(6,1);3,545(5,8);3,533(3,5);3,346(3,1);3,335(4,2);3,320(6,5 );3,307(6,8);3,297(6,7);3,285(4,0);3,280(3,4);3,269(2,7);2,464(2,3);2,452(2,9);2,442(2,9);2,430(2,8);2,420(2,0);2, 229(3,2);2,202(3,7);2,194(3,2);2,167(2,6);2,058(1,4);2,047(2,2);2,035(2,1);2,023(3,6);2,012(4,8);1,999(3,6);1,98 7(4,2);1,952(2,1);1,940(3,6);1,930(3,5);1,918(4,4);1,905(3,8);1,883(2,0);1,509(8,9);0,146(1,3);0,000(252,0);-0,150(1,8) |
| Verbindung 289a erythro: 1H-NMR (400,0 MHz, CDCl3): δ = 8,401(3,2);8,395(3,4);7,975(2,5);7,380(0,9);7,375(1,1);7,369(0,8);7,358(1,0);7,352(1,2);7,347(0,8);7,269(0,5);7,2 68(0,6);7,267(0,7);7,261(42,5);7,170(0,8);7,149(1,4);7,146(0,9);7,129(1,1);7,125(1,3);7,104(0,8);7,037(0,7);7,03 1(0,7);7,019(0,7);7,012(0,9);7,005(0,7);6,993(0,6);6,987(0,7);6,890(0,5);6,887(0,7);6,881(0,9);6,877(0,7);6,871(0 ,6);6,865(0,6);6,860(0,7);6,856(0,5);5,299(16,0);4,487(2,1);4,472(2,1);3,633(0,9);3,616(0,9);3,092(0,9);3,072(0,9 );3,049(1,4);3,029(1,3);2,907(1,4);2,891(1,4);2,864(1,0);2,848(0,9);0,000(15,4) |
| Verbindung 107a: 1H-NMR (400,0 MHz, CDCl3): δ = 8,492(0,7);8,489(1,4);8,485(0,8);8,481(0,8);8,477(1,4);8,474(0,8);8,419(0,7);8,416(1,3);8,412(0,8);8,408(0,8);8,4 05(1,3);8,401(0,7);7,434(0,8);7,430(0,8);7,416(1,4);7,413(1,4);7,410(2,6);7,407(1,1);7,399(1,3);7,393(1,4);7,390( 1,2);7,386(1,1);7,295(1,3);7,2843(1,6);7,2835(1,5);7,273(1,7);7,271(0,5);7,270(0,5);7,269(0,5);7,268(0,6);7,2674 (0,7);7,2666(0,8);7,266(1,0);7,261(77,3);7,2543(1,0);7,2536(1,0);7,252(1,2);7,248(0,6);7,243(0,7);7,241(0,8);7,2 37(1,2);7,233(1,0);7,227(0,9);7,221(1,1);7,220(1,5);7,216(2,1);7,197(1,3);7,193(1,2);7,177(1,4);7,172(2,5);7,166( 3,3);7,155(2,4);7,151(3,2);7,145(1,2);7,134(0,7);7,130(1,2);7,009(1,0);7,004(1,0);6,997(1,2);6,992(1,0);6,986(1,8 );6,965(2,4);6,944(0,8);4,345(1,8);4,337(1,7);4,321(2,0);4,311(1,9);4,148(1,1);4,130(3,3);4,112(3,4);4,094(1,1);3, 906(0,7);3,896(0,6);3,892(0,8);3,889(0,6);3,881(0,8);3,661(0,8);3,648(1,0);3,634(1,0);3,620(0,6);3,535(0,7);3,52 2(1,0);3,508(1,1);3,500(0,6);3,494(0,7);3,486(0,5);3,480(0,6);3,466(0,7);3,410(0,6);3,397(0,7);3,390(0,7);3,377(0 ,7);2,410(0,9);2,406(0,6);2,401(0,6);2,396(1,0);2,387(0,9);2,374(0,9);2,043(16,0);2,036(0,5);2,024(0,5);1,841(0,5 );1,830(0,5);1,625(0,8);1,286(0,7);1,284(0,7);1,276(4,9);1,258(10,4);1,241(4,8);0,008(0,8);0,000(27,6);-0,009(0,8) |
| Verbindung 597a: 1H-NMR (400,0 MHz, CDCl3): δ = 8,373(13,1);8,185(16,0);7,421(0,8);7,419(0,7);7,406(1,0);7,402(1,7);7,400(1,4);7,388(1,5);7,384(1,6);7,381(1,2); 7,370(0,7);7,366(1,0);7,293(0,6);7,261(45,8);7,239(1,0);7,224(1,7);7,219(1,4);7,215(1,6);7,211(0,9);7,205(1,8);7, 202(1,7);7,198(1,6);7,194(1,0);7,183(1,7);7,174(1,8);7,171(2,9);7,168(2,0);7,151(2,1);7,117(3,6);7,114(3,5);7,09 5(4,4);7,092(4,2);7,076(1,2);7,074(1,2);7,070(0,7);6,997(0,5);6,969(3,1);6,954(4,2);6,949(5,1);6,941(2,5);6,936(3 ,2);6,933(3,4);6,928(2,4);6,923(1,5);6,920(1,7);6,914(0,7);5,298(2,5);4,274(3,2);4,249(3,7);4,205(3,6);4,178(4,6); 3,996(1,0);3,987(1,0);3,968(1,7);3,959(1,7);3,941(0,8);3,931(0,8);3,909(0,8);3,897(0,8);3,883(1,3);3,871(1,3);3,8 57(0,7);3,845(0,7);3,768(1,1);3,755(1,8);3,741(2,2);3,728(2,0);3,715(1,3);3,607(0,9);3,594(1,5);3,581(2,0);3,567( 1,8);3,554(1,1);3,480(1,2);3,468(1,4);3,457(1,5);3,453(1,3);3,446(1,5);3,441(1,3);3,430(1,2);3,419(1,1);3,395(1,0 );3,383(1,1);3,372(1,2);3,368(1,0);3,360(1,2);3,356(1,0);3,346(0,9);3,334(0,8);2,955(2,8);2,879(2,4);2,564(0,6);2, 554(0,7);2,550(0,7);2,541(1,0);2,529(1,2);2,518(1,2);2,506(1,3);2,497(0,8);2,493(0,7);2,483(0,7);2,317(0,5);2,30 6(1,0);2,293(0,7);2,290(0,7);2,286(0,6);2,278(1,1);2,274(1,0);2,271(1,0);2,267(0,8);2,259(0,6);2,255(0,5);2,243(0 ,7);2,073(0,6);2,061(0,5);2,050(0,8);2,047(0,8);2,042(0,7);2,038(1,0);2,035(0,8);2,024(0,7);2,012(0,8);1,932(0,6); |
| 1,919(0,9);1,906(0,8);1,897(1,2);1,884(1,1);1,874(0,5);1,862(0,6);1,516(1,5);0,008(0,8);0,000(18,0);-0,009(0,7) |
| Verbindung 599a: 1H-NMR (400,0 MHz, CDCl3): δ = 8,379(14,7);8,197(16,0);7,519(0,8);7,392(0,8);7,387(1,2);7,375(14,4);7,370(10,1);7,358(5,7);7,352(1,3);7,341(1,0 );7,336(1,7);7,295(0,5);7,280(2,3);7,275(3,5);7,270(2,3);7,269(2,3);7,267(1,9);7,260(141,7);7,254(2,3);7,236(1,1) ;7,233(1,6);7,231(1,9);7,228(2,2);7,212(9,1);7,209(9,1);7,193(3,1);7,190(2,0);7,174(4,3);7,172(3,5);7,156(1,7);7, 153(1,8);7,075(1,9);7,071(2,9);7,056(1,4);7,053(2,3);6,996(0,9);4,249(3,8);4,224(4,4);4,180(3,9);4,152(5,1);3,98 9(0,9);3,979(0,9);3,961(1,6);3,952(1,5);3,934(0,8);3,924(0,7);3,898(0,8);3,886(0,8);3,872(1,4);3,860(1,4);3,845(0 ,7);3,834(0,7);3,773(1,0);3,760(1,6);3,746(2,0);3,733(1,9);3,720(1,2);3,610(0,9);3,597(1,5);3,583(2,1);3,570(1,8); 3,557(1,2);3,481(1,1);3,470(1,2);3,458(1,3);3,454(1,2);3,447(1,3);3,442(1,2);3,431(1,1);3,420(1,0);3,396(1,1);3,3 84(1,1);3,374(1,2);3,369(1,0);3,362(1,2);3,357(1,0);3,347(0,9);3,335(0,9);2,560(0,5);2,550(0,6);2,546(0,6);2,537( 1,0);2,525(1,1);2,514(1,1);2,502(1,3);2,492(0,8);2,489(0,8);2,479(0,7);2,308(0,9);2,296(0,6);2,292(0,7);2,284(0,8 );2,280(1,0);2,277(0,9);2,273(0,9);2,269(0,8);2,261(0,5);2,257(0,5);2,245(0,7);2,068(0,6);2,045(0,8);2,041(0,9);2, 037(0,8);2,033(1,1);2,029(0,9);2,018(0,7);2,006(0,9);1,922(0,7);1,908(0,9);1,896(0,8);1,887(1,3);1,873(1,2);1,86 4(0,6);1,852(0,6);0,008(1,6);0,000(53,0);-0,009(1,6) |
| Verbindung 100a: 1H-NMR (400,0 MHz, CDCl3): δ = 8,503(1,6);8,500(2,5);8,497(1,6);8,491(1,7);8,488(2,5);8,418(2,2);8,414(1,4);8,409(1,4);8,406(2,2);7,519(0,9);7,4 69(1,1);7,465(1,2);7,447(1,8);7,445(2,4);7,424(1,5);7,421(1,5);7,325(1,6);7,313(2,5);7,303(2,3);7,292(1,9);7,282( 1,7);7,260(161,3);7,242(2,3);7,239(2,3);7,224(1,4);7,219(2,8);7,216(3,0);7,206(1,4);7,198(3,4);7,191(2,6);7,187( 3,3);7,176(3,5);7,166(1,9);7,155(0,9);7,150(1,5);7,115(1,1);7,110(1,4);7,095(0,8);7,089(0,9);7,025(0,9);7,004(1,7 );7,001(1,2);6,996(1,1);6,984(1,5);6,980(2,5);6,974(1,1);6,959(1,6);6,956(1,3);6,947(1,1);6,934(0,9);6,929(1,0);6, 881(0,9);6,876(1,2);6,871(1,0);6,865(0,9);6,854(0,9);4,351(3,0);4,337(2,6);4,326(3,3);4,311(2,9);4,150(1,2);4,13 2(3,5);4,115(3,5);4,097(1,2);3,950(0,6);3,939(0,6);3,928(1,4);3,918(1,4);3,904(1,6);3,893(1,5);3,880(0,7);3,871(0 ,6);3,696(0,7);3,682(1,3);3,668(1,5);3,654(1,8);3,641(1,0);3,557(0,8);3,543(1,5);3,529(2,1);3,515(2,7);3,501(1,7); 3,491(0,7);3,485(0,8);3,472(0,7);3,423(1,1);3,410(1,2);3,402(1,3);3,395(0,9);3,389(1,2);3,382(0,9);3,375(0,9);3,3 62(0,8);2,427(0,8);2,417(1,5);2,403(1,9);2,394(1,8);2,381(1,9);2,368(0,8);2,233(7,8);2,107(1,2);2,091(0,8);2,079( 0,8);2,067(1,0);2,056(1,3);2,046(16,0);2,032(1,1);2,019(0,5);1,882(0,6);1,867(0,7);1,862(0,7);1,857(0,7);1,847(1, 0);1,837(1,0);1,827(0,6);1,277(4,2);1,260(8,4);1,242(4,1);0,069(5,8);0,008(2,6);0,000(57,3);-0,009(2,1) |
| Verbindung 620a: 1H-NMR (400,0 MHz, CDCl3): δ = 8,381(16,0);8,369(1,1);8,243(1,1);8,214(15,4);7,519(5,4);7,293(0,6);7,289(0,8);7,260(956,7);7,250(5,0); 7,244(2,3);7,229(3,0);7,226(3,7);7,218(2,0);7,209(3,2);7,205(3,4);7,200(1,8);7,185(2,0);7,147(0,6);7,128(1,3);7,1 22(1,8);7,118(1,4);7,112(1,2);7,101(1,3);7,093(1,8);7,087(1,4);7,071(2,0);7,061(1,4);7,050(3,1);7,043(1,5);7,030( 1,9);7,026(2,2);7,006(1,7);6,996(5,4);6,921(1,6);5,299(2,1);4,265(3,9);4,240(4,4);4,184(3,4);4,157(4,4);3,974(0,8 );3,965(0,8);3,946(1,4);3,936(1,4);3,917(0,7);3,908(0,6);3,887(0,8);3,875(0,8);3,861(1,4);3,848(1,4);3,836(0,9);3, 823(0,7);3,779(0,9);3,766(1,4);3,753(1,8);3,740(1,7);3,726(1,1);3,636(1,0);3,623(1,6);3,610(2,0);3,597(1,9);3,58 3(1,2);3,478(0,9);3,467(1,1);3,456(1,1);3,444(1,2);3,428(1,0);3,411(1,0);3,399(1,1);3,388(1,2);3,377(1,2);3,362(0 ,9);3,350(0,9);2,544(0,6);2,535(0,9);2,522(1,0);2,510(1,0);2,499(1,3);2,490(0,7);2,476(0,6);2,294(0,8);2,266(0,9); 2,231(0,7);2,040(1,1);2,013(0,9);2,005(0,7);1,936(0,7);1,923(1,0);1,912(0,9);1,901(1,4);1,888(1,2);1,878(0,7);1,8 65(0,7);1,543(11,9);1,371(0,9);1,333(1,7);1,284(2,4);1,256(2,2);0,146(1,0);0,008(9,1);0,000(336,9);-0,009(9,9);-0,150(1,0) |
| Verbindung 84a: 1H-NMR (400,0 MHz, CDCl3): δ = 8,385(4,0);8,377(5,5);8,374(7,4);8,368(6,9);8,201(3,7);7,959(6,9);7,351(2,4);7,345(3,2);7,340(2,5);7,328(3,5);7,3 22(3,5);7,316(2,8);7,307(3,5);7,291(4,2);7,286(4,9);7,278(2,6);7,272(6,6);7,262(66,2);7,251(4,4);7,244(1,5);7,04 1(1,1);7,034(1,4);7,029(1,9);7,022(3,0);7,013(2,3);7,010(3,1);7,008(3,1);7,003(3,4);7,001(3,5);6,995(1,3);6,989(1 ,6);6,987(1,6);6,982(1,7);6,966(2,4);6,956(1,5);6,950(3,1);6,947(3,6);6,932(1,6);6,927(1,8);6,922(1,0);6,887(4,0); 6,868(3,8);6,858(2,8);6,853(3,1);6,848(1,8);6,835(2,4);6,830(3,0);6,825(1,8);5,299(16,0);4,307(5,9);4,292(6,1);4, 057(3,2);4,040(3,4);3,835(1,2);3,798(1,6);3,785(3,4);3,772(3,5);3,758(4,1);3,745(2,0);3,690(0,9);3,679(1,1);3,67 5(1,1);3,664(1,6);3,653(1,3);3,648(1,2);3,638(1,0);3,547(1,9);3,535(2,2);3,526(2,3);3,520(2,0);3,514(2,4);3,509(2 ,1);3,499(2,4);3,487(2,3);3,474(2,4);3,460(2,7);3,446(2,0);3,437(2,3);3,422(1,1);3,385(1,0);3,373(1,2);3,361(1,4); 3,349(1,4);3,346(1,2);3,335(1,0);3,323(0,9);2,320(0,5);2,315(0,5);2,305(0,9);2,295(1,0);2,284(0,9);2,280(1,0);2,2 70(1,2);2,259(1,1);2,245(1,5);2,237(0,8);2,231(0,9);2,223(2,2);2,209(2,6);2,202(1,6);2,195(1,5);2,188(2,0);2,174( 1,3);2,170(1,1);2,162(1,3);2,150(2,3);2,138(2,2);2,127(2,8);2,114(2,5);2,102(1,8);2,091(1,6);2,084(0,8);2,078(1,0 );2,073(1,2);2,065(1,0);2,054(0,6);2,048(0,6);2,043(0,8);2,038(0,8);2,005(0,9);1,752(1,5);1,371(0,6);1,286(0,9);1, 258(1,4);0,008(1,0);0,000(25,2);-0,009(1,2) |
| Verbindung 295a: 1H-NMR (400,0 MHz, CDCl3): δ = 8,492(0,8);8,488(1,6);8,485(0,9);8,480(0,9);8,477(1,6);8,473(0,9);8,421(0,8);8,417(1,5);8,414(0,9);8,410(0,8);8,4 06(1,5);8,403(0,8);7,557(1,4);7,551(1,5);7,541(1,4);7,535(1,5);7,431(0,9);7,428(0,9);7,410(1,3);7,407(1,5);7,404( 1,0);7,387(1,2);7,383(1,1);7,308(1,4);7,302(1,5);7,299(0,9);7,293(3,2);7,287(1,8);7,282(2,5);7,278(1,2);7,272(2,5 );7,266(1,7);7,261(74,3);7,250(1,0);7,239(0,7);7,236(0,8);7,218(1,4);7,216(0,9);7,215(1,4);7,213(0,9);7,195(1,4); 7,192(1,3);7,177(1,4);7,166(2,4);7,155(1,6);7,148(2,0);7,145(1,3);7,134(0,7);7,127(2,8);7,107(1,5);7,079(0,6);7,0 73(0,6);7,067(0,7);7,062(0,6);7,057(0,9);7,051(0,9);7,046(0,9);7,040(0,8);6,965(1,9);6,944(2,7);6,923(1,3);4,342( 2,0);4,332(1,8);4,318(2,2);4,306(2,0);4,148(1,1);4,130(3,3);4,112(3,3);4,094(1,1);3,903(0,9);3,892(0,7);3,889(0,9 );3,878(0,9);3,662(0,9);3,648(1,0);3,634(1,0);3,621(0,7);3,535(0,8);3,522(1,1);3,508(1,2);3,501(0,7);3,494(0,7);3, 486(0,6);3,481(0,7);3,467(0,7);3,453(0,5);3,411(0,6);3,398(0,7);3,390(0,7);3,383(0,5);3,377(0,7);2,419(0,5);2,40 9(0,8);2,405(0,6);2,396(1,0);2,387(1,1);2,374(1,2);2,095(0,9);2,057(0,5);2,043(16,0);2,035(0,6);2,023(0,6);1,842( 0,6);1,831(0,6);1,588(0,7);1,333(0,7);1,284(1,1);1,276(4,9);1,258(10,4);1,240(4,8);0,008(0,8);0,000(27,5);-0,009(0,7) |
| Verbindung 619a: 1H-NMR (400,0 MHz, CDCl3): δ = 8,384(14,1);8,219(16,0);7,588(2,5);7,582(2,7);7,572(2,6);7,566(2,7);7,519(1,1);7,431(2,4);7,425(2,6);7,415(2,5); 7,410(2,6);7,325(1,3);7,319(1,3);7,314(1,5);7,308(1,4);7,304(1,9);7,298(1,9);7,293(2,0);7,287(1,7);7,260(176,1); 7,191(3,4);7,170(4,9);7,150(2,8);7,122(1,3);7,116(1,2);7,111(1,3);7,105(1,3);7,101(1,7);7,095(1,7);7,089(1,7);7,0 84(1,6);7,010(3,9);6,996(1,3);6,990(5,2);6,969(2,6);4,258(3,5);4,233(4,0);4,176(3,8);4,148(4,9);3,972(0,9);3,962( 0,9);3,944(1,5);3,935(1,5);3,917(0,7);3,907(0,7);3,879(0,8);3,867(0,8);3,853(1,4);3,841(1,4);3,828(0,8);3,816(0,7 );3,780(1,1);3,767(1,7);3,753(2,0);3,740(2,0);3,727(1,3);3,632(1,0);3,619(1,6);3,606(2,1);3,593(2,0);3,580(1,3);3, 477(1,2);3,466(1,3);3,454(1,4);3,450(1,3);3,443(1,4);3,439(1,3);3,427(1,2);3,415(1,1);3,409(1,2);3,397(1,2);3,38 6(1,3);3,382(1,1);3,375(1,3);3,371(1,1);3,360(1,0);3,348(1,0);2,553(0,6);2,543(0,6);2,539(0,6);2,530(1,0);2,518(1 ,1);2,507(1,1);2,495(1,2);2,485(0,8);2,482(0,8);2,472(0,6);2,297(0,9);2,282(0,6);2,270(1,0);2,266(0,9);2,262(0,9); 2,258(0,8);2,234(0,7);2,071(0,7);2,059(0,5);2,047(0,8);2,044(0,9);2,036(1,1);2,021(0,8);2,009(0,9);1,997(0,5);1,9 28(0,7);1,915(1,0);1,904(0,9);1,893(1,3);1,880(1,2);1,870(0,7);1,858(0,7);1,845(0,5);1,672(0,8);1,621(0,6);0,008( 2,0);0,000(66,8);-0,009(2,0) |
| Verbindung 289a: 1H-NMR (400,0 MHz, CDCl3): δ = 7,518(15,3);7,294(5,1);7,260(2831,6);7,227(5,7);7,209(4,0);6,996(16,0);1,537(809,6);1,256(2,7);0,146(3,8);0,069 (19,1);0,008(40,2);0,000(960,5);-0,008(36,7);-0,150(3,9) |
| Verbindung 296a: 1H-NMR (400,0 MHz, CDCl3): δ = 8,410(3,1);8,403(7,8);8,396(5,2);8,205(2,5);7,969(4,4);7,519(3,1);7,373(2,2);7,365(1,3);7,359(1,9);7,353(1,7);7,3 42(1,5);7,336(1,9);7,331(1,5);7,289(1,9);7,283(2,1);7,278(1,7);7,272(4,2);7,260(579,5);7,244(1,7);7,227(0,9);7,2 09(0,9);7,197(2,7);7,191(2,8);7,181(2,6);7,175(2,6);7,121(1,2);7,100(3,7);7,093(2,9);7,079(2,5);7,072(6,5);7,050( 4,8);7,044(1,2);7,039(1,3);7,033(1,1);7,029(0,7);7,023(0,6);7,018(0,6);7,011(0,6);6,996(3,3);6,955(1,6);6,949(1,6 );6,945(1,8);6,939(1,6);6,934(1,3);6,928(1,3);6,923(1,2);6,917(1,1);5,299(16,0);4,286(3,8);4,271(3,9);4,039(2,3); 4,022(2,5);3,835(0,9);3,814(1,0);3,802(2,3);3,788(2,2);3,775(2,9);3,762(1,3);3,713(0,6);3,703(0,8);3,687(1,0);3,6 76(0,9);3,672(0,8);3,662(0,7);3,561(1,2);3,550(1,5);3,540(1,4);3,535(1,3);3,529(1,5);3,523(1,3);3,513(1,2);3,502( 1,1);3,440(1,1);3,426(1,5);3,404(1,4);3,387(1,2);3,374(0,9);3,360(0,9);3,347(0,7);3,336(0,7);2,615(1,1);2,289(0,7 );2,278(0,6);2,264(0,7);2,254(0,9);2,235(0,7);2,229(0,8);2,221(0,6);2,213(1,5);2,200(1,5);2,192(1,1);2,186(1,0);2, 178(1,2);2,170(0,9);2,164(0,8);2,155(0,9);2,143(1,4);2,131(1,3);2,120(1,7);2,107(1,5);2,098(0,7);2,087(0,9);2,07 1(0,8);2,060(0,8);2,053(0,6);2,044(0,6);2,025(0,6);2,005(0,6);1,552(6,2);1,333(0,9);1,284(1,0);1,259(0,9);0,146(0 ,6);0,113(0,8);0,008(6,2);0,000(194,3);-0,009(5,5);-0,150(0,6) |
| Verbindung 613a: 1H-NMR (400,0 MHz, CDCl3): δ = 8,381(16,0);8,369(1,2);8,243(1,1);8,214(15,4);7,519(5,4);7,293(0,7);7,289(0,8);7,260(956,7);7,250(5,0);7,244(2,3 );7,229(3,0);7,226(3,7);7,218(2,0);7,209(3,2);7,205(3,4);7,200(1,9);7,185(2,0);7,147(0,6);7,128(1,3);7,122(1,8);7, 118(1,4);7,112(1,2);7,101(1,3);7,093(1,8);7,087(1,4);7,071(2,0);7,061(1,4);7,050(3,1);7,043(1,5);7,030(1,9);7,02 6(2,2);7,006(1,7);6,996(5,4);6,921(1,6);5,299(2,1);4,265(4,0);4,240(4,4);4,184(3,4);4,157(4,4);3,974(0,8);3,965(0 ,8);3,946(1,4);3,936(1,4);3,917(0,7);3,908(0,6);3,887(0,8);3,875(0,8);3,861(1,4);3,848(1,4);3,836(0,9);3,823(0,7); 3,779(0,9);3,766(1,4);3,753(1,8);3,740(1,8);3,726(1,1);3,636(1,0);3,623(1,6);3,610(2,0);3,597(2,0);3,583(1,2);3,4 78(0,9);3,467(1,1);3,456(1,2);3,444(1,2);3,428(1,0);3,411(1,0);3,399(1,1);3,388(1,2);3,377(1,2);3,362(0,9);3,350( 0,9);2,544(0,6);2,535(0,9);2,522(1,0);2,510(1,0);2,499(1,3);2,490(0,7);2,476(0,6);2,294(0,8);2,266(0,9);2,231(0,7 );2,040(1,1);2,013(0,9);2,005(0,7);1,936(0,7);1,923(1,0);1,912(0,9);1,901(1,4);1,888(1,2);1,878(0,7);1,865(0,7);1, 543(11,9);1,371(0,9);1,333(1,7);1,284(2,4);1,256(2,2);0,146(1,0);0,008(9,1);0,000(336,9);-0,009(9,9);-0,150(1,0) |
| Verbindung 86a: 1H-NMR (400,0 MHz, CDCl3): δ = 8,399(5,2);8,392(5,9);8,388(7,3);8,381(7,0);8,213(3,8);7,967(6,9);7,519(1,3);7,347(2,5);7,342(3,2);7,336(2,6);7,3 24(2,9);7,319(4,3);7,313(4,2);7,305(3,8);7,295(5,5);7,290(4,5);7,285(7,2);7,280(10,6);7,277(8,4);7,260(230,5);7, 250(9,2);7,242(3,1);7,231(7,3);7,226(3,4);7,221(4,7);7,216(2,8);7,211(3,4);7,175(1,0);7,137(4,4);7,133(7,2);7,12 8(3,9);7,061(2,7);7,042(2,0);6,996(1,8);6,959(3,8);6,940(3,4);5,299(16,0);4,491(0,6);4,477(0,6);4,278(5,6);4,263( 5,8);4,038(3,3);4,021(3,5);3,835(1,0);3,802(1,5);3,789(3,1);3,776(3,2);3,762(3,8);3,749(1,9);3,695(0,9);3,684(1,4 );3,669(1,6);3,658(1,3);3,654(1,2);3,643(1,2);3,553(1,7);3,542(2,0);3,532(2,1);3,526(1,8);3,520(2,1);3,515(1,7);3, 505(1,6);3,493(2,0);3,473(0,9);3,463(2,2);3,447(2,7);3,437(2,1);3,425(2,2);3,408(1,1);3,389(1,0);3,377(1,0);3,36 5(1,3);3,353(1,3);3,339(0,9);3,327(0,8);2,964(0,8);2,945(0,8);2,911(0,5);2,315(0,5);2,300(0,6);2,291(0,9);2,281(1 ,0);2,266(1,0);2,255(1,4);2,240(1,4);2,232(1,3);2,225(0,9);2,218(2,1);2,204(2,5);2,196(1,5);2,190(1,5);2,183(1,9); 2,168(1,2);2,158(1,3);2,145(2,3);2,134(2,0);2,122(2,7);2,110(2,5);2,099(1,8);2,087(1,4);2,074(1,4);2,065(0,9);2,0 48(0,6);2,037(0,8);1,570(1,7);1,371(1,1);1,333(0,9);1,286(1,7);1,257(2,5);0,880(0,5);0,008(3,5);0,000(88,5);-0,009(3,7) |
| Verbindung 619bab: 1H-NMR (400,0 MHz, CDCl3): δ = 8,421(9,4);8,250(10,9);7,595(1,5);7,590(1,6);7,580(1,5);7,574(1,5);7,417(1,6);7,411(1,7);7,401(1,6);7,396(1,6);7, 328(0,7);7,322(0,7);7,317(0,8);7,311(0,7);7,307(1,1);7,301(1,1);7,296(1,1);7,290(1,0);7,261(61,7);7,217(1,9);7,1 96(2,6);7,176(1,3);7,113(0,7);7,107(0,7);7,102(0,8);7,096(0,8);7,092(1,1);7,086(1,1);7,081(1,1);7,075(0,9);7,019( 2,2);6,999(3,0);6,978(1,4);4,267(0,6);4,254(1,3);4,239(1,2);4,225(1,7);4,216(2,4);4,190(2,5);4,164(2,5);4,136(3,1 );4,117(1,3);4,102(1,2);4,088(1,5);4,075(0,9);4,046(0,9);4,034(1,0);4,024(1,1);4,017(0,9);4,012(1,0);4,006(0,8);3, 995(0,7);3,983(0,7);3,961(16,0);3,926(14,2);3,908(0,8);3,901(0,7);3,896(0,8);3,890(0,6);3,879(0,6);3,868(0,6);3, 845(0,6);3,836(0,6);3,817(1,0);3,807(1,0);3,789(0,5);3,779(0,5);3,769(0,5);3,741(0,8);3,731(0,8);2,682(0,6);2,66 9(0,8);2,659(0,8);2,646(0,8);2,637(0,5);2,464(0,5);2,437(0,6);2,183(0,6);2,015(0,6);2,004(0,6);1,992(0,7);1,980(0 ,7);1,586(0,6);1,255(0,7);0,008(0,8);0,000(23,4);-0,008(0,8) |
| Verbindung 599bae: 1H-NMR (400,0 MHz, CDCl3): δ = 8,409(5,4);8,215(5,9);7,400(1,0);7,396(2,3);7,394(2,0);7,392(1,5);7,378(3,0);7,274(0,8);7,269(1,2);7,261(39,2);7, 252(1,0);7,247(0,6);7,242(0,5);7,240(0,5);7,237(0,6);7,226(0,7);7,222(1,0);7,221(1,3);7,217(1,1);7,196(2,7);7,17 7(1,7);7,157(0,7);7,052(0,9);7,033(0,7);4,214(1,4);4,206(0,7);4,188(1,8);4,177(0,9);4,163(1,7);4,135(1,7);4,058(0 ,6);4,043(0,6);4,029(1,2);4,016(0,7);3,922(8,4);3,889(0,5);3,882(4,2);3,878(4,3);3,849(0,5);3,822(0,5);3,812(0,5); 3,407(16,0);3,387(14,7);0,000(14,8) |
| Verbindung 289a threo-1: 1H-NMR (400,0 MHz, CDCl3): δ = 8,395(14,8);8,389(14,9);8,193(14,6);7,521(1,2);7,295(0,8);7,285(0,6);7,284(0,6);7,283(0,6);7,2823(0,5); 7,2815(0,5);7,281(0,6);7,2783(0,9);7,2775(0,9);7,277(1,0);7,276(1,2);7,272(6,5);7,2663(10,5);7,2655(11,5);7,262 (205,0);7,256(2,0);7,255(1,7);7,254(1,5);7,253(1,4);7,252(1,4);7,249(5,9);7,244(7,3);7,238(5,5);7,155(5,5);7,134( 9,2);7,131(6,3);7,114(7,2);7,110(9,2);7,089(6,6);7,083(5,1);7,077(5,3);7,065(5,2);7,059(6,1);7,057(6,0);7,050(5,4 );7,038(4,9);7,032(5,1);6,998(1,2);6,923(3,3);6,918(4,3);6,913(6,2);6,908(4,4);6,902(4,4);6,897(3,7);6,892(5,0);6, 887(3,5);6,882(2,5);5,299(8,7);4,045(15,0);4,027(16,0);3,708(4,0);3,698(4,8);3,694(4,9);3,682(6,6);3,671(5,8);3, 667(5,6);3,657(4,8);3,482(2,7);3,472(2,9);3,464(2,8);3,454(5,3);3,445(3,1);3,437(2,9);3,427(2,6);3,391(4,8);3,38 0(5,6);3,367(6,2);3,365(5,4);3,356(6,1);3,353(5,8);3,341(4,8);3,329(4,4);2,323(2,1);2,313(2,3);2,309(2,5);2,299(4 ,3);2,288(4,4);2,285(2,9);2,278(3,4);2,274(4,4);2,264(5,6);2,254(3,1);2,250(2,9);2,240(2,6);2,096(2,7);2,085(5,0); 2,074(2,9);2,068(2,8);2,058(5,3);2,050(4,3);2,047(3,4);2,039(2,1);2,033(2,2);2,022(3,7);2,011(1,9);1,592(5,5);1,3 33(1,0);1,284(1,4);1,255(1,6);0,008(2,0);0,000(66,6);-0,009(2,0) |
| Verbindung 512a: 1H-NMR (400,0 MHz, CDCl3): δ = 8,564(1,2);8,544(11,3);8,529(3,7);8,495(16,0);8,471(1,0);8,440(0,7);8,425(1,0);8,412(7,1);8,400(7,1);8,393(2,6); 8,381(2,6);8,372(10,3);8,360(10,3);7,521(0,9);7,500(4,9);7,495(5,3);7,484(5,1);7,478(5,3);7,453(3,8);7,447(4,1); 7,436(3,9);7,430(4,1);7,373(0,8);7,333(3,3);7,327(3,3);7,322(3,5);7,316(3,6);7,311(4,6);7,305(4,5);7,300(4,5);7,2 94(4,6);7,286(3,0);7,281(3,3);7,275(3,5);7,270(5,2);7,262(109,3);7,254(4,7);7,242(1,5);7,219(6,0);7,202(11,1);7, 198(12,8);7,187(8,0);7,181(12,3);7,176(4,7);7,160(5,4);7,127(0,7);7,105(8,9);7,092(8,6);6,998(0,8);5,299(5,0);4, 417(7,0);4,402(7,1);4,052(5,4);4,034(5,5);3,868(0,5);3,851(0,5);3,811(3,1);3,801(3,1);3,784(3,4);3,773(3,4);3,72 7(0,5);3,690(13,9);3,564(3,2);3,546(3,4);3,537(5,8);3,519(6,0);3,501(3,9);3,489(4,1);3,481(6,1);3,472(8,7);3,462( 3,5);3,454(3,5);3,445(3,1);3,090(1,7);3,071(3,2);3,051(2,1);3,021(3,2);3,011(3,4);2,987(4,8);2,977(4,8);2,885(1,8 );2,866(5,4);2,852(4,7);2,838(5,9);2,827(7,5);2,811(5,2);2,804(4,4);2,792(1,7);2,777(1,4);2,702(2,3);2,683(3,6);2, 664(1,9);2,494(0,9);2,483(1,5);2,470(2,0);2,465(1,9);2,460(2,2);2,451(2,2);2,446(1,9);2,441(2,4);2,430(2,3);2,42 2(2,0);2,413(2,7);2,404(2,5);2,397(2,6);2,387(2,4);2,379(1,5);2,370(1,3);2,360(0,7);1,840(1,0);1,255(0,5);0,008(1 ,6);0,000(40,9);-0,009(1,7) |
| Beispiel 1808(lba): ¹H-NMR(400,0 MHz, CDCl₃): δ = 7,835(12,2);7,822(15,8);7,520(1,4);7,301(3,3);7,295(3,3);7,290(0,7);7,284(3,4);7,278(3,9);7,261(251,6);7,254(6,1 );7,248(5,0);7,237(4,5);7,231(4,6);7,128(5,3);7,107(14,7);7,086(1,0);7,078(2,8);7,072(2,5);7,066(2,8);7,061(2,6); 7,056(1,4);7,050(1,3);7,044(3,7);7,039(3,6);7,033(3,1);7,027(2,8);7,023(1,9);7,016(1,8);7,012(1,7);7,006(1,6);6,9 97(1,5);6,923(0,5);6,852(16,0);6,835(11,7);4,225(6,0);4,210(6,2);4,133(4,8);4,114(5,1);3,813(1,7);3,801(3,9);3,78 7(3,6);3,774(4,8);3,761(2,4);3,757(1,6);3,746(1,8);3,743(1,7);3,730(2,1);3,719(2,1);3,716(2,0);3,705(1,6);3,621(1 ,6);3,601(3,4);3,595(2,6);3,589(4,9);3,582(3,9);3,573(4,2);3,571(3,5);3,563(3,3);3,557(2,9);3,547(2,4);3,544(1,7); 3,530(1,9);3,491(1,7);3,480(1,9);3,467(2,0);3,464(1,7);3,456(2,0);3,453(1,8);3,440(1,6);3,429(1,5);2,232(0,6);2,2 22(0,6);2,218(0,7);2,208(1,3);2,197(1,5);2,187(1,3);2,183(1,4);2,173(2,2);2,163(1,2);2,159(1,2);2,149(1,0);2,119( 3,8);2,112(1,6);2,105(6,0);2,100(7,1);2,091(3,3);2,087(7,1);2,074(2,8);2,070(3,5);2,067(1,9);2,064(1,6);2,056(0,7 );2,050(0,8);2,039(1,3);2,028(0,6);1,528(1,8);0,008(3,1);0,000(98,2);-0,009(3,0) |
| Beispiel 1774(lba): ¹H-NMR(400,0 MHz, CDCl3): δ = 8,741(9,4);8,726(15,0);8,004(1,0);7,586(9,7);7,467(16,0);7,265(66,6);7,160(1,7);7,142(3,3);7,139(3,3);7,135(2,1) ;7,121(5,3);7,118(5,3);7,115(3,4);7,101(3,9);7,097(5,3);7,094(4,0);7,087(2,0);7,076(4,2);7,069(2,1);7,061(1,8);7, 048(1,6);7,043(1,6);7,010(2,5);7,004(2,7);6,992(2,6);6,986(3,3);6,977(2,8);6,965(2,6);6,960(3,7);6,950(2,1);6,94 6(1,5);6,940(1,5);6,934(1,2);6,929(1,6);6,925(1,2);6,920(0,8);6,893(1,8);6,889(2,4);6,884(3,1);6,879(2,4);6,873(2 ,4);6,868(2,1);6,863(2,6);6,858(1,9);6,853(1,3);4,293(7,5);4,279(7,8);4,041(4,7);4,024(5,4);3,834(2,2);3,822(5,2); 3,816(1,8);3,808(5,2);3,795(7,2);3,786(5,0);3,782(4,4);3,777(3,1);3,771(3,1);3,763(3,9);3,749(3,1);3,739(1,7);3,7 35(1,7);3,725(1,8);3,722(2,0);3,712(1,9);3,708(1,9);3,698(1,6);3,603(2,7);3,592(3,1);3,581(3,1);3,576(2,6);3,570( 3,2);3,565(2,6);3,554(2,4);3,543(2,3);3,483(3,6);3,469(1,6);3,458(1,8);3,445(2,1);3,443(1,9);3,434(2,1);3,432(1,9 );3,418(1,6);3,407(1,4);2,958(8,7);2,880(7,4);2,879(7,4);2,352(0,7);2,343(0,8);2,338(0,8);2,328(1,5);2,318(1,5);2, 314(1,0);2,308(1,1);2,304(1,5);2,293(1,8);2,284(1,0);2,279(1,0);2,274(1,1);2,270(1,0);2,261(1,4);2,252(1,1);2,24 7(1,2);2,238(3,0);2,225(3,4);2,217(2,1);2,212(1,9);2,203(2,5);2,190(1,6);2,158(1,7);2,147(2,5);2,135(3,1);2,123(3 ,3);2,111(2,9);2,099(1,8);2,088(1,3);2,076(0,9);2,013(0,9);2,003(1,6);1,993(0,9);1,985(1,0);1,975(1,7);1,968(1,5); 1,965(1,2);1,958(0,8);1,951(0,8);1,940(1,3);1,930(0,7);1,816(4,8);0,008(0,9);0,000(25,3);-0,009(0,8) |
| Beispiel 192(lba): ¹H-NMR(400,0 MHz, CDCl3): δ = 8,579(6,2);8,575(6,5);8,568(6,5);8,564(6,4);8,524(6,7);8,520(6,8);8,512(6,9);8,508(6,8);7,734(7,2);7,730(7,3);7,7 14(7,9);7,710(7,7);7,525(8,3);7,521(8,4);7,519(2,9);7,505(9,2);7,501(8,9);7,383(2,3);7,368(2,5);7,363(4,5);7,349( 4,6);7,343(3,3);7,329(3,1);7,293(0,6);7,260(329,1);7,225(8,3);7,214(8,2);7,205(10,0);7,194(7,9);7,190(3,1);7,185 (5,3);7,170(9,4);7,165(4,2);7,155(3,4);7,150(6,8);7,107(3,1);7,104(12,0);7,097(3,5);7,092(9,6);7,084(10,9);7,079( 4,5);7,072(12,1);7,068(2,3);7,066(2,0);7,053(4,5);7,051(4,4);7,047(3,4);7,045(3,0);7,033(2,3);7,030(2,2);7,026(1, 7);7,024(1,6);6,996(1,9);6,971(3,9);6,968(5,1);6,965(4,7);6,952(3,4);6,948(4,3);6,946(3,8);6,926(2,1);6,924(2,1); 6,920(2,7);6,918(2,5);6,905(3,4);6,903(3,5);6,899(4,7);6,897(4,3);6,884(2,0);6,880(4,5);6,875(6,2);6,869(2,5);6,8 56(3,7);6,852(4,1);6,846(2,4);4,395(7,9);4,370(16,0);4,346(8,9);4,181(2,7);4,170(5,1);4,157(5,1);4,146(8,8);4,13 5(5,1);4,121(4,4);4,111(2,1);3,630(2,1);3,615(3,1);3,602(5,5);3,588(4,9);3,574(3,2);3,499(4,6);3,485(6,0);3,480(3 ,8);3,471(5,8);3,466(4,2);3,457(6,9);3,452(2,9);3,442(3,3);3,438(2,7);3,399(3,3);3,385(3,7);3,379(3,8);3,371(2,2); 3,366(3,6);3,358(2,2);3,352(2,3);3,338(2,0);2,467(0,6);2,454(1,2);2,440(0,8);2,433(2,5);2,419(4,4);2,413(2,8);2,4 10(3,2);2,406(2,9);2,402(3,3);2,396(5,1);2,387(3,1);2,383(4,7);2,378(2,8);2,368(2,6);2,359(0,8);2,348(0,5);2,344( 0,7);2,105(1,2);2,091(2,3);2,081(1,3);2,078(1,4);2,071(1,8);2,067(2,4);2,057(3,2);2,047(1,7);2,043(1,6);2,033(3,0 );2,019(1,4);2,005(0,6);1,873(1,5);1,863(1,7);1,858(1,7);1,854(1,8);1,848(1,8);1,843(1,9);1,839(2,6);1,828(2,6);1, 824(1,5);1,819(1,5);1,813(1,3);1,809(1,4);1,804(1,2);1,793(1,2);1,622(4,0);0,008(3,8);0,000(122,5);-0,009(3,6) |
| Beispiel 215(lba): ¹H-NMR(400,0 MHz, CDCl3): δ = 8,577(10,7);8,573(11,0);8,565(11,1);8,562(10,9);8,524(6,5);8,520(6,7);8,512(6,8);8,509(6,5);7,729(11,1);7,725(1 1,2);7,708(12,4);7,705(11,8);7,539(8,0);7,535(7,9);7,518(15,9);7,515(8,7);7,414(8,9);7,409(9,8);7,398(9,1);7,392 (9,5);7,359(1,4);7,292(2,8);7,260(1373,1);7,251(10,7);7,245(7,0);7,239(5,8);7,235(7,8);7,227(15,1);7,224(8,3);7, 216(14,9);7,210(5,7);7,207(12,2);7,196(13,8);7,192(6,9);7,181(6,4);7,175(6,8);7,166(13,1);7,158(1,8);7,145(21,2 );7,124(8,8);7,115(9,4);7,103(9,0);7,094(8,5);7,083(8,6);7,046(2,6);7,040(2,5);7,035(2,9);7,029(2,6);7,025(4,6);7, 019(4,5);7,013(4,6);7,008(4,4);6,996(8,2);6,984(8,8);6,963(12,5);6,942(4,6);6,914(1,0);4,362(13,3);4,357(9,1);4, 338(16,0);4,332(10,9);4,163(2,9);4,144(7,3);4,133(5,4);4,128(3,7);4,120(10,4);4,109(8,1);4,103(2,9);4,096(3,7);4 ,085(3,6);3,637(2,4);3,623(4,6);3,609(5,6);3,596(6,3);3,581(3,2);3,524(3,3);3,510(6,7);3,496(7,6);3,492(4,3);3,48 2(10,3);3,476(5,7);3,468(5,7);3,458(3,7);3,448(3,5);3,430(2,6);3,415(5,5);3,401(6,5);3,395(6,4);3,387(3,9);3,382( 6,1);3,374(3,9);3,367(4,2);3,354(3,6);2,413(4,9);2,398(7,4);2,382(6,8);2,379(8,4);2,365(4,9);2,110(1,9);2,097(3,6 );2,087(2,2);2,073(4,2);2,062(5,0);2,052(2,8);2,038(4,6);2,025(2,2);2,005(2,4);1,882(2,8);1,870(3,1);1,866(3,3);1, 862(3,3);1,855(3,1);1,851(3,6);1,847(4,9);1,836(4,7);1,832(2,8);1,827(2,8);1,821(2,6);1,816(2,5);1,801(2,0);1,58 |
| 7(6,3);0,157(2,0);0,146(1,6);0,008(16,7);0,000(516,3);-0,009(15,4);-0,050(1,6);-0,149(1,6) |
| Beispiel 1484(lba): ¹H-NMR(400,0 MHz, CDCl3): δ = 8,003(1,8);7,301(2,2);7,295(2,4);7,284(2,4);7,279(2,3);7,263(50,3);7,220(4,5);7,214(4,8);7,203(4,6);7,197(4,6);7, 131(1,3);7,110(6,2);7,089(14,8);7,083(2,2);7,077(2,4);7,068(7,4);7,062(0,8);7,056(0,6);7,050(0,6);7,019(3,1);7,0 14(3,1);7,009(3,5);7,000(6,4);6,997(6,9);6,992(2,2);6,987(2,1);6,981(2,0);6,973(11,3);6,969(11,7);6,718(14,0);6, 717(14,2);6,715(14,3);4,190(6,7);4,176(7,0);4,010(3,4);3,993(3,7);3,813(1,8);3,801(4,0);3,787(3,7);3,774(5,2);3, 761(2,5);3,750(0,9);3,741(1,1);3,736(1,1);3,724(1,4);3,714(1,3);3,709(1,3);3,699(1,1);3,623(2,4);3,612(2,7);3,60 2(2,8);3,596(2,2);3,590(2,8);3,585(2,2);3,575(2,6);3,564(2,9);3,556(1,0);3,546(3,2);3,532(3,6);3,522(2,5);3,517(3 ,5);3,508(3,8);3,493(3,1);3,484(15,8);3,479(1,4);3,466(1,1);3,454(1,0);2,958(16,0);2,880(13,6);2,879(13,4);2,279 (0,5);2,270(0,6);2,264(0,6);2,255(1,1);2,244(0,9);2,240(0,6);2,235(0,8);2,230(0,9);2,220(1,3);2,211(0,7);2,205(0, 6);2,196(0,6);2,189(0,7);2,176(1,2);2,168(0,7);2,163(0,9);2,154(2,6);2,141(3,5);2,133(2,0);2,127(1,8);2,119(2,9); 2,114(1,8);2,106(1,9);2,102(3,3);2,090(3,1);2,078(3,4);2,066(2,6);2,054(1,2);2,042(1,2);2,030(0,6);2,018(0,6);2,0 07(1,0);1,996(0,7);1,989(0,6);1,983(0,7);1,979(1,1);1,972(0,9);1,968(0,7);1,954(0,5);1,944(0,8);1,496(6,6);0,008( 0,6);0,000(19,0) |
| Beispiel 127 threo: ¹H-NMR(600,1 MHz, CDCl3): δ = 7,660(1,8);7,648(3,2);7,635(2,0);7,444(0,5);7,432(0,4);7,282(3,0);7,269(3,4);7,261(50,0);7,211(3,3) ;7,205(1,1);7,199(3,4);7,194(1,7);7,189(1,4);7,183(1,9);7,180(1,6);7,175(0,9);7,172(0,9);7,166(1,2);7,152(0,4); 7,088(1,1);7,084(1,2);7,074(0,9);7,017(0,3);7,001(0,4);6,819(0,4);6,806(0,4);4,362(0,4);4,345(0,4);4,270(2,7); 4,254(2,8);3,555(0,7);3,548(1,0);3,538(1,4); 3,529(1,2);3,520(0,8);3,456(0,7);3,449(0,8);3,439(1,4);3,432(1,3);3,422(0,9);3,415(0,7);3,304(0,8);3,296(0,9);3,2 88(1,1);3,286(0,9);3,280(1,0);3,278(0,9);3,270(0,8);3,262(0,7);2,003(0,4);1,995(0,7);1,987(0,5);1,978(0,8);1,972( 1,0);1,964(0,6);1,955(0,8);1,947(0,4);1,771(0,4);1,764(0,5);1,761(0,5);1,755(0,8);1,748(0,8);1,739(0,8);1,732(0,6 );1,725(0,5);1,723(0,4);1,716(0,3);1,528(4,5);0,005(0,8);0,000(14,4);-0,006(0,5) |
| Beispiel127erythro: ¹H-NMR(600,1 MHz, CDCl3): δ = 7,457(1,2);7,444(2,2);7,431(1,4);7,261(50,0);7,166(2,0);7,152(1,8);7,032(0,5);7,017(1,5);7,004(1,4);7,01(1,7);6,9 95(0,6);6,987(1,0);6,983(0,5);6,864(0,8);6,857(0,6);6,850(0,6);6,819(1,9);6,807(1,8);4,362(1,8);4,346(1,9);3,701( 0,5);3,694(0,7);3,691(0,6);3,684(0,8);3,675(0,7);3,673(0,7);3,666(0,5);3,445(0,5);3,440(0,5);3,428(0,9);3,423(0,9 );3,410(0,5);3,405(0,5);3,351(0,5);3,343(0,6);3,335(0,8);3,327(0,8);3,317(0,5);3,309(0,5);2,404(0,5);2,397(0,5);2, 388(0,5);2,381(0,6);2,375(0,4);2,371(0,3);2,271(0,3);2,263(0,6);2,256(0,4);2,252(0,4);2,245(0,7);2,240(0,6);2,22 2(0,4);1,546(10,9);0,005(0,7);0,000(14,2) |
| Beispiel1758(lba):¹H-NMR(400,0MHz,CDCl₃): δ = 8,730(9,1);8,710(15,1);8,004(0,7);7,588(9,6);7,449(16,0);7,334(1,6);7,319(1,8);7,312(3,8);7,297(4,1);7,291(5,1); 7,277(5,2);7,271(4,2);7,265(70,6);7,257(3,8);7,049(1,2);7,047(1,4);7,043(1,4);7,041(1,6);7,032(2,2);7,030(2,6);7, 026(4,7);7,022(3,0);7,020(3,3);7,011(6,1);7,009(7,3);7,005(6,1);7,003(4,9);6,999(2,0);6,991(4,6);6,984(2,2);6,98 2(2,1);6,975(2,3);6,970(2,5);6,964(1,5);6,952(2,2);6,946(2,7);6,941(4,6);6,940(4,7);6,937(5,2);6,935(4,8);6,919(3 ,6);6,918(4,6);6,916(4,4);6,892(3,3);6,887(4,1);6,881(2,5);6,869(3,2);6,863(4,1);6,858(2,5);4,300(8,2);4,286(8,6); 4,063(4,9);4,046(5,7);3,842(1,2);3,833(1,5);3,826(2,9);3,823(3,2);3,813(5,7);3,810(6,6);3,804(3,6);3,797(6,5);3,7 89(5,0);3,783(6,5);3,775(2,2);3,770(3,0);3,730(1,3);3,720(1,6);3,716(1,6);3,704(2,0);3,693(1,9);3,689(1,8);3,679( 1,6);3,596(2,7);3,585(3,1);3,575(3,1);3,569(2,6);3,563(3,2);3,558(2,6);3,548(2,5);3,536(2,3);3,481(4,2);3,458(1,6 );3,447(1,8);3,434(2,1);3,432(1,7);3,423(2,0);3,420(1,8);3,407(1,6);3,396(1,4);2,9563(6,8);2,9558(6,7);2,879(5,8) ;2,878(5,8);2,354(0,7);2,345(0,8);2,339(0,8);2,330(1,6);2,319(1,3);2,315(0,9);2,310(1,1);2,305(1,3);2,295(1,9);2, 286(1,0);2,280(1,1);2,277(1,2);2,271(1,0);2,263(1,7);2,255(1,1);2,250(1,2);2,242(3,3);2,228(3,8);2,220(2,1);2,21 5(1,9);2,206(2,8);2,193(1,6);2,157(1,7);2,146(2,7);2,134(3,2);2,122(3,6);2,110(3,1);2,098(1,8);2,086(1,5);2,074(0 ,9);2,027(0,9);2,017(1,4);2,006(1,0);1,999(0,9);1,992(1,0);1,989(1,5);1,982(1,3);1,978(1,1);1,971(0,8);1,964(0,8); 1,954(1,2);1,943(0,7);1,808(3,8);0,008(0,8);0,000(27,4);-0,009(0,8) |
| Beispiel181(lba):¹H-NMR(400,0MHz,CDCl₃): δ = 8,523(3,7);8,520(1,5);8,517(1,6);8,513(3,7);8,481(5,3);8,472(5,3);7,263(50,0);7,259(12,4);7,200(1,1);7,186(2,3); 7,180(1,4);7,176(1,6);7,170(2,8);7,166(4,8);7,162(5,3);7,157(5,1);7,154(4,0);7,147(1,2);7,086(0,3);7,063(1,6);7,0 61(1,3);7,057(1,2);7,046(2,2);7,032(2,7);7,016(2,6);7,002(1,5);6,990(1,4);6,987(1,5);6,978(1,5);6,974(2,0);6,969( 1,5);6,960(1,4);6,957(1,4);6,930(6,1);6,927(5,8);6,866(1,9);6,863(1,6);6,859(1,5);6,852(1,6);4,381(4,8);4,364(5,0 );4,317(3,8);4,301(3,9);3,684(1,3);3,677(1,8);3,675(1,8);3,667(2,2);3,658(2,1);3,657(2,0);3,649(1,5);3,563(1,0);3, 554(1,7);3,545(2,1);3,536(2,0);3,527(1,2);3,488(4,1);3,458(1,0);3,451(1,1);3,442(2,0);3,435(2,4);3,426(1,3);3,42 0(2,9);3,415(2,5);3,404(1,4);3,398(1,3);3,377(1,5);3,370(1,7);3,361(2,0);3,359(1,8);3,354(2,0);3,352(1,8);3,343(1 ,5);3,336(1,4);3,330(1,2);3,322(1,3);3,315(1,4);3,312(1,3);3,307(1,4);3,304(1,3);3,297(1,2);3,289(1,0);2,403(0,6); 2,397(0,7);2,393(0,7);2,387(1,2);2,380(1,3);2,371(1,3);2,364(1,6);2,358(1,0);2,354(0,9);2,349(0,8);2,261(0,9);2,2 54(1,7);2,246(1,0);2,243(1,0);2,236(1,9);2,230(1,6);2,223(0,7);2,220(0,7);2,213(1,2);2,205(0,6);2,001(0,7);1,994( 1,2);1,985(1,2);1,977(1,7);1,970(2,0);1,962(1,7);1,953(2,1);1,945(1,7);1,915(2,2);1,833(0,9);1,825(1,1);1,817(1,2 );1,810(1,4);1,801(1,4);1,794(0,9);1,787(0,8);1,778(0,6);0,005(0,6);0,000(12,8);-0,006(0,5) |
| Beispiel1515(lba):¹H-NMR(400,0MHz,CDCl₃): δ = 7,361(0,5);7,351(0,7);7,348(1,0);7,338(2,5);7,328(2,1);7,324(3,6);7,315(3,2);7,311(2,1);7,301(1,9);7,291(4,1);7,2 62(50,0);7,114(12,4);7,092(0,4);7,085(0,3);7,072(0,5);7,068(0,6);7,058(1,0);7,052(1,8);7,047(1,8);7,036(3,3);7,0 33(4,1);7,019(2,6);7,010(0,8);7,007(1,0);6,995(0,8);6,992(1,0);6,969(3,8);6,956(3,5);6,930(2,2);6,927(3,0);6,914( 2,2);6,911(2,9);4,280(5,6);4,271(5,7);4,061(1,8);4,050(1,9);3,851(1,5);3,843(3,0);3,834(2,9);3,825(3,5);3,816(1,7 );3,761(0,6);3,755(1,0);3,749(1,1);3,739(2,5);3,730(3,8);3,723(2,5);3,714(2,9);3,705(1,4);3,640(1,7);3,633(1,9);3, 625(2,1);3,622(1,9);3,618(2,1);3,615(1,8);3,607(1,6);3,600(1,5);3,495(0,6);3,490(2,8);3,478(0,9);3,471(0,9);3,46 0(0,5);3,453(0,5);2,259(0,5);2,252(0,6);2,243(0,6);2,236(0,6);2,230(1,0);2,221(1,4);2,215(0,9);2,212(0,9);2,207(2 ,1);2,198(2,3);2,192(1,3);2,189(1,2);2,183(1,7);2,174(0,9);2,110(1,0);2,103(1,7);2,095(1,9);2,087(2,3);2,079(2,1); 2,071(1,3);2,064(1,1);2,055(0,6);1,939(0,5);1,932(0,3);1,928(0,3);1,921(0,6);1,916(0,6);1,897(0,5);1,556(7,6);0,0 00(14,4);-0,006(0,5) |
| Beispiel1461(lba):¹H-NMR(400,0MHz,CDCl₃): δ = 8,004(1,0);7,343(1,1);7,328(1,4);7,324(3,8);7,309(4,0);7,304(5,0);7,289(4,9);7,284(3,1);7,269(3,2);7,261(50,1);7, 054(0,9);7,052(1,1);7,047(1,1);7,045(1,5);7,043(1,9);7,041(2,0);7,036(2,1);7,034(2,6);7,031(2,5);7,027(3,6);7,02 4(4,7);7,022(5,5);7,020(4,4);7,016(3,6);7,013(3,8);7,009(2,2);7,004(3,9);7,001(2,8);6,999(2,0);6,995(1,9);6,992(2 ,1);6,988(6,3);6,985(7,2);6,982(2,3);6,976(1,2);6,963(2,1);6,956(15,1);6,952(16,0);6,934(3,7);6,933(3,5);6,904(2, 7);6,899(3,4);6,893(2,1);6,880(2,7);6,875(3,3);6,870(2,1);6,727(5,1);6,726(5,0);6,724(5,1);6,722(4,8);6,708(9,2); 6,707(9,3);6,704(9,4);4,202(6,9);4,187(7,2);4,043(3,7);4,026(4,0);3,800(1,8);3,787(3,8);3,774(3,8);3,761(4,9);3,7 48(2,4);3,733(1,0);3,724(1,2);3,719(1,2);3,707(1,7);3,697(1,5);3,692(1,4);3,682(1,2);3,614(2,4);3,603(2,7);3,593( 3,6);3,587(2,5);3,581(4,9);3,576(3,3);3,566(6,3);3,554(4,4);3,543(3,5);3,528(1,7);3,508(1,3);3,496(1,5);3,484(10, 9);3,472(1,6);3,469(1,4);3,457(1,2);3,445(1,1);2,956(9,3);2,879(7,8);2,878(7,6);2,281(0,6);2,272(0,6);2,266(0,6); 2,258(1,2);2,247(0,9);2,243(0,7);2,238(0,8);2,232(0,9);2,223(1,5);2,214(0,8);2,208(0,7);2,199(0,7);2,187(0,7);2,1 74(1,3);2,166(0,7);2,161(0,9);2,152(2,7);2,139(3,7);2,131(2,0);2,125(1,8);2,118(3,2);2,111(1,8);2,104(1,8);2,099( 3,4);2,087(3,0);2,075(3,4);2,063(2,5);2,052(1,2);2,039(1,8);2,028(1,6);2,017(0,8);2,010(0,7);2,004(0,7);2,000(1,0 );1,993(0,9);1,989(0,8);1,982(0,5);1,976(0,6);1,965(0,7);1,954(0,5);1,449(4,9);0,008(0,6);0,000(19,6);-0,009(0,5) |
| Beispiel1677(lba):¹H-NMR(400,0MHz,CDCl₃): δ = 8,001(1,8);7,334(1,9);7,327(3,8);7,319(2,2);7,312(5,7);7,306(6,6);7,300(4,3);7,292(6,9);7,286(5,1);7,279(2,8);7,2 72(5,1);7,262(65,8);7,043(1,6);7,040(4,0);7,037(4,2);7,034(4,5);7,031(3,7);7,019(9,5);7,016(8,0);7,013(7,7);7,01 0(6,5);7,007(2,8);7,006(2,9);7,005(3,1);7,003(3,7);7,001(4,5);6,999(4,0);6,998(4,2);6,995(4,0);6,992(3,7);6,989(3 ,0);6,978(4,9);6,977(5,7);6,974(9,2);6,971(6,4);6,964(2,2);6,958(4,9);6,957(5,1);6,955(6,4);6,954(6,1);6,953(5,9); 6,952(6,1);6,946(3,0);6,940(1,8);6,915(4,5);6,910(5,5);6,905(3,5);6,892(4,5);6,886(5,5);6,881(3,5);6,394(3,5);6,3 93(3,5);6,385(7,2);6,383(5,0);6,380(7,1);6,378(7,3);6,376(5,3);6,375(5,0);6,370(13,2);6,362(7,7);6,360(7,3);6,26 6(12,9);6,261(13,1);6,256(11,0);6,251(12,0);6,250(8,3);6,245(6,7);6,240(5,7);6,236(5,6);4,150(11,5);4,136(12,0); 3,992(6,1);3,974(6,5);3,803(3,0);3,790(6,3);3,776(6,6);3,763(8,5);3,750(5,6);3,741(1,9);3,736(1,9);3,724(2,7);3,7 14(2,4);3,709(2,3);3,699(2,1);3,641(4,2);3,629(4,7);3,620(4,9);3,614(3,6);3,608(4,8);3,602(3,5);3,593(3,6);3,581( 3,3);3,549(2,2);3,537(2,5);3,526(2,6);3,522(2,1);3,514(2,6);3,510(2,2);3,499(2,0);3,487(1,9);3,481(6,3);3,467(1,9 );3,461(2,6);3,452(4,1);3,446(4,0);3,443(3,6);3,437(3,2);3,433(3,2);3,428(3,5);3,422(3,7);3,414(1,8);3,408(1,5);2, 956(16,0);2,878(14,1);2,877(14,0);2,276(0,9);2,267(0,9);2,261(1,0);2,252(1,8);2,243(1,2);2,241(1,4);2,238(1,1);2 ,232(1,3);2,229(1,2);2,226(1,4);2,218(2,2);2,209(1,2);2,203(1,1);2,194(1,0);2,142(1,1);2,129(2,3);2,121(1,2);2,11 6(1,4);2,107(4,5);2,094(6,2);2,086(3,3);2,080(2,9);2,072(5,4);2,064(3,1);2,059(2,9);2,052(5,8);2,040(5,3);2,028(5 ,7);2,016(3,7);2,004(2,2);1,992(2,3);1,980(1,1);1,972(1,1);1,960(1,6);1,950(1,2);1,943(1,1);1,937(1,1);1,932(1,6); |
| 1,925(1,4);1,921(1,2);1,915(0,9);1,908(0,9);1,898(1,2);1,886(0,8);1,498(12,3);0,008(0,7);0,000(25,4);-0,009(0,7) |
| Beispiel1153(lba):¹H-NMR(400,0MHz,CDCl₃): δ = 8,095(2,9);8,090(5,8);8,087(3,3);8,082(5,6);7,264(29,0);7,127(1,4);7,119(0,9);7,113(3,1);7,105(1,7);7,097(3,0);7, 089(1,6);7,083(1,6);7,075(0,7);7,065(0,7);7,061(0,7);7,053(0,8);7,048(1,1);7,043(0,8);7,035(0,7);7,031(0,7);7,01 3(1,5);7,010(1,6);7,001(1,7);6,997(2,3);6,992(1,7);6,984(1,5);6,980(1,5);6,914(1,0);6,907(0,8);6,900(0,9);6,886(2 ,2);6,879(1,7);6,871(1,9);6,666(2,1);6,664(2,4);6,658(5,6);6,652(4,2);6,650(3,9);6,499(3,5);6,476(7,7);4,192(5,3); 4,181(5,3);4,031(0,4);3,987(2,4);3,974(2,5);3,912(50,0);3,724(1,4);3,715(2,9);3,706(3,0);3,698(3,3);3,689(1,6);3, 659(0,6);3,652(0,8);3,649(0,8);3,642(1,3);3,634(0,9);3,631(0,9);3,624(0,7);3,494(1,7);3,488(3,2);3,480(1,9);3,47 6(1,7);3,471(1,9);3,468(1,7);3,462(1,5);3,454(1,4);3,381(0,7);3,373(0,8);3,365(1,0);3,357(1,0);3,348(0,7);3,340(0 ,6);3,278(0,7);3,268(1,6);3,259(3,3);3,253(2,3);3,248(2,2);3,242(2,8);3,232(1,3);2,272(0,3);2,266(0,4);2,262(0,4); 2,256(0,7);2,249(0,7);2,240(0,7);2,233(0,8);2,227(0,5);2,223(0,4);2,217(0,4);2,110(0,6);2,101(1,1);2,096(0,8);2,0 91(1,1);2,087(2,4);2,077(3,6);2,070(3,8);2,063(6,4);2,054(6,4);2,046(6,1);2,038(4,2);2,031(2,1);2,022(2,0);2,014( 1,1);0,000(8,3) |
| Beispiel532Threo(lba):¹H-NMR(400,0MHz,CDCl₃): δ = 8,341(15,5);8,339(15,4);8,328(16,0);8,326(15,5);8,308(0,8);7,519(4,8);7,379(0,6);7,310(1,0);7,293(1,7);7,260(90 3,9);7,229(0,7);7,210(1,3);7,163(5,1);7,142(8,1);7,138(6,3);7,121(7,6);7,116(20,9);7,112(19,5);7,102(5,3);7,097( 10,7);7,084(5,1);7,078(5,8);7,070(5,1);7,058(4,8);7,052(4,9);7,032(12,4);7,028(11,3);7,018(11,4);7,015(10,7);6,9 96(5,2);6,975(0,8);6,918(3,2);6,913(4,0);6,909(5,6);6,904(4,3);6,898(4,3);6,892(3,7);6,887(4,7);6,883(3,3);6,877( 2,4);5,299(4,5);4,032(13,8);4,014(14,6);3,709(2,1);3,696(3,1);3,683(4,1);3,672(3,6);3,658(2,6);3,384(4,7);3,374( 5,3);3,366(5,1);3,356(10,5);3,346(7,1);3,338(5,6);3,328(5,2);2,288(2,0);2,278(2,2);2,273(2,4);2,263(4,3);2,252(4, 3);2,242(3,4);2,238(4,3);2,228(5,8);2,218(3,2);2,214(3,0);2,204(2,6);2,170(0,6);2,088(2,8);2,077(5,2);2,067(3,0); 2,061(2,7);2,050(5,2);2,042(4,5);2,031(2,0);2,025(2,2);2,014(3,4);2,004(1,9);1,541(60,1);1,333(3,2);1,284(6,3);1, 256(5,0);0,880(0,8);0,146(1,0);0,008(9,2);0,000(342,5);-0,006(4,9);-0,009(11,0);-0,051(0,6);-0,150(1,1) |
| Beispiel1288(lba):¹H-NMR(400,0MHz,CDCl₃): δ = 8,620(14,8);8,617(14,8);8,565(12,7);8,562(12,6);8,189(16,0);8,185(15,7);8,010(0,7);7,963(13,7);7,960(13,4);7,52 0(1,1);7,261(212,1);7,215(2,8);7,211(0,6);7,195(5,3);7,191(3,5);7,180(3,4);7,174(7,3);7,170(5,7);7,162(3,3);7,15 4(4,1);7,149(5,2);7,135(3,0);7,129(3,1);7,077(2,7);7,056(4,8);7,053(3,7);7,046(3,1);7,041(4,3);7,036(6,4);7,032(6 ,8);7,023(4,7);7,017(4,5);7,011(4,5);7,005(2,9);6,997(4,1);6,991(2,8);6,978(2,5);6,973(2,6);6,860(1,8);6,856(2,2); 6,851(3,2);6,846(2,3);6,840(2,2);6,835(2,0);6,829(2,5);6,825(1,8);6,820(1,3);4,292(9,2);4,285(8,4);4,270(9,6);4,2 60(8,7);3,728(2,0);3,717(2,8);3,701(2,9);3,690(3,2);3,676(2,3);3,645(2,3);3,633(4,5);3,619(4,6);3,605(6,3);3,594( 6,0);3,583(3,3);3,577(4,0);3,570(6,1);3,561(3,6);3,552(2,9);3,548(3,1);3,543(2,5);3,536(2,5);3,346(2,7);3,335(3,0 );3,323(5,3);3,319(3,3);3,312(5,8);3,308(3,2);3,297(5,0);3,286(4,9);3,272(2,3);3,261(2,1);2,958(5,6);2,882(4,8);2, 489(1,1);2,480(1,2);2,475(1,2);2,466(1,8);2,454(2,0);2,441(1,8);2,431(2,2);2,422(1,4);2,417(1,3);2,408(1,1);2,23 6(1,3);2,225(2,7);2,215(1,5);2,209(1,4);2,198(2,9);2,191(2,4);2,180(1,1);2,174(1,1);2,163(2,0);2,153(0,9);2,063(1 ,0);2,052(1,9);2,040(1,5);2,028(3,0);2,017(3,8);2,004(2,8);1,992(3,3);1,980(1,6);1,960(1,7);1,948(2,9);1,935(2,4); 1,925(3,6);1,913(3,1);1,902(1,3);1,890(1,5);1,878(0,9);1,485(1,6);0,008(2,6);0,000(79,4);-0,009(2,3) |
| Beispiel1785(lba):¹H-NMR(400,0MHz,CDCl₃): δ = 7,832(12,1);7,818(16,0);7,519(2,3);7,351(0,5);7,338(3,8);7,323(4,4);7,317(6,8);7,303(6,9);7,297(5,3);7,283(5,7); 7,260(409,8);7,228(0,5);7,053(1,9);7,051(2,7);7,047(3,2);7,044(3,0);7,041(2,0);7,032(3,6);7,030(4,9);7,026(5,9); 7,023(5,4);7,020(3,4);7,005(5,2);7,002(5,8);6,996(2,9);6,983(3,3);6,974(2,8);6,968(6,0);6,951(4,6);6,947(6,4);6,9 26(3,0);6,920(3,6);6,915(2,2);6,902(2,8);6,897(3,6);6,892(2,1);6,827(15,9);6,821(11,6);4,240(7,1);4,225(7,5);4,1 63(5,3);4,144(5,6);3,802(2,0);3,789(4,4);3,775(4,2);3,762(5,4);3,749(2,6);3,742(1,6);3,731(1,8);3,728(1,8);3,716( 2,4);3,704(2,2);3,701(2,2);3,690(1,8);3,648(1,7);3,632(3,2);3,628(2,9);3,611(3,5);3,596(3,4);3,589(1,7);3,580(3,9 );3,565(3,2);3,563(2,6);3,553(2,3);3,547(3,0);3,538(2,5);3,536(1,9);3,533(1,8);3,520(2,2);3,487(2,0);3,475(2,2);3, 463(2,4);3,460(1,9);3,452(2,3);3,448(1,9);3,436(1,8);3,425(1,7);2,241(0,7);2,231(0,8);2,227(0,7);2,217(1,3);2,20 6(1,5);2,196(1,4);2,192(1,6);2,183(2,3);2,173(1,3);2,169(1,3);2,159(1,3);2,130(1,4);2,120(3,9);2,109(5,7);2,102(7 ,1);2,096(3,5);2,090(7,4);2,085(4,3);2,074(2,9);2,072(3,3);2,056(1,3);2,045(0,6);1,540(5,3);0,157(0,6);0,146(0,5); 0,008(5,3);0,000(153,6);-0,009(4,4) |
| Beispiel1801(lba):¹H-NMR(400,0MHz,CDCl₃): δ = 7,832(12,4);7,820(16,0);7,265(65,4);7,167(2,3);7,163(1,8);7,146(4,0);7,142(5,1);7,138(2,2);7,126(3,1);7,122(5,9) ;7,117(3,1);7,101(2,7);7,097(2,2);7,091(1,6);7,085(1,7);7,073(1,6);7,067(1,9);7,065(1,9);7,059(1,7);7,045(2,8);7, 040(3,0);7,027(2,0);7,021(2,4);7,012(2,2);7,000(2,1);6,994(2,1);6,953(1,1);6,949(1,4);6,944(1,9);6,939(1,5);6,93 3(1,4);6,926(2,2);6,922(3,0);6,919(2,5);6,916(3,2);6,912(2,3);6,905(1,8);6,900(1,5);6,894(2,0);6,890(1,5);6,885(1 ,0);6,839(15,3);6,821(11,4);4,238(5,9);4,223(6,2);4,139(4,7);4,120(5,0);3,805(1,7);3,792(3,8);3,778(3,4);3,765(4, 8);3,752(2,3);3,748(1,5);3,737(1,7);3,734(1,7);3,721(2,0);3,710(2,0);3,707(1,9);3,696(1,6);3,625(1,6);3,609(2,8); 3,605(3,1);3,598(1,7);3,591(3,5);3,585(3,0);3,580(3,0);3,572(2,3);3,564(4,3);3,561(2,3);3,553(2,4);3,547(2,5);3,5 37(2,5);3,534(1,6);3,520(1,9);3,484(0,7);3,481(1,7);3,470(1,9);3,457(2,0);3,454(1,7);3,446(2,0);3,443(1,8);3,430( 1,6);3,419(1,5);2,959(2,7);2,881(2,3);2,880(2,3);2,232(0,6);2,222(0,7);2,218(0,7);2,209(1,3);2,197(1,4);2,187(1,2 );2,184(1,4);2,174(2,1);2,164(1,1);2,160(1,1);2,150(1,0);2,115(3,9);2,108(1,6);2,102(5,6);2,097(8,1);2,088(2,8);2, 084(6,7);2,080(5,3);2,067(3,9);2,062(1,7);2,059(1,3);2,051(0,7);2,045(0,7);2,035(1,2);2,024(0,6);1,685(2,4);0,00 8(0,8);0,000(24,1);-0,009(0,7) |
| Beispiel215(lba):¹H-NMR(400,0MHz,CDCl₃): δ = 8,574(6,6);8,570(6,9);8,562(6,8);8,558(6,8);8,521(6,7);8,518(7,0);8,510(6,9);8,506(6,9);8,012(0,9);7,724(7,7);7,7 20(7,8);7,704(8,5);7,700(8,3);7,537(8,5);7,533(8,6);7,521(1,0);7,516(9,5);7,512(9,3);7,412(5,5);7,406(6,0);7,395( 5,7);7,389(5,9);7,262(113,0);7,253(2,8);7,247(2,4);7,242(2,7);7,236(2,7);7,232(4,1);7,224(9,9);7,221(4,6);7,215( 4,4);7,212(9,3);7,203(8,4);7,198(6,0);7,192(14,0);7,181(6,0);7,175(6,3);7,164(8,0);7,143(12,5);7,121(5,1);7,113( 9,6);7,101(9,4);7,092(8,8);7,081(8,6);7,044(2,3);7,039(2,0);7,033(2,5);7,027(2,4);7,023(4,6);7,017(4,5);7,012(4,4 );7,006(4,1);6,998(0,8);6,984(8,6);6,962(12,5);6,941(4,5);4,359(8,7);4,355(9,2);4,335(10,5);4,329(11,3);4,161(2, 7);4,143(5,5);4,136(2,6);4,131(3,3);4,127(3,2);4,118(7,4);4,108(5,1);4,102(2,6);4,095(2,4);4,084(2,3);3,632(2,1); 3,618(4,4);3,604(5,3);3,590(6,2);3,576(3,1);3,522(1,9);3,507(3,7);3,494(4,5);3,485(16,0);3,480(6,3);3,470(5,4);3, 468(3,6);3,465(3,7);3,459(2,6);3,453(3,6);3,443(3,6);3,425(2,4);3,412(3,2);3,398(3,7);3,392(3,8);3,384(2,3);3,37 9(3,6);3,371(2,4);3,365(2,4);3,351(2,1);2,955(9,0);2,881(7,6);2,880(7,7);2,410(4,9);2,395(7,7);2,379(6,9);2,376(8 ,4);2,362(5,0);2,108(1,1);2,094(2,3);2,084(1,2);2,081(1,3);2,073(1,9);2,071(2,5);2,060(3,2);2,050(1,8);2,046(1,6); 2,036(3,0);2,022(1,4);1,882(1,5);1,871(1,7);1,867(1,8);1,862(1,8);1,856(1,8);1,852(2,0);1,847(2,8);1,837(2,7);1,8 33(1,5);1,828(1,5);1,822(1,3);1,817(1,4);1,813(1,3);1,802(1,1);1,629(2,8);0,008(1,3);0,000(42,2);-0,009(1,3) |
| Beispiel1477(lba):¹H-NMR(400,0MHz,CDCl₃): δ = 8,003(1,7);7,262(60,8);7,166(1,4);7,152(2,8);7,145(2,5);7,142(1,8);7,131(4,7);7,127(3,4);7,121(2,6);7,111(3,5);7, 106(4,6);7,100(2,0);7,097(1,6);7,091(1,6);7,086(3,2);7,079(1,4);7,073(1,6);7,071(1,5);7,065(1,4);7,052(1,3);7,04 6(1,4);7,016(2,3);7,010(2,5);6,997(3,0);6,994(7,3);6,991(9,2);6,983(2,8);6,971(2,9);6,966(15,2);6,962(14,1);6,95 8(2,2);6,954(1,4);6,947(1,2);6,941(1,0);6,936(1,3);6,931(1,0);6,927(0,7);6,903(1,7);6,899(2,1);6,894(2,9);6,889(2 ,2);6,883(2,2);6,878(1,8);6,873(2,3);6,868(1,7);6,863(1,2);6,722(10,6);6,721(11,5);6,719(11,3);6,718(11,0);6,715 (6,8);6,714(6,4);6,711(6,2);6,710(5,8);4,199(7,1);4,185(7,4);4,010(3,9);3,993(4,2);3,813(2,0);3,800(4,4);3,787(4, 0);3,773(5,7);3,761(2,7);3,749(1,1);3,740(1,3);3,735(1,3);3,723(1,7);3,713(1,6);3,708(1,6);3,698(1,4);3,622(2,7); 3,610(3,0);3,601(3,1);3,595(2,4);3,589(3,1);3,583(2,4);3,574(3,0);3,567(1,4);3,562(2,5);3,559(1,3);3,549(3,4);3,5 36(4,0);3,526(2,6);3,521(3,1);3,515(2,3);3,512(4,1);3,504(1,9);3,498(2,0);3,492(2,0);3,489(1,7);3,485(7,5);3,480( 1,9);3,478(1,6);3,465(1,4);3,454(1,3);2,958(15,6);2,957(16,0);2,880(13,7);2,878(14,0);2,288(0,6);2,279(0,7);2,27 3(0,7);2,264(1,3);2,253(1,1);2,249(0,8);2,244(0,9);2,239(1,1);2,229(1,6);2,220(0,9);2,214(0,8);2,205(0,7);2,192(0 ,7);2,178(1,2);2,171(0,7);2,165(0,9);2,156(2,9);2,143(3,8);2,135(2,3);2,130(2,1);2,121(3,4);2,107(4,4);2,095(3,4); 2,083(3,8);2,071(2,9);2,060(1,3);2,048(1,3);2,036(0,7);2,019(0,8);2,008(1,2);1,998(0,8);1,991(0,8);1,984(0,8);1,9 80(1,3);1,974(1,1);1,970(0,9);1,963(0,6);1,956(0,6);1,946(0,9);1,935(0,6);1,508(3,9);0,008(0,7);0,000(24,0);-0,009(0,7) |
| Beispiel1542(lba):¹H-NMR(400,0MHz,CDCl₃): δ = 7,996(2,0);7,368(1,2);7,353(1,7);7,347(4,4);7,332(5,0);7,327(5,9);7,313(5,9);7,307(3,6);7,292(3,2);7,266(32,8);7, 259(11,9);7,087(20,3);7,078(1,6);7,076(1,6);7,072(1,6);7,070(1,7);7,056(4,1);7,050(4,7);7,036(8,0);7,032(6,2);7, 015(6,6);7,010(4,4);6,991(2,4);6,980(6,2);6,960(4,9);6,941(3,4);6,936(4,3);6,931(2,6);6,918(3,3);6,913(4,3);6,90 8(2,6);4,294(8,1);4,280(8,5);4,068(4,7);4,052(5,1);3,853(2,1);3,840(4,3);3,826(4,1);3,813(5,5);3,800(2,7);3,761(1 ,2);3,751(1,5);3,747(1,6);3,734(3,2);3,724(3,1);3,719(2,7);3,709(5,0);3,695(4,8);3,686(3,2);3,681(3,8);3,672(4,3); 3,658(2,2);3,647(2,7);3,636(3,1);3,625(3,1);3,620(2,7);3,614(3,2);3,609(2,6);3,598(2,4);3,587(2,2);3,506(1,5);3,4 95(1,7);3,480(8,0);3,470(2,1);3,454(1,4);3,443(1,3);2,959(16,0);2,877(13,6);2,288(0,7);2,278(0,8);2,273(0,8);2,2 64(1,3);2,253(1,4);2,249(1,0);2,239(2,3);2,228(2,1);2,218(1,8);2,212(1,6);2,203(3,3);2,190(3,2);2,182(2,1);2,176( 1,9);2,168(2,3);2,155(1,5);2,116(1,6);2,104(2,4);2,092(3,0);2,080(3,2);2,069(2,9);2,057(1,7);2,046(1,3);2,033(0,9 );1,949(0,8);1,939(1,5);1,929(0,9);1,922(0,9);1,911(1,7);1,904(1,5);1,894(0,7);1,887(0,7);1,876(1,2);1,866(0,7);1, |
| 729(4,4);0,008(0,5);0,000(12,4) |
| Beispiel1565(lba):¹H-NMR(400,0MHz,CDCl₃): δ = 8,005(1,3);7,351(2,1);7,345(2,2);7,334(2,0);7,328(2,0);7,2724(9,5);7,2715(9,3);7,264(57,7);7,258(4,0);7,252(3,7) ;7,163(1,3);7,142(4,7);7,117(17,9);7,109(2,4);7,103(2,1);7,097(5,7);7,088(0,6);7,082(0,5);7,060(2,4);7,054(2,3);7 ,050(2,6);7,044(2,4);7,039(1,5);7,033(1,5);7,028(1,4);7,022(1,3);4,269(5,0);4,255(5,2);4,046(3,2);4,029(3,5);3,86 6(1,4);3,854(2,9);3,840(2,7);3,827(3,7);3,814(1,8);3,783(0,8);3,773(1,0);3,769(1,0);3,759(1,1);3,756(1,2);3,746(1 ,2);3,742(1,2);3,732(1,0);3,713(0,8);3,704(0,9);3,697(0,8);3,686(1,6);3,676(2,2);3,669(1,1);3,661(3,4);3,658(2,8); 3,653(2,1);3,647(3,6);3,638(3,1);3,636(3,0);3,630(2,0);3,625(3,6);3,620(1,9);3,609(1,7);3,598(1,5);3,521(1,1);3,5 10(1,2);3,496(1,5);3,487(16,0);3,469(1,0);3,459(0,9);2,960(11,7);2,881(9,8);2,880(9,9);2,263(0,5);2,259(0,5);2,2 49(0,8);2,238(1,0);2,232(0,9);2,228(0,8);2,224(1,1);2,219(1,0);2,217(1,0);2,213(1,2);2,210(0,9);2,204(1,3);2,196( 2,0);2,190(0,8);2,183(2,0);2,174(1,4);2,169(1,3);2,162(1,3);2,160(1,4);2,147(1,0);2,112(1,1);2,101(1,5);2,100(1,5 );2,089(2,0);2,077(2,0);2,066(1,9);2,054(1,1);2,043(0,8);2,041(0,8);2,030(0,6);1,939(0,5);1,929(1,1);1,918(0,6);1, 911(0,6);1,901(1,2);1,893(1,0);1,866(0,9);1,564(3,9);0,008(0,7);0,000(21,5);-0,009(0,6) |
| Beispiel1516(lba):¹H-NMR(400,0MHz,CDCl₃): δ = 7,262(50,0);7,256(5,1);7,222(1,7);7,214(1,9);7,208(2,1);7,200(1,9);7,150(2,6);7,142(3,0);7,136(3,5);7,127(3,2);7, 100(7,5);7,085(0,5);7,077(0,6);7,071(2,2);7,057(3,9);7,051(3,9);7,047(1,7);7,043(2,4);7,037(5,9);7,022(2,6);4,25 9(3,2);4,250(3,3);4,038(2,0);4,027(2,1);3,847(0,9);3,839(1,9);3,830(1,8);3,821(2,2);3,813(1,0);3,787(0,4);3,758(0 ,6);3,751(0,7);3,748(0,7);3,741(0,9);3,730(1,3);3,724(1,2);3,719(0,6);3,712(1,0);3,706(0,6);3,700(0,7);3,694(1,4); 3,685(1,7);3,678(1,2);3,669(1,7);3,660(0,9);3,634(1,1);3,627(1,2);3,620(1,3);3,616(1,2);3,612(1,3);3,609(1,1);3,6 02(1,0);3,595(0,9);3,499(0,6);3,492(0,9);3,490(0,8);3,483(1,0);3,476(1,0);3,465(0,6);3,458(0,5);2,285(0,3);2,279( 0,3);2,276(0,4);2,269(0,6);2,262(0,6);2,253(0,6);2,246(0,7);2,239(0,4);2,236(0,4);2,230(0,4);2,227(0,5);2,218(0,7 );2,212(0,6);2,209(0,6);2,203(1,3);2,195(1,4);2,189(0,8);2,186(0,7);2,180(1,0);2,171(0,6);2,099(0,6);2,092(1,0);2, 084(1,2);2,076(1,4);2,068(1,3);2,060(0,8);2,052(0,7);2,044(0,4);1,927(0,3);1,920(0,6);1,913(0,4);1,908(0,4);1,90 1(0,7);1,896(0,7);1,890(0,3);1,885(0,3);1,878(0,5);1,541(6,6);0,005(0,7);0,000(13,6);-0,006(0,5) |
| Beispiel208(lba):¹H-NMR(400,0MHz,CDCl₃): δ = 8,570(11,1);8,567(11,4);8,559(11,4);8,555(11,2);8,516(6,7);8,512(6,9);8,505(6,9);8,501(6,8);7,718(14,0);7,715(1 3,8);7,698(15,5);7,694(14,7);7,535(8,8);7,531(8,7);7,522(1,0);7,515(9,8);7,511(9,4);7,263(118,6);7,219(15,9);7,2 14(5,0);7,207(18,3);7,199(16,3);7,196(6,0);7,190(6,5);7,187(19,0);7,181(8,5);7,180(8,7);7,176(4,9);7,169(4,8);7, 164(5,4);7,160(7,3);7,155(7,5);7,135(6,4);7,110(12,6);7,102(5,9);7,098(13,8);7,089(11,1);7,078(10,7);7,071(1,9); 7,024(2,6);7,003(4,9);6,999(3,7);6,995(3,1);6,989(3,4);6,982(4,3);6,977(6,2);6,970(3,6);6,968(3,3);6,962(3,4);6,9 58(4,1);6,949(2,8);6,944(3,1);6,906(2,2);6,903(2,5);6,901(2,3);6,896(3,5);6,893(2,6);6,891(2,3);6,887(2,3);6,881( 1,9);6,875(2,3);6,871(1,7);6,866(1,3);4,363(8,6);4,356(12,9);4,337(11,1);4,333(16,0);4,158(2,7);4,141(8,1);4,131 (5,7);4,123(3,5);4,117(9,3);4,107(8,1);4,098(2,7);4,094(3,9);4,083(3,6);3,625(1,9);3,611(3,9);3,597(4,7);3,583(5, 5);3,569(2,8);3,519(2,8);3,505(5,6);3,491(6,7);3,483(11,2);3,478(9,2);3,464(6,9);3,455(2,4);3,449(3,2);3,437(3,0) ;3,421(2,2);3,408(4,6);3,394(5,3);3,388(5,5);3,380(3,5);3,374(5,2);3,367(3,4);3,360(3,5);3,347(3,1);2,955(4,5);2, 880(3,8);2,878(3,7);2,405(5,2);2,390(7,5);2,388(7,6);2,374(6,6);2,372(7,7);2,356(4,7);2,103(2,0);2,089(4,0);2,07 9(2,2);2,075(2,3);2,068(3,3);2,065(4,4);2,055(5,6);2,052(2,8);2,045(3,0);2,041(2,8);2,031(5,3);2,017(2,5);1,883(2 ,5);1,872(2,8);1,868(3,0);1,863(3,0);1,857(3,0);1,853(3,3);1,848(4,5);1,838(4,5);1,834(2,5);1,829(2,4);1,823(2,2); 1,818(2,3);1,814(2,0);1,803(1,8);1,624(2,7);0,008(1,4);0,000(44,8);-0,009(1,2) |
| Beispiel208Threo(lba):¹H-NMR(400,0MHz,CDCl₃): δ = 8,574(1,9);8,570(2,0);8,563(2,0);8,559(2,0);8,519(10,6);8,515(11,0);8,508(10,9);8,504(10,9);7,723(2,4);7,720(2, 4);7,703(2,6);7,699(2,5);7,538(13,3);7,534(13,7);7,517(15,0);7,513(14,7);7,261(206,1);7,223(2,7);7,217(0,9);7,2 11(3,7);7,202(3,2);7,199(1,0);7,191(3,4);7,182(1,6);7,178(1,0);7,172(0,9);7,167(1,1);7,162(1,4);7,157(1,5);7,138( 1,2);7,112(16,2);7,100(15,5);7,091(14,3);7,080(14,1);7,024(4,2);7,003(7,8);7,000(5,1);6,995(4,9);6,989(5,3);6,98 3(6,8);6,978(9,3);6,971(5,7);6,968(5,2);6,962(5,5);6,958(6,5);6,950(4,2);6,944(4,8);6,909(3,6);6,905(3,9);6,903(3 ,7);6,899(5,6);6,895(4,2);6,893(3,7);6,889(3,7);6,884(2,9);6,877(3,7);6,874(2,7);6,868(2,1);4,366(12,7);4,360(2,8 );4,341(16,0);4,160(4,3);4,143(7,2);4,134(4,0);4,126(4,6);4,117(5,6);4,109(1,8);4,100(3,6);4,085(0,7);3,631(3,3); 3,617(6,8);3,603(8,4);3,589(9,6);3,575(4,9);3,523(0,6);3,509(1,1);3,495(1,5);3,488(5,1);3,481(2,1);3,472(7,5);3,4 61(3,9);3,454(5,6);3,443(5,1);3,427(3,7);3,412(1,0);3,398(1,1);3,392(1,1);3,384(0,7);3,378(1,1);3,371(0,7);3,364( 0,7);3,351(0,6);2,409(8,5);2,393(12,7);2,377(12,6);2,361(7,8);2,093(0,7);2,069(0,8);2,058(1,0);2,048(0,5);2,045( 0,5);2,034(0,9);1,873(0,5);1,868(0,6);1,864(0,6);1,858(0,6);1,853(0,6);1,849(0,8);1,838(0,8);1,607(3,5);0,008(2,1 );0,000(77,2);-0,009(2,2) |
| Beispiel1531(lba):¹H-NMR(400,0MHz,CDCl₃): δ = 7,288(3,5);7,262(50,0);7,188(0,4);7,174(1,0);7,168(1,6);7,155(3,2);7,139(3,2);7,131(12,3);7,125(1,8);7,117(0,8); 7,112(0,6);7,109(0,5);7,104(0,5);7,100(0,4);7,085(0,3);7,051(1,5);7,047(1,6);7,039(1,7);7,034(2,4);7,029(1,6);7,0 21(1,5);7,017(1,4);6,980(0,6);6,973(0,5);6,966(0,6);6,922(2,2);6,915(1,7);6,907(1,9);4,268(5,1);4,260(5,2);4,040( 1,4);4,029(1,5);3,857(1,4);3,849(2,9);3,840(2,7);3,831(3,3);3,823(1,6);3,772(0,4);3,765(0,6);3,763(0,5);3,756(0,6 );3,747(0,7);3,745(0,7);3,738(0,7);3,731(0,5);3,724(0,8);3,718(0,5);3,710(1,6);3,701(2,7);3,694(1,9);3,692(1,7);3, 685(2,7);3,676(1,4);3,642(1,7);3,635(1,9);3,628(2,1);3,624(1,9);3,621(2,1);3,618(1,7);3,610(1,5);3,603(1,4);3,50 4(0,5);3,497(0,6);3,490(2,3);3,481(0,8);3,470(0,4);3,463(0,4);2,252(0,4);2,245(0,5);2,236(0,5);2,228(0,5);2,222(0 ,9);2,214(1,2);2,208(0,9);2,204(0,9);2,199(1,9);2,190(2,2);2,184(1,3);2,181(1,2);2,175(1,5);2,166(0,9);2,107(1,0); 2,100(1,6);2,092(1,9);2,084(2,0);2,076(1,9);2,068(1,3);2,061(1,0);2,052(0,6);1,925(0,5);1,907(0,6);1,901(0,6);1,8 83(0,4);1,566(5,3);0,000(14,6);-0,006(0,5) |
| Beispiel1272(lba):¹H-NMR(400,0MHz,CDCl₃): δ = 8,623(13,3);8,620(13,8);8,565(11,5);8,561(11,5);8,162(16,0);8,159(15,6);7,926(13,9);7,922(13,8);7,519(6,5);7,38 3(2,4);7,369(2,7);7,364(5,2);7,349(5,1);7,344(3,4);7,329(3,5);7,310(1,5);7,308(0,7);7,291(1,3);7,286(1,7);7,277(2 ,8);7,271(5,2);7,270(5,8);7,269(7,2);7,260(1202,0);7,247(4,0);7,231(2,5);7,226(5,9);7,212(4,5);7,206(3,8);7,198( 1,2);7,192(2,8);7,169(1,0);7,081(8,0);7,078(6,4);7,075(4,7);7,060(7,4);7,058(7,4);7,054(6,3);7,052(4,4);7,043(4,2 );7,038(5,1);7,033(4,7);7,020(3,9);7,016(4,1);7,010(2,3);6,996(6,6);6,969(2,1);6,967(2,1);6,963(2,2);6,960(2,4);6, 948(3,4);6,946(3,8);6,942(3,6);6,939(4,2);6,927(1,8);6,925(1,9);6,921(1,9);6,918(2,0);6,888(5,0);6,886(4,5);6,88 1(3,7);6,876(4,2);6,870(6,5);6,858(3,5);6,852(3,5);6,847(2,1);4,302(8,8);4,280(14,4);4,254(8,3);3,725(2,1);3,711( 2,6);3,698(3,0);3,687(2,9);3,684(3,0);3,673(2,5);3,639(2,2);3,626(4,1);3,613(6,3);3,599(5,5);3,590(5,8);3,586(5,1 );3,581(5,0);3,566(3,9);3,556(3,7);3,348(2,6);3,337(4,0);3,326(4,5);3,321(2,9);3,314(5,9);3,310(4,4);3,303(3,3);3, 299(4,3);3,287(4,6);3,276(2,4);2,502(1,0);2,493(1,2);2,488(1,4);2,479(2,1);2,467(1,8);2,453(1,9);2,444(2,7);2,43 5(1,6);2,429(1,4);2,421(1,3);2,276(1,5);2,265(3,0);2,254(1,4);2,249(1,5);2,238(3,0);2,230(2,5);2,220(1,2);2,214(1 ,2);2,203(2,1);2,192(1,0);2,089(1,0);2,078(2,0);2,065(1,6);2,053(2,7);2,043(4,0);2,030(2,7);2,017(3,6);2,006(2,1); 1,977(1,8);1,964(2,6);1,954(2,2);1,952(2,4);1,942(3,3);1,929(3,0);1,919(1,2);1,908(1,3);1,894(0,9);1,529(6,9);1,2 55(1,0);0,331(1,0);0,238(0,8);0,157(1,8);0,146(1,2);0,008(12,9);0,000(468,1);-0,009(13,6);-0,150(1,4) |
| Beispiel1781(lba):¹H-NMR(400,0MHz,CDCl₃): δ = 8,749(7,1);8,735(14,0);7,600(7,8);7,480(16,0);7,301(2,8);7,296(2,9);7,285(2,9);7,279(3,0);7,263(83,3);7,224(5,4) ;7,218(5,7);7,207(5,5);7,201(5,6);7,125(1,6);7,104(5,9);7,101(5,9);7,083(7,3);7,079(14,8);7,072(3,1);7,067(2,7);7 ,058(8,6);7,051(0,8);7,046(0,7);7,007(3,7);7,001(3,7);6,996(4,1);6,990(3,7);6,986(2,6);6,980(2,4);6,975(2,4);6,96 9(2,1);4,283(8,0);4,269(8,4);4,038(4,1);4,020(4,7);3,839(2,3);3,826(5,3);3,813(5,2);3,806(1,6);3,799(7,5);3,787(4 ,7);3,783(4,5);3,773(2,9);3,769(3,2);3,759(4,1);3,753(1,6);3,744(2,8);3,739(1,5);3,727(1,7);3,717(1,6);3,712(1,6); 3,702(1,3);3,609(2,8);3,598(3,2);3,587(3,2);3,582(2,7);3,576(3,2);3,571(2,7);3,560(2,5);3,549(2,3);3,486(3,4);3,4 74(1,3);3,463(1,5);3,449(1,8);3,438(1,8);3,436(1,6);3,423(1,3);3,412(1,2);2,958(3,8);2,881(3,2);2,880(3,1);2,347( 0,6);2,338(0,7);2,333(0,7);2,323(1,2);2,313(1,2);2,309(0,8);2,303(1,0);2,298(1,2);2,288(1,5);2,278(1,0);2,274(1,6 );2,262(1,7);2,253(1,1);2,248(1,2);2,239(3,1);2,226(3,6);2,217(2,2);2,212(2,0);2,204(2,6);2,191(1,6);2,158(1,7);2, 146(2,7);2,135(3,2);2,123(3,5);2,111(3,0);2,099(1,8);2,088(1,4);2,076(0,9);2,015(0,7);2,005(1,3);1,994(0,8);1,98 7(0,8);1,977(1,4);1,970(1,2);1,959(0,6);1,952(0,7);1,942(1,0);1,932(0,6);1,753(6,0);0,008(1,1);0,000(31,9);-0,009(0,9) |
| Beispiel1700(lba):¹H-NMR(400,0MHz,CDCl₃): δ = 8,005(1,8);7,291(3,2);7,285(3,4);7,274(3,2);7,269(3,7);7,262(60,2);7,230(6,1);7,224(6,4);7,213(6,3);7,207(6,3);7, 123(1,7);7,116(5,3);7,102(6,4);7,094(14,3);7,086(3,5);7,081(7,6);7,073(11,9);7,069(3,7);7,065(1,2);7,059(1,0);7, 053(0,8);7,044(4,5);7,038(4,3);7,032(4,8);7,027(4,4);7,022(2,8);7,016(2,5);7,011(2,4);7,005(2,2);6,998(0,5);6,37 8(3,1);6,377(3,1);6,373(5,8);6,372(5,8);6,369(6,4);6,364(10,8);6,355(6,5);6,282(10,0);6,278(10,1);6,272(8,2);6,2 68(8,1);6,261(5,1);6,256(5,1);6,251(4,3);6,247(4,2);4,138(9,2);4,124(9,5);3,950(5,1);3,931(5,5);3,819(2,4);3,806( 5,3);3,792(5,1);3,779(7,2);3,770(1,8);3,766(3,6);3,761(1,7);3,756(1,6);3,744(2,1);3,734(1,9);3,729(1,9);3,719(1,7 );3,653(3,3);3,641(3,7);3,632(3,8);3,626(2,9);3,620(3,8);3,614(2,9);3,605(2,8);3,593(2,6);3,562(1,7);3,550(1,9);3, 538(2,1);3,535(1,7);3,526(2,1);3,523(1,8);3,511(1,6);3,499(1,4);3,485(6,8);3,458(0,7);3,450(1,0);3,441(1,0);3,43 3(2,1);3,429(2,2);3,420(3,2);3,413(3,5);3,404(3,2);3,396(3,3);3,390(2,6);3,381(1,3);3,375(1,2);2,958(16,0);2,880( 13,4);2,879(12,9);2,281(0,7);2,272(0,7);2,267(0,8);2,258(1,5);2,247(1,2);2,243(0,9);2,237(1,0);2,232(1,2);2,223( 1,8);2,214(0,9);2,208(0,9);2,199(0,8);2,146(0,8);2,133(1,7);2,125(0,9);2,120(1,1);2,111(3,5);2,098(4,8);2,090(2,8 );2,085(2,4);2,077(4,2);2,071(2,5);2,063(2,5);2,059(4,6);2,047(4,3);2,035(4,6);2,023(3,4);2,012(1,7);2,000(1,7);1, 988(0,8);1,952(0,9);1,941(1,4);1,930(0,9);1,923(0,9);1,917(1,0);1,913(1,5);1,906(1,3);1,902(1,0);1,896(0,8);1,88 8(0,8);1,878(1,1);1,867(0,7);1,489(10,1);0,008(0,7);0,000(22,5);-0,009(0,7) |
| Beispiel1538(lba):¹H-NMR(400,0MHz,CDCl₃): δ = 7,345(0,7);7,341(0,7);7,334(0,7);7,330(0,7);7,300(2,5);7,262(50,0);7,253(2,3);7,250(2,1);7,154(0,6);7,142(8,3);7, 130(2,0);7,126(1,2);7,116(4,3);7,107(0,7);7,102(2,8);7,093(0,3);7,085(0,4);7,045(1,3);7,041(1,4);7,038(1,5);7,03 4(1,4);7,032(1,1);7,027(1,0);7,024(1,0);7,020(0,8);4,258(3,3);4,249(3,4);4,039(1,1);4,028(1,1);3,858(0,9);3,850(1 ,9);3,841(1,8);3,832(2,2);3,824(1,1);3,773(0,3);3,766(0,4);3,764(0,4);3,755(0,5);3,748(0,4);3,746(0,5);3,740(0,5); 3,735(0,4);3,730(0,3);3,723(0,6);3,716(0,4);3,711(0,4);3,706(1,2);3,697(1,8);3,690(1,2);3,687(1,1);3,681(1,8);3,6 72(0,9);3,644(1,1);3,638(1,2);3,630(1,3);3,627(1,2);3,623(1,3);3,620(1,2);3,612(1,0);3,605(0,9);3,505(0,4);3,498( 0,4);3,491(2,3);3,481(0,5);3,471(0,3);2,243(0,3);2,236(0,4);2,227(0,4);2,220(0,7);2,211(0,9);2,206(0,6);2,203(0,7 );2,197(1,3);2,188(1,4);2,182(0,8);2,179(0,8);2,173(1,0);2,164(0,6);2,103(0,6);2,095(1,0);2,088(1,2);2,080(1,4);2, 072(1,3);2,064(0,8);2,056(0,6);2,049(0,4);1,925(0,3);1,906(0,4);1,901(0,4);1,556(3,1);0,005(0,7);0,000(14,1);-0,006(0,5) |
| Beispiel532Erythro(lba):¹H-NMR(400,0MHz,CDCl₃): δ = 8,341(1,2);8,321(12,7);8,320(12,5);8,309(12,9);8,307(12,7);7,519(4,3);7,360(0,9);7,309(0,9);7,292(1,8);7,286(1, 4);7,283(1,6);7,271(3,9);7,269(5,3);7,260(812,3);7,210(2,6);7,163(0,5);7,156(4,4);7,135(7,0);7,132(5,1);7,115(6, 6);7,111(7,8);7,097(1,1);7,090(4,7);7,036(13,8);7,033(14,0);7,032(16,0);7,019(4,5);7,013(4,3);7,001(4,1);6,996(8 ,9);6,992(14,5);6,988(11,4);6,979(10,3);6,975(11,6);6,969(4,0);6,866(2,4);6,861(3,4);6,856(4,5);6,852(3,3);6,845 (3,5);6,840(2,9);6,834(3,8);6,830(2,7);6,825(1,9);5,299(12,0);4,260(11,2);4,244(11,5);4,032(1,0);4,014(1,0);3,78 3(2,3);3,770(2,5);3,757(2,8);3,748(2,8);3,735(1,3);3,531(1,2);3,520(2,3);3,509(2,5);3,498(2,9);3,472(1,9);3,461(1 ,1);3,351(3,1);3,336(5,6);3,328(3,8);3,320(3,4);3,313(5,7);3,297(2,9);2,194(1,1);2,180(1,7);2,173(1,4);2,167(1,5); 2,159(4,4);2,146(5,1);2,137(3,7);2,135(2,9);2,131(3,1);2,123(3,9);2,115(3,0);2,110(3,0);2,103(4,9);2,092(4,3);2,0 80(5,2);2,068(4,3);2,056(1,7);2,044(3,6);2,032(1,2);1,541(142,4);1,491(0,8);1,440(1,0);1,425(1,0);1,362(5,0);1,3 33(2,7);1,284(3,9);1,256(5,8);0,880(1,2);0,146(1,0);0,032(0,8);0,008(9,6);0,000(325,0);-0,009(9,2);-0,050(1,0);-0,150(0,9) |
| Beispiel647(lba):¹H-NMR(600,1MHz,CDCl₃): δ = 8,185(0,3);8,181(0,3);8,041(0,3);8,031(0,4);8,027(0,4);8,018(0,3);7,449(0,3);7,446(0,3);7,438(0,3);7,433(0,4);7,2 61(50,0);7,211(0,4);7,197(0,7);7,058(0,3);7,054(0,3);7,051(0,3);7,047(0,5);7,037(0,4);7,032(0,4);7,025(0,7);7,01 1(0,8);6,842(0,4);6,833(0,4);6,828(0,4);6,819(0,3);4,211(0,4);4,194(0,4);4,144(0,7);4,126(0,7);3,758(0,4);3,749(0 ,4);3,740(0,4);1,557(3,5);0,000(13,9);-0,006(0,5) |
| Beispiel1137(lba):¹H-NMR(600,1MHz,CDCl₃): δ = 8,095(3,2);8,086(8,3);8,076(5,9);7,303(1,9);7,290(4,4);7,279(4,1);7,277(4,2);7,263(42,9);7,024(1,7);7,021(1,8);7, 010(4,0);7,007(3,4);6,996(3,2);6,983(3,0);6,970(2,1);6,952(2,0);6,946(4,5);6,933(4,6);6,900(3,2);6,884(3,2);6,70 0(2,4);6,691(2,4);6,671(4,7);6,663(4,6);6,528(4,4);6,487(8,6);4,193(5,8);4,181(5,9);4,027(0,4);4,012(3,0);4,000(3 ,0);3,908(50,0);3,708(1,5);3,699(3,0);3,690(3,2);3,681(3,4);3,672(1,7);3,645(0,8);3,638(1,0);3,635(1,0);3,628(1,6 );3,620(1,1);3,617(1,0);3,610(0,9);3,488(3,6);3,485(2,0);3,476(2,0);3,471(2,0);3,467(1,8);3,462(2,1);3,458(1,8);3, 453(1,6);3,445(1,5);3,378(0,9);3,370(1,0);3,362(1,3);3,354(1,2);3,345(0,9);3,337(0,8);3,298(1,9);3,288(3,2);3,28 2(3,3);3,277(2,4);3,272(2,9);3,262(1,9);2,265(0,4);2,259(0,5);2,255(0,5);2,249(0,8);2,242(0,9);2,232(0,9);2,226(1 ,0);2,220(0,6);2,216(0,6);2,210(0,5);2,107(0,8);2,097(1,5);2,095(1,4);2,083(2,9);2,074(4,4);2,066(4,7);2,060(6,6); 2,050(6,6);2,043(6,4);2,034(4,8);2,027(2,9);2,019(2,3);2,011(1,4);0,000(11,5) |
| Beispiel1558(lba):¹H-NMR(400,0MHz,CDCl₃): δ = 7,997(2,2);7,266(38,5);7,258(12,2);7,199(1,5);7,179(3,9);7,159(6,1);7,136(6,6);7,125(2,3);7,115(4,9);7,105(20,5) ;7,093(1,9);7,067(2,6);7,061(2,8);7,049(2,8);7,042(3,8);7,035(3,0);7,022(2,6);7,017(2,7);6,997(1,3);6,992(1,7);6, 987(2,2);6,983(1,9);6,977(1,8);6,971(1,5);6,966(1,8);6,961(1,5);6,939(2,9);6,934(3,6);6,929(3,0);6,923(2,8);6,91 8(2,6);6,913(3,0);6,908(2,4);4,283(7,9);4,269(8,2);4,050(4,7);4,033(5,2);3,861(2,1);3,849(4,3);3,835(4,2);3,822(5 ,4);3,809(2,7);3,779(1,3);3,769(1,6);3,765(1,7);3,752(2,0);3,742(2,0);3,738(2,0);3,728(1,6);3,716(1,4);3,707(1,6); 3,699(1,4);3,689(2,8);3,680(3,7);3,666(4,8);3,657(3,6);3,652(5,4);3,642(5,9);3,630(5,3);3,625(3,3);3,619(3,5);3,6 14(2,8);3,603(2,4);3,592(2,2);3,517(1,5);3,506(1,8);3,491(2,6);3,483(7,9);3,466(1,5);3,455(1,4);2,961(16,0);2,87 8(13,9);2,280(0,7);2,270(0,8);2,266(0,9);2,256(1,3);2,245(1,6);2,232(2,5);2,220(2,5);2,210(2,1);2,205(1,9);2,196( 3,5);2,183(3,2);2,174(2,2);2,169(2,1);2,160(2,4);2,148(1,5);2,113(1,7);2,101(2,5);2,090(3,1);2,078(3,3);2,067(2,9 );2,055(1,7);2,043(1,4);2,031(0,9);1,937(0,9);1,927(1,6);1,917(1,0);1,910(0,9);1,900(1,9);1,892(1,7);1,882(0,8);1, 875(0,8);1,864(1,4);1,854(0,7);1,706(4,1);0,008(0,7);0,000(14,6);-0,008(0,6) |
| Beispiel1295(Iba):¹H-NMR(400,0MHz,CDCl₃): δ = 8,623(14,4);8,620(15,2);8,570(12,8);8,566(13,8);8,199(15,6);8,196(16,0);7,971(13,3);7,968(14,3);7,519(4,0);7,38 5(7,9);7,367(6,7);7,260(668,8);7,237(14,3);7,231(13,7);7,220(11,5);7,214(10,7);7,171(18,4);7,168(19,9);7,157(1 1,6);7,153(17,4);7,038(5,1);7,017(11,7);6,996(12,9);6,981(5,7);6,976(6,1);6,970(6,5);6,965(6,1);4,281(15,1);4,26 0(12,8);4,256(11,9);3,733(2,4);3,721(4,4);3,706(4,1);3,693(4,5);3,681(3,3);3,645(3,0);3,632(5,5);3,619(5,8);3,60 5(8,3);3,593(7,4);3,579(7,2);3,569(8,6);3,556(6,1);3,545(5,8);3,533(3,5);3,346(3,1);3,335(4,2);3,320(6,5);3,307(6 ,8);3,297(6,7);3,285(4,0);3,280(3,4);3,269(2,7);2,464(2,3);2,452(2,9);2,442(2,9);2,430(2,8);2,420(2,0);2,229(3,2); 2,202(3,7);2,194(3,2);2,167(2,6);2,058(1,4);2,047(2,2);2,035(2,1);2,023(3,6);2,012(4,8);1,999(3,6);1,987(4,2);1,9 52(2,1);1,940(3,6);1,930(3,5);1,918(4,4);1,905(3,8);1,883(2,0);1,509(8,9);0,146(1,3);0,000(252,0);-0,150(1,8) |
| Beispiel289Erythro1(lba):¹H-NMR(400,0MHz,CDCl₃): δ = 8,401(3,2);8,395(3,4);7,975(2,5);7,380(0,9);7,375(1,1);7,369(0,8);7,358(1,0);7,352(1,2);7,347(0,8);7,269(0,5);7,2 68(0,6);7,267(0,7);7,261(42,5);7,170(0,8);7,149(1,4);7,146(0,9);7,129(1,1);7,125(1,3);7,104(0,8);7,037(0,7);7,03 1(0,7);7,019(0,7);7,012(0,9);7,005(0,7);6,993(0,6);6,987(0,7);6,890(0,5);6,887(0,7);6,881(0,9);6,877(0,7);6,871(0 ,6);6,865(0,6);6,860(0,7);6,856(0,5);5,299(16,0);4,487(2,1);4,472(2,1);3,633(0,9);3,616(0,9);3,092(0,9);3,072(0,9 );3,049(1,4);3,029(1,3);2,907(1,4);2,891(1,4);2,864(1,0);2,848(0,9);0,000(15,4) |
| Beispiel107(lba):¹H-NMR(400,0MHz,CDCl₃): δ = 8,492(0,7);8,489(1,4);8,485(0,8);8,481(0,8);8,477(1,4);8,474(0,8);8,419(0,7);8,416(1,3);8,412(0,8);8,408(0,8);8,4 05(1,3);8,401(0,7);7,434(0,8);7,430(0,8);7,416(1,4);7,413(1,4);7,410(2,6);7,407(1,1);7,399(1,3);7,393(1,4);7,390( 1,2);7,386(1,1);7,295(1,3);7,2843(1,6);7,2835(1,5);7,273(1,7);7,271(0,5);7,270(0,5);7,269(0,5);7,268(0,6);7,2674 (0,7);7,2666(0,8);7,266(1,0);7,261(77,3);7,2543(1,0);7,2536(1,0);7,252(1,2);7,248(0,6);7,243(0,7);7,241(0,8);7,2 37(1,2);7,233(1,0);7,227(0,9);7,221(1,1);7,220(1,5);7,216(2,1);7,197(1,3);7,193(1,2);7,177(1,4);7,172(2,5);7,166( 3,3);7,155(2,4);7,151(3,2);7,145(1,2);7,134(0,7);7,130(1,2);7,009(1,0);7,004(1,0);6,997(1,2);6,992(1,0);6,986(1,8 );6,965(2,4);6,944(0,8);4,345(1,8);4,337(1,7);4,321(2,0);4,311(1,9);4,148(1,1);4,130(3,3);4,112(3,4);4,094(1,1);3, 906(0,7);3,896(0,6);3,892(0,8);3,889(0,6);3,881(0,8);3,661(0,8);3,648(1,0);3,634(1,0);3,620(0,6);3,535(0,7);3,52 2(1,0);3,508(1,1);3,500(0,6);3,494(0,7);3,486(0,5);3,480(0,6);3,466(0,7);3,410(0,6);3,397(0,7);3,390(0,7);3,377(0 ,7);2,410(0,9);2,406(0,6);2,401(0,6);2,396(1,0);2,387(0,9);2,374(0,9);2,043(16,0);2,036(0,5);2,024(0,5);1,841(0,5 );1,830(0,5);1,625(0,8);1,286(0,7);1,284(0,7);1,276(4,9);1,258(10,4);1,241(4,8);0,008(0,8);0,000(27,6);-0,009(0,8) |
| Beispiel597(lba):¹H-NMR(400,0MHz,CDCl₃): δ = 8,373(13,1);8,185(16,0);7,421(0,8);7,419(0,7);7,406(1,0);7,402(1,7);7,400(1,4);7,388(1,5);7,384(1,6);7,381(1,2); 7,370(0,7);7,366(1,0);7,293(0,6);7,261(45,8);7,239(1,0);7,224(1,7);7,219(1,4);7,215(1,6);7,211(0,9);7,205(1,8);7, 202(1,7);7,198(1,6);7,194(1,0);7,183(1,7);7,174(1,8);7,171(2,9);7,168(2,0);7,151(2,1);7,117(3,6);7,114(3,5);7,09 5(4,4);7,092(4,2);7,076(1,2);7,074(1,2);7,070(0,7);6,997(0,5);6,969(3,1);6,954(4,2);6,949(5,1);6,941(2,5);6,936(3 ,2);6,933(3,4);6,928(2,4);6,923(1,5);6,920(1,7);6,914(0,7);5,298(2,5);4,274(3,2);4,249(3,7);4,205(3,6);4,178(4,6); 3,996(1,0);3,987(1,0);3,968(1,7);3,959(1,7);3,941(0,8);3,931(0,8);3,909(0,8);3,897(0,8);3,883(1,3);3,871(1,3);3,8 57(0,7);3,845(0,7);3,768(1,1);3,755(1,8);3,741(2,2);3,728(2,0);3,715(1,3);3,607(0,9);3,594(1,5);3,581(2,0);3,567( 1,8);3,554(1,1);3,480(1,2);3,468(1,4);3,457(1,5);3,453(1,3);3,446(1,5);3,441(1,3);3,430(1,2);3,419(1,1);3,395(1,0 );3,383(1,1);3,372(1,2);3,368(1,0);3,360(1,2);3,356(1,0);3,346(0,9);3,334(0,8);2,955(2,8);2,879(2,4);2,564(0,6);2, 554(0,7);2,550(0,7);2,541(1,0);2,529(1,2);2,518(1,2);2,506(1,3);2,497(0,8);2,493(0,7);2,483(0,7);2,317(0,5);2,30 6(1,0);2,293(0,7);2,290(0,7);2,286(0,6);2,278(1,1);2,274(1,0);2,271(1,0);2,267(0,8);2,259(0,6);2,255(0,5);2,243(0 ,7);2,073(0,6);2,061(0,5);2,050(0,8);2,047(0,8);2,042(0,7);2,038(1,0);2,035(0,8);2,024(0,7);2,012(0,8);1,932(0,6); 1,919(0,9);1,906(0,8);1,897(1,2);1,884(1,1);1,874(0,5);1,862(0,6);1,516(1,5);0,008(0,8);0,000(18,0);-0,009(0,7) |
| Beispiel491(lba):¹H-NMR(400,0MHz,CDCl₃): δ = 8,608(13,1);8,526(7,1);8,514(16,0);8,501(13,0);8,495(15,1);7,521(1,3);7,422(11,2);7,410(11,2);7,397(11,9);7,396 (12,0);7,392(8,0);7,351(6,5);7,345(5,1);7,340(7,1);7,331(16,3);7,328(18,1);7,325(11,9);7,321(10,0);7,314(14,3);7 ,306(4,9);7,303(5,8);7,301(5,7);7,299(5,6);7,295(4,8);7,286(8,0);7,283(9,7);7,281(10,0);7,278(9,6);7,271(8,9);7,2 62(219,4);7,249(3,3);7,231(6,7);7,212(10,2);7,192(5,0);7,154(8,4);6,998(3,2);6,990(4,3);6,972(3,7);5,298(3,5);4, 333(6,1);4,317(7,1);4,157(7,3);4,142(8,4);4,044(2,1);3,944(2,3);3,934(2,7);3,916(2,9);3,906(2,8);3,892(2,1);3,71 7(1,8);3,703(3,5);3,690(4,1);3,676(4,5);3,663(2,3);3,613(2,8);3,602(3,2);3,597(3,4);3,586(6,4);3,575(4,1);3,570(4 ,1);3,559(3,5);3,514(1,8);3,486(2,6);3,455(1,4);3,380(3,7);3,367(4,1);3,358(4,5);3,353(3,5);3,345(4,3);3,340(3,6); 3,331(3,5);3,318(3,2);2,285(1,1);2,273(1,8);2,260(1,4);2,250(2,2);2,245(2,2);2,238(3,1);2,225(2,4);2,222(2,6);2,2 09(3,5);2,197(2,6);2,168(2,3);2,153(3,2);2,144(3,2);2,137(3,0);2,130(3,2);2,120(3,9);2,106(5,4);2,095(4,5);2,084( 4,8);2,071(4,0);2,060(1,8);2,048(1,8);2,035(1,0);1,605(1,9);1,255(0,7);0,008(2,4);0,000(79,2);-0,009(2,3) |
| Beispiel511(lbs):¹H-NMR(400,0MHz,CDCl₃): δ = 8,609(12,9);8,530(15,0);8,517(15,4);8,502(14,8);7,598(4,4);7,592(4,6);7,582(4,5);7,577(4,5);7,521(1,1);7,435 (8,8);7,422(8,7);7,348(3,6);7,331(6,6);7,318(4,1);7,302(2,3);7,296(2,3);7,291(2,5);7,285(2,4);7,281(3,1);7,274(3, 2);7,269(3,9);7,262(187,8);7,254(0,9);7,2533(0,7);7,2525(0,6);7,252(0,5);7,141(5,9);7,120(8,7);7,100(4,6);7,046( 1,3);7,025(6,5);7,006(7,9);6,998(3,6);5,299(16,0);4,354(4,8);4,338(5,6);4,140(3,9);4,124(4,5);4,030(1,5);3,927(1, 4);3,918(1,7);3,913(1,5);3,900(2,0);3,889(1,7);3,884(1,6);3,874(1,3);3,740(1,3);3,727(2,6);3,714(2,9);3,700(3,3); 3,688(1,9);3,632(1,8);3,620(2,2);3,616(2,3);3,605(4,1);3,594(2,6);3,589(2,8);3,578(2,3);3,497(1,9);3,387(2,4);3,3 74(2,7);3,365(3,0);3,360(2,4);3,352(2,9);3,347(2,5);3,338(2,3);3,325(2,1);2,278(0,7);2,266(1,2);2,250(0,9);2,242( 1,6);2,238(1,6);2,231(2,3);2,215(2,0);2,203(2,7);2,191(2,1);2,161(2,2);2,151(2,1);2,146(2,0);2,137(2,2);2,125(2,8 );2,113(3,9);2,101(3,2);2,090(3,6);2,078(2,9);2,067(1,4);2,054(1,4);2,042(0,7);1,600(0,9);0,008(2,1);0,000(69,7);-0,009(2,1) |
| Beispiel599(lba):¹H-NMR(400,0MHz,CDCl₃): δ = 8,379(14,7);8,197(16,0);7,519(0,8);7,392(0,8);7,387(1,2);7,375(14,4);7,370(10,1);7,358(5,7);7,352(1,3);7,341(1, 0);7,336(1,7);7,295(0,5);7,280(2,3);7,275(3,5);7,270(2,3);7,269(2,3);7,267(1,9);7,260(141,7);7,254(2,3);7,236(1, 1);7,233(1,6);7,231(1,9);7,228(2,2);7,212(9,1);7,209(9,1);7,193(3,1);7,190(2,0);7,174(4,3);7,172(3,5);7,156(1,7); 7,153(1,8);7,075(1,9);7,071(2,9);7,056(1,4);7,053(2,3);6,996(0,9);4,249(3,8);4,224(4,4);4,180(3,9);4,152(5,1);3,9 89(0,9);3,979(0,9);3,961(1,6);3,952(1,5);3,934(0,8);3,924(0,7);3,898(0,8);3,886(0,8);3,872(1,4);3,860(1,4);3,845( 0,7);3,834(0,7);3,773(1,0);3,760(1,6);3,746(2,0);3,733(1,9);3,720(1,2);3,610(0,9);3,597(1,5);3,583(2,1);3,570(1,8 );3,557(1,2);3,481(1,1);3,470(1,2);3,458(1,3);3,454(1,2);3,447(1,3);3,442(1,2);3,431(1,1);3,420(1,0);3,396(1,1);3, 384(1,1);3,374(1,2);3,369(1,0);3,362(1,2);3,357(1,0);3,347(0,9);3,335(0,9);2,560(0,5);2,550(0,6);2,546(0,6);2,53 7(1,0);2,525(1,1);2,514(1,1);2,502(1,3);2,492(0,8);2,489(0,8);2,479(0,7);2,308(0,9);2,296(0,6);2,292(0,7);2,284(0 ,8);2,280(1,0);2,277(0,9);2,273(0,9);2,269(0,8);2,261(0,5);2,257(0,5);2,245(0,7);2,068(0,6);2,045(0,8);2,041(0,9); 2,037(0,8);2,033(1,1);2,029(0,9);2,018(0,7);2,006(0,9);1,922(0,7);1,908(0,9);1,896(0,8);1,887(1,3);1,873(1,2);1,8 64(0,6);1,852(0,6);0,008(1,6);0,000(53,0);-0,009(1,6) |
| Beispiel100(lba):¹H-NMR(400,0MHz,CDCl₃): δ = 8,503(1,6);8,500(2,5);8,497(1,6);8,491(1,7);8,488(2,5);8,418(2,2);8,414(1,4);8,409(1,4);8,406(2,2);7,519(0,9);7,4 69(1,1);7,465(1,2);7,447(1,8);7,445(2,4);7,424(1,5);7,421(1,5);7,325(1,6);7,313(2,5);7,303(2,3);7,292(1,9);7,282( 1,7);7,260(161,3);7,242(2,3);7,239(2,3);7,224(1,4);7,219(2,8);7,216(3,0);7,206(1,4);7,198(3,4);7,191(2,6);7,187( 3,3);7,176(3,5);7,166(1,9);7,155(0,9);7,150(1,5);7,115(1,1);7,110(1,4);7,095(0,8);7,089(0,9);7,025(0,9);7,004(1,7 );7,001(1,2);6,996(1,1);6,984(1,5);6,980(2,5);6,974(1,1);6,959(1,6);6,956(1,3);6,947(1,1);6,934(0,9);6,929(1,0);6, 881(0,9);6,876(1,2);6,871(1,0);6,865(0,9);6,854(0,9);4,351(3,0);4,337(2,6);4,326(3,3);4,311(2,9);4,150(1,2);4,13 2(3,5);4,115(3,5);4,097(1,2);3,950(0,6);3,939(0,6);3,928(1,4);3,918(1,4);3,904(1,6);3,893(1,5);3,880(0,7);3,871(0 ,6);3,696(0,7);3,682(1,3);3,668(1,5);3,654(1,8);3,641(1,0);3,557(0,8);3,543(1,5);3,529(2,1);3,515(2,7);3,501(1,7); 3,491(0,7);3,485(0,8);3,472(0,7);3,423(1,1);3,410(1,2);3,402(1,3);3,395(0,9);3,389(1,2);3,382(0,9);3,375(0,9);3,3 62(0,8);2,427(0,8);2,417(1,5);2,403(1,9);2,394(1,8);2,381(1,9);2,368(0,8);2,233(7,8);2,107(1,2);2,091(0,8);2,079( 0,8);2,067(1,0);2,056(1,3);2,046(16,0);2,032(1,1);2,019(0,5);1,882(0,6);1,867(0,7);1,862(0,7);1,857(0,7);1,847(1, 0);1,837(1,0);1,827(0,6);1,277(4,2);1,260(8,4);1,242(4,1);0,069(5,8);0,008(2,6);0,000(57,3);-0,009(2,1) |
| Beispiel289erythro1(lba):¹H-NMR(400,0MHz,CDCl₃): δ = 8,393(7,4);8,195(7,2);7,521(1,0);7,295(0,6);7,262(185,3);7,246(6,1);7,155(4,0);7,134(7,9);7,131(5,5);7,114(6,1); 7,110(7,9);7,090(5,1);7,083(4,2);7,078(4,3);7,065(4,4);7,059(5,5);7,051(4,4);7,039(4,0);7,033(4,0);6,998(1,1);6,9 18(4,2);6,914(5,2);6,909(4,4);6,903(3,9);6,897(3,7);6,892(4,3);6,888(3,4);5,299(16,0);4,046(10,1);4,028(10,6);3, 708(2,7);3,697(3,8);3,694(3,8);3,682(5,1);3,671(4,4);3,667(4,2);3,657(3,2);3,483(2,2);3,474(2,6);3,466(2,7);3,45 6(4,3);3,447(2,9);3,439(2,6);3,429(2,1);3,391(3,0);3,380(3,4);3,367(4,6);3,356(4,6);3,341(2,9);3,330(2,7);2,324(1 ,3);2,310(1,7);2,300(2,6);2,289(3,2);2,276(3,3);2,265(3,4);2,255(2,2);2,241(1,5);2,096(1,7);2,086(3,2);2,075(2,1); 2,069(2,1);2,058(3,9);2,050(3,4);2,040(1,6);2,033(1,6);2,023(2,3);2,012(1,2);1,622(3,8);1,333(0,8);1,284(1,1);1,2 56(1,6);0,008(2,0);0,000(64,2);-0,006(0,6);-0,007(0,5);-0,009(1,8) |
| Beispiel620(lba):¹H-NMR(400,0MHz,CDCl₃): δ = 8,381(16,0);8,369(1,1);8,243(1,1);8,214(15,4);7,519(5,4);7,293(0,6);7,289(0,8);7,260(956,7);7,250(5,0);7,244 (2,3);7,229(3,0);7,226(3,7);7,218(2,0);7,209(3,2);7,205(3,4);7,200(1,8);7,185(2,0);7,147(0,6);7,128(1,3);7,122(1, 8);7,118(1,4);7,112(1,2);7,101(1,3);7,093(1,8);7,087(1,4);7,071(2,0);7,061(1,4);7,050(3,1);7,043(1,5);7,030(1,9); 7,026(2,2);7,006(1,7);6,996(5,4);6,921(1,6);5,299(2,1);4,265(3,9);4,240(4,4);4,184(3,4);4,157(4,4);3,974(0,8);3,9 65(0,8);3,946(1,4);3,936(1,4);3,917(0,7);3,908(0,6);3,887(0,8);3,875(0,8);3,861(1,4);3,848(1,4);3,836(0,9);3,823( 0,7);3,779(0,9);3,766(1,4);3,753(1,8);3,740(1,7);3,726(1,1);3,636(1,0);3,623(1,6);3,610(2,0);3,597(1,9);3,583(1,2 );3,478(0,9);3,467(1,1);3,456(1,1);3,444(1,2);3,428(1,0);3,411(1,0);3,399(1,1);3,388(1,2);3,377(1,2);3,362(0,9);3, 350(0,9);2,544(0,6);2,535(0,9);2,522(1,0);2,510(1,0);2,499(1,3);2,490(0,7);2,476(0,6);2,294(0,8);2,266(0,9);2,23 1(0,7);2,040(1,1);2,013(0,9);2,005(0,7);1,936(0,7);1,923(1,0);1,912(0,9);1,901(1,4);1,888(1,2);1,878(0,7);1,865(0 ,7);1,543(11,9);1,371(0,9);1,333(1,7);1,284(2,4);1,256(2,2);0,146(1,0);0,008(9,1);0,000(336,9);-0,009(9,9);-0,150(1,0) |
| Beispiel84(lba):¹H-NMR(400,0MHz,CDCl₃): δ = 8,385(4,0);8,377(5,5);8,374(7,4);8,368(6,9);8,201(3,7);7,959(6,9);7,351(2,4);7,345(3,2);7,340(2,5);7,328(3,5);7,3 22(3,5);7,316(2,8);7,307(3,5);7,291(4,2);7,286(4,9);7,278(2,6);7,272(6,6);7,262(66,2);7,251(4,4);7,244(1,5);7,04 1(1,1);7,034(1,4);7,029(1,9);7,022(3,0);7,013(2,3);7,010(3,1);7,008(3,1);7,003(3,4);7,001(3,5);6,995(1,3);6,989(1 ,6);6,987(1,6);6,982(1,7);6,966(2,4);6,956(1,5);6,950(3,1);6,947(3,6);6,932(1,6);6,927(1,8);6,922(1,0);6,887(4,0); 6,868(3,8);6,858(2,8);6,853(3,1);6,848(1,8);6,835(2,4);6,830(3,0);6,825(1,8);5,299(16,0);4,307(5,9);4,292(6,1);4, 057(3,2);4,040(3,4);3,835(1,2);3,798(1,6);3,785(3,4);3,772(3,5);3,758(4,1);3,745(2,0);3,690(0,9);3,679(1,1);3,67 5(1,1);3,664(1,6);3,653(1,3);3,648(1,2);3,638(1,0);3,547(1,9);3,535(2,2);3,526(2,3);3,520(2,0);3,514(2,4);3,509(2 ,1);3,499(2,4);3,487(2,3);3,474(2,4);3,460(2,7);3,446(2,0);3,437(2,3);3,422(1,1);3,385(1,0);3,373(1,2);3,361(1,4); 3,349(1,4);3,346(1,2);3,335(1,0);3,323(0,9);2,320(0,5);2,315(0,5);2,305(0,9);2,295(1,0);2,284(0,9);2,280(1,0);2,2 70(1,2);2,259(1,1);2,245(1,5);2,237(0,8);2,231(0,9);2,223(2,2);2,209(2,6);2,202(1,6);2,195(1,5);2,188(2,0);2,174( 1,3);2,170(1,1);2,162(1,3);2,150(2,3);2,138(2,2);2,127(2,8);2,114(2,5);2,102(1,8);2,091(1,6);2,084(0,8);2,078(1,0 );2,073(1,2);2,065(1,0);2,054(0,6);2,048(0,6);2,043(0,8);2,038(0,8);2,005(0,9);1,752(1,5);1,371(0,6);1,286(0,9);1, 258(1,4);0,008(1,0);0,000(25,2);-0,009(1,2) |
| Beispiel295(lba):¹H-NMR(400,0MHz,CDCl₃): δ = 8,492(0,8);8,488(1,6);8,485(0,9);8,480(0,9);8,477(1,6);8,473(0,9);8,421(0,8);8,417(1,5);8,414(0,9);8,410(0,8);8,4 06(1,5);8,403(0,8);7,557(1,4);7,551(1,5);7,541(1,4);7,535(1,5);7,431(0,9);7,428(0,9);7,410(1,3);7,407(1,5);7,404( 1,0);7,387(1,2);7,383(1,1);7,308(1,4);7,302(1,5);7,299(0,9);7,293(3,2);7,287(1,8);7,282(2,5);7,278(1,2);7,272(2,5 );7,266(1,7);7,261(74,3);7,250(1,0);7,239(0,7);7,236(0,8);7,218(1,4);7,216(0,9);7,215(1,4);7,213(0,9);7,195(1,4); 7,192(1,3);7,177(1,4);7,166(2,4);7,155(1,6);7,148(2,0);7,145(1,3);7,134(0,7);7,127(2,8);7,107(1,5);7,079(0,6);7,0 73(0,6);7,067(0,7);7,062(0,6);7,057(0,9);7,051(0,9);7,046(0,9);7,040(0,8);6,965(1,9);6,944(2,7);6,923(1,3);4,342( 2,0);4,332(1,8);4,318(2,2);4,306(2,0);4,148(1,1);4,130(3,3);4,112(3,3);4,094(1,1);3,903(0,9);3,892(0,7);3,889(0,9 );3,878(0,9);3,662(0,9);3,648(1,0);3,634(1,0);3,621(0,7);3,535(0,8);3,522(1,1);3,508(1,2);3,501(0,7);3,494(0,7);3, 486(0,6);3,481(0,7);3,467(0,7);3,453(0,5);3,411(0,6);3,398(0,7);3,390(0,7);3,383(0,5);3,377(0,7);2,419(0,5);2,40 9(0,8);2,405(0,6);2,396(1,0);2,387(1,1);2,374(1,2);2,095(0,9);2,057(0,5);2,043(16,0);2,035(0,6);2,023(0,6);1,842( 0,6);1,831(0,6);1,588(0,7);1,333(0,7);1,284(1,1);1,276(4,9);1,258(10,4);1,240(4,8);0,008(0,8);0,000(27,5);-0,009(0,7) |
| Beispiel619(lba):¹H-NMR(400,0MHz,CDCl₃): δ = 8,384(14,1);8,219(16,0);7,588(2,5);7,582(2,7);7,572(2,6);7,566(2,7);7,519(1,1);7,431(2,4);7,425(2,6);7,415(2,5); 7,410(2,6);7,325(1,3);7,319(1,3);7,314(1,5);7,308(1,4);7,304(1,9);7,298(1,9);7,293(2,0);7,287(1,7);7,260(176,1); 7,191(3,4);7,170(4,9);7,150(2,8);7,122(1,3);7,116(1,2);7,111(1,3);7,105(1,3);7,101(1,7);7,095(1,7);7,089(1,7);7,0 84(1,6);7,010(3,9);6,996(1,3);6,990(5,2);6,969(2,6);4,258(3,5);4,233(4,0);4,176(3,8);4,148(4,9);3,972(0,9);3,962( 0,9);3,944(1,5);3,935(1,5);3,917(0,7);3,907(0,7);3,879(0,8);3,867(0,8);3,853(1,4);3,841(1,4);3,828(0,8);3,816(0,7 );3,780(1,1);3,767(1,7);3,753(2,0);3,740(2,0);3,727(1,3);3,632(1,0);3,619(1,6);3,606(2,1);3,593(2,0);3,580(1,3);3, 477(1,2);3,466(1,3);3,454(1,4);3,450(1,3);3,443(1,4);3,439(1,3);3,427(1,2);3,415(1,1);3,409(1,2);3,397(1,2);3,38 6(1,3);3,382(1,1);3,375(1,3);3,371(1,1);3,360(1,0);3,348(1,0);2,553(0,6);2,543(0,6);2,539(0,6);2,530(1,0);2,518(1 ,1);2,507(1,1);2,495(1,2);2,485(0,8);2,482(0,8);2,472(0,6);2,297(0,9);2,282(0,6);2,270(1,0);2,266(0,9);2,262(0,9); 2,258(0,8);2,234(0,7);2,071(0,7);2,059(0,5);2,047(0,8);2,044(0,9);2,036(1,1);2,021(0,8);2,009(0,9);1,997(0,5);1,9 28(0,7);1,915(1,0);1,904(0,9);1,893(1,3);1,880(1,2);1,870(0,7);1,858(0,7);1,845(0,5);1,672(0,8);1,621(0,6);0,008( 2,0);0,000(66,8);-0,009(2,0) |
| Beispiel489(lba):¹H-NMR(400,0MHz,CDCl₃): δ = 8,597(1,1);8,576(1,6);8,561(1,5);8,526(1,7);8,494(2,3);8,485(2,3);8,469(1,4);8,444(4,3);8,371(2,7);8,358(2,7);8,3 05(4,3);8,255(0,6);7,844(2,4);7,831(2,4);7,714(2,8);7,701(2,7);7,519(1,6);7,311(2,9);7,286(4,4);7,260(284,4);7,2 11(2,8);7,191(1,7);7,054(1,7);7,042(1,8);7,015(1,5);6,996(2,3);6,961(1,6);6,941(1,4);6,878(1,7);6,826(0,9);6,805( 1,2);6,700(0,6);5,298(1,2);4,469(0,7);4,444(1,3);4,429(1,2);4,419(1,1);4,404(0,8);4,380(0,6);4,365(0,8);4,348(0,7 );4,335(0,7);4,254(1,1);4,084(1,0);4,063(1,0);3,947(0,6);3,795(0,9);3,782(0,7);3,690(0,9);3,609(0,9);3,540(1,5);3, 531(1,6);3,513(1,6);3,495(2,2);3,471(16,0);3,440(1,7);3,105(0,6);3,082(0,6);2,924(0,5);2,473(2,5);2,457(2,3);2,4 49(2,4);2,423(1,2);2,186(1,7);2,163(1,7);1,253(0,9);0,007(4,2);0,000(100,3);-0,001(97,6);-0,008(5,1);-0,150(0,5) |
| Beispiel511(lba):¹H-NMR(400,0MHz,CDCl₃): δ = 8,620(14,0);8,556(12,5);8,543(12,3);8,521(13,4);7,920(7,6);7,906(8,6);7,898(8,8);7,884(7,8);7,843(1,2);7,836(9, 9);7,831(3,8);7,823(10,5);7,819(5,1);7,817(5,0);7,814(11,0);7,806(3,8);7,800(10,4);7,793(1,1);7,603(5,1);7,597(5 ,3);7,588(5,2);7,582(5,1);7,525(5,9);7,519(4,6);7,512(5,8);7,495(4,0);7,483(3,7);7,342(3,4);7,309(3,1);7,303(3,0); 7,298(3,2);7,292(3,4);7,288(3,9);7,282(4,0);7,277(4,2);7,270(4,7);7,260(243,0);7,151(6,7);7,130(10,9);7,127(16, 0);7,121(5,0);7,110(11,4);7,104(25,6);7,098(5,6);7,089(6,2);7,083(22,9);7,077(5,3);7,065(3,9);7,060(11,9);7,037( 6,2);7,017(5,3);6,996(4,1);5,298(10,2);4,412(1,9);4,401(2,1);4,386(2,2);4,300(3,2);4,286(3,6);4,275(3,1);4,260(4, 2);4,246(4,4);4,232(5,2);4,217(2,5);4,179(1,8);4,164(3,1);4,150(3,0);4,135(2,9);4,119(2,0);4,104(5,1);4,088(6,8); 4,073(3,5);4,067(3,7);4,058(2,9);4,053(3,4);4,045(3,0);4,039(3,2);4,025(3,1);3,910(1,6);3,901(1,9);3,881(2,0);3,8 73(1,9);3,782(1,5);2,541(0,7);2,526(1,3);2,512(1,4);2,497(1,6);2,490(2,2);2,476(1,9);2,461(2,1);2,448(1,4);2,392( 3,5);2,367(3,7);2,359(3,1);2,347(3,5);2,332(2,4);2,323(2,1);2,311(1,4);2,286(1,5);1,256(1,5);0,994(0,6);0,974(1,6 );0,950(6,5);0,930(16,0);0,922(0,6);0,910(7,9);0,611(0,7);0,591(0,7);0,547(2,5);0,528(7,3);0,508(6,7);0,488(1,8); 0,008(3,2);0,000(91,7);-0,009(2,6) |
| Beispiel289(lba):¹H-NMR(400,0MHz,CDCl₃): δ = 7,518(15,3);7,294(5,1);7,260(2831,6);7,227(5,7);7,209(4,0);6,996(16,0);1,537(809,6);1,256(2,7);0,146(3,8);0,069 (19,1);0,008(40,2);0,000(960,5);-0,008(36,7);-0,150(3,9) |
| Beispiel505(lba):¹H-NMR(400,0MHz,CDCl₃): δ = 8,607(13,2);8,526(13,9);8,513(14,5);8,499(16,0);7,520(1,2);7,439(7,7);7,426(7,6);7,327(2,9);7,314(2,9);7,295(0, 9);7,261(218,8);7,251(2,0);7,239(1,9);7,232(2,5);7,224(1,9);7,212(2,1);7,207(3,3);7,185(3,2);7,166(3,1);7,161(3, 1);7,148(1,0);7,142(2,9);7,112(3,7);7,091(4,5);7,066(3,7);7,045(2,2);6,997(2,6);6,978(2,2);6,955(1,4);6,849(2,0); 5,299(12,2);4,358(4,3);4,342(4,9);4,148(3,0);4,132(3,5);4,038(1,4);3,928(1,1);3,918(1,4);3,902(1,7);3,890(1,4);3, 876(1,0);3,726(1,6);3,712(1,8);3,699(2,0);3,614(1,4);3,602(1,7);3,587(1,6);3,498(1,7);3,353(1,4);2,280(0,6);2,26 7(1,0);2,244(1,3);2,232(1,8);2,216(1,6);2,204(2,2);2,192(1,7);2,158(1,9);2,149(1,8);2,143(1,6);2,134(1,9);2,127(2 ,4);2,114(3,1);2,102(2,4);2,091(3,3);2,079(2,4);2,067(1,2);2,056(1,3);2,043(0,7);2,005(0,7);1,586(1,7);1,492(2,0); 1,318(1,6);1,255(0,9);0,008(2,6);0,0064(1,0);0,0056(1,0);0,005(1,1);0,000(80,3);-0,006(0,8);-0,007(0,7);-0,009 (2,3) |
| Beispiel296(lba):¹H-NMR(400,0MHz,CDCl₃): δ = 8,410(3,1);8,403(7,8);8,396(5,2);8,205(2,5);7,969(4,4);7,519(3,1);7,373(2,2);7,365(1,3);7,359(1,9);7,353(1,7);7,3 42(1,5);7,336(1,9);7,331(1,5);7,289(1,9);7,283(2,1);7,278(1,7);7,272(4,2);7,260(579,5);7,244(1,7);7,227(0,9);7,2 09(0,9);7,197(2,7);7,191(2,8);7,181(2,6);7,175(2,6);7,121(1,2);7,100(3,7);7,093(2,9);7,079(2,5);7,072(6,5);7,050( 4,8);7,044(1,2);7,039(1,3);7,033(1,1);7,029(0,7);7,023(0,6);7,018(0,6);7,011(0,6);6,996(3,3);6,955(1,6);6,949(1,6 );6,945(1,8);6,939(1,6);6,934(1,3);6,928(1,3);6,923(1,2);6,917(1,1);5,299(16,0);4,286(3,8);4,271(3,9);4,039(2,3); 4,022(2,5);3,835(0,9);3,814(1,0);3,802(2,3);3,788(2,2);3,775(2,9);3,762(1,3);3,713(0,6);3,703(0,8);3,687(1,0);3,6 76(0,9);3,672(0,8);3,662(0,7);3,561(1,2);3,550(1,5);3,540(1,4);3,535(1,3);3,529(1,5);3,523(1,3);3,513(1,2);3,502( 1,1);3,440(1,1);3,426(1,5);3,404(1,4);3,387(1,2);3,374(0,9);3,360(0,9);3,347(0,7);3,336(0,7);2,615(1,1);2,289(0,7 );2,278(0,6);2,264(0,7);2,254(0,9);2,235(0,7);2,229(0,8);2,221(0,6);2,213(1,5);2,200(1,5);2,192(1,1);2,186(1,0);2, 178(1,2);2,170(0,9);2,164(0,8);2,155(0,9);2,143(1,4);2,131(1,3);2,120(1,7);2,107(1,5);2,098(0,7);2,087(0,9);2,07 1(0,8);2,060(0,8);2,053(0,6);2,044(0,6);2,025(0,6);2,005(0,6);1,552(6,2);1,333(0,9);1,284(1,0);1,259(0,9);0,146(0 ,6);0,113(0,8);0,008(6,2);0,000(194,3);-0,009(5,5);-0,150(0,6) |
| Beispiel613(lba):¹H-NMR(400,0MHz,CDCl₃): δ = 8,381(16,0);8,369(1,2);8,243(1,1);8,214(15,4);7,519(5,4);7,293(0,7);7,289(0,8);7,260(956,7);7,250(5,0);7,244(2, 3);7,229(3,0);7,226(3,7);7,218(2,0);7,209(3,2);7,205(3,4);7,200(1,9);7,185(2,0);7,147(0,6);7,128(1,3);7,122(1,8); 7,118(1,4);7,112(1,2);7,101(1,3);7,093(1,8);7,087(1,4);7,071(2,0);7,061(1,4);7,050(3,1);7,043(1,5);7,030(1,9);7,0 26(2,2);7,006(1,7);6,996(5,4);6,921(1,6);5,299(2,1);4,265(4,0);4,240(4,4);4,184(3,4);4,157(4,4);3,974(0,8);3,965( 0,8);3,946(1,4);3,936(1,4);3,917(0,7);3,908(0,6);3,887(0,8);3,875(0,8);3,861(1,4);3,848(1,4);3,836(0,9);3,823(0,7 );3,779(0,9);3,766(1,4);3,753(1,8);3,740(1,8);3,726(1,1);3,636(1,0);3,623(1,6);3,610(2,0);3,597(2,0);3,583(1,2);3, 478(0,9);3,467(1,1);3,456(1,2);3,444(1,2);3,428(1,0);3,411(1,0);3,399(1,1);3,388(1,2);3,377(1,2);3,362(0,9);3,35 0(0,9);2,544(0,6);2,535(0,9);2,522(1,0);2,510(1,0);2,499(1,3);2,490(0,7);2,476(0,6);2,294(0,8);2,266(0,9);2,231(0 ,7);2,040(1,1);2,013(0,9);2,005(0,7);1,936(0,7);1,923(1,0);1,912(0,9);1,901(1,4);1,888(1,2);1,878(0,7);1,865(0,7); 1,543(11,9);1,371(0,9);1,333(1,7);1,284(2,4);1,256(2,2);0,146(1,0);0,008(9,1);0,000(336,9);-0,009(9,9);-0,150(1,0) |
| Beispiel289threo2(lba):¹H-NMR(400,0MHz,CDCl₃): δ = 8,393(7,4);8,195(7,2);7,521(1,0);7,295(0,6);7,262(185,3);7,246(6,1);7,155(4,0);7,134(7,9);7,131(5,5);7,114(6,1); 7,110(7,9);7,090(5,1);7,083(4,2);7,078(4,3);7,065(4,4);7,059(5,5);7,051(4,4);7,039(4,0);7,033(4,0);6,998(1,1);6,9 18(4,2);6,914(5,2);6,909(4,4);6,903(3,9);6,897(3,7);6,892(4,3);6,888(3,4);5,299(16,0);4,046(10,1);4,028(10,6);3, 708(2,7);3,697(3,8);3,694(3,8);3,682(5,1);3,671(4,4);3,667(4,2);3,657(3,2);3,483(2,2);3,474(2,6);3,466(2,7);3,45 6(4,3);3,447(2,9);3,439(2,6);3,429(2,1);3,391(3,0);3,380(3,4);3,367(4,6);3,356(4,6);3,341(2,9);3,330(2,6);2,324(1 ,3);2,310(1,7);2,300(2,6);2,289(3,2);2,276(3,3);2,265(3,4);2,255(2,2);2,241(1,5);2,096(1,7);2,086(3,2);2,075(2,1); 2,069(2,1);2,058(3,8);2,050(3,4);2,040(1,6);2,033(1,6);2,023(2,3);2,012(1,2);1,622(3,8);1,333(0,8);1,284(1,1);1,2 56(1,6);0,008(2,0);0,000(64,2);-0,006(0,6);-0,007(0,5);-0,009(1,8) |
| Beispiel86(lba):¹H-NMR(400,0MHz,CDCl₃): δ = 8,399(5,2);8,392(5,9);8,388(7,3);8,381(7,0);8,213(3,8);7,967(6,9);7,519(1,3);7,347(2,5);7,342(3,2);7,336(2,6);7,3 24(2,9);7,319(4,3);7,313(4,2);7,305(3,8);7,295(5,5);7,290(4,5);7,285(7,2);7,280(10,6);7,277(8,4);7,260(230,5);7, 250(9,2);7,242(3,1);7,231(7,3);7,226(3,4);7,221(4,7);7,216(2,8);7,211(3,4);7,175(1,0);7,137(4,4);7,133(7,2);7,12 8(3,9);7,061(2,7);7,042(2,0);6,996(1,8);6,959(3,8);6,940(3,4);5,299(16,0);4,491(0,6);4,477(0,6);4,278(5,6);4,263( 5,8);4,038(3,3);4,021(3,5);3,835(1,0);3,802(1,5);3,789(3,1);3,776(3,2);3,762(3,8);3,749(1,9);3,695(0,9);3,684(1,4 );3,669(1,6);3,658(1,3);3,654(1,2);3,643(1,2);3,553(1,7);3,542(2,0);3,532(2,1);3,526(1,8);3,520(2,1);3,515(1,7);3, 505(1,6);3,493(2,0);3,473(0,9);3,463(2,2);3,447(2,7);3,437(2,1);3,425(2,2);3,408(1,1);3,389(1,0);3,377(1,0);3,36 5(1,3);3,353(1,3);3,339(0,9);3,327(0,8);2,964(0,8);2,945(0,8);2,911(0,5);2,315(0,5);2,300(0,6);2,291(0,9);2,281(1 ,0);2,266(1,0);2,255(1,4);2,240(1,4);2,232(1,3);2,225(0,9);2,218(2,1);2,204(2,5);2,196(1,5);2,190(1,5);2,183(1,9); 2,168(1,2);2,158(1,3);2,145(2,3);2,134(2,0);2,122(2,7);2,110(2,5);2,099(1,8);2,087(1,4);2,074(1,4);2,065(0,9);2,0 48(0,6);2,037(0,8);1,570(1,7);1,371(1,1);1,333(0,9);1,286(1,7);1,257(2,5);0,880(0,5);0,008(3,5);0,000(88,5);-0,009(3,7) |
| Beispiel619(lbab):¹H-NMR(400,0MHz,CDCl₃): δ = 8,421(9,4);8,250(10,9);7,595(1,5);7,590(1,6);7,580(1,5);7,574(1,5);7,417(1,6);7,411(1,7);7,401(1,6);7,396(1,6);7, 328(0,7);7,322(0,7);7,317(0,8);7,311(0,7);7,307(1,1);7,301(1,1);7,296(1,1);7,290(1,0);7,261(61,7);7,217(1,9);7,1 96(2,6);7,176(1,3);7,113(0,7);7,107(0,7);7,102(0,8);7,096(0,8);7,092(1,1);7,086(1,1);7,081(1,1);7,075(0,9);7,019( 2,2);6,999(3,0);6,978(1,4);4,267(0,6);4,254(1,3);4,239(1,2);4,225(1,7);4,216(2,4);4,190(2,5);4,164(2,5);4,136(3,1 );4,117(1,3);4,102(1,2);4,088(1,5);4,075(0,9);4,046(0,9);4,034(1,0);4,024(1,1);4,017(0,9);4,012(1,0);4,006(0,8);3, 995(0,7);3,983(0,7);3,961(16,0);3,926(14,2);3,908(0,8);3,901(0,7);3,896(0,8);3,890(0,6);3,879(0,6);3,868(0,6);3, 845(0,6);3,836(0,6);3,817(1,0);3,807(1,0);3,789(0,5);3,779(0,5);3,769(0,5);3,741(0,8);3,731(0,8);2,682(0,6);2,66 9(0,8);2,659(0,8);2,646(0,8);2,637(0,5);2,464(0,5);2,437(0,6);2,183(0,6);2,015(0,6);2,004(0,6);1,992(0,7);1,980(0 ,7);1,586(0,6);1,255(0,7);0,008(0,8);0,000(23,4);-0,008(0,8) |
| Beispiel599(lbae):¹H-NMR(400,0MHz,CDCl₃): δ = 8,409(5,4);8,215(5,9);7,400(1,0);7,396(2,3);7,394(2,0);7,392(1,5);7,378(3,0);7,274(0,8);7,269(1,2);7,261(39,2);7, 252(1,0);7,247(0,6);7,242(0,5);7,240(0,5);7,237(0,6);7,226(0,7);7,222(1,0);7,221(1,3);7,217(1,1);7,196(2,7);7,17 7(1,7);7,157(0,7);7,052(0,9);7,033(0,7);4,214(1,4);4,206(0,7);4,188(1,8);4,177(0,9);4,163(1,7);4,135(1,7);4,058(0 ,6);4,043(0,6);4,029(1,2);4,016(0,7);3,922(8,4);3,889(0,5);3,882(4,2);3,878(4,3);3,849(0,5);3,822(0,5);3,812(0,5); 3,407(16,0);3,387(14,7);0,000(14,8) |
| Beispiel289Threo1(lba):¹H-NMR(400,0MHz,CDCl₃): δ = 8,395(14,8);8,389(14,9);8,193(14,6);7,521(1,2);7,295(0,8);7,285(0,6);7,284(0,6);7,283(0,6);7,2823(0,5);7,2815(0 ,5);7,281(0,6);7,2783(0,9);7,2775(0,9);7,277(1,0);7,276(1,2);7,272(6,5);7,2663(10,5);7,2655(11,5);7,262(205,0); 7,256(2,0);7,255(1,7);7,254(1,5);7,253(1,4);7,252(1,4);7,249(5,9);7,244(7,3);7,238(5,5);7,155(5,5);7,134(9,2);7,1 31(6,3);7,114(7,2);7,110(9,2);7,089(6,6);7,083(5,1);7,077(5,3);7,065(5,2);7,059(6,1);7,057(6,0);7,050(5,4);7,038( 4,9);7,032(5,1);6,998(1,2);6,923(3,3);6,918(4,3);6,913(6,2);6,908(4,4);6,902(4,4);6,897(3,7);6,892(5,0);6,887(3,5 );6,882(2,5);5,299(8,7);4,045(15,0);4,027(16,0);3,708(4,0);3,698(4,8);3,694(4,9);3,682(6,6);3,671(5,8);3,667(5,6) ;3,657(4,8);3,482(2,7);3,472(2,9);3,464(2,8);3,454(5,3);3,445(3,1);3,437(2,9);3,427(2,6);3,391(4,8);3,380(5,6);3, 367(6,2);3,365(5,4);3,356(6,1);3,353(5,8);3,341(4,8);3,329(4,4);2,323(2,1);2,313(2,3);2,309(2,5);2,299(4,3);2,28 8(4,4);2,285(2,9);2,278(3,4);2,274(4,4);2,264(5,6);2,254(3,1);2,250(2,9);2,240(2,6);2,096(2,7);2,085(5,0);2,074(2 ,9);2,068(2,8);2,058(5,3);2,050(4,3);2,047(3,4);2,039(2,1);2,033(2,2);2,022(3,7);2,011(1,9);1,592(5,5);1,333(1,0); 1,284(1,4);1,255(1,6);0,008(2,0);0,000(66,6);-0,009(2,0) |
| Beispiel512(lba):¹H-NMR(400,0MHz,CDCl₃): δ = 8,564(1,2);8,544(11,3);8,529(3,7);8,495(16,0);8,471(1,0);8,440(0,7);8,425(1,0);8,412(7,1);8,400(7,1);8,393(2,6); 8,381(2,6);8,372(10,3);8,360(10,3);7,521(0,9);7,500(4,9);7,495(5,3);7,484(5,1);7,478(5,3);7,453(3,8);7,447(4,1); 7,436(3,9);7,430(4,1);7,373(0,8);7,333(3,3);7,327(3,3);7,322(3,5);7,316(3,6);7,311(4,6);7,305(4,5);7,300(4,5);7,2 94(4,6);7,286(3,0);7,281(3,3);7,275(3,5);7,270(5,2);7,262(109,3);7,254(4,7);7,242(1,5);7,219(6,0);7,202(11,1);7, 198(12,8);7,187(8,0);7,181(12,3);7,176(4,7);7,160(5,4);7,127(0,7);7,105(8,9);7,092(8,6);6,998(0,8);5,299(5,0);4, 417(7,0);4,402(7,1);4,052(5,4);4,034(5,5);3,868(0,5);3,851(0,5);3,811(3,1);3,801(3,1);3,784(3,4);3,773(3,4);3,72 7(0,5);3,690(13,9);3,564(3,2);3,546(3,4);3,537(5,8);3,519(6,0);3,501(3,9);3,489(4,1);3,481(6,1);3,472(8,7);3,462( 3,5);3,454(3,5);3,445(3,1);3,090(1,7);3,071(3,2);3,051(2,1);3,021(3,2);3,011(3,4);2,987(4,8);2,977(4,8);2,885(1,8 );2,866(5,4);2,852(4,7);2,838(5,9);2,827(7,5);2,811(5,2);2,804(4,4);2,792(1,7);2,777(1,4);2,702(2,3);2,683(3,6);2, 664(1,9);2,494(0,9);2,483(1,5);2,470(2,0);2,465(1,9);2,460(2,2);2,451(2,2);2,446(1,9);2,441(2,4);2,430(2,3);2,42 2(2,0);2,413(2,7);2,404(2,5);2,397(2,6);2,387(2,4);2,379(1,5);2,370(1,3);2,360(0,7);1,840(1,0);1,255(0,5);0,008(1 ,6);0,000(40,9);-0,009(1,7) |
| Beispiel 619(lbab): ¹H-NMR(400,0 MHz, CDCl₃): δ = 8,421(9,4);8,250(10,9);7,595(1,5);7,590(1,6);7,580(1,5);7,574(1,5);7,417(1,6);7,411(1,7);7,401(1,6);7,396(1,6);7, 328(0,7);7,322(0,7);7,317(0,8);7,311(0,7);7,307(1,1);7,301(1,1);7,296(1,1);7,290(1,0);7,261(61,7);7,217(1,9);7,1 96(2,6);7,176(1,3);7,113(0,7);7,107(0,7);7,102(0,8);7,096(0,8);7,092(1,1);7,086(1,1);7,081(1,1);7,075(0,9);7,019( 2,2);6,999(3,0);6,978(1,4);4,267(0,6);4,254(1,3);4,239(1,2);4,225(1,7);4,216(2,4);4,190(2,5);4,164(2,5);4,136(3,1 );4,117(1,3);4,102(1,2);4,088(1,5);4,075(0,9);4,046(0,9);4,034(1,0);4,024(1,1);4,017(0,9);4,012(1,0);4,006(0,8);3, 995(0,7);3,983(0,7);3,961(16,0);3,926(14,2);3,908(0,8);3,901(0,7);3,896(0,8);3,890(0,6);3,879(0,6);3,868(0,6);3, 845(0,6);3,836(0,6);3,817(1,0);3,807(1,0);3,789(0,5);3,779(0,5);3,769(0,5);3,741(0,8);3,731(0,8);2,682(0,6);2,66 9(0,8);2,659(0,8);2,646(0,8);2,637(0,5);2,464(0,5);2,437(0,6);2,183(0,6);2,015(0,6);2,004(0,6);1,992(0,7);1,980(0 ,7);1,586(0,6);1,255(0,7);0,008(0,8);0,000(23,4);-0,008(0,8) |
| Beispiel 6198(lba): ¹H-NMR(400,0 MHz, CDCl₃): δ = 8,384(14,1);8,219(16,0);7,588(2,5);7,582(2,7);7,572(2,6);7,566(2,7);7,519(1,1);7,431(2,4);7,425(2,6);7,415(2,5); 7,410(2,6);7,325(1,3);7,319(1,3);7,314(1,5);7,308(1,4);7,304(1,9);7,298(1,9);7,293(2,0);7,287(1,7);7,260(176,1); 7,191(3,4);7,170(4,9);7,150(2,8);7,122(1,3);7,116(1,2);7,111(1,3);7,105(1,3);7,101(1,7);7,095(1,7);7,089(1,7);7,0 84(1,6);7,010(3,9);6,996(1,3);6,990(5,2);6,969(2,6);4,258(3,5);4,233(4,0);4,176(3,8);4,148(4,9);3,972(0,9);3,962( 0,9);3,944(1,5);3,935(1,5);3,917(0,7);3,907(0,7);3,879(0,8);3,867(0,8);3,853(1,4);3,841(1,4);3,828(0,8);3,816(0,7 );3,780(1,1);3,767(1,7);3,753(2,0);3,740(2,0);3,727(1,3);3,632(1,0);3,619(1,6);3,606(2,1);3,593(2,0);3,580(1,3);3, 477(1,2);3,466(1,3);3,454(1,4);3,450(1,3);3,443(1,4);3,439(1,3);3,427(1,2);3,415(1,1);3,409(1,2);3,397(1,2);3,38 6(1,3);3,382(1,1);3,375(1,3);3,371(1,1);3,360(1,0);3,348(1,0);2,553(0,6);2,543(0,6);2,539(0,6);2,530(1,0);2,518(1 ,1);2,507(1,1);2,495(1,2);2,485(0,8);2,482(0,8);2,472(0,6);2,297(0,9);2,282(0,6);2,270(1,0);2,266(0,9);2,262(0,9); 2,258(0,8);2,234(0,7);2,071(0,7);2,059(0,5);2,047(0,8);2,044(0,9);2,036(1,1);2,021(0,8);2,009(0,9);1,997(0,5);1,9 28(0,7);1,915(1,0);1,904(0,9);1,893(1,3);1,880(1,2);1,870(0,7);1,858(0,7);1,845(0,5);1,672(0,8);1,621(0,6);0,008( 2,0);0,000(66,8);-0,009(2,0) |
| Beispiel 208threo(lba): ¹H-NMR(400,0 MHz, CDCl₃): δ = 8,574(1,9);8,570(2,0);8,563(2,0);8,559(2,0);8,519(10,6);8,515(11,0);8,508(10,9);8,504(10,9);7,723(2,4);7,720(2, 4);7,703(2,6);7,699(2,5);7,538(13,3);7,534(13,7);7,517(15,0);7,513(14,7);7,261(206,1);7,223(2,7);7,217(0,9);7,2 11(3,7);7,202(3,2);7,199(1,0);7,191(3,4);7,182(1,6);7,178(1,0);7,172(0,9);7,167(1,1);7,162(1,4);7,157(1,5);7,138( 1,2);7,112(16,2);7,100(15,5);7,091(14,3);7,080(14,1);7,024(4,2);7,003(7,8);7,000(5,1);6,995(4,9);6,989(5,3);6,98 3(6,8);6,978(9,3);6,971(5,7);6,968(5,2);6,962(5,5);6,958(6,5);6,950(4,2);6,944(4,8);6,909(3,6);6,905(3,9);6,903(3 ,7);6,899(5,6);6,895(4,2);6,893(3,7);6,889(3,7);6,884(2,9);6,877(3,7);6,874(2,7);6,868(2,1);4,366(12,7);4,360(2,8 );4,341(16,0);4,160(4,3);4,143(7,2);4,134(4,0);4,126(4,6);4,117(5,6);4,109(1,8);4,100(3,6);4,085(0,7);3,631(3,3); 3,617(6,8);3,603(8,4);3,589(9,6);3,575(4,9);3,523(0,6);3,509(1,1);3,495(1,5);3,488(5,1);3,481(2,1);3,472(7,5);3,4 61(3,9);3,454(5,6);3,443(5,1);3,427(3,7);3,412(1,0);3,398(1,1);3,392(1,1);3,384(0,7);3,378(1,1);3,371(0,7);3,364( 0,7);3,351(0,6);2,409(8,5);2,393(12,7);2,377(12,6);2,361(7,8);2,093(0,7);2,069(0,8);2,058(1,0);2,048(0,5);2,045( 0,5);2,034(0,9);1,873(0,5);1,868(0,6);1,864(0,6);1,858(0,6);1,853(0,6);1,849(0,8);1,838(0,8);1,607(3,5);0,008(2,1 );0,000(77,2);-0,009(2,2) |
| Beispiel 1693(lba): ¹H-NMR(400,0 MHz, CDCl₃): δ = 8,012(0,8);7,261(52,9);7,160(0,8);7,156(1,4);7,139(1,4);7,135(3,0);7,131(1,6);7,118(1,2);7,115(2,8);7,110(2,3);7, 094(1,0);7,090(1,6);7,086(0,7);7,080(0,7);7,068(0,6);7,062(0,8);7,059(0,7);7,053(0,7);7,041(0,6);7,035(0,7);7,02 5(1,1);7,019(1,3);7,007(1,2);7,001(1,4);6,998(1,4);6,992(1,3);6,980(1,1);6,974(1,3);6,963(0,6);6,958(0,8);6,954(0 ,6);6,948(0,6);6,937(0,6);6,926(0,9);6,921(1,1);6,915(1,4);6,911(1,1);6,905(1,1);6,899(0,9);6,894(1,2);6,889(0,8); 6,885(0,6);6,377(3,2);6,368(5,6);6,359(3,7);6,279(4,2);6,275(4,3);6,269(3,5);6,265(3,5);6,257(2,2);6,252(2,2);6,2 47(1,9);6,242(1,8);4,144(3,5);4,130(3,6);3,951(1,9);3,933(2,0);3,821(1,0);3,808(2,2);3,795(2,1);3,781(2,9);3,769( 1,5);3,762(0,7);3,757(0,6);3,745(0,8);3,735(0,8);3,730(0,8);3,720(0,7);3,655(1,4);3,643(1,5);3,633(1,6);3,628(1,2 );3,622(1,6);3,616(1,2);3,606(1,2);3,595(1,1);3,563(0,7);3,551(0,8);3,539(0,9);3,536(0,7);3,528(0,9);3,525(0,8);3, 512(0,7);3,501(0,6);3,488(1,2);3,437(0,7);3,432(1,1);3,424(1,4);3,418(1,3);3,408(1,2);3,404(1,0);3,400(1,2);3,39 4(1,0);3,386(0,5);2,957(7,8);2,956(7,8);2,882(6,7);2,881(6,7);2,264(0,6);2,229(0,7);2,137(0,7);2,115(1,4);2,102(2 ,0);2,094(1,1);2,088(1,0);2,080(1,8);2,078(1,3);2,066(2,2);2,053(1,8);2,042(1,9);2,030(1,4);2,018(0,7);2,005(16,0 );1,943(0,6);1,915(0,6);1,908(0,5);1,462(2,5);0,008(0,6);0,000(19,5);-0,009(0,6) |
| Beispiel 1700(lba): ¹H-NMR(400,0 MHz, CDCl₃): δ = 8,005(1,8);7,291(3,2);7,285(3,4);7,274(3,2);7,269(3,7);7,262(60,2);7,230(6,1);7,224(6,4);7,213(6,3);7,207(6,3);7, 123(1,7);7,116(5,3);7,102(6,4);7,094(14,3);7,086(3,5);7,081(7,6);7,073(11,9);7,069(3,7);7,065(1,2);7,059(1,0);7, 053(0,8);7,044(4,5);7,038(4,3);7,032(4,8);7,027(4,4);7,022(2,8);7,016(2,5);7,011(2,4);7,005(2,2);6,998(0,5);6,37 8(3,1);6,377(3,1);6,373(5,8);6,372(5,8);6,369(6,4);6,364(10,8);6,355(6,5);6,282(10,0);6,278(10,1);6,272(8,2);6,2 68(8,1);6,261(5,1);6,256(5,1);6,251(4,3);6,247(4,2);4,138(9,2);4,124(9,5);3,950(5,1);3,931(5,5);3,819(2,4);3,806( 5,3);3,792(5,1);3,779(7,2);3,770(1,8);3,766(3,6);3,761(1,7);3,756(1,6);3,744(2,1);3,734(1,9);3,729(1,9);3,719(1,7 );3,653(3,3);3,641(3,7);3,632(3,8);3,626(2,9);3,620(3,8);3,614(2,9);3,605(2,8);3,593(2,6);3,562(1,7);3,550(1,9);3, 538(2,1);3,535(1,7);3,526(2,1);3,523(1,8);3,511(1,6);3,499(1,4);3,485(6,8);3,458(0,7);3,450(1,0);3,441(1,0);3,43 3(2,1);3,429(2,2);3,420(3,2);3,413(3,5);3,404(3,2);3,396(3,3);3,390(2,6);3,381(1,3);3,375(1,2);2,958(16,0);2,880( 13,4);2,879(12,9);2,281(0,7);2,272(0,7);2,267(0,8);2,258(1,5);2,247(1,2);2,243(0,9);2,237(1,0);2,232(1,2);2,223( 1,8);2,214(0,9);2,208(0,9);2,199(0,8);2,146(0,8);2,133(1,7);2,125(0,9);2,120(1,1);2,111(3,5);2,098(4,8);2,090(2,8 );2,085(2,4);2,077(4,2);2,071(2,5);2,063(2,5);2,059(4,6);2,047(4,3);2,035(4,6);2,023(3,4);2,012(1,7);2,000(1,7);1, 988(0,8);1,952(0,9);1,941(1,4);1,930(0,9);1,923(0,9);1,917(1,0);1,913(1,5);1,906(1,3);1,902(1,0);1,896(0,8);1,88 8(0,8);1,878(1,1);1,867(0,7);1,489(10,1);0,008(0,7);0,000(22,5);-0,009(0,7) |

Die in der nachfolgenden Tabelle Rtz für das jeweilige Enantiomer der Formel (Ia) angegebene Retentionszeit (Rtz) ist in Minuten (min) angegeben, wobei der hinter der Retentionszeit nach dem Schrägstrich angegebene Buchstabe a, b, c bzw. d sich auf die oben angegebenen Bedingungen für die HPLC an chiraler Säule und das betreffende oben angegebene Elutionsmittel bezieht, also Eluent a, Eluent b, Eluent c bzw. Eluent d.

**Tabelle Rtz: Rtz in min.**

| Nr. | Q(R²)ₙ | (R³)ₘ | Erythro-1 | Erythro- 2 | Threo-1 | Threo-2 |
|---|---|---|---|---|---|---|
| 289a | 3,4-F₂ | 5-Fluorpyridin-3-yl | 7,070/c | 7,385/c | 11,894/c | 14,023/c |

### Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis ca. 277 °C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man

| | | |
|---|---|---|
| 75 | Gewichtsteile | einer Verbindung der Formel (I), |
| 10 | Gewichtsteile | ligninsulfonsaures Calcium, |
| 5 | Gewichtsteile | Natriumlaurylsulfat, |
| 3 | Gewichtsteile | Polyvinylalkohol, und |
| 7 | Gewichtsteile | Kaolin |

mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man

| | | |
|---|---|---|
| 25 | Gewichtsteile | einer Verbindung der Formel (I), |
| 5 | Gewichtsteile | 2,2'-dinaphthylmethan-6,6'-disulfonsaures Natrium |
| 2 | Gewichtsteile | oleoylmethyltaurinsaures Natrium, |
| 1 | Gewichtsteil | Polyvinylalkohol, |
| 17 | Gewichtsteile | Calciumcarbonat, und |
| 50 | Gewichtsteile | Wasser |

auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### Biologische Beispiele

### Beispiel PE - Herbizide Wirkung im Vorauflauf

Samen von mono- bzw. dikotylen Unkraut- bzw. Kulturpflanzen wurden in Holzfasertöpfen in sandiger Lehmerde ausgelegt und mit Erde abgedeckt. Die in Form von benetzbaren Pulvern (WP) formulierten erfindungsgemäßen Verbindungen (I) wurden dann als wäßrige Suspension bzw. Emulsion mit einer Wasseraufwandmenge von umgerechnet 600 l/ha unter Zusatz von 0,2% Netzmittel auf die Oberfläche der Abdeckerde appliziert.

Nach der Behandlung wurden die Töpfe im Gewächshaus aufgestellt und unter guten Wachstumsbedingungen für die Testpflanzen gehalten. Nach ca. 3 Wochen wurde die Wirkung visuell im Vergleich zu unbehandelten Kontrollen in Prozentwerten bonitiert. Beispielsweise bedeutet 100% Wirkung = Pflanzen sind abgestorben, 50% herbizide Wirkung oder Schaden = Pflanzen zu 50% reduziert bzw. Pflanzenmasse um 50% reduziert, 0 % Wirkung = wie Kontrollpflanzen.

Die gefundenen Ergebnisse zeigen, dass erfindungsgemäße Verbindungen (I) und deren Salze, insbesondere die als bevorzugt gekennzeichneten erfindungsgemäßen Verbindungen, eine gute bis sehr gute herbizide Wirksamkeit im Vorauflauf gegen Schadpflanzen bei einer Aufwandmenge von 320 g oder weniger pro Hektar aufweisen, regelmäßig auch noch bei einer jeweiligen Aufwandmenge von 80 g/ha.

Die biologischen Tests wurden jeweils separat mit den folgenden erfindungsgemäßen Verbindungen durchgeführt, die auf Grund ihrer guten bis sehr guten herbiziden Wirksamkeit erfindungsgemäß besonders bevorzugt sind:
Verbindung Nr. 532a threo, 532a erythro, 1288a, 1272a, 1295a, 208 threo, 208a, 208a threo, 215a, 1477a, 1461a, 1484a, 1542a, 1558a, 1565a, 1677a, 1531a, 1515a, 1538a, 181a, 647a, 1153a, 1137a, 1516a, 289a, 289a threo-1, 289 erythro-1, 100a, 107a, 296a, 613a, 620a, 86a, 597a, 512a, 599a und 599bae.

Diese erfindungsgemäßen Verbindungen wurden dabei jeweils als Bestandteil eines Spritzpulvers (WP-Formulierung) in den biologischen Tests eingesetzt.

Alle genannten erfindungsgemäßen Verbindungen zeigten bei einer Aufwandmenge von 320 g/ha in den biologischen Tests eine 80%ige bis 100%ige herbizide Wirkung gegen eine, mehrere oder sämtliche der folgenden Schadpflanzen:
ALOMY = Alopecurus myosuroides
AVEFA = Avena fatua
CYPES = Cyperus esculentus
ECHCG = Echinochloa crus-galli
LOLMU = Lolium multiflorum
SETVI = Setaria viridis
ABUTH = Abutilon theophrasti
AMARE = Amaranthus retroflexus
POLCO = Polygonum convolvulus (= Fallopia convolvulus)
STEME = Stellaria media
VIOTR = Viola tricolor
VERPE = Veronica persica

Bestimmt wurde die jeweilige herbizide Wirkung zum jeweils gleichen Zeitpunkt nach Applikation der jeweiligen Formulierung, d.h. die Schädigung der jeweiligen Schadpflanze in %.

Die vorstehend genannten erfindungsgemäßen Verbindungen wurden ferner jeweils in den genannten Aufwandmengen auf folgende Nutzpflanzen angewendet:
ORYSA = Oryza sativa (gemeiner Reis)
TRZAS = Triticum aestivum (spring) (Sommerweizen)
ZEAMX = Zea mays (Mais)
BRSNW = Brassica napus subsp. napus (winter) (Winterraps)

Die beobachtete Schädigung dieser Nutzpflanzen lag dabei jeweils im akzeptablen Bereich und wurde als allgemein niedrig eingestuft (regelmäßig im Bereich von 0 bis 20%, zumeist im Bereich von 0 bis 10%).

### Beispiel PO - Herbizide Wirkung im Nachauflauf

Samen von mono- bzw. dikotylen Unkraut- bzw. Kulturpflanzen wurden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat wurden die Versuchspflanzen im Einblattstadium behandelt, wobei die in Form von benetzbaren Pulvern (WP) formulierten erfindungsgemäßen Verbindungen als wäßrige Suspension bzw. Emulsion mit einer Wasseraufwandmenge von umgerechnet 600 l/ha unter Zusatz von 0,2% Netzmittel auf die grünen Pflanzenteile gesprüht wurden. Nach ca. 3 Wochen Standzeit der Versuchspflanzen im Gewächshaus unter optimalen Wachstumsbedingungen wurde die Wirkung der Präparate visuell im Vergleich zu unbehandelten Kontrollen in Prozentwerten bonitiert. Beispielsweise bedeutet 100% Wirkung = Pflanzen sind abgestorben, 50% herbizide Wirkung oder Schaden = Pflanzen zu 50% reduziert bzw. Pflanzenmasse um 50% reduziert, 0 % Wirkung = wie Kontrollpflanzen.

Die biologischen Tests wurden jeweils separat mit den folgenden erfindungsgemäßen Verbindungen durchgeführt, die auf Grund ihrer guten bis sehr guten herbiziden Wirksamkeit erfindungsgemäß besonders bevorzugt sind:
Verbindung Nr. 532a threo, 1288a, 1542a, 1558a, 1700, 1700a, 1531a, 151a, 1538a, 181a, 647a, 1516a, 289a, 289 erythro-1, 289 threo-2, 100a, 107a, 296a, 613a, 620a, 86a, 84a, 295a, 599a, 599bae, 619, 619lbab, 619a und 619bab.

Die gefundenen Ergebnisse zeigen, dass erfindungsgemäße Verbindungen (I) und deren Salze, insbesondere die als bevorzugt gekennzeichneten erfindungsgemäßen Verbindungen, eine gute bis sehr gute herbizide Wirksamkeit gegen Schadpflanzen im Nachauflauf bei einer Aufwandmenge von 320 g oder weniger pro Hektar aufweisen, regelmäßig auch noch bei einer jeweiligen Aufwandmenge von 80 g/ha.

Alle genannten erfindungsgemäßen Verbindungen zeigten bei einer Aufwandmenge von 320 g/ha in den biologischen Tests eine 80%ige bis 100%ige herbizide Wirkung gegen eine, mehrere oder sämtliche der in obigem biologischen Beispiel PE genannten Schadpflanzen. Die beobachtete Schädigung der Nutzpflanzen ORYSA, TRZAS, ZEAMX und BRSNW lag dabei jeweils wie im obigen biologischen Beispiel PE ebenfalls im akzeptablen Bereich.

## Patentansprüche

1. Verbindung der Formel (I) und/oder deren Salz 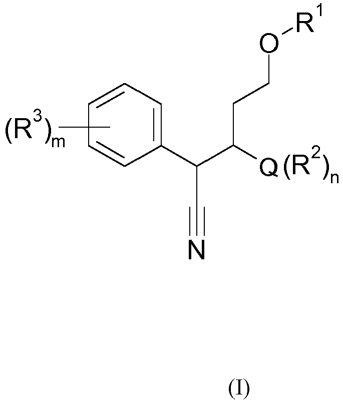 worin
Q einen heteroaromatischen Rest bedeutet, ausgewählt aus der Gruppe bestehend aus Pyridinyl, Pyrazinyl, Thienyl und Thiazolyl,
R¹ Wasserstoff oder eine Gruppe bestehend aus:
Acetyl, Propanoyl, Butanoyl, Pentanoyl, Hexanoyl, 2-Methylpropanoyl (= Isopropanoyl), 2,2-Difluoracetyl, 2,2,2-Trifluoracetyl, C(=O)OMe, Cyclopropancarbonyl, 1-Methylcyclopropancarbonyl, Acryl, Prop-2-inoyl, But-2-inoyl, 2-Methylacryl, Benzoyl, 4-Chlorbenzoyl, 3-Chlorbenzoyl, 2-Chlorbenzoyl, 4-Fluorbenzoyl, 3-Fluorbenzoyl, 2-Fluorbenzoyl, 2,2-Dimethylpropanoyl (= Pivaloyl), 3,3-Dimethylbutanoyl, 2-Nitrobenzoyl, 2-Fluoracetyl, 2-Chloracetyl, 2-Bromacetyl, 2,2-Dichloracetyl, 2-Methoxyacetyl, 2,6-Difluorbenzoyl, C(O)C(=O)OMe, und C(O)CH₂C(O)OMe bedeutet,
(R²)ₙ n Substituenten R² bedeuten, wobei R² (wenn n = 1) bzw. jeder der Substituenten R² (wenn n größer als 1 ist) unabhängig voneinander Halogen, Cyano, Nitro, (C₁-C₈)Alkyl, (C₁-C₈)Alkoxy, (C₁-C₈)Alkylthio, (C₁-C₈)Alkylsulfinyl, (C₁-C₈)Alkylsulfonyl, (C₁-C₆)Haloalkyl, (C₂-C₆)Haloalkenyl, (C₁-C₆)Haloalkoxy, (C₁-C₆)Haloalkylthio, (C₁-C₆)Haloalkylsulfinyl, (C₁-C₆)Haloalkylsulfonyl, (C₂-C₆)Alkenyl, (C₂-C₆)Alkinyl, Tri-[(C₁-C₄)alkyl]-silyl oder Tri-[(C₁-C₄)alkyl]-silyl-(C₁-C₄)alkyl bedeutet,
oder jeweils zwei am heteroaromatischen Rest Q unmittelbar benachbarte R² gemeinsam eine Gruppe der Formel -Z¹-A*¹-Z²- bedeuten, in welcher
A* für eine Alkylengruppe mit 1 bis 4 C-Atomen steht, die gegebenenfalls durch einen oder mehrere Reste ausgewählt aus der Gruppe bestehend aus Halogen, (C₁-C₄)Alkyl, (C₁-C₄)Haloalkyl, (C₁-C₄)Alkoxy und (C₁-C₄)Haloalkoxy substituiert ist,
Z¹ für eine direkte Bindung, O oder S steht und
Z² für eine direkte Bindung, O oder S steht,
wobei die Gruppe -Z¹-A*-Z²- zusammen mit den an diese Gruppe gebundenen Atomen des heteroaromatischen Restes Q einen 5 oder 6 Ring bilden, und
(R³)ₘ m Substituenten R³ bedeutet,
wobei R³ (wenn m = 1) bzw. jeder der Substituenten R³ (wenn m größer als 1) unabhängig voneinander Halogen, Cyano, Nitro, (C₁-C₈)Alkyl, (C₁-C₈)Alkoxy, (C₁-C₈)Alkylthio, (C₁-C₈)Alkylsulfinyl, (C₁-C₈)Alkylsulfonyl, (C₁-C₆)Haloalkyl, (C₂-C₆)Haloalkenyl, (C₁-C₆)Haloalkoxy, (C₁-C₆)Haloalkylthio, (C₁-C₆)Haloalkylsulfinyl, (C₁-C₆)Haloalkylsulfonyl, (C₂-C₆)Alkenyl, (C₂-C₆)Alkinyl, Tri-[(C₁-C₄)alkyl]-silyl, Tri-[(C₁-C₄)alkyl]-silyl-(C₁-C₄)alkyl oder -NR*R** bedeutet, wobei R* und R** unabhängig voneinander und unabhängig von gegebenenfalls vorhandenen weiteren Resten -NR*R** jeweils ausgewählt sind aus der Gruppe bestehend aus H, (C₁-C₈)Alkyl, (C₂-C₈)Alkenyl, (C₂-C₈)Alkinyl, (C₁-C₄)Alkoxy-(C₁-C₄)alkyl, (C₁-C₆)Alkanoyl, [(C₁-C₄)Haloalkyl]-carbonyl, [(C₁-C₄)Alkoxy]-carbonyl, [(C₁-C₄)Haloalkoxy]-carbonyl, (C₃-C₆)Cycloalkyl, (C₃-C₆)Cycloalkyl-(C₁-C₄)alkyl, (C₃-C₆)Cycloalkenyl, (C₃-C₆)Cycloalkenyl-(C₁-C₄)alkyl, Phenyl und Phenyl-(C₁-C₄)alkyl, wobei jeder der genannten Reste (C₃-C₆)Cycloalkyl, (C₃-C₆)Cycloalkyl-(C₁-C₄)alkyl, (C₃-C₆)Cycloalkenyl, (C₃-C₆)Cycloalkenyl-(C₁-C₄)alkyl, Phenyl und Phenyl-(C₁-C₄)alkyl im Cyclus gegebenenfalls durch einen oder mehrere gleiche oder verschiedene Reste R^{bb} substituiert ist, wobei
R^{bb} jeweils Halogen, (C₁-C₄)Alkyl, (C₁-C₄)Haloalkyl, (C₁-C₄)Alkoxy oder (C₁-C₄)Haloalkoxy bedeutet, und im Falle von (C₃-C₆)Cycloalkyl, (C₃-C₆)Cycloalkyl-(C₁-C₄)alkyl, (C₃-C₆)Cycloalkenyl, (C₃-C₆)Cycloalkenyl-(C₁-C₄)alkyl R^{bb} zusätzlich Oxo bedeuten kann, oder
NR*R** einen 3- bis 8-gliedrigen Heterocyclus bedeutet, welcher gegebenenfalls zusätzlich zu diesem N-Atom ein oder zwei weitere Heteroringatome ausgewählt aus der Gruppe bestehend aus N, O und S umfasst, und welcher unsubstituiert oder durch einen oder mehrere Reste ausgewählt aus der Gruppe bestehend aus (C₁-C₄)Alkyl, (C₁-C₄)Haloalkyl und Oxo substituiert ist,
oder wobei jeweils zwei am Ring unmittelbar benachbarte Gruppen R³ gemeinsam eine Gruppe der Formel -Z³-A**-Z⁴- bedeuten, in welcher
A** für eine Alkylengruppe mit 1 bis 4 C-Atomen steht, die gegebenenfalls durch einen oder mehrere Reste ausgewählt aus der Gruppe bestehend aus Halogen, (C₁-C₄)Alkyl, (C₁-C₄)Haloalkyl, (C₁-C₄)Alkoxy und (C₁-C₄)Haloalkoxy substituiert ist,
Z³ für eine direkte Bindung, O oder S steht und
Z⁴ für eine direkte Bindung, O oder S steht,
wobei die Gruppe -Z³-A**-Z⁴- zusammen mit den an diese Gruppe gebundenen C-Atomen des Phenylrings einen 5 oder 6 Ring bilden,
n 0, oder eine ganze Zahl im Bereich von 1 bis 5, vorzugsweise 0, 1, 2 oder 3 bedeutet, und
m 0, 1, 2, 3, 4 oder 5, vorzugsweise 0, 1, 2, oder 3 bedeutet.

2. Verbindung der Formel (I) und/oder deren Salz gemäß Anspruch 1, **dadurch gekennzeichnet, dass** die Verbindung der Formel (I) der nachfolgend definierten Formel (I-1), (I-2) oder (I-3) entspricht 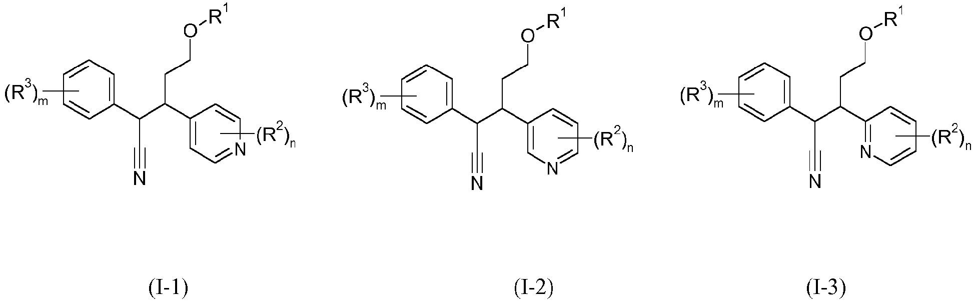 wobei
R¹, R², R³, m und n die in Anspruch 1 definierte Bedeutung haben.

3. Verbindung der Formel (I) und/oder deren Salz gemäß einem der Ansprüche 1 bis 2, **dadurch gekennzeichnet, dass**
(R²)ₙ n Substituenten R² bedeutet,
wobei R² (wenn n = 1) oder jeder derSubstituenten R² (wenn n größer als 1) unabhängig voneinander Halogen, Cyano, Nitro, (C₁-C₆)Alkyl, (C₁-C₆)Alkoxy, (C₁-C₆)Alkylthio, (C₁-C₆)Alkylsulfinyl, (C₁-C₆)Alkylsulfonyl, (C₁-C₆)Haloalkyl, (C₂-C₆)Haloalkenyl, (C₁-C₆)Haloalkoxy, (C₁-C₆)Haloalkylthio, (C₁-C₆)Haloalkylsulfinyl, (C₁-C₆)Haloalkylsulfonyl, (C₂-C₆)Alkenyl, (C₂-C₆)Alkinyl, Tri-[(C₁-C₄)alkyl]-silyl oder Tri-[(C₁-C₄)alkyl]-silyl-(C₁-C₄)alkyl bedeutet,
und/oder
(R³)ₘ m Substituenten R³ bedeutet,
wobei R³ (wenn m = 1) oder jeder der Substituenten R³ (wenn m größer als 1) unabhängig voneinander Halogen, Cyano, Nitro, (C₁-C₆)Alkyl, (C₁-C₆)Alkoxy, (C₁-C₆)Alkylthio, (C₁-C₆)Alkylsulfinyl, (C₁-C₆)Alkylsulfonyl, (C₁-C₆)Haloalkyl, (C₂-C₆)Haloalkenyl, (C₁-C₆)Haloalkoxy, (C₁-C₆)Haloalkylthio, (C₁-C₆)Haloalkylsulfinyl, (C₁-C₆)Haloalkylsulfonyl, (C₂-C₆)Alkenyl, (C₂-C₆)Alkinyl, NR*R**, Tri-[(C₁-C₄)alkyl]-silyl oder Tri-[(C₁-C₄)alkyl]-silyl-(C₁-C₄)alkyl bedeutet
oder wobei jeweils zwei am Ring unmittelbar benachbarte Gruppen R³ gemeinsam eine Gruppe der Formel -Z³-A**-Z⁴- bedeuten, in welcher
A** für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z³ für eine direkte Bindung, O oder S steht und
Z⁴ für eine direkte Bindung, O oder S steht,
wobei die Gruppe -Z³-A**-Z⁴- zusammen mit den an die Gruppe gebundenen C-Atomen des Phenylrings einen ankondensierten 5 oder 6 Ring bilden,
R*, R** jeweils unabhängig voneinander oder gemeinsam mit dem N-Atom die oben genannte Bedeutung haben und
n, m jeweils unabhängig voneinander 0, 1, 2, 3, 4 oder 5, vorzugsweise 0, 1, 2 oder 3, insbesondere 0, 1 oder 2 bedeuten.

4. Verbindung der Formel (I) und/oder deren Salz gemäß einem der Ansprüche 1 bis 3, **dadurch gekennzeichnet, dass**
(R²)ₙ n Substituenten R² bedeutet, wobei, im Falle dass n = 1 ist, der Substituent R² oder, im Falle dass n größer als 1 ist, jeder der Substituenten R² unabhängig voneinander
Halogen, Cyano, Nitro, (C₁-C₄)Alkyl, (C₁-C₄)Alkoxy, (C₁-C₄)Alkylthio, (C₁-C₄)Alkylsulfinyl, (C₁-C₄)Alkylsulfonyl, (C₁-C₄)Haloalkyl, (C₂-C₄)Haloalkenyl, (C₁-C₄)Haloalkoxy, (C₁-C₄)Haloalkylthio, (C₁-C₄)Haloalkylsulfinyl, (C₁-C₄)Haloalkylsulfonyl, (C₂-C₄)Alkenyl, (C₂-C₄)Alkinyl, Tri-[(C₁-C₄)Alkyl]-silyl oder Tri-[(C₁-C₄)Alkyl]-silyl -(C₁-C₄)Alkyl bedeutet, und
n 0, 1, 2, 3, oder 4, vorzugsweise 0, 1, 2 oder 3, insbesondere 0, 1 oder 2 bedeutet.

5. Verbindung der Formel (I) und/oder deren Salz gemäß einem der Ansprüche 1 bis 4, **dadurch gekennzeichnet, dass**
(R³)ₘ m Substituenten R³ bedeutet,
wobei, im Falle dass m = 1 ist, der Substituent R³ oder, im Falle dass m größer als 1 ist, jeder der Substituenten R³ unabhängig voneinander Halogen, Cyano, Nitro, (C₁-C₄)Alkyl, (C₁-C₄)Alkoxy, (C₁-C₄)Alkylthio, (C₁-C₄)Alkylsulfinyl, (C₁-C₄)Alkylsulfonyl, (C₁-C₄)Haloalkyl, (C₂-C₄)Haloalkenyl, (C₁-C₄)Haloalkoxy, (C₁-C₄)Haloalkylthio, (C₁-C₄)Haloalkylsulfinyl, (C₁-C₄)Haloalkylsulfonyl, (C₂-C₆)Alkenyl, (C₂-C₆)Alkinyl oder Tri-[(C₁-C₄)Alkyl]silyl-Z^{b}-, wobei Z^{b} = für eine kovalente Bindung oder (C₁-C₄)Alkylen steht, bedeutet oder
jeweils zwei am Ring unmittelbar benachbarte Gruppen R³ gemeinsam eine Gruppe der Formel -Z³-A**-Z⁴- bedeuten,
wobei
A** für eine Alkylengruppe steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z³ für O oder S steht und
Z⁴ für O oder S steht,
wobei die Gruppe -Z³-A**-Z⁴- zusammen mit den an die Gruppe gebundenen C-Atomen des Phenylrings einen ankondensierten 5 oder 6 Ring bilden,
m 0, 1, 2, 3, 4 oder 5, vorzugsweise 0, 1, 2, 3 oder 4, insbesondere 0, 1, 2 oder 3 bedeutet.

6. Verbindung der Formel (I) und/oder deren Salz gemäß einem der Ansprüche 1 bis 5, **dadurch gekennzeichnet, dass**
(R²)ₙ n Substituenten R² bedeutet,
wobei, im Falle dass n = 1 ist, der Substituent R² oder, im Falle dass n größer als 1 ist, jeder der Substituenten R² unabhängig voneinander Fluor, Chlor, Brom, Iod, Cyano, Nitro, Methyl, Ethyl, Methoxy, Methylthio, Methylsulfinyl, Methylsulfonyl, Trifluormethyl, Trifluormethoxy, Trifluoralkylthio, Trifluormethylsulfinyl oder Trifluormethylsulfonyl bedeutet, und
(R³)ₘ m Substituenten R³ bedeutet,
wobei, im Falle dass m = 1 ist, der Substituent Rest R³ oder, im Falle dass m größer als 1 ist, jeder der Substituenten R³ unabhängig voneinander Fluor, Chlor, Brom, Iod, Cyano, Nitro, Methyl, Methoxy, Methylthio, Methylsulfinyl, Methylsulfonyl, Trifluormethyl, Trifluormethoxy, Trifluoralkylthio, Trifluormethylsulfinyl oder Trifluormethylsulfonyl bedeutet, und
m 0, 1, 2, 3 oder 4, bevorzugt 0, 1, 2 oder 3 bedeutet, und
n 0, 1, 2 oder 3, bevorzugt 0, 1 oder 2 bedeutet.

7. Verwendung einer Verbindung der Formel (I) und/oder deren Salz wie in einem der Ansprüche 1 bis 6 definiert,
als Herbizid und/oder Pflanzenwachstumsregulator, vorzugsweise in Kulturen von Nutz- und/oder Zierpflanzen.

8. Herbizides oder pflanzenwachstumsregulierendes Mittel, **dadurch gekennzeichnet, dass** das Mittel eine oder mehrere Verbindungen der Formel (I) und/oder deren Salze enthält wie in einem der Ansprüche 1 bis 6 definiert,
und ein oder mehrere weitere Stoffe ausgewählt aus den Gruppen (i) und/oder (ii):
(i) ein oder mehrere weitere agrochemisch wirksame Stoffe, vorzugsweise ausgewählt aus der Gruppe bestehend aus Insektiziden, Akariziden, Nematiziden, weiteren Herbiziden, Fungiziden, Safenern, Düngemitteln und/oder weiteren Wachstumsregulatoren,
(ii) ein oder mehrere im Pflanzenschutz übliche Formulierungshilfsmittel.

9. Verfahren zur Bekämpfung von Schadpflanzen oder zur Wachstumsregulierung von Pflanzen, **dadurch gekennzeichnet, dass** eine wirksame Menge
- einer oder mehrerer Verbindungen der Formel (I) und/oder deren Salzen, wie in einem der Ansprüche 1 bis 6 definiert,
- eines Mittels nach Anspruch 8,
auf die Pflanzen, Pflanzensamen, den Boden, in dem oder auf dem die Pflanzen wachsen, oder die Anbaufläche appliziert wird.

10. Verfahren zur Herstellung einer Verbindung der Formel (I), wie sie gemäß einem der Ansprüche 1 bis 6 definiert ist, oder deren Salz, **dadurch gekennzeichnet, dass** eine Verbindung der Formel (E) 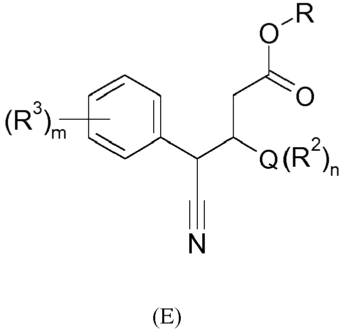 mittels Reduktion zu einer Verbindung der Formel (Ia) 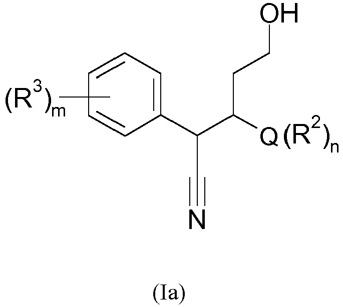 umgesetzt wird, und Verbindung (Ia) gegebenenfalls weiter zu einer Verbindung der Formel (I) umgesetzt wird, sofern R¹ in Formel (I) nicht Wasserstoff ist, wobei
R Wasserstoff oder ein organischer Rest ist, vorzugsweise ein Rest ist ausgewählt aus der Gruppe der für R¹ definierten Reste gemäß einem der Ansprüche 1 bis 6,
und
Q, R², R³, m und n jeweils die in einem der Ansprüche 1 bis 6 definierte Bedeutung haben.

## Claims

1. Compound of the formula (I) and/or salt thereof, 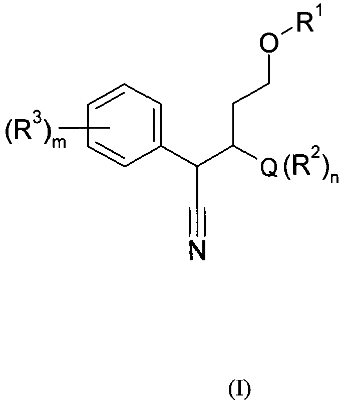 in which
Q represents a heteroaromatic radical selected from the group consisting of pyridinyl, pyrazinyl, thienyl and thiazolyl,
R¹ represents hydrogen or a group consisting of:
acetyl, propanoyl, butanoyl, pentanoyl, hexanoyl, 2-methylpropanoyl (= isopropanoyl), 2,2-difluoroacetyl, 2,2,2-trifluoroacetyl, C(=O)OMe, cyclopropanecarbonyl, 1-methylcyclopropanecarbonyl, acryl, prop-2-ynoyl, but-2-ynoyl, 2-methylacryl, benzoyl, 4-chlorobenzoyl, 3-chlorobenzoyl, 2-chlorobenzoyl, 4-fluorobenzoyl, 3-fluorobenzoyl, 2-fluorobenzoyl, 2,2-dimethylpropanoyl (= pivaloyl), 3,3-dimethylbutanoyl, 2-nitrobenzoyl, 2-fluoroacetyl, 2-chloroacetyl, 2-bromoacetyl, 2,2-dichloroacetyl, 2-methoxyacetyl, 2,6-difluorobenzoyl, C(O)C(=O)OMe and C(O)CH₂C(O)OMe,
(R²)ₙ represent n substituents R², where R² (if n = 1) or each of the substituents R² (if n is greater than 1) independently of one another represents halogen, cyano, nitro, (C₁-C₈)alkyl, (C₁-C₈)alkoxy, (C₁-C₈)alkylthio, (C₁-C₈)alkylsulfinyl, (C₁-C₈) alkylsulfonyl, (C₁-C₆)haloalkyl, (C₂-C₆) haloalkenyl, (C₁-C₆)haloalkoxy, (C₁-C₆)haloalkylthio, (C₁-C₆)haloalkylsulfinyl, (C₁-C₆)haloalkylsulfonyl, (C₂-C₆) alkenyl, (C₂-C₆)alkynyl, tri-[(C₁-C₄)alkyl] silyl or tri-[(C₁-C₄)alkyl] silyl- (C₁-C₄)alkyl,
or in each case two R² directly adjacent on the heteroaromatic radical Q are together a group of the formula -Z¹-A*¹-Z²-, in which
A* represents an alkylene group having 1 to 4 carbon atoms which is optionally substituted by one or more radicals selected from the group consisting of halogen, (C₁-C₄)alkyl, (C₁-C₄)haloalkyl, (C₁-C₉)alkoxy and (C₁-C₄)haloalkoxy,
Z¹ represents a direct bond, O or S and
Z² represents a direct bond, O or S,
where the group -Z¹-A*-Z²- together with the atoms of the heteroaromatic radical Q bonded to this group Q form a 5- or 6-membered ring, and
(R³)ₘ represents m substituents R³,
where R³ (if m = 1) or each of the substituents R³ (if m is greater than 1) independently of one another represents halogen, cyano, nitro, (C₁-C₈)alkyl, (C₁-C₈)alkoxy, (C₁-C₈)alkylthio, (C₁-C₈)alkylsulfinyl, (C₁-C₈)alkylsulfonyl, (C₁-C₆)haloalkyl, (C₂-C₆) haloalkenyl, (C₁-C₆)haloalkoxy, (C₁-C₆)haloalkylthio, (C₁-C₆)haloalkylsulfinyl, (C₁-C₆)haloalkylsulfonyl, (C₂-C₆) alkenyl, (C₂-C₆)alkynyl, tri-[(C₁-C₄)alkyl] silyl, tri-[(C₁-C₄)alkyl]silyl-(C₁-C₄)alkyl or -NR*R**, where R* and R**, independently of one another and independently of any other radicals -NR*R** present, are in each case selected from the group consisting of H, (C₁-C₈) alkyl, (C₂-C₈) alkenyl, (C₂-C₈)alkynyl, (C₁-C₄)alkoxy- (C₁-C₄)alkyl, (C₁-C₆)alkanoyl, [(C₁-C₄)haloalkyl] carbonyl, [(C₁-C₄)alkoxy] carbonyl, [(C₁-C₄)haloalkoxy] carbonyl, (C₃-C₆)cycloalkyl, (C₃-C₆)cycloalkyl- (C₁-C₄)alkyl, (C₃-C₆)cycloalkenyl, (C₃-C₆)cycloalkenyl- (C₁-C₄)alkyl, phenyl and phenyl- (C₁-C₄)alkyl, where each of the specified radicals (C₃-C₆)cycloalkyl, (C₃-C₆)cycloalkyl- (C₁-C₄)alkyl, (C₃-C₆)cycloalkenyl, (C₃-C₆)cycloalkenyl-(C₁-C₄)alkyl, phenyl and phenyl-(C₁-C₄)alkyl is substituted in the cycle optionally by one or more identical or different radicals R^{bb}, where
R^{bb} in each case represents halogen, (C₁-C₄)alkyl, (C₁-C₄)haloalkyl, (C₁-C₄)alkoxy or (C₁-C₄)haloalkoxy and, in the case of (C₃-C₆)cycloalkyl, (C₃-C₆)cycloalkyl- (C₁-C₄)alkyl, (C₃-C₆)cycloalkenyl, (C₃-C₆)cycloalkenyl- (C₁-C₄)alkyl, R^{bb} may additionally represent oxo, or
NR*R** represents a 3- to 8-membered heterocycle which, in addition to this nitrogen atom, optionally contains one or two further ring heteroatoms selected from the group consisting of N, O and S and which is unsubstituted or substituted by one or more radicals selected from the group consisting of (C₁-C₄)alkyl, (C₁-C₄)haloalkyl and oxo,
or where in each case two groups R³ directly adjacent on the ring together represent a group of the formula -Z³-A**-Z⁴-, in which
A** represents an alkylene group having 1 to 4 carbon atoms which is optionally substituted by one or more radicals selected from the group consisting of halogen, (C₁-C₄)alkyl, (C₁-C₄)haloalkyl, (C₁-C₄)alkoxy and (C₁-C₄)haloalkoxy,
Z³ represents a direct bond, O or S and
Z⁴ represents a direct bond, O or S,
where the group -Z³-A**-Z⁴ together with the carbon atoms, bonded to this group, of the phenyl ring form a 5- or 6-membered ring,
n represents 0, or an integer in the range from 1 to 5, preferably 0, 1, 2 or 3, and
m represents 0, 1, 2, 3, 4 or 5, preferably 0, 1, 2 or 3.

2. Compound of the formula (I) and/or salt thereof according to Claim 1, **characterized in that** the compound of the formula (I) corresponds to the formula (I-1), (I-2) or (I-3) defined below 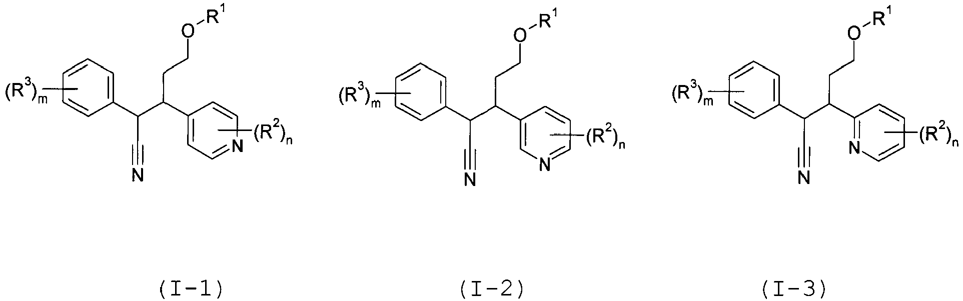 where
R¹, R², R³, m and n have the meaning defined in Claim 1.

3. Compound of the formula (I) and/or salt thereof according to one of Claims 1 to 2, **characterized in that**
(R²)ₙ represents n substituents R²,
where R² (if n = 1) or each of the substituents R² (if n is greater than 1) independently of the others represents halogen, cyano, nitro, (C₁-C₆)alkyl, (C₁-C₆)alkoxy, (C₁-C₆)alkylthio, (C₁-C₆)alkylsulfinyl, (C₁-C₆)alkylsulfonyl, (C₁-C₆)haloalkyl, (C₂-C₆) haloalkenyl, (C₁-C₆)haloalkoxy, (C₁-C₆)haloalkylthio, (C₁-C₆)haloalkylsulfinyl, (C₁-(C₁-C₆)haloalkylthio, (C₁-C₆)haloalkylsulfinyl, (C₁-C₆)haloalkylsulfonyl, (C₂-C₆)alkenyl, (C₂-C₆)alkynyl, tri[(C₁-C₄)alkyl] silyl or tri[(C₁-C₄)alkyl]silyl-(C₁-C₄)alkyl,
and/or
(R³)ₘ represents m substituents R³,
where R³ (if m = 1) or each of the substituents R³ (if m is greater than 1) independently of one another represents halogen, cyano, nitro, (C₁-C₆)alkyl, (C₁-C₆)alkoxy, (C₁-C₆)alkylthio, (C₁-C₆)alkylsulfinyl, (C₁-C₆)alkylsulfonyl, (C₁-C₆)haloalkyl, (C₂-C₆) haloalkenyl, (C₁-C₆)haloalkoxy, (C₁-C₆)haloalkylthio, (C₁-C₆)haloalkylsulfinyl, (C₁-C₆)haloalkylsulfonyl, (C₂-C₆) alkenyl, (C₂-C₆)alkynyl, NR*R**, tri [(C₁-C₄)alkyl] silyl or tri[(C₁-C₄)alkyl]silyl(C₁-C₄)alkyl
or where in each case two groups R³ directly adjacent on the ring together represent a group of the formula -Z³-A**-Z⁴-, in which
A** represents an alkylene group having 1 to 4 carbon atoms which is optionally substituted by one or more radicals from the group consisting of halogen, (C₁-C₄)alkyl, (C₁-C₄)haloalkyl, (C₁-C₄)alkoxy and (C₁-C₄)haloalkoxy,
Z³ represents a direct bond, O or S and
Z⁴ represents a direct bond, O or S,
where the group -Z³-A**-Z⁴ together with the carbon atoms, bonded to the group, of the phenyl ring form a fused-on 5- or 6-membered ring,
R*, R** each independently of one another or together with the nitrogen atom have the meaning mentioned above and
n, m in each case independently of one another represent 0, 1, 2, 3, 4 or 5, preferably 0, 1, 2 or 3, in particular 0, 1 or 2.

4. Compound of the formula (I) and/or salt thereof according to one of Claims 1 to 3, **characterized in that**
(R²)ₙ represents n substituents R², where, in the case that n = 1, the substituent R² or, in the case that n is greater than 1, each of the substituents R² independently of the others represents
halogen, cyano, nitro, (C₁-C₄)alkyl, (C₁-C₄)alkoxy, (C₁-C₄)alkylthio, (C₁-C₄)alkylsulfinyl, (C₁-C₄)alkylsulfonyl, (C₁-C₄)haloalkyl, (C₂-C₄)haloalkenyl, (C₁-C₄)haloalkoxy, (C₁-C₄)haloalkylthio, (C₁-C₄)haloalkylsulfinyl, (C₁-C₄)haloalkylsulfonyl, (C₂-C₄)alkenyl, (C₂-C₄)alkynyl, tri [(C₁-C₄)alkyl] silyl or tri[(C₁-C₄)alkyl]silyl(C₁-C₄)alkyl, and
n represents 0, 1, 2, 3, or 4, preferably 0, 1, 2 or 3, in particular 0, 1 or 2.

5. Compound of the formula (I) and/or salt thereof according to one of Claims 1 to 4, **characterized in that**
(R³)ₘ represents m substituents R³,
where in the case that m = 1, the substituent R³ or, in the case that m is greater than 1, each of the substituents R³ independently of one another represents halogen, cyano, nitro, (C₁-C₄)alkyl, (C₁-C₄)alkoxy, (C₁-C₄)alkylthio, (C₁-C₄)alkylsulfinyl, (C₁-C₄)alkylsulfonyl, (C₁-C₄)haloalkyl, (C₂-C₄)haloalkenyl, (C₁-C₄)haloalkoxy, (C₁-C₄)haloalkylthio, (C₁-C₄)haloalkylsulfinyl, (C₁-C₄)haloalkylsulfonyl, (C₂-C₆) alkenyl, (C₂-C₆) alkynyl or tri[(C₁-C₄)alkyl]silyl-Z^{b}-, where Z^{b} = is a covalent bond or (C₁-C₄)alkylene, or
in each case two groups R³ directly adjacent to one another at the ring together represent a group of the formula -Z³-A**-Z⁴-,
where
A** represents an alkylene group which is optionally substituted by one or more radicals from the group halogen, (C₁-C₄)alkyl, (C₁-C₄)haloalkyl, (C₁-C₄)alkoxy and (C₁-C₄)haloalkoxy,
Z³ represents O or S and
Z⁴ represents O or S,
where the group -Z³-A**-Z⁴- together with the carbon atoms, attached to the group, of the phenyl ring form a fused-on 5- or 6-membered ring,
m represents 0, 1, 2, 3, 4 or 5, preferably 0, 1, 2, 3 or 4, in particular 0, 1, 2 or 3.

6. Compound of the formula (I) and/or salt thereof according to one of Claims 1 to 5, **characterized in that**
(R²)ₙ represents n substituents R²,
where, in the case that n = 1, the substituent R² or, in the case that n is greater than 1, each of the substituents R² independently of one another represents fluorine, chlorine, bromine, iodine, cyano, nitro, methyl, ethyl, methoxy, methylthio, methylsulfinyl, methylsulfonyl, trifluoromethyl, trifluoromethoxy, trifluoroalkylthio, trifluoromethylsulfinyl or trifluoromethylsulfonyl, and
(R³)ₘ represents m substituents R³ ,
where, in the case that m = 1, the substituent R³ or, in the case that m is greater than 1, each of the substituents R³ independently of the others represents fluorine, chlorine, bromine, iodine, cyano, nitro, methyl, methoxy, methylthio, methylsulfinyl, methylsulfonyl, trifluoromethyl, trifluoromethoxy, trifluoroalkylthio, trifluoromethylsulfinyl or trifluoromethylsulfonyl, and
m represents 0, 1, 2, 3 or 4, preferably 0, 1, 2 or 3, and
n represents 0, 1, 2 or 3, preferably 0, 1 or 2.

7. Use of a compound of the formula (I) and/or salt thereof as defined in any of Claims 1 to 6,
as herbicide and/or plant growth regulator, preferably in crops of useful plants and/or ornamental plants.

8. Herbicidal or plant growth-regulating composition, **characterized in that** the composition comprises one or more compounds of the formula (I) and/or salts thereof as defined in any of Claims 1 to 6,
and one or more further substances selected from groups (i) and/or (ii):
(i) one or more further agrochemically active substances, preferably selected from the group consisting of insecticides, acaricides, nematicides, further herbicides, fungicides, safeners, fertilizers and/or further growth regulators,
(ii) one or more formulation auxiliaries customary in crop protection.

9. Method for controlling harmful plants or for regulating the growth of plants, **characterized in that** an effective amount
- of one or more compounds of the formula (I) and/or salts thereof, as defined in any of Claims 1 to 6,
- of a composition according to Claim 8,
is applied to the plants, seeds of plants, the soil in which or on which the plants grow or the area under cultivation.

10. Process for preparing a compound of the formula (I) as defined in any of Claims 1 to 6 or a salt thereof, **characterized in that** a compound of the formula (E) 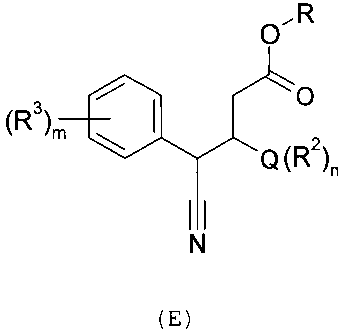 is converted by reduction into a compound of the formula (Ia) 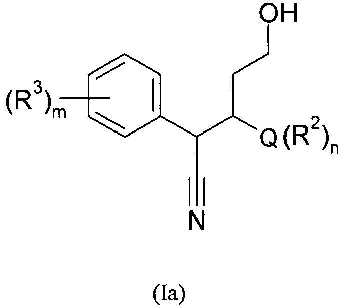 and the compound (Ia) is optionally reacted further to give a compound of the formula (I), provided R¹ in formula (I) does not represent hydrogen, where
R represents hydrogen or an organic radical, preferably a radical selected from the group of the radicals defined for R¹ according to any of Claims 1 to 6,
and
Q, R², R³, m and n in each case have the meaning defined in any of Claims 1 to 6.

## Revendications

1. Composé de formule (I) ou son sel 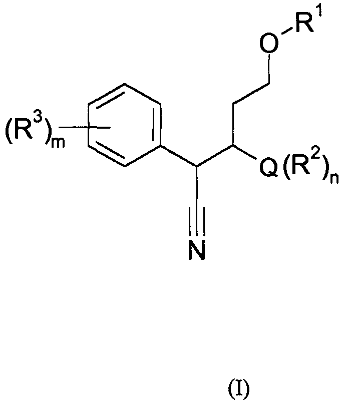 dans laquelle
Q signifie un radical hétéroaromatique, choisi dans le groupe constitué par pyridinyle, pyrazinyle, thiényle et thiazolyle,
R¹ signifie hydrogène ou un radical choisi dans le groupe constitué par :
acétyle, propanoyle, butanoyle, pentanoyle, hexanoyle, 2-méthylpropanoyle (= isopropanoyle), 2,2-difluoroacétyle, 2,2,2-trifluoroacétyle, C(=O)OMe, cyclopropanecarbonyle, 1-méthylcyclopropanecarbonyle, acryle, prop-2-ynoyle, but-2-ynoyle, 2-méthylacryle, benzoyle, 4-chlorobenzoyle, 3-chlorobenzoyle, 2-chlorobenzoyle, 4-fluorobenzoyle, 3-fluorobenzoyle, 2-fluorobenzoyle, 2,2-diméthylpropanoyle (= pivaloyle), 3,3-diméthylbutanoyle, 2-nitrobenzoyle, 2-fluoroacétyle, 2-chloroacétyle, 2-bromoacétyle, 2,2-dichloroacétyle, 2-méthoxyacétyle, 2,6-difluorobenzoyle, C(O)C(=O)OMe et C(O)CH₂C(O)OMe,
(R²)ₙ signifie n substituants R², R² (lorsque n = 1) ou chaque substituant R² (lorsque n est supérieur à 1) signifiant, indépendamment, halogéno, cyano, nitro, (C₁-C₈)alkyle, (C₁-C₈)alcoxy, (C₁-C₈)alkylthio, (C₁-C₈)alkylsulfinyle, (C₁-C₈)alkylsulfonyle, (C₁-C₆)halogénoalkyle, (C₂-C₆)halogénoalcényle, (C₁-C₆)halogénoalcoxy, (C₁-C₆)halogénoalkylthio, (C₁-C₆)halogénoalkylsulfinyle, (C₁-C₆)halogénoalkylsulfonyle, (C₂-C₆) alcényle, (C₂-C₆)alcynyle, tri-[(C₁-C₄)alkyl]-silyle ou tri-[(C₁-C₄)alkyl] -silyl- (C₁-C₄)alkyle, ou à chaque fois deux R² directement adjacents au radical hétéroaromatique Q formant ensemble un groupe de formule -Z¹-A*¹-Z²-, dans laquelle
A* représente un groupe alkylène comprenant 1 à 4 atomes de carbone, qui est le cas échéant substitué par un ou plusieurs radicaux choisis dans le groupe constitué par halogéno, (C₁-C₄)alkyle, (C₁-C₄)halogénoalkyle, (C₁-C₄)alcoxy et (C₁-C₄)halogénoalcoxy,
Z¹ représente une liaison directe, O ou S et
Z² représente une liaison directe, O ou S,
le groupe -Z¹-A*-Z²- formant un cycle de 5 ou 6 chaînons ensemble avec les atomes du radical hétéroaromatique Q liés à ce groupe,
(R³)ₘ signifie m substituants R³,
R³ (lorsque m = 1) ou chaque substituant R³ (lorsque m est supérieur à 1) signifiant, indépendamment, halogéno, cyano, nitro, (C₁-C₈)alkyle, (C₁-C₈)alcoxy, (C₁-C₈)alkylthio, (C₁-C₈)alkylsulfinyle, (C₁-C₈)alkylsulfonyle, (C₁-C₆)halogénoalkyle, (C₂-C₆) halogénoalcényle, (C₁-C₆)halogénoalcoxy, (C₁-C₆)halogénoalkylthio, (C₁-C₆)halogénoalkylsulfinyle, (C₁-C₆)halogénoalkylsulfonyle, (C₂-C₆) alcényle, (C₂-C₆)alcynyle, tri-[(C₁-C₄)alkyl]-silyle, tri-[(C₁-C₄)alkyl] -silyl- (C₁-C₉)alkyle ou -NR*R**, R* et R** étant choisis, indépendamment l'un de l'autre et indépendamment d'autres radicaux -NR*R** éventuellement présents, à chaque fois dans le groupe constitué par H, (C₁-C₈)alkyle, (C₂-C₈)alcényle, (C₂-C₈)alcynyle, (C₁-C₄)alcoxy- (C₁-C₄)alkyle, (C₁-C₆)alcanoyle, [(C₁-C₄)halogénoalkyle]-carbonyle, [(C₁-C₄)alcoxy]-carbonyle, [(C₁-C₄)halogénoalcoxy]-carbonyle, (C₃-C₆)cycloalkyle, (C₃-C₆)cycloalkyl-(C₁-C₄)alkyle, (C₃-C₆)cycloalcényle, (C₃-C₆)cycloalcényl-(C₁-C₄)alkyle, phényle et phényl-(C₁-C₄)alkyle, chacun des radicaux (C₃-C₆)cycloalkyle, (C₃-C₆)cycloalkyl-(C₁-C₄)alkyle, (C₃-C₆)cycloalcényle, (C₃-C₆)cycloalcényl-(C₁-C₄)alkyle, phényle et phényl-(C₁-C₄)alkyle mentionnés étant le cas échéant substitué dans le cycle par un ou plusieurs radicaux R^{bb} identiques ou différents,
R^{bb} pouvant signifier, à chaque fois, halogéno, (C₁-C₄)alkyle, (C₁-C₄)halogénoalkyle, (C₁-C₄)alcoxy ou (C₁-C₄)halogénoalcoxy et, dans le cas de (C₃-C₆)cycloalkyle, (C₃-C₆)cycloalkyl-(C₁-C₄)alkyle, (C₃-C₆)cycloalcényle, (C₃-C₆)cycloalcényl-(C₁-C₄)alkyle, R^{bb} pouvant en outre signifier oxo, ou NR*R** signifiant un hétérocycle de 3 à 8 chaînons, qui comprend le cas échéant, en plus de cet atome de N, un ou deux autres hétéroatomes de cycle choisis dans le groupe constitué par N, O et S et qui est non substitué ou substitué par un ou plusieurs radicaux choisis dans le groupe constitué par (C₁-C₄)alkyle, (C₁-C₄)halogénoalkyle et oxo,
ou à chaque fois deux groupes R³ directement adjacents au cycle signifiant ensemble un groupe de formule -Z³-A**-Z⁴- dans laquelle
A** représente un groupe alkylène comprenant 1 à 4 atomes de carbone, qui est le cas échéant substitué par un ou plusieurs radicaux choisis dans le groupe constitué par halogéno, (C₁-C₄)alkyle, (C₁-C₄)halogénoalkyle, (C₁-C₄)alcoxy et (C₁-C₄)halogénoalcoxy,
Z³ représente une liaison directe, O ou S et
Z⁴ représente une liaison directe, O ou S,
le groupe -Z³-A**-Z⁴- formant un cycle de 5 ou 6 chaînons avec les atomes de carbone du cycle phényle liés à ce groupe,
n signifie 0 ou un nombre entier dans la plage de 1 à 5, de préférence 0, 1, 2 ou 3 et
m signifie 0, 1, 2, 3, 4 ou 5, de préférence 0, 1, 2 ou 3.

2. Composé de formule (I) et/ou son sel selon la revendication 1, **caractérisé en ce que** le composé de formule (I) correspond aux formules (I-1), (I-2) ou (I-3) définies dans la suite 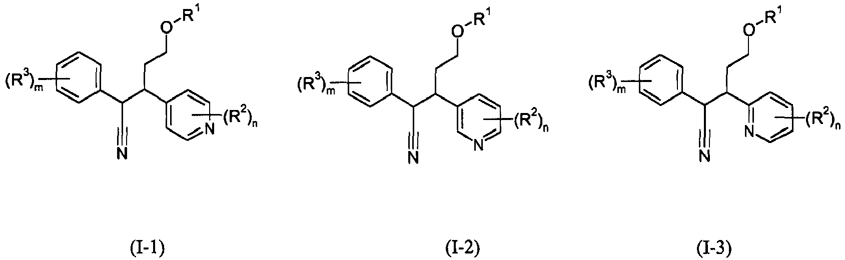 où R¹, R², R³, m et n ont la signification définie dans la revendication 1.

3. Composé de formule (I) et/ou son sel selon l'une quelconque des revendications 1 à 2, **caractérisé en ce que**
(R²)ₙ signifie n substituants R²,
R² (lorsque n = 1) ou chaque substituant R² (lorsque n est supérieur à 1) signifiant, indépendamment, halogéno, cyano, nitro, (C₁-C₆)alkyle, (C₁-C₆)alcoxy, (C₁-C₆)alkylthio, (C₁-C₆)alkylsulfinyle, (C₁-C₈)alkylsulfonyle, (C₁-C₆)halogénoalkyle, (C₂-C₆) halogénoalcényle, (C₁-C₆)halogénoalcoxy, (C₁-C₆)halogénoalkylthio, (C₁-C₆)halogénoalkylsulfinyle, (C₁-C₆)halogénoalkylsulfonyle, (C₂-C₆) alcényle, (C₂-C₆)alcynyle, tri-[(C₁-C₄)alkyl]-silyle ou tri-[(C₁-C₄)alkyl] -silyl- (C₁-C₄)alkyle et/ou
(R³)ₘ signifie m substituants R³,
R³ (lorsque m = 1) ou chaque substituant R³ (lorsque m est supérieur à 1) signifiant, indépendamment, halogéno, cyano, nitro, (C₁-C₆)alkyle, (C₁-C₆)alcoxy, (C₁-C₆)alkylthio, (C₁-C₆)alkylsulfinyle, (C₁-C₈)alkylsulfonyle, (C₁-C₆)halogénoalkyle, (C₂-C₆)halogénoalcényle, (C₁-C₆)halogénoalcoxy, (C₁-C₈)halogénoalkylthio, (C₁-C₆)halogénoalkylsulfinyle, (C₁-C₆)halogénoalkylsulfonyle, (C₂-C₆) alcényle, (C₂-C₆)alcynyle, NR*R**, tri-[(C₁-C₄)alkyl]-silyle ou tri-[(C₁-C₄)alkyl] -silyl- (C₁-C₄)alkyle ou à chaque fois deux groupes R³ directement adjacents au cycle signifiant ensemble un groupe de formule -Z³-A**-Z⁴- dans laquelle
A** représente un groupe alkylène comprenant 1 à 4 atomes de carbone, qui est le cas échéant substitué par un ou plusieurs radicaux du groupe formé par halogène, (C₁-C₄)alkyle, (C₁-C₄)halogénoalkyle, (C₁-C₄)alcoxy et (C₁-C₄)halogénoalcoxy,
Z³ représente une liaison directe, O ou S et
Z⁴ représente une liaison directe, O ou S,
le groupe -Z³-A**-Z⁴- formant conjointement avec les atomes de carbone liés au groupe du cycle phényle, un cycle condensé de 5 ou 6 chaînons,
R*, R** présentent à chaque fois indépendamment l'un de l'autre ou ensemble avec l'atome de N la signification susmentionnée et
n, m signifient à chaque fois indépendamment l'un de l'autre 0, 1, 2, 3, 4 ou 5, de préférence 0, 1, 2 ou 3, en particulier 0, 1 ou 2.

4. Composé de formule (I) et/ou son sel selon l'une quelconque des revendications 1 à 3, **caractérisé en ce que**
(R²)ₙ signifie n substituants R² où, dans le cas où n = 1, le substituant R², ou, dans le cas où n est supérieur à 1, chaque substituant R² signifie indépendamment halogéno, cyano, nitro, (C₁-C₄)alkyle, (C₁-C₄)alcoxy, (C₁-C₄)alkylthio, (C₁-C₄)alkylsulfinyle, (C₁-C₄)alkylsulfonyle, (C₁-C₄)halogénoalkyle, (C₂-C₄)halogénoalcényle, (C₁-C₄)halogénoalcoxy, (C₁-C₄)halogénoalkylthio, (C₁-C₄)halogénoalkylsulfinyle, (C₁-C₄)halogénoalkylsulfonyle, (C₂-C₄)alcényle, (C₂-C₄)alcynyle, tri-[(C₁-C₄)alkyl]-silyle ou tri-[(C₁-C₄)alkyl] -silyl- (C₁-C₄)alkyle, et
n signifie 0, 1, 2, 3, ou 4, de préférence 0, 1, 2 ou 3, en particulier 0, 1 ou 2.

5. Composé de formule (I) et/ou son sel selon l'une quelconque des revendications 1 à 4, **caractérisé en ce que**
(R³)ₘ signifie m substituants R³,
où dans le cas où m = 1, le substituant R³ ou, dans le cas où m est supérieur à 1, chaque substituant R³ signifie indépendamment halogène, cyano, nitro, (C₁-C₄)alkyle, (C₁-C₄)alcoxy, (C₁-C₄)alkylthio, (C₁-C₄)alkylsulfinyle, (C₁-C₄)alkylsulfonyle, (C₁-C₄)halogénoalkyle, (C₂-C₄)halogénoalcényle, (C₁-C₄)halogénoalcoxy, (C₁-C₄)halogénoalkylthio, (C₁-C₄)halogénoalkylsulfinyle, (C₁-C₄)halogénoalkylsulfonyle, (C₂-C₆) alcényle, (C₂-C₆)alcynyle ou tri-[(C₁-C₄)alkyl]silyl-Z^{b}-, où Z^{b} = une liaison covalente ou (C₁-C₄)alkylène, ou
à chaque fois deux groupes R³ directement adjacents au cycle signifiant ensemble un groupe de formule -Z³-A**-Z⁴-dans laquelle
A** représente un groupe alkylène, qui est le cas échéant substitué par un ou plusieurs radicaux du groupe formé par halogène, (C₁-C₄)alkyle, (C₁-C₄)halogénoalkyle, (C₁-C₄)alcoxy et (C₁-C₄)halogénoalcoxy,
Z³ représente O ou S, et
Z⁴ représente O ou S,
le groupe -Z³-A**-Z⁴- formant conjointement avec les atomes de carbone liés au groupe du cycle phényle, un cycle condensé de 5 ou 6 chaînons,
m signifie 0, 1, 2, 3, 4 ou 5, de préférence 0, 1, 2, 3 ou 4, en particulier 0, 1, 2 ou 3.

6. Composé de formule (I) et/ou son sel selon l'une quelconque des revendications 1 à 5, **caractérisé en ce que**
(R²)ₙ signifie n substituants R², où, dans le cas où n = 1, le substituant R² ou, dans le cas où n est supérieur à 1, chaque substituant R² signifie indépendamment fluor, chlore, brome, iode, cyano, nitro, méthyle, éthyle, méthoxy, méthylthio, méthylsulfinyle, méthylsulfonyle, trifluorométhyle, trifluorométhoxy, trifluoroalkylthio, trifluorométhylsulfinyle ou trifluorométhylsulfonyle et
(R³)ₘ signifie m substituants R³, où, dans le cas où m = 1, le substituant R³ ou, dans le cas où m est supérieur à 1, chaque substituant R³ signifie indépendamment fluor, chlore, brome, iode, cyano, nitro, méthyle, méthoxy, méthylthio, méthylsulfinyle, méthylsulfonyle, trifluorométhyle, trifluorométhoxy, trifluoroalkylthio, trifluorométhylsulfinyle ou trifluorométhylsulfonyle et
m signifie 0, 1, 2, 3 ou 4, de préférence 0, 1, 2 ou 3, et
n signifie 0, 1, 2 ou 3, de préférence 0, 1 ou 2.

7. Utilisation d'un composé de formule (I) et/ou de son sel tel que défini dans l'une quelconque des revendications 1 à 6 comme herbicide et/ou régulateur de la croissance de plantes, de préférence dans la culture de plantes utiles et/ou décoratives.

8. Agent herbicide ou de régulation de la croissance de plantes, caractérisé
en ce l'agent contient un ou plusieurs composés de formule (I) et/ou leurs sels, tels qu'ils sont définis dans l'une quelconque des revendications 1 à 6 et une ou plusieurs substances choisies dans les groupes (i) et/ou (ii) :
(i) une ou plusieurs substances actives d'un point de vue agrochimique, de préférence choisies dans le groupe constitué par les insecticides, les acaricides, les nématicides, d'autres herbicides, les fongicides, les phytoprotecteurs, les engrais et/ou d'autres régulateurs de croissance,
(ii) un ou plusieurs adjuvants de formulation usuels dans la protection des plantes.

9. Procédé pour lutter contre des plantes nuisibles ou pour la régulation de la croissance de plantes, **caractérisé en ce qu'**on applique
- un ou plusieurs composés de formule (I) et/ou leurs sels tels que définis dans l'une quelconque des revendications 1 à 6
- un agent selon la revendication 8,
sur les plantes, les graines de plantes, le sol dans ou sur lequel les plantes croissent ou sur la surface de culture.

10. Procédé pour la préparation d'un composé de formule (I), tel qu'il est défini selon l'une quelconque des revendications 1 à 6, **caractérisé en ce qu'**on transforme un composé de formule (E) 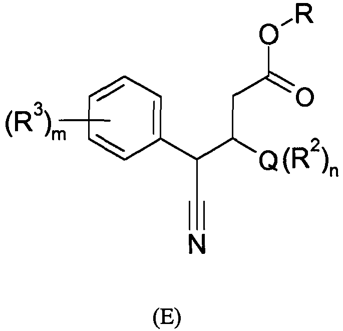 par réduction en un composé de formule (Ia) 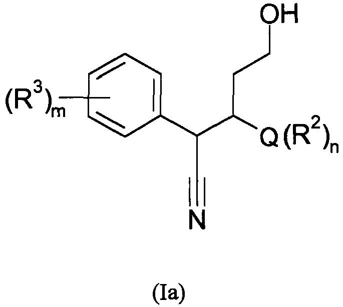 et on transforme le composé (Ia) le cas échéant davantage en un composé de formule (I), pour autant que R¹ dans la formule (I) ne signifie pas hydrogène,
R représentant hydrogène ou un radical organique, de préférence un radical choisi dans le groupe des radicaux définis pour R¹ selon l'une quelconque des revendications 1 à 6, et
Q, R², R³, m et n présentent à chaque fois la signification définie dans l'une quelconque des revendications 1 à 6.
